(19) Europäisches Patentamt
European Patent Office
Office européen des brevets

(11) **EP 4 253 549 A2**

(12) **EUROPEAN PATENT APPLICATION**

(43) Date of publication:
04.10.2023 Bulletin 2023/40

(21) Application number: 23178221.0

(22) Date of filing: **17.09.2019**

(51) International Patent Classification (IPC):
**C12N 15/66** (2006.01)

(52) Cooperative Patent Classification (CPC):
(C-Sets available)
**C12N 9/22; C12N 15/1031; C12N 15/1058;
C12N 15/66; C40B 40/08; C40B 50/06;** C40B 20/04

(Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.09.2018 US 201862733410 P**

(62) Document number(s) of the earlier application(s) in
accordance with Art. 76 EPC:
**19863047.7 / 3 853 363**

(71) Applicant: **The University of Hong Kong
Hong Kong (CN)**

(72) Inventors:
• **WONG, Alan Siu Lun
Hong Kong (CN)**

• **CHOI, Gigi Ching Gee
Hong Kong (CN)**

(74) Representative: **HGF
HGF Limited
1 City Walk
Leeds LS11 9DX (GB)**

Remarks:
•The complete document including Reference
Table(s) and the Sequence Listing(s) can be
downloaded from the EPO website
•This application was filed on 08-06-2023 as a
divisional application to the application mentioned
under INID code 62.
•Claims filed after the date of filing of the application
/ after the date of receipt of the divisional application
(Rule 68(4) EPC)

(54) **IMPROVED HIGH-THROUGHPUT COMBINATORIAL GENETIC MODIFICATION SYSTEM AND OPTIMIZED CAS9 ENZYME VARIANTS**

(57) The present invention provides to an improved high-throughput system and method for generating and screening of genetic variants by combinatorial modifications. Also provided are optimized SpCas9 enzyme variants produced by this system.

Figure 1a

EP 4 253 549 A2

**(Cont. next page)**

(52) Cooperative Patent Classification (CPC): (Cont.)

C-Sets
**C12N 15/1031, C12Q 2521/313, C12Q 2521/501,
C12Q 2563/179**

**Description**

RELATED APPLICATIONS

This application claims priority to U.S. Provisional Patent Application No. 62/733,410, filed September 19, 2018, the contents of which are hereby incorporated by reference in the entirety for all purposes.

BACKGROUND

Recombinant proteins are of an increasingly significant importance in a wide variety of applications including uses in industrial and medical contexts. As the functionalities of recombinant proteins, especially enzymes and antibodies, may be improved by genetic mutations, continuous efforts have been made to generate and select a broad spectrum of possible genetic variants of recombinant proteins in order to identify those with more desirable characteristics such that improved efficiency may be achieved in their applications.

Cas9 (CRISPR-associated protein 9) is an RNA-guided DNA endonuclease associated with the CRISPR (Clustered Regularly Interspaced Short Palindromic Repeats) adaptive immunity system in bacteria such as *Streptococcus pyogenes,* a species of Gram-positive bacterium in the genus *Streptococcus.* Because of the increased use of CRISPR for genetic editing in the recent years, Cas9 is an enzyme of intense interest many seek to improve the performance of by way of genetic modification. The currently available systems for systematically generating and screening of a large number of genetic variants of any particular protein are, however, often cumbersome, labor-intensive, and therefore inefficient.

As such, there exists a distinct need for new high-throughput combinatorial genetic modification systems/methods as well as for engineered proteins (such as the Cas9 enzyme) with improved properties. The present invention fulfills this and other related needs.

SUMMARY OF THE INVENTION

Previously, the research group headed by the present inventors devised a system for high-throughput functional analysis of a high-order barcoded combinatorial genetic library, termed combinatorial genetic *en masse* or CombiGEM. This system has been used for generating, for example, a library of barcoded dual guide-RNA (gRNA) combinations and a library of two-wise or three-wise barcoded human microRNA (miRNA) precursors, to be further screened for desired functionalities, see *e.g.,* Wong et al. (Nat. Biotechnol. 2015 September; 33(9):952-961), Wong et al. (Proc. Nat. Acad. Sci., March 1, 2016, 113(9):2544-2549), WO2016/070037, and WO2016/115033. See also, U.S. Patent No. 9,315,806. The inventors have now made further modifications to the CombiGEM system and developed the improved Comb in SEAL platform, which provides seamless connection between any two adjacent genetic components of each member of a high-order combinatorial mutant library. In other words, this platform does not introduce any artificial or extraneous amino acid sequence at each of the junction sites, thus permitting the generation of a large collection of protein variants containing combinatorial mutations while otherwise maintaining the native amino acid sequence of the wild-type protein.

As such, the present invention firstly provides an improved high-throughput genetic modification system for systematically generating and screening of combinatorial mutants. In one aspect, the invention provides a DNA construct that comprises in the direction of from 5' to 3' of a DNA strand: a first recognition site for a first type IIS restriction enzyme; a DNA element; a first and a second recognition sites for a second type IIS restriction enzyme, a barcode uniquely assigned to the DNA element; and a second recognition site for the first type IIS restriction enzyme. In some embodiments, the DNA construct is a linear construct; in other embodiments, the DNA construct is a circular construct or a DNA vector including a bacteria-based DNA plasmid or a DNA viral vector. The DNA construct is preferably isolated, *i.e.,* in the absence of any significant amount of other DNA sequences. In some embodiments, the invention provides a library including at least two possibly more of the DNA constructs described above and herein, each of the library members has a distinct DNA element having a distinct polynucleotide sequence along with an uniquely assigned bar code.

In another aspect of this invention, another DNA construct is provided: the DNA construct comprising in the direction of from 5' to 3' of a DNA strand: a recognition site for a first type IIS restriction enzyme; a plurality of DNA elements; a primer binding site; and a plurality of barcodes each uniquely assigned to one of the plurality of DNA elements, and a recognition site for a second type IIS restriction enzyme, wherein the plurality of DNA elements are connected to each other to form a coding sequence for a protein (such as a coding sequence for a native or wild-type protein) without any extraneous sequence at any connection point between any two of the plurality of DNA elements, and wherein the plurality of barcodes are placed in the reverse order of their assigned DNA elements. In some embodiments, the DNA construct is a linear one; in other embodiments, the DNA construct is a circular one, such as a DNA vector including a bacteria-based DNA plasmid or DNA viral vector. A library of such constructs is also provided to include at least two possibly more of the constructs, each member having a distinct set of DNA elements of distinct polynucleotide sequences and a set of uniquely assigned bar codes.

In some embodiments of the either DNA contructs describe above and herein, the first type IIS restriction enzyme and the second type IIS restriction enzyme generate compatible ends upon cleaving a DNA molecule. In some embodiments, the first type IIS restriction enzyme is BsaI. In some embodiments, the second type IIS restriction enzyme is BbsI.

In one further aspect, the present invention relates to a method for generating a combinatorial genetic construct. The method includes these steps: (a) cleaving a first DNA vector of claim 2 with the first type IIS restriction enzyme to release a first DNA fragment comprising the first DNA segment, the first and second recognition sites for a second type IIS restriction enzyme, and the first barcode flanked by a first and a second ends generated by the first type IIS restriction enzyme; (b) cleaving an initial expression vector comprising a promoter with the second type IIS restriction enzyme to linearize the initial expression vector near 3' end of the promoter and generate two ends that are compatible with the first and second ends of DNA fragment of (a); (c) annealing and ligating the first DNA fragment of (a) into the linearized expression vector of (b) to form a 1-way composite expression vector in which the first DNA fragment and the first barcode are operably linked to the promoter at its 3' end; (d) cleaving a second DNA vector of claim 2 with the first type IIS restriction enzyme to release a second DNA fragment comprising the second DNA segment, the first and second recognition sites for the second type IIS restriction enzyme, and the second barcode flanked by a first and a second ends generated by the first type IIS restriction enzyme; (e) cleaving the composite expression vector of (c) with the second type IIS restriction enzyme to linearize the composite expression vector between the first DNA element and the first barcode and generate two ends that are compatible with the first and second ends of DNA fragment of (d); and (f) annealing and ligating the second DNA fragment of (d) into linearized composite expression vector of (e) between the first DNA element and the first barcode to form a 2-way composite expression vector in which the first DNA fragment, the second DNA fragment, the second barcode, and the first barcode are operably linked in this order to the promoter at its 3' end, wherein the first and second DNA elements encode the first and second segments of a pre-selected protein from its N-terminus that are immediately adjacent to each other, and wherein the first and second DNA fragments are joined to each other in the 2-way composite expression vector without any extraneous nucleotide sequence resulting in any amino acid residue not found in the pre-selected protein, and wherein each of the first and second DNA elements comprises one or more mutations.

In some embodiments of this method, steps (d) to (f) are repeated until the nth time to incorporate the nth DNA fragment comprising the nth DNA element, the first and second recognition sites for the second type IIS restriction enzyme, and the nth barcode into an n-way composite expression vector, the nth DNA elment encoding for the nth or the second to the last segment of the pre-selected protein from its C-terminus. The method further includes the steps of: (x) providing a final DNA vector, which comprises between a first and a second recognition sites for a first type IIS restriction enzyme, a (n+1)th DNA element, a primer-binding site, and a (n+1)th barcode; (y) cleaving the final DNA vector with the first type IIS restriction enzyme to release a final DNA fragment comprising from 5' to 3': the (n+1)th DNA element, the primer-binding site, and the (n+1)th barcode, flanked by a first and a second ends generated by the first type IIS restriction enzyme; (z) annealing and ligating the final DNA fragment into the n-way composite expression vector, which is produced after steps (d) to (f) are repeated for the nth time and has been linearized by the second type IIS restriction enzyme, to form a final composite expression vector, wherein the first, second, and so on up to the nth and the (n+1)th DNA elements encode the first, second, and so on up to the nth and the last segments of the pre-selected protein from its N-terminus that are immediately adjacent to each other, and wherein the first, second, and so on up to the nth and the last DNA fragments are joined to each other in the final composite expression vector without any extraneous nucleotide sequence resulting in any amino acid residue not found in the pre-selected protein, and wherein each of the DNA elements comprises one or more mutations.

In some embodiments of the methods described above or herein, the first type IIS restriction enzyme and the second type IIS restriction enzyme generate compatible ends upon cleaving a DNA molecule. In some embodiments, the first type IIS restriction enzyme is BsaI. In some embodiments, the second type IIS restriction enzyme is BbsI.

In an additional aspect, the present invention provides a library that includes at least two possibly more of the final composite expression vectors generated by the methods described above and herein.

Secondly, the present invention provides SpCas9 mutants that possess improved on-target cleavage and reduced off-target cleavage capability, which are generated and identified by using the improved high-throughput genetic modification system described above and herein. In one aspect, the invention provies a polypeptide (preferably isolated polypeptide) comprising the amino acid sequence set forth in any one of SEQ ID NOs:1 and 4-13, which serves as the base sequence, wherein at least one possibly more residues corresponding to residue(s) 661, 695, 848, 923, 924, 926, 1003, or 1060 of SEQ ID NO:1 is modified, e.g., by substitution. Some exemplary polypeptides of the present invention are provided in Table 2 of this disclosure. In some embodiments, the residue corresponding to residue 1003 of SEQ ID NO:1 is substituted and residue corresponding to residue 661 of SEQ ID NO:1 is substituted. In some embodiments, the polypeptide further has a substitution at the residue corresponding to residue 926 of SEQ ID NO:1. For example, the polypeptide has the residue corresponding to residue 1003 of SEQ ID NO:1 substituted with Histidine and the residue corresponding to residue 661 of SEQ ID NO:1 substituted with Alanine. In another example, the polypeptide has the base amino acid sequence set forth in SEQ ID NO:1, wherein residue 1003 is substituted with Histidine and residue 661 is substituted with Alanine, which optionally further includes a substitution with Alanine at residue 926. In a further example, the polypeptide has the base amino acid sequence set forth in SEQ ID NO:1, wherein residues 695, 848, and 926 are substituted with Alanine, residue 923 is substitued with Methionine, and residue 924 is substituted with Valine. A composition is also provided, which comprises (1) the polypeptide described above and herein; and (2) a physiologically acceptable excipient.

In another aspect, the present invention provides a nucleic acid (preferably isolated nucleic acid) that comprises a polynucleotide sequence encoding the polypeptide described above and herein as well as a composition containing the nucleic acid. The invention also provides an expression cassette comprising a promoter operably linked to a polynucleotide sequence encoding the polypeptide of this invention, and a vector (such as a bacteria-based plasmid or a virus-based vector) that comprises the expression cassette, a host cell comprising the expression cassette of or the polypeptide of the present invention.

In a further aspect, the present invention provides a method for cleaving a DNA molecule at a target site. The method includes the step of contacting the DNA molecule comprising the target DNA site with a polypeptide describe above and herein, plus a short guide RNA (sgRNA) that specifically binds the target DNA site, thereby causing the DNA molecule to be cleaved at the target DNA site. In some embodiments of the method, the DNA molecule is a genomic DNA within a live cell, and the cell has been transfected with polynucleotide sequences encoding the sgRNA and the polypeptide. In some cases, the cell has been transfected with a first vector encoding the sgRNA and a second vector encoding the polypeptide. In other cases, the cell has been transfected with a vector that encodes both the sgRNA and the polypeptide. In some embodiments of the method, each of the first and second vectors is a viral vector, such as a retrovial vector especially a lentiviral vector.

The high-throughput combinatorial genetic modification systems, methods, and related compositions described above and herein are sutiable, with modifications when appropriarte, for use in either prokaryotic cells and eukaryotic cells. Some equivalents can also be derived from the description above and herein. For instance, the placement of the DNA element and its corresponding barcode in each of the DNA constructs can be switched, *i.e.,* the DNA construct comprises from 5' to 3': a first recognition site for a first type IIS restriction enzyme, a barcode uniquely assigned to a DNA element,, a first and a second recognition sites for a second type IIS restriction enzyme, the DNA element, and a second recognition site for the first type IIS restriction enzyme. The DNA construct and a library of such DNA constructs can be used in the same fashion as described herein to generate intermediate and final vectors similar to those described herein, except for the relative locations of the DNA elements and barcodes in these vectors are switched accordingly.

BRIEF DESCRIPTION OF THE DRAWINGS

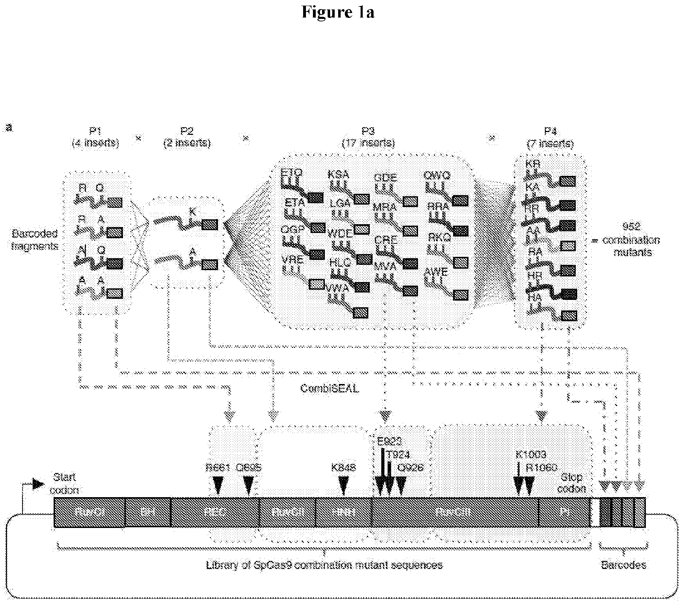

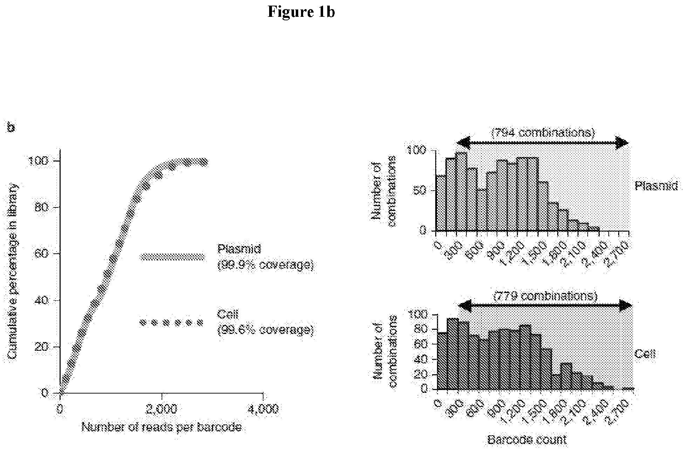

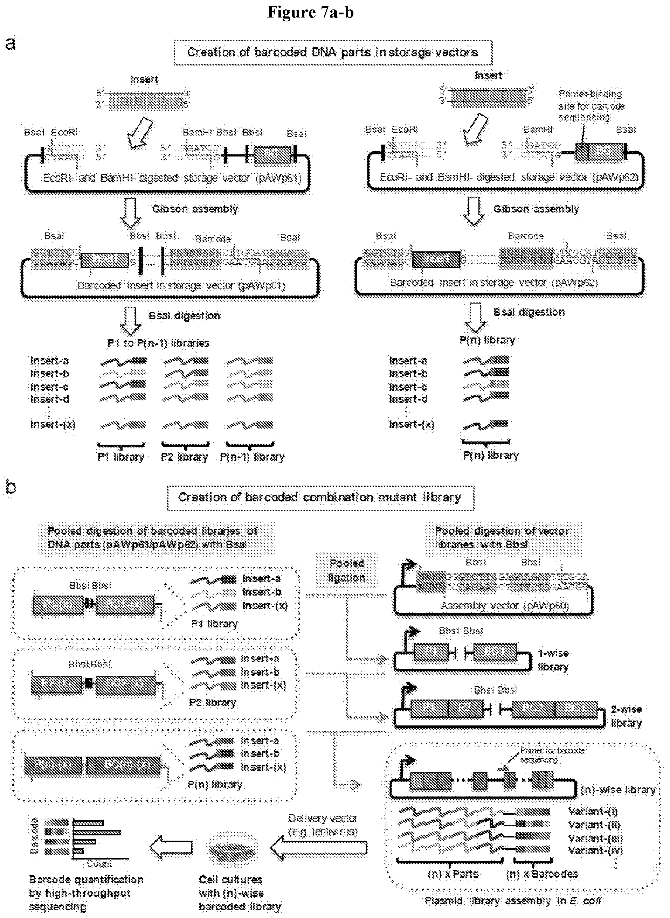

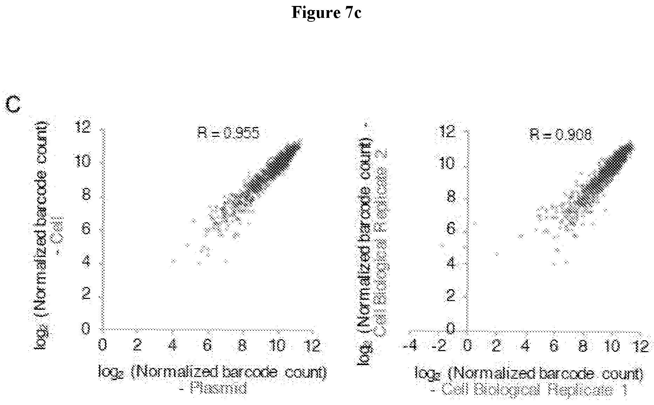

**Fig. 1 Generation of high-coverage combination mutant library of SpCas9 and efficient delivery of the library to human cells, a,** Strategy for assembling combination mutant library of SpCas9. SpCas9's coding sequence was modularized into four composable parts (i.e., P1 to P4), each comprising a repertoire of barcoded fragments encoding predetermined amino acid residue mutations at defined positions as depicted in the diagram. A library of 952 SpCas9 variants was assembled by consecutive rounds of one-pot seamless ligation of the parts, and concatenated barcodes that uniquely tagged each variant were generated (See **Fig. 7** for details). **b,** Cumulative distribution of sequencing reads for the barcoded combination mutant library in the plasmid pool extracted from *E. coli* and the infected OVCAR8-ADR cell pools. High coverage of the library within the plasmid and infected cell pools (~99.9% and ~99.6%, respectively) was detected from ~0.8 million reads per sample, and most combinations were detected with at least 300 absolute barcode reads (highlighted by the shaded areas).

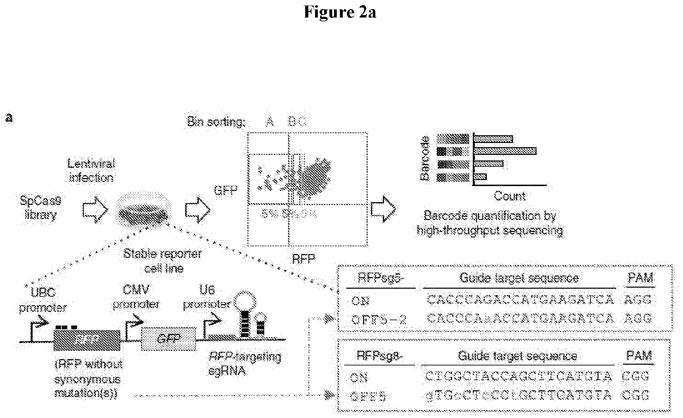

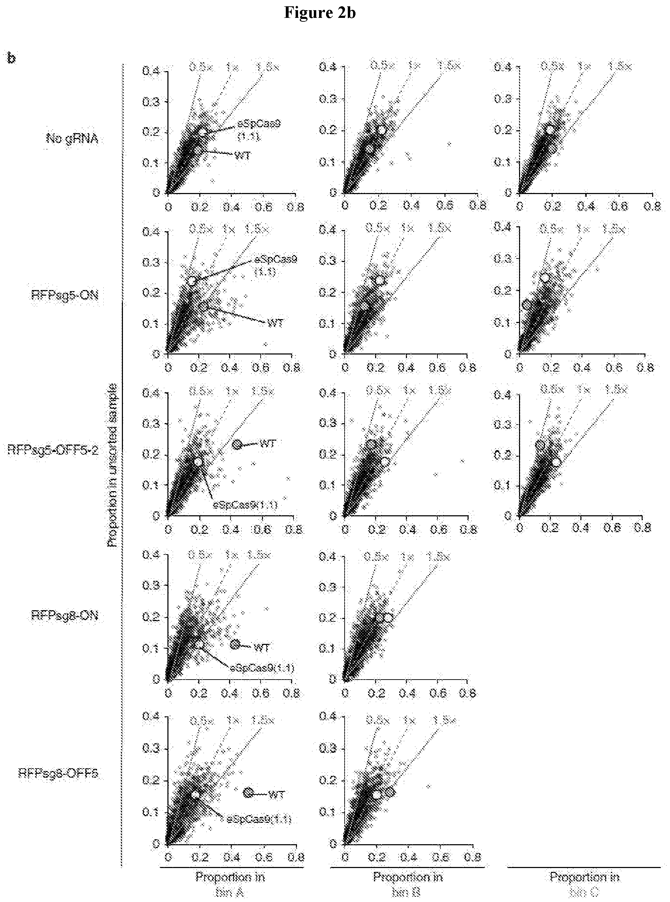

**Fig. 2 Strategy for profiling on- and off- target activities of SpCas9 variants in human cells, a,** The SpCas9 library was delivered via lentiviruses at multiplicity of infection of ~0.3 to OVCAR8-ADR reporter cell lines that express *RFP* and *GFP* genes driven by UBC and CMV promoters, respectively, and a tandem U6 promoter-driven expression cassette of gRNA targeting *RFP* (RFPsg5 or RFPsg8) site. RFP and GFP expression were analyzed under flow cytometry. On-target activity of SpCas9 was measured when the gRNA spacer sequence completely matches with the *RFP* target site, while its off-target activity was measured when the *RFP* target site harbors synonymous mutations. Cells harboring an active SpCas9 variant were expected to lose RFP fluorescence. Cells were sorted into bins encompassing ~5% of the population based on RFP fluorescence, and their genomic DNA was extracted for quantification of the barcoded SpCas9 variant by Illumina HiSeq. **b,** Scatterplots comparing the barcode count of each SpCas9 variant between the sorted bins (i.e., A, B, and C) and the unsorted population. Each dot represents a SpCas9 variant, and WT SpCas9 and eSpCas9(1.1) are labeled in the plots. Solid reference lines denote 1.5-fold enrichment and 0.5-fold depletion in barcode counts, and the dotted reference line indicates no change in barcode count, in the sorted bin compared to the unsorted population.

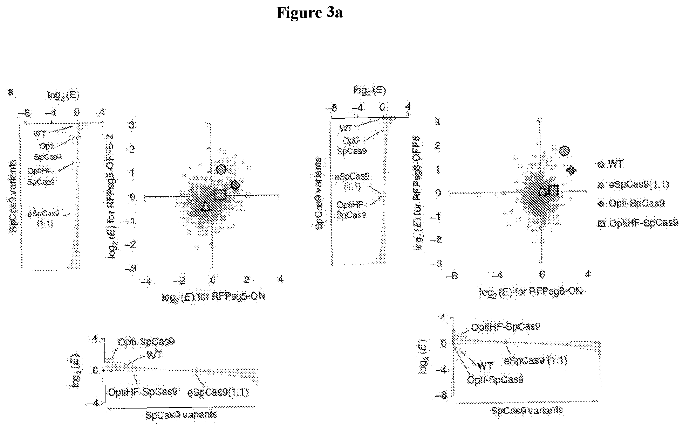

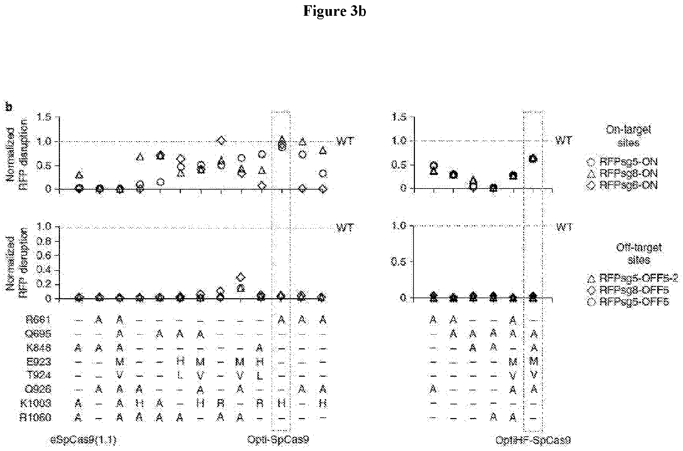

**Fig. 3 High-throughput profiling reveals the broad-spectrum specificity and efficiency of SpCas9 combination mutants. a,** Combination mutants of SpCas9 were ranked by their log-transformed enrichment ratios (*i.e.*, $\log_2(E)$) representing their relative abundance in the sorted RFP-depleted cell population for each of the on- (x-axis) and off- (y-axis) target reporter cell lines based on the profiling data from two biological replicates (See **Table 2** and **METHODS** for details). Each dot in the scatterplots represents a SpCas9 variant, and WT SpCas9, eSpCas9(1.1), Opti-SpCas9, and OptiHF-SpCas9 are labeled. >99% of the combination mutants had a lower $\log_2(E)$ than WT in the two off-target reporter lines RFPsg5-OFF5-2 and RFPsg8-OFF5, while 16.2% and 2.5% of the mutants had a higher $\log_2(E)$ than WT in the two on-target reporter lines RFPsg5-ON and RFPsg8-ON, respectively. **b,** OVCAR8-ADR reporter cells harboring on- (upper panel) and off- (lower panel) target sites were infected with individual SpCas9 combination mutants. Editing efficiencies of SpCas9 variants were measured by cell percentage with depleted RFP level and compared to WT.

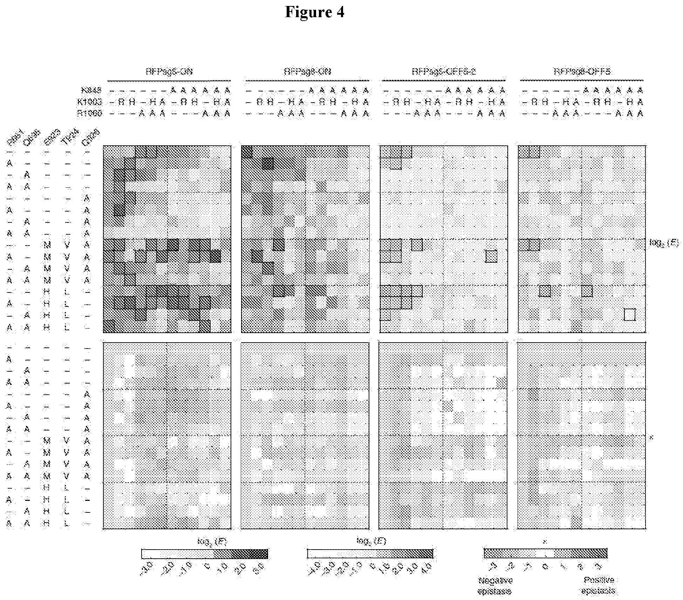

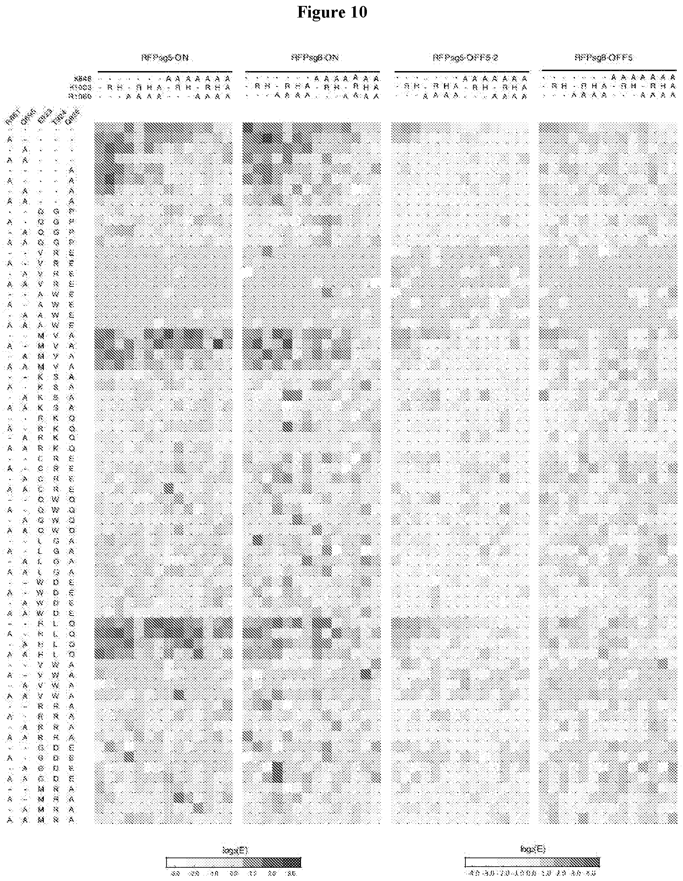

**Fig. 4 Heatmaps depicting editing efficiency and epistasis for the on- and off-target sites.** Editing efficiency (upper panel; measured by $\log_2(E)$) and epistasis (lower panel; $\varepsilon$) scores were determined for each SpCas9 combination mutant as described in Methods. Amino acid residues that are predicted to make contacts with the target DNA strand or located at the linker region connecting SpCas9's HNH and RuvC domains are grouped on the y-axis, while those predicted to interact with the non-target DNA strand are presented on the x-axis, to aid visualization. The P-value for $\log_2(E)$ of each combination is computed by comparing the $\log_2(E)$ with those contained within the whole population obtained from two independent biological replicates using the two-sample, two-tailed Student's t-test (MATLAB function 'ttest2'). The adjusted P-values (i.e., Q-values) are calculated based on the distribution of P-values (MATLAB function 'mafdr') to correct for multiple hypothesis testing. A $\log_2(E)$ was considered as statistically significant relative to the entire population based on a Q-value cutoff at <0.1, and are boxed. The full heatmaps are presented in full in **Fig. 10.** The combinations for which no enrichment ratio or epistasis score was measured are indicated in grey.

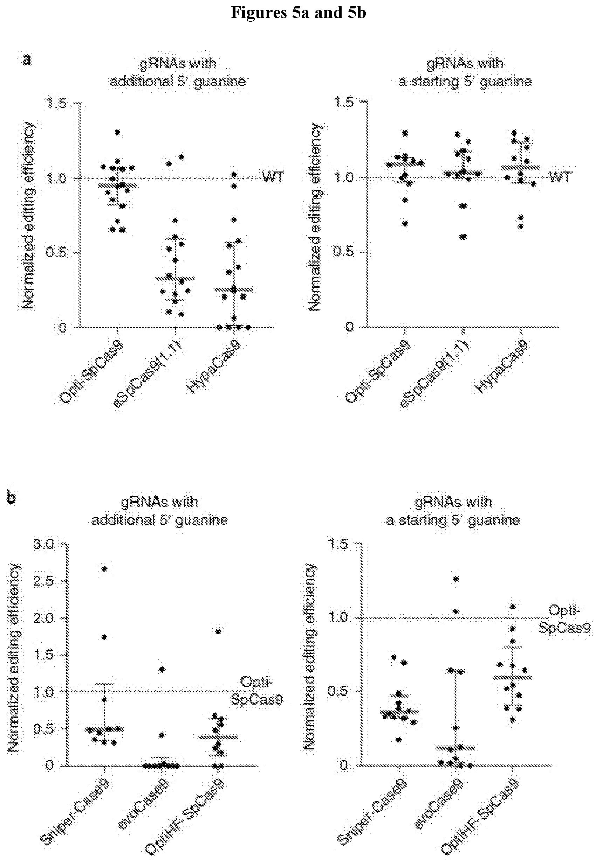

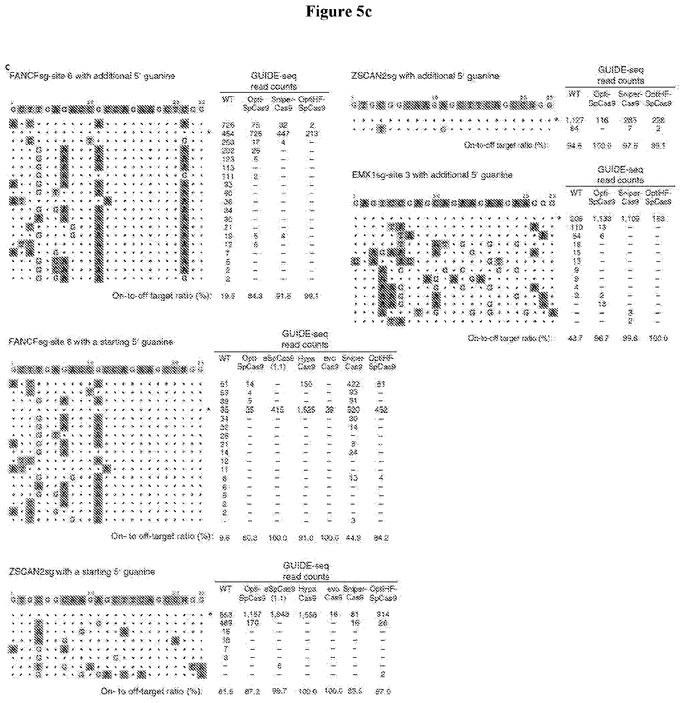

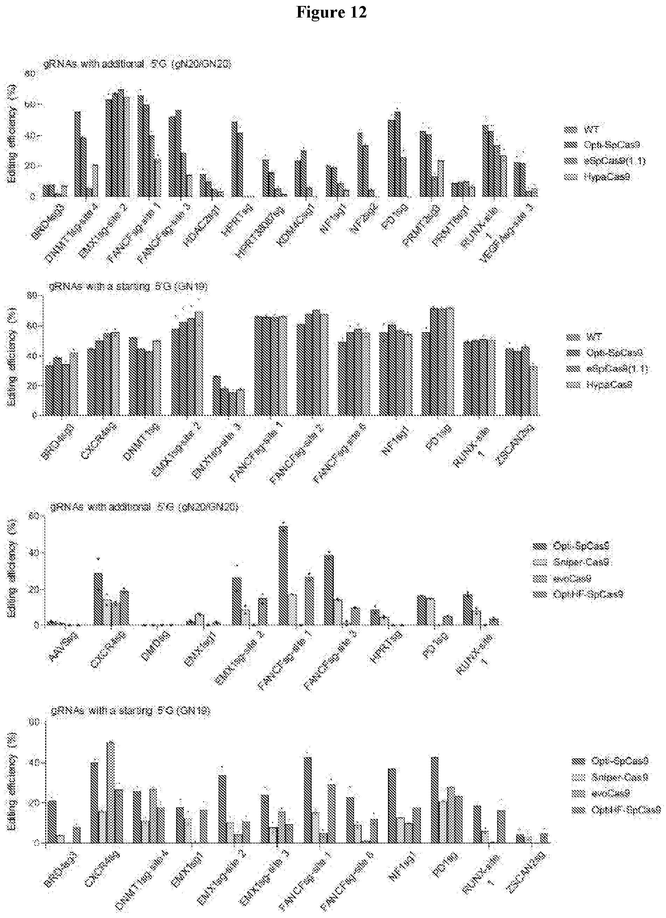

**Fig. 5 Opti-SpCas9 exhibits robust on-target and reduced off target activities, a-b,** Assessment of SpCas9 variants for efficient on-target editing with gRNAs targeting endogenous loci. Percentage of indel was measured using T7 endonuclease I (T7E1) assay. Ratio of on-target activity of SpCas9 variants to WT (in (**a**)) and to Opti-SpCas9 (in (**b**)) was determined, and the median and interquartile ranges for the normalized percentage of indel formation are shown for the 10 to 16 loci tested. Each locus was measured once or twice, and full dataset are presented in **Fig. 12. c,** GUIDE-Seq genome-wide specificity profiles for the panel of SpCas9 variants each paired with indicated gRNAs. Mismatched positions in off-target sites are highlighted in color, and GUIDE-Seq read counts were used as a measure of the cleavage efficiency at a given site. The list of gRNA sequences used is presented in **Table 5.**

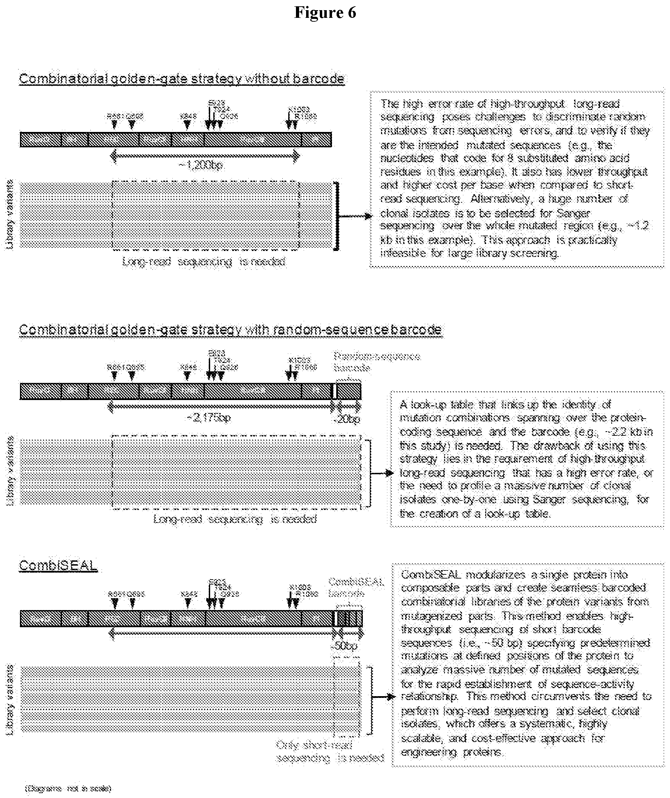

**Fig. 6 Examples of strategies for characterizing combinatorial mutations on a protein sequence.**

**Fig. 7 Strategy for seamless assembly of the barcoded combination mutant library pool, a,** To create barcoded DNA parts in storage vectors, genetic inserts were generated by PCR or synthesis, and cloned in the storage vectors harboring a random barcode (pAWp61 and pAWp62; digested with EcoRI and BamHI) with Gibson assembly reactions. BsaI digestion was performed to generate the barcoded DNA parts (*i.e.*, P1, P2,..., P(n)). BbsI sites and a primer-binding site for barcode sequencing were introduced in between the insert and the barcode for pAWp61 and pAWp62, respectively, **b,** To create the barcoded combination mutant library, the pooled DNA parts and destination assembly vectors were digested with BsaI and BbsI, respectively. A one-pot ligation created a pooled vector library, which was further iteratively digested and ligated with the subsequent pool of DNA parts to generate higher-order combination mutants. The barcoded inserts were linked with compatible overhangs that are originated from the protein-coding sequence after digestion with type IIS restriction enzymes (*i.e.,* BsaI and BbsI), thereby no fusion scar is formed in the ligation reactions. All barcodes were localized into a contiguous stretch of DNA. The final combination mutant library was encoded in lentiviruses and delivered into targeted human cells. The integrated barcodes representing each combination were amplified from the genomic DNA within the pooled cell populations in an unbiased fashion and quantified using high-throughput sequencing to identify shifts in representation under different experimental conditions. **c,** To show a highly reproducible representation between the plasmid and infected cell pools, as well as between biological replicates of infected cell pools.

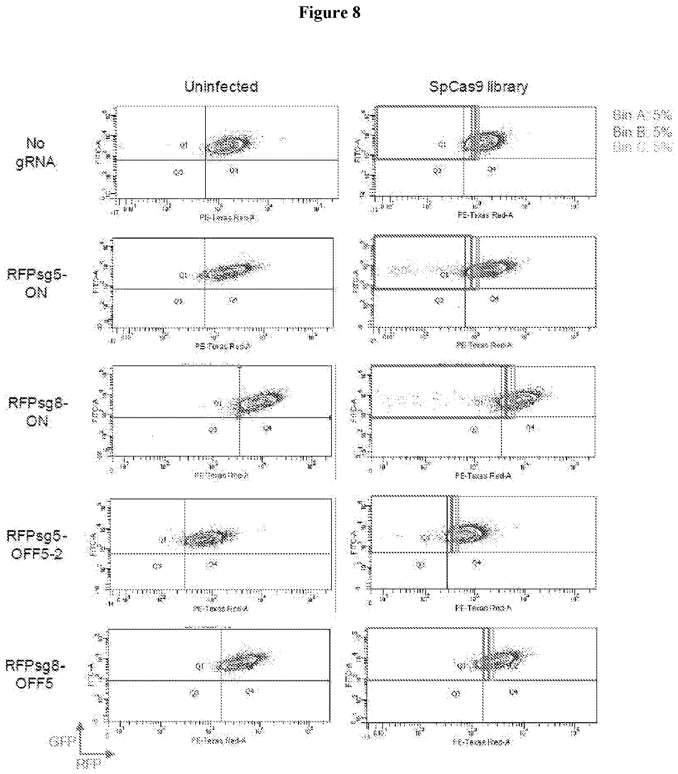

**Fig. 8 Fluorescence-activated cell sorting of SpCas9 library-infected human cells harboring on- and off-target reporters.** OVCAR8-ADR reporter cell lines that express RFP and GFP genes driven by UBC and CMV promoters, respectively, and a tandem U6 promoter-driven expression cassette of gRNA targeting the RFP site (RFPsg5 or RFPsg8) were either uninfected or infected with the SpCas9 library. RFPsg5-ON and RFPsg8-ON lines harbor sites that match completely with the gRNA sequence, while RFPsg5-OFF5-2 and RFPsg8-OFF5 lines contain synonymous mutations on the RFP and are mismatched to the gRNA. Cells were sorted under flow cytometry into bins each encompassing ~5% of the population with low RFP fluorescence. These experiments were repeated independently twice with similar results.

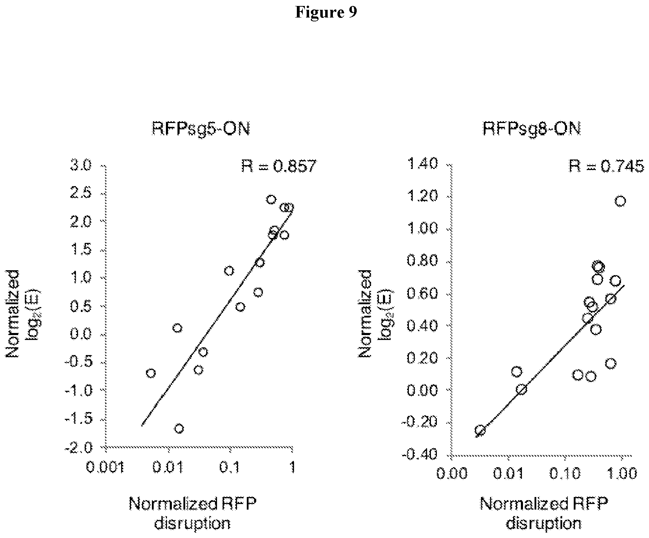

**Fig. 9 Positive correlation between enrichment score determined from the pooled** screen **and individual validation data.** The normalized $\log_2(E)$ for each SpCas9 combination mutant is the mean score determined from the pooled screens in two biological replicates, and the normalized RFP disruption value is the mean cell percentage with depleted RFP level when compared to WT determined from three biological replicates. R is the Pearson's r.

**Fig. 10 Heatmaps depicting editing efficiency for the on- and off- target sites.** Editing efficiency was measured by the log-transformed enrichment ratio ($\log_2(E)$) determined for each SpCas9 combination mutant. Enriched and depleted mutants have >0 and <0, respectively. To aid visualization, amino acid residues that are predicted to make contacts with the target DNA strand or located at the linker region connecting SpCas9's HNH and RuvC domains are grouped on the yaxis, while those predicted to make contacts with the non-target DNA strand are presented on the x-axis. The combinations for those with no enrichment are indicated in grey.

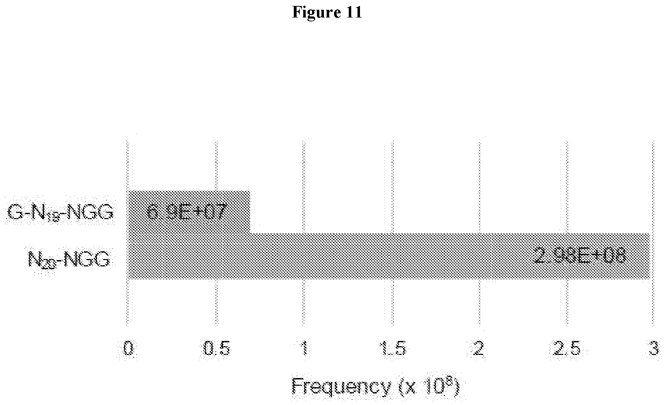

**Fig. 11 Frequency of N20-NGG and G-N19-NGG sites in the reference human genome.** A custom Python code was used to find the occurrence of $N_{20}$-NGG and $G$-$N_{19}$-NGG sites in both strands of the reference human genome hg19, as an estimate of the targeting ranges of Opti-SpCas9 and other engineered SpCas9 variants including eSpCas9(1.1), SpCas9-HF1, HypaCas9, and evoCas9, respectively. $N_{20}$-NGG sites are about 4.3 times more frequent than $G$-$N_{19}$-NGG sites in the human genome.

**Fig. 12 Summary of T7 endonuclease I (T7E1) assay results for DNA mismatch cleavage in OVCAR8-ADR cells.** Cells were infected with an SpCas9 variant and the indicated gRNA, and genomic DNA were collected for T7E1 assay after 11 to 16 days post-infection. Indel quantification for the infected samples is displayed as a bar graph.

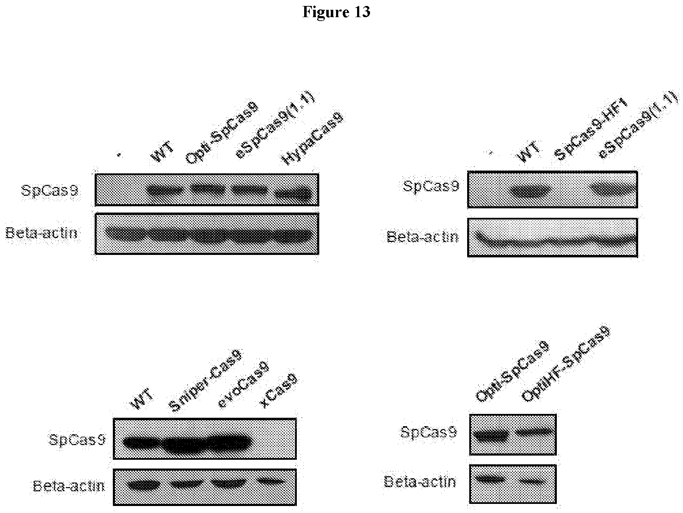

**Fig. 13 Expression of SpCas9 variants in OVCAR8-ADR cells.** Cells were infected with lentiviruses encoding WT SpCas9, Opti-SpCas9, eSpCas9(1.1), HypaCas9, SpCas9-HF1, Sniper-Cas9, evoCas9, xCas9, or OptiHF-SpCas9. Protein lysates were extracted for Western blot analysis, and immunoblotted with anti-SpCas9 antibodies. Beta-actin was used as loading control. Expression of SpCas9-HF1 and xCas9 was not detected in OVCAR8-ADR cells, which could be due to their non-optimized sequence for expression in mammalian cells[24,49], and thus SpCas9-HF1 and xCas9 were not included in other activity assays. These experiments were repeated independently for three times with similar results.

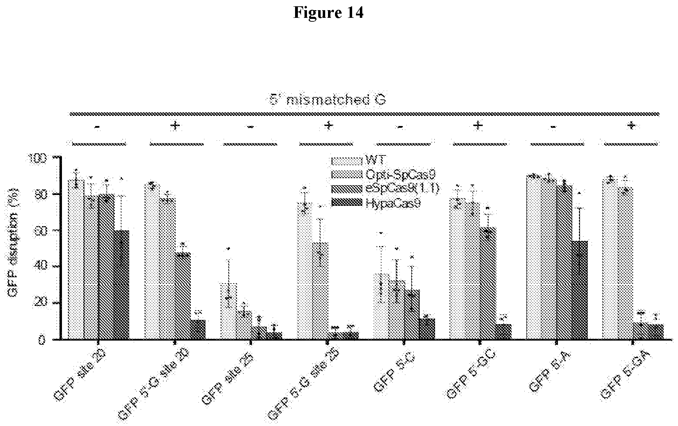

**Fig. 14 Evaluation of the editing efficiency of SpCas9 variants with gRNAs bearing or lacking an additional mismatched 5' guanine (5'G) using GFP disruption assay.** OVCAR8-ADR cells expressing WT SpCas9, Opti-SpCas9, eSpCas9(1.1), or HypaCas9 were infected with lentiviruses encoding gRNAs carrying or lacking an additional mismatched 5'G. Editing efficiency was measured by cell percentage with depleted GFP level using flow cytometry. Values and error bars reflect the mean and s.d. of four independent biological replicates.

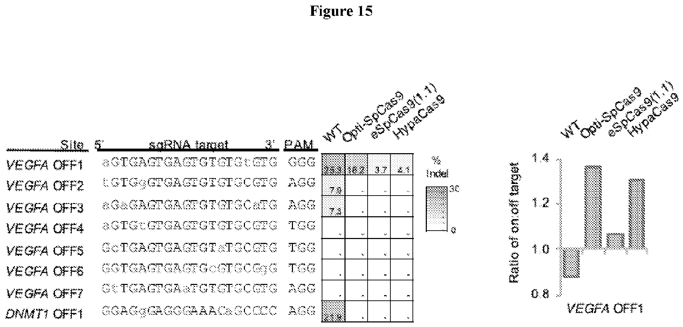

**Fig. 15 Opti-SpCas9 exhibits reduced off-target activity when compared to wild-type SpCas9.** Assessment of SpCas9 variants for off-target editing brought by *VEGFA* site 3 or *DNMT1* site 4 gRNA at eight endogenous loci. Percentage of indel was measured using T7E1 assay, averaged from three independent experiments. Dash indicates none detected. Specificity of WT SpCas9 and its variants with *VEGFA* site 3 gRNA at OFF1 loci is plotted as the ratio of on-target to off-target activity (on-target activity data was obtained from Fig. 12).

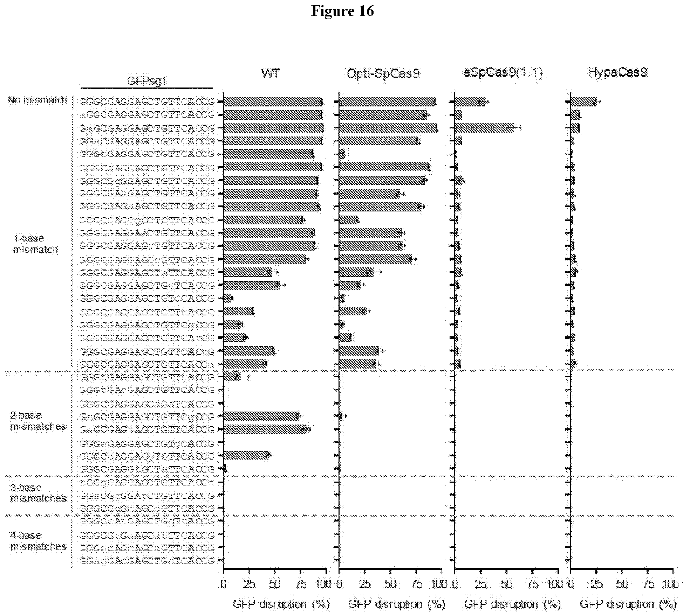

**Fig. 16 Characterization of SpCas9 variants for editing target sites harboring sequences that are perfectly matched with the gRNA's spacer or contain mismatch(es) using GFP disruption assay.** OVCAR8-ADR cells expressing WT SpCas9, Opti-SpCas9, eSpCas9(1.1), or HypaCas9 were infected with lentiviruses encoding gRNAs carrying no or one- to four-base mismatch(es) against the target. Editing efficiency was measured by cell percentage with depleted GFP level using flow cytometry. Values and error bars reflect the mean and s.d. of three independent biological replicates.

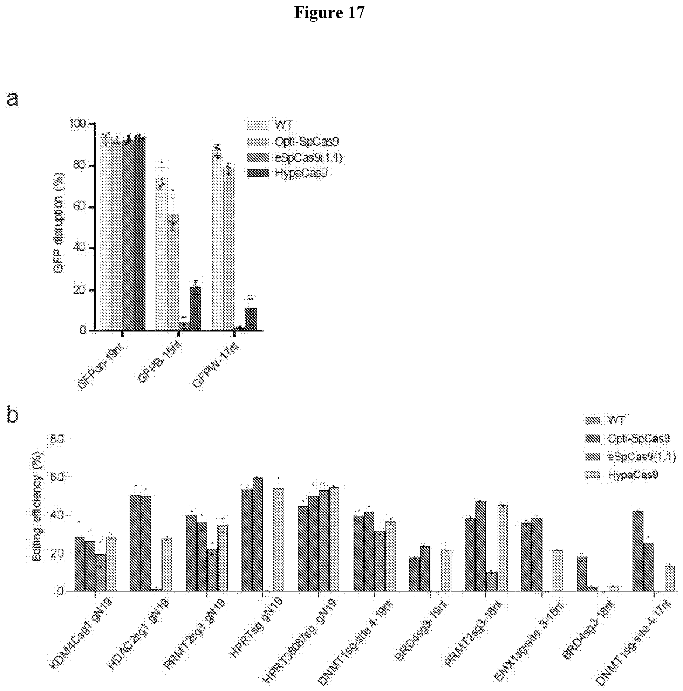

**Fig. 17 On-target editing activity of SpCas9 variants using truncated gRNAs. a, b,** OVCAR8-ADR cells expressing WT SpCas9, Opti-SpCas9, eSpCas9(1.1), or HypaCas9 were infected with lentiviruses encoding gRNAs of varied length (17 to 19 nucleotides) targeting the GFP sequence (**a**) and endogenous loci (**b**). Editing efficiency was measured by cell percentage with depleted GFP level using flow cytometry (**a**) and T7E1 assay (**b**). The list of gRNA sequences used is presented in Table 5. For (**a**), values and error bars reflect the mean and s.d. of four independent biological replicates.

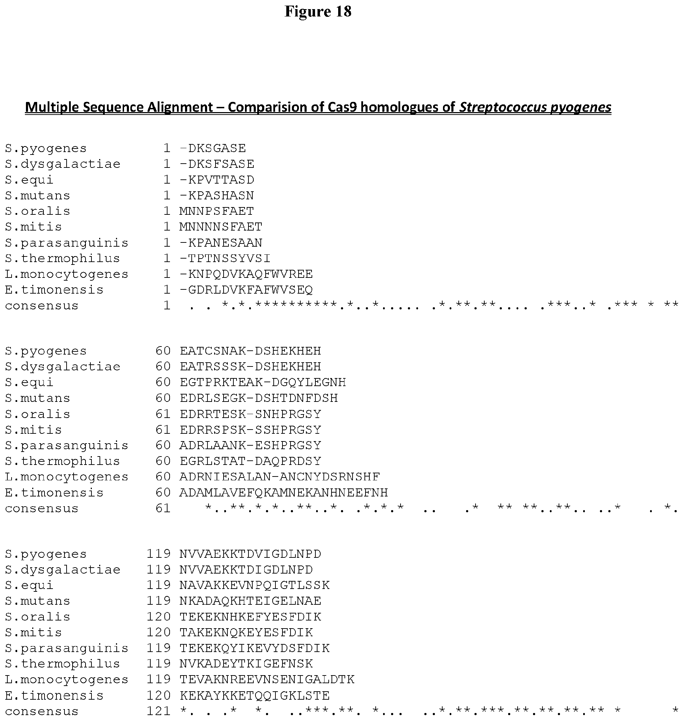

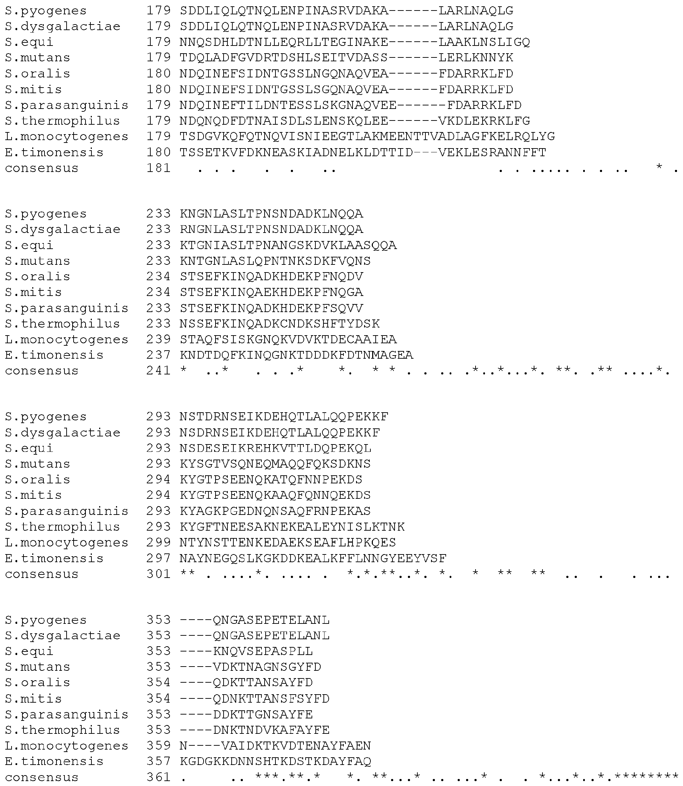

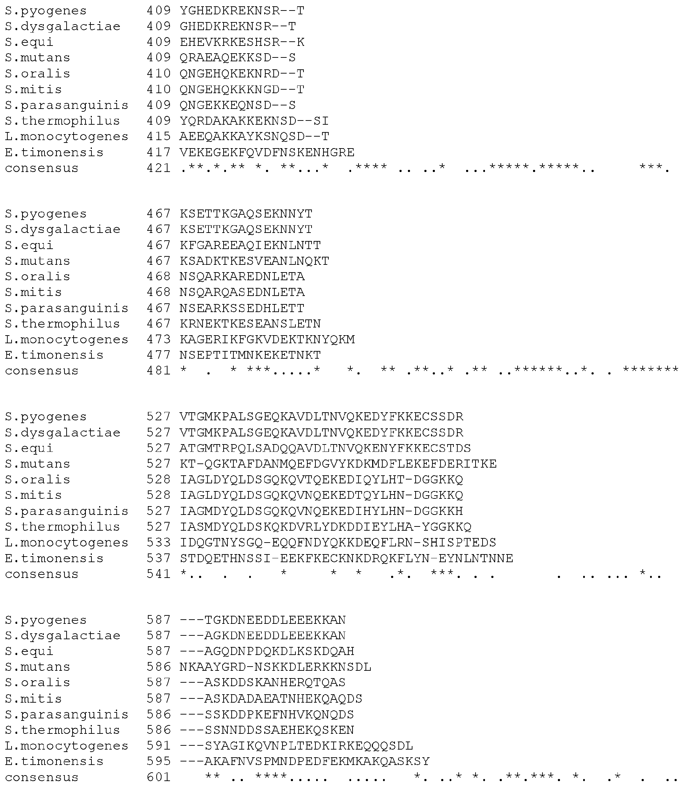

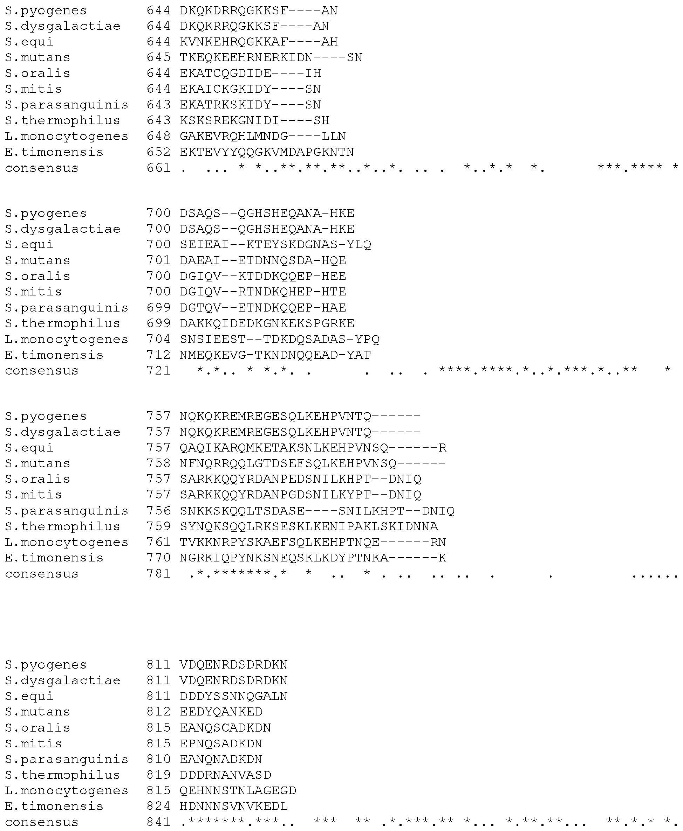

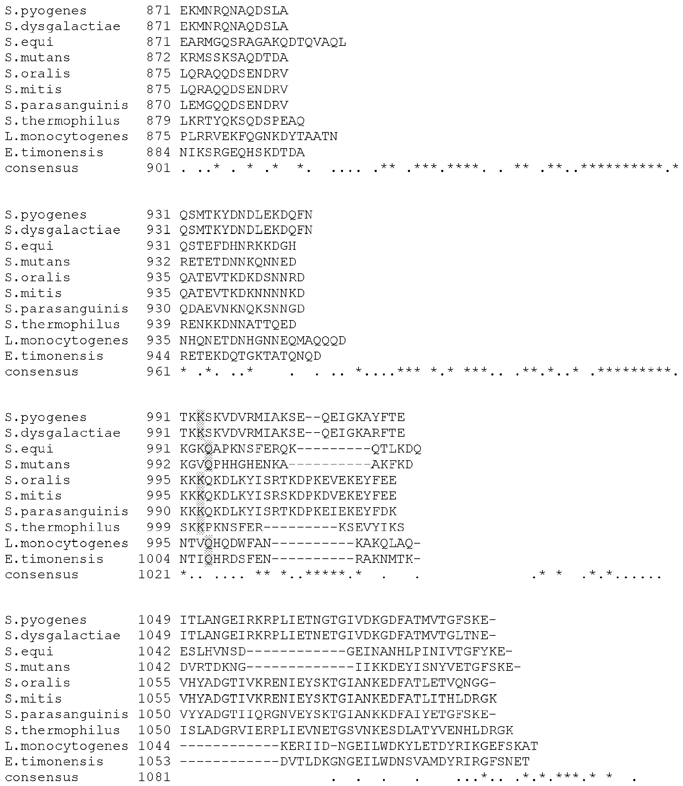

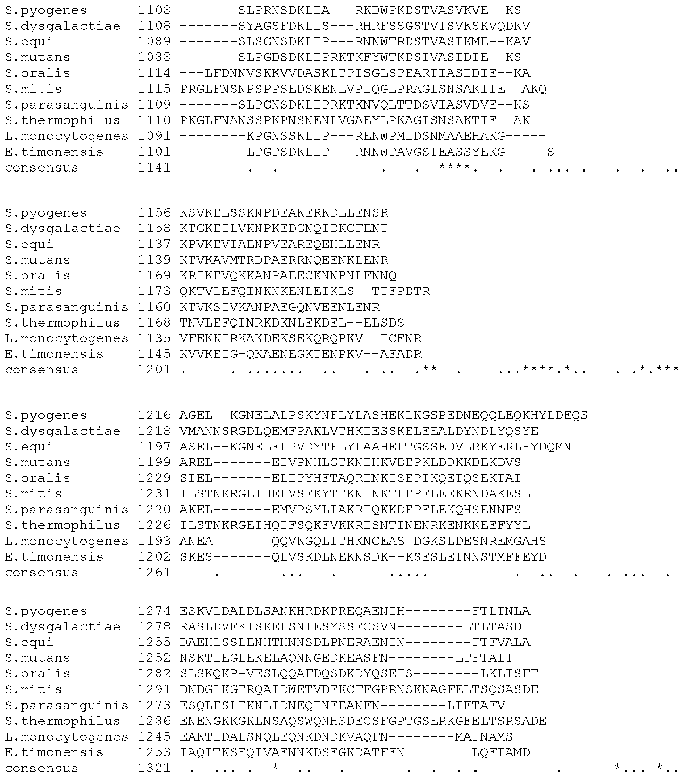

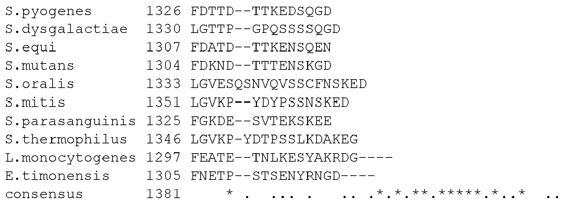

**Fig. 18 Multiple Sequence Alignment - Comparison of Cas9 homologues of *Streptococcus pyogenes.*** Conserved amino acid residues among the Cas9 homologues, especially those corresponding to SpCas9 residues 661 and 1003, are marked.

DEFINITIONS

**"CRISPR-Cas9"** or **"Cas9"** as used herein refers to a CRISPR associated protein 9, an RNA-guided DNA endonuclease enzyme associated with the CRISPR (Clustered Regularly Interspaced Short Palindromic Repeats) adaptive immunity system found in some bacteria species, including *Streptococcus pyogenes.* SpCas9, the Cas9 protein of the *Streptococcus pyogenes* origin, has the amino acid sequence set forth in SEQ ID NO:1, which is encoded by the polynucleotide sequence set forth in SEQ ID NO:2. Additional Cas9 enzymes with significant sequence homology including at least some (*e.g.*, at least two, three, four, five or more, such as at least half but not necessarily all) of the known key conserved residues such as residues 661, 695, 848, 923, 924, 926, 1003, and 1060 of SEQ ID NO:1, see sequence alignment in Figure 18. As used herein, the term "Cas9 protein" encompasses any RNA-guided DNA endonuclease enzyme that share substantial amino acid sequence identity with SEQ ID NO:1, *e.g.,* at least 50%, 60%, 70%, 75%, up to 80%, 85% or more overall sequence identity. Exemplary wild-type Cas9 proteins include those from bacterial species *Streptococcus mutans, Streptococcus dysgalactiae, Streptococcus equi, Streptococcus oralis, Streptococcus mitis, Listeria monocytogenes, Enterococcus timonensis, Streptococcus thermophilus,* and *Streptococcus parasanguinis*, having the amino acid sequences set forth in SEQ ID NOs:4-13, respectively.

The term **"nucleic acid"** or **"polynucleotide"** refers to deoxyribonucleotides or ribonucleotides and polymers thereof in either single- or double-stranded form. Unless specifically limited, the term encompasses nucleic acids containing known analogues of natural nucleotides which have similar binding properties as the reference nucleic acid and are metabolized in a manner similar to naturally occurring nucleotides. Unless otherwise indicated, a particular nucleic acid sequence also implicitly encompasses conservatively modified variants thereof (*e.g.*, degenerate codon substitutions) and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res., 19:5081 (1991); Ohtsuka et al., J. Biol. Chem., 260:2605-2608 (1985); and Cassol *et al.,* (1992); Rossolini et al., Mol. Cell. Probes, 8:91-98 (1994)). The terms nucleic acid and polynucleotide are used interchangeably with gene, cDNA, and mRNA encoded by a gene.

The terms **"polypeptide," "peptide,"** and **"protein"** are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymers. As used herein, the terms encompass amino acid chains of any length, including full length proteins (*i.e.*, antigens), wherein the amino acid residues are linked by covalent peptide bonds.

The term **"amino acid"** refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, *e.g.*, hydroxyproline, γ-carboxyglutamate, and O-phosphoserine. Amino acid analogs refers to compounds that have the same basic chemical structure as a naturally occurring amino acid, *i.e.,* an α carbon that is bound to a hydrogen, a carboxyl group, an amino group, and an R group, *e.g.*, homoserine, norleucine, methionine sulfoxide, methionine methyl sulfonium. Such analogs have modified R groups (*e.g.*, norleucine) or modified peptide backbones, but retain the same basic chemical structure as a naturally occurring amino acid. "Amino acid mimetics" refers to chemical compounds that have a structure that is different from the general chemical structure of an amino acid, but that functions in a manner similar to a naturally occurring amino acid.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

An **"expression cassette"** is a nucleic acid construct, generated recombinantly or synthetically, with a series of specified nucleic acid elements that permit transcription of a particular polynucleotide sequence in a host cell. An expression cassette may be part of a plasmid, viral genome, or nucleic acid fragment. Typically, an expression cassette includes a polynucleotide to be transcribed, operably linked to a promoter. "Operably linked" in this context means two or more genetic elements, such as a polynucleotide coding sequence and a promoter, placed in relative positions that permit the proper biological functioning of the elements, such as the promoter directing transcription of the coding sequence. Other elements that may be present in an expression cassette include those that enhance transcription (*e.g.,* enhancers) and terminate transcription (*e.g.,* terminators), as well as those that confer certain binding affinity or antigenicity to the recombinant protein produced from the expression cassette.

A **"vector"** is a circular nucleic acid construct recombinantly produced from a bacteria-based structure (*e.g.,* a plasmid) or virus-based structure (*e.g.,* a viral genome). Typically a vector contains an origin for self-replication, in addition to one or more genetic components of interest (*e.g.,* polynucleotide sequences encoding one or more proteins). In some cases, a vector may contain an expression cassette, making the vector an expression vector. In other cases, a vector may not contain an apparatus for the expression of a coding sequence but rather acts as a carrier or shuttle for the storage and/or transfer of one or more genetic components of interest (*e.g.,* coding sequences) from one genetic construct to another. Optionally, a vector may further include one or more selection or identification marker-coding sequences, which may encode for proteins such as antibiotic-resistant proteins (*e.g.,* for detection of a bacterial host cell) or fluorescent proteins (*e.g.,* for detection of a eukaryotic host cell) so as to allow ready detection of transformed or transfected host cells that harbor the vector and permit protein expression from the vector.

The term **"heterologous,"** when used in the context of describing the relationship between two elements such as two polynucleotide sequences or two polypeptide sequences in a recombinant construct, describes the two elements as being derived from two different origins and now being placed in a position relative to each other not found in nature. For example, a "heterologous" promoter directing the expression of a protein coding sequence is a promoter not found in nature to direct the expression of the coding sequence. As another example, in the case of a peptide fused with a "heterologous" peptide to form a recombinant polypeptide, the two peptide sequences are either derived from two different parent proteins or derived from the same protein but two separate parts not immediately adjacent to each other. In other words, the placement of two elements "heterologous" to each other does not result in a longer polynucleotide or polypeptide sequence that can be found in nature.

As used herein, the term **"barcode"** refers to a short stretch of polynucleotide sequence (typically no longer than 30 nucleotides, *e.g.,* between about 4 or 5 to about 6, 7, 8, 9, 10, 12, 20, or 25 nucleotides) that is uniquely assigned to another, pre-determined polynucleotide sequence (for example, one segment of the coding sequence for a protein of interest, such as SpCas9) so as to allow detection/identification of the pre-determined polynucleotide sequence or its encoded amino acid sequence based on the presence of the barcode.

**"Type IIS restriction enzymes"** are endonucleases that recognize asymmetric DNA sequences and cleave outside (to the 3' or 5') of their recognition sequences. They act in contrast to type IIP restriction enzymes, which recognize symmetric or palindromic DNA sequences and cleave within their recognition sequences. Because type IIS restriction enzymes cut DNA strands outside of their recognition sequences, they can generate overhangs of virtually any sequences independent of their recognition sequences. It is thus possible to use two different type IIS restriction enzymes to generate not only the same size and same direction overhangs (*i.e.,* the overhangs are both 3' or 5' overhangs and have the same number of nucleotides) but also matched overhangs or compatible ends (*i.e.,* the overhangs on the two opposite strands are fully complementary), which would allow annealing and ligation between two ends generated by the two different type IIS restriction enzymes.

As used herein, the term **"short guide RNA"** or **"sgRNA"** refers to an RNA molecule of about 15-50 (*e.g.,* 20, 25, or 30) nucleotides in length that specifically binds to a DNA molecule at a pre-determined target site and guides a CRISPR nuclease to cleave the DNA molecule adjacent to the target site.

A nucleotide sequence **"binds specifically"** to anther when the two polynucleotide sequences, especially two single-stranded DNA or RNA sequences, complex with each other to form a double-stranded structure based on substantial or complete (*e.g.,* at least about 80%, 85%, 90%, 95%, 96%, 97%, 98%, 99%, or up to 100%) Watson-Crick complementarity between the two sequences.

**"Physiologically acceptable excipient/carrier"** and "pharmaceutically acceptable excipient/carrier" refer to a substance that aids the administration of an active agent to - and often absorption by - a delivery target (cells, tissue, or a live organism) and can be included in the compositions of the present invention without causing an significant effect on the recipient. Non-limiting examples of physiologically/pharmaceutically acceptable excipients include water, NaCl, normal saline solutions, lactated Ringer's, normal sucrose, normal glucose, binders, fillers, disintegrants, lubricants, coatings, sweeteners, flavoring and coloring agents, and the like. As used herein, the term "physiologically/pharmaceutically acceptable excipient/carrier" is intended to include any and all solvents, dispersion media, coatings, antibacterial and antifungal agents, isotonic and absorption delaying agents, and the like, compatible with the intended use.

The term **"about"** when used in reference to a pre-determined value denotes a range encompassing ±10% of the value.

DETAILED DESCRIPTION

**I. GENERAL**

The present invention relates to a newly improved high-order genetic modification and screening platform for high-efficiency generation and identification of recombinant proteins with desirable biological functionalities. This invention also provides a recombinant protein produced by the platform.

A. Recombinant Technology

Basic texts disclosing general methods and techniques in the field of recombinant genetics include Sambrook and Russell, Molecular Cloning, A Laboratory Manual (3rd ed. 2001); Kriegler, Gene Transfer and Expression: A Laboratory Manual (1990); and Ausubel et al., eds., Current Protocols in Molecular Biology (1994).

For nucleic acids, sizes are given in either kilobases (kb) or base pairs (bp). These are estimates derived from agarose or acrylamide gel electrophoresis, from sequenced nucleic acids, or from published DNA sequences. For proteins, sizes are given in kilodaltons (kDa) or amino acid residue numbers. Proteins sizes are estimated from gel electrophoresis, from sequenced proteins, from derived amino acid sequences, or from published protein sequences.

Oligonucleotides that are not commercially available can be chemically synthesized, *e.g.*, according to the solid phase phosphoramidite triester method first described by Beaucage & Caruthers, Tetrahedron Lett. 22: 1859-1862 (1981), using an automated synthesizer, as described in Van Devanter et. al., Nucleic Acids Res. 12: 6159-6168 (1984). Purification of oligonucleotides is performed using any art-recognized strategy, *e.g.*, native acrylamide gel electrophoresis or anion-exchange HPLC as described in Pearson & Reanier, J. Chrom. 255: 137-149 (1983).

The polynucleotide sequence encoding a polypeptide of interest, *e.g.*, an SpCas9 protein or a fragment thereof, and synthetic oligonucleotides can be verified after cloning or subcloning using, *e.g.*, the chain termination method for sequencing double-stranded templates of Wallace et al., Gene 16: 21-26 (1981).

B. Modification of a Polynucleotide Coding Sequence

Given the known amino acid sequence of a pre-selected protein of interest (*e.g.*, SpCas9), modifications can be made in order to achieve a desirable feature or improved biological functionality of the protein, as may be determined by *in vitro* or *in vivo* methods known in the field as well as described herein. Possible modifications to the amino acid sequence may include substitutions (conservative or non-conservative); deletion or addition of one or more amino acid residues at one or more locations of the amino acid sequence.

A variety of mutation-generating protocols are established and described in the art, and can be readily used to modify a polynucleotide sequence encoding a protein of interest. *See, e.g.,* Zhang et al., Proc. Natl. Acad. Sci. USA, 94: 4504-4509 (1997); and Stemmer, Nature, 370: 389-391 (1994). The procedures can be used separately or in combination to produce variants of a set of nucleic acids, and hence variants of encoded proteins.

Mutational methods of generating diversity include, for example, site-directed mutagenesis (Botstein and Shortle, Science, 229: 1193-1201 (1985)), mutagenesis using uracil-containing templates (Kunkel, Proc. Natl. Acad. Sci. USA, 82: 488-492 (1985)), oligonucleotide-directed mutagenesis (Zoller and Smith, Nucl. Acids Res., 10: 6487-6500 (1982)), phosphorothioate-modified DNA mutagenesis (Taylor et al., Nucl. Acids Res., 13: 8749-8764 and 8765-8787 (1985)), and mutagenesis using gapped duplex DNA (Kramer *et al., Nucl. Acids Res.,* **12**: 9441-9456 (1984)).

Other possible methods for generating mutations include point mismatch repair (Kramer et al., Cell, 38: 879-887 (1984)), mutagenesis using repair-deficient host strains (Carter et al., Nucl. Acids Res., 13: 4431-4443 (1985)), deletion mutagenesis (Eghtedarzadeh and Henikoff, Nucl. Acids Res., 14: 5115 (1986)), restriction-selection and restriction-purification (Wells et al., Phil. Trans. R. Soc. Land. A, 317: 415-423 (1986)), mutagenesis by total gene synthesis (Nambiar et al., Science, 223: 1299-1301 (1984)), double-strand break repair (Mandecki, Proc. Natl. Acad. Sci. USA, 83: 7177-7181 (1986)), mutagenesis by polynucleotide chain termination methods (U.S. Patent No. 5,965,408), and error-prone PCR (Leung et al., Biotechniques, 1: 11-15 (1989)).

C. Modification of Nucleic Acids for Preferred Codon Usage

The polynucleotide sequence encoding a protein of interest or a fragment thereof can be further altered based on the principle of codon degeneracy to coincide with the preferred codon usage either to enhance recombinant expression in a particular type of host cells or to facilitate further genetic manipulation such as to allow construction of restriction endonuclease recognition sequences at desirable sites for potential cleavage/re-ligation. The latter usage is of particular importance in the present invention as seamless connection of multiple coding segments of a target protein (*e.g.*, SpCas9 protein) undergoing combinatorial mutagenesis relies on the digestion of the coding segments by type IIS restriction enzymes to generate overhangs that are specifically derived from the coding sequences of the native protein so as to eliminate any extraneous sequences or the so-called scar sequences at the junctures between any two of these segments.

At the completion of modification, the coding sequences are verified by sequencing and are then subcloned into an appropriate vector for further manipulation or for recombinant expression of the protein.

## D. Expression of Recombinant Polypeptides

A recombinant polypeptide of interest (*e.g.*, an improved Cas9 protein) can be expressed using routine techniques in the field of recombinant genetics, relying on the polynucleotide sequences encoding the polypeptide as disclosed herein.

### (i) Expression Systems

To obtain high level expression of a nucleic acid encoding a polypeptide of interest, one typically subclones the polynucleotide coding sequence into an expression vector that contains a strong promoter to direct transcription, a transcription/translation terminator and a ribosome binding site for translational initiation. Suitable bacterial promoters are well known in the art and described, *e.g.*, in Sambrook and Russell, *supra,* and Ausubel *et al., supra.* Bacterial expression systems for expressing recombinant polypeptides are available in, *e.g., E. coli, Bacillus sp., Salmonella*, and *Caulobacter.* Kits for such expression systems are commercially available. Eukaryotic expression systems for mammalian cells, yeast, and insect cells are well known in the art and are also commercially available. Some exemplary eukaryotic expression vectors include adenoviral vectors, adeno-associated vectors, and retroviral vectors such as viral vectors derived from lentiviruses.

The promoter used to direct expression of a heterologous polynucleotide sequence encoding a protein of interest depends on the particular application. The promoter is optionally positioned about the same distance from the heterologous transcription start site as it is from the transcription start site in its natural setting. As is known in the art, however, some variation in this distance can be accommodated without loss of promoter function.

In addition to the promoter, the expression vector typically includes a transcription unit or expression cassette that contains all the additional elements required for the expression of the desired polypeptide in host cells. A typical expression cassette thus contains a promoter operably linked to the nucleic acid sequence encoding the polypeptide and signals required for efficient polyadenylation of the transcript, ribosome binding sites, and translation termination. In the case of recombinant expression of a secreted protein, the polynucleotide sequence encoding the protein is typically linked to a cleavable signal peptide sequence to promote secretion of the recombinant polypeptide by the transformed cell. If, on the other hand, a recombinant polypeptide is intended to be expressed on the host cell surface, an appropriate anchoring sequence is used in concert with the coding sequence. Additional elements of the cassette may include enhancers and, if genomic DNA is used as the structural gene, introns with functional splice donor and acceptor sites.

In addition to a promoter sequence, the expression cassette should also contain a transcription termination region downstream of the coding sequence to provide for efficient termination. The termination region may be obtained from the same gene as the promoter sequence or may be obtained from different genes.

Expression vectors containing regulatory elements from eukaryotic viruses are typically used in eukaryotic expression vectors, e.g., SV40 vectors, papilloma virus vectors, lentivirus vectors, and vectors derived from Epstein-Barr virus. Other exemplary eukaryotic vectors include pMSG, pAV009/A$^+$, pMTO10/A$^+$, pMAMneo-5, baculovirus pDSVE, and any other vector allowing expression of proteins under the direction of the SV40 early promoter, SV40 later promoter, metallothionein promoter, murine mammary tumor virus promoter, Rous sarcoma virus promoter, polyhedrin promoter, or other promoters shown effective for expression in eukaryotic cells.

The elements that are typically included in expression vectors may also include a replicon that functions in *E. coli,* a gene encoding antibiotic resistance to permit selection of bacteria that harbor recombinant plasmids, and unique restriction sites in nonessential regions of the plasmid to allow insertion of eukaryotic sequences. The particular antibiotic resistance gene chosen is not critical, any of the many resistance genes known in the art are suitable. The prokaryotic sequences are optionally chosen such that they do not interfere with the replication of the DNA in eukaryotic cells, if necessary. Similar to antibiotic resistance selection markers, metabolic selection markers based on known metabolic pathways may also be used as a means for selecting transformed host cells.

As discussed above, a person skilled in the art will recognize that various conservative substitutions can be made to a protein or its coding sequence while still retaining the biological activity of the protein. Moreover, modifications of a polynucleotide coding sequence may also be made to accommodate preferred codon usage in a particular expression host or to generate a restriction enzyme cleavage site without altering the resulting amino acid sequence.

*(ii) Transfection Methods*

Standard transfection methods are used to produce bacterial, mammalian, yeast, insect, or plant cell lines that express large quantities of a recombinant polypeptide, which are then purified using standard techniques (*see, e.g.*, Colley et al., J. Biol. Chem. 264: 17619-17622 (1989); Guide to Protein Purification, in Methods in Enzymology, vol. 182 (Deutscher, ed., 1990)). Transformation of eukaryotic and prokaryotic cells are performed according to standard techniques (*see, e.g.*, Morrison, J. Bact. 132: 349-351 (1977); Clark-Curtiss & Curtiss, Methods in Enzymology 101: 347-362 (Wu *et al.*, eds, 1983).

Any of the well-known procedures for introducing foreign nucleotide sequences into host cells may be used. These include the use of calcium phosphate transfection, polybrene, protoplast fusion, electroporation, liposomes, microinjection, plasma vectors, viral vectors and any of the other well-known methods for introducing cloned genomic DNA, cDNA, synthetic DNA, or other foreign genetic material into a host cell (*see, e.g.*, Sambrook and Russell, *supra*). It is only necessary that the particular genetic engineering procedure used be capable of successfully introducing at least one gene into the host cell capable of expressing the recombinant polypeptide.

## II. IMPROVED COMBINATORIAL GENETIC MODIFICATION SYSTEM

Based on previously developed high-throughput CombiGEM combinatorial genetic modification system and the like, the present inventors have made further modifications to these systems with a goal to seamlessly join DNA elements encoding protein segments, each corresponding to a portion of a protein of interest (*e.g.*, SpCas9) and containing at least one, possibly multiple, mutations in its amino acid sequence, such that the resultant composite protein variants will have no extraneous amino acid residues except for the intentionally introduced mutations. As the previous methodologies utilize type IIP restriction endonucleases to cleave and religate DNA sequences (which encode segments of the combinatorial protein variant), the nature of this type of endonucleases (binding to and cleaving within a short palindromic stretch of nucleotide sequence) typically requires the user to engineer cleavage sites by introducing extra nucleotides, which in turn results in extraneous amino acid residue(s), or a "scar" sequence, at each junction point between two segments in the protein variants generated by the systems. These extraneous amino acid residues further alter the protein sequence and can potentially interfere with functional screening of the variants.

In their effort to avoid introducing these unwanted extra amino acid residues, the present inventors discovered that, if type IIS restriction enzymes are instead used for constructing and ligating multiple DNA coding sequences encoding segments of a protein to build a library of combinatorial genetic variants, such undesirable "scar" sequences between the segments can be entirely eliminated. This strategy takes advantage of the fact that the type IIS endonucleases are able to cleave DNA strands outside of their asymmetric recognition sites, which allows compatible ends or matched overhangs having a portion of the native DNA coding sequence for the wild-type protein to be generated after DNA cleavage by these enzymes. The use of native protein-derived coding sequence in the compatible ends or matched overhangs not only supports seamless junctures between protein segments but also allows for specific directional ligation, further enhancing efficiency in the process of constructing combinatorial protein variants.

### A. Generation of Libraries of DNA Segments Encoding Protein Segments

The first step in generating a library of combinatorial protein variants is to generate a library for each one of the segments of the protein: a protein variant can be designed such that it is to be produced by joining end to end a predetermined number (for example, 3, 4, 5, 6, or more) of protein segments or modules. As in this disclosure the predetermined number is expressed as n+1, then for a protein of interest is devised to consist of 6 segments, n=5. A library or a collection of individual members of DNA elements encoding the first protein segment, which corresponds to the most-N-terminal portion of the wild-type protein and contains one or more possible mutations in this portion of the protein, may be first generated by known methods such as recombinant production or chemical synthesis, and then incorporated into a DNA vector (a so-called storage vector for its purpose) that contains the appropriate restriction enzyme sites as well as a barcode sequence uniquely assigned to a DNA element harboring a pre-determined mutation (or a predetermined set of mutations). If the DNA element is relatively long, it may be first made by joining shorter fragments by known methods such as Gibson assembly before being incorporated into a storage vector. As discussed above, methods of generating DNA sequence mutations are well-known to those of skill in the art and can be readily employed to create sequence variants by modifying the native version or wild-type sequence, *e.g.*, by deletion, insertion, and/or substitution of one or more nucleotides.

Figure 5a depicts an example of how a DNA element encoding a protein segment is inserted and ligated into a vector to form a DNA construct that includes, from 5' to 3', a first recognition site for a first type IIS restriction enzyme (*e.g.*, BsaI), the DNA element, a first and a second recognition sites for a second type IIS restriction enzyme (*e.g.*, BbsI), a barcode uniquely assigned to the DNA element for the specific mutation(s) it harbors, and a second recognition site for the first type IIS restriction enzyme (*e.g.*, BsaI). For a protein that has been designed or "deconstructed" to have (n+1) segments or modules for combinatorial mutation studies, a library of storage vectors containing DNA segments can be constructed in the same fashion for each of the subsequent DNA elements, the second, third, and so forth until the nth DNA element (encoding the second, third, and so forth until the nth protein segment, respectively), the nth protein segment corresponding to the second to the last or the most-C-terminal portion of the protein.

For the DNA element encoding the last or most-C-terminal segment of the protein, a structurally different storage vector is employed in constructing the library of vectors containing the (n+1)th DNA elements. As exemplified in Figure 5a, the last or the (n+1)th DNA element is inserted into this storage vector to form a DNA construct that includes, from 5' to 3', a first recognition site for a first type IIS restriction enzyme (*e.g.*, BsaI), the (n+1)th DNA element, a short stretch of nucleotide sequence serving as a primer-binding site, a barcode uniquely assigned to the DNA element for the specific mutation(s) it harbors, and a second recognition site for the first type IIS restriction enzyme (*e.g.*, BsaI). The presence and placement of the primer-binding site allows for rapid sequencing of the combined barcodes utilizing a universal primer (which binds specifically to the primer-binding site) after a composite coding sequence (combining all n+1 DNA elements) for a protein variant is generated, so as to permit easy identification of the mutations harbored in the variant, making it unnecessary to perform the laborious task of sequencing the entire composite coding sequence.

In order to ensure equal opportunity for each potential combinatorial protein variant in the library, DNA elements each harboring a unique set of mutations are preferably present in a library at an equal molar ratio.

B. Generation of Combinatorial Protein Mutant Library

Once the libraries of storage vectors containing the first, second, and so forth until the nth, and the (n+1)th DNA elements have been constructed, DNA fragments containing the DNA elements encoding the protein segments or modules are first released by way of enzymatic digestion of the storage vectors, for example, by using the first type IIS restriction endonuclease (*e.g.,* BsaI) to cleave the vectors at two sites. The digestion of the storage vectors releases DNA fragments each containing the DNA element encoding a protein segment (harboring mutations) and its uniquely assigned barcode, with the two type IIS restriction enzyme (*e.g.*, BbsI) recognition sites sandwiched in between. The two ends of the DNA fragments have overhangs produced by the first type IIS restriction enzyme cleavage.

In the meantime, a DNA vector that is intended to carry and express the final composite DNA elements encoding an entire protein variant (a so-called destination vector for its purpose) is an expression vector containing all necessary genetic elements for the expression of a DNA coding sequence. As discussed in an earlier section, one essential element for transcription is a promoter that is to be operably linked to a coding sequence in order to direct transcription of the sequence. Typically, the promoter is a heterologous promoter to the coding sequence.

In order to receive DNA fragments produced from the storage vector libraries, the destination vector is linearized, also by way of digestion by a type IIS restriction enzyme, at a site that is a suitable distance downstream from the promoter so as to permit insertion/ligation of the DNA fragment and place the DNA element (which encodes the protein segment) within the DNA fragment under the control of the promoter for transcription. Often the type IIS restriction enzyme used to linearize the destination vector is different from that used to release the DNA fragments from the storage vectors. But they preferably generate the same size and matched overhangs so as to allow ligation of the DNA fragments into the destination vector.

As illustrated in Figure 5b, when the library of storage vectors containing the full variety of the first DNA elements encoding the full variety of the first protein segments are digested by the first type IIS restriction enzyme, a library of DNA fragments containing the full variety of the first DNA elements along with their corresponding barcodes are released from their storage vectors. This library of these first DNA fragments, preferably at equal molar ratio for each sequence variety, are then ligated into the linearized the destination vector, resulting in a 1-wise library. Each member of the resultant 1-wise library will contain a functional expression cassette in which the promoter is operably linked to the first DNA element and capable of directing the expression of the first or most-N-terminal protein segment encoded by the first DNA element.

The 1-wise library is subsequently digested again with a type IIS restriction enzyme, cleaving each member of the library twice between the first DNA element and its barcode, generating two overhangs at each cleavage site.

Meanwhile the library of storage vectors containing the full variety of the second DNA elements encoding the full variety of the second protein segments are digested by the first type IIS restriction enzyme, a library of DNA fragments containing the full variety of the second DNA elements along with their corresponding barcodes are released from their storage vectors. This library of these second DNA fragments, preferably at equal molar ratio for each sequence variety, are then ligated into the linearized 1-wise expression vector between the first DNA element and its corresponding barcode, resulting in a new library of 2-wise expression vectors. Each member of the resultant 2-wise library will contain an functional expression cassette in which the promoter is operably linked to the first DNA element fused with the second DNA element and capable of directing the expression of the fused first and second protein segments encoded by the fusion of the first DNA element and the second DNA element. To eliminate any extraneous amino acid residue or "scar" sequence at the fusion point between the first and second protein segments, the two cleavage sites located between the first DNA element and its barcode must be carefully designed so as to ensure (1) there is a perfect match (both in sequence and size/direction of overhangs) between the overhangs of the two ends of the linearized 1-way vector and the overhangs of the two ends of the second DNA fragments released from the library of the storage vectors containing the full variety of the second DNA elements; and (2) the matched overhang sequence between the tail or 3' end of the first DNA element and the head or 5' end of the second DNA element upon their ligation encodes for a stretch of amino acid sequence found in the wild-type protein of interest at the same location. In other words, the design of the cleavage sites ensures the seamless connection of two adjacent protein segments.

At the completion of ligation of the library of the second DNA fragments released from the library of the second storage vectors into the linearized 1-wise expression vector library, a library of 2-wise composite expression vectors is now constructed. Repeating the cycle of the steps outlined in the last two paragraphs, one can continue to incorporate into the composite expression vectors the third DNA fragment, and so forth until the nth and the (n+1)th DNA fragments to obtain a library of the final composite expression vectors, which contain a full array of DNA coding sequences encoding full length protein variants containing all possible combinations of mutations, each variant coding sequence followed by a composite barcode sequence, which will have all of the barcodes corresponding to their uniquely assigned to DNA elements but in the reverse order of how the DNA elements are fused.

### C. Functional Screening of Protein Variants

Since the final library of destination vectors are expression vectors each with a promoter operably linked to a composite DNA coding sequence containing all n+1 DNA elements to encode a full length protein variant containing a specific set of mutations, these protein variants can be readily expressed, screened, and selected for any particular desirable functional features in an appropriate reporting system. For example, a viral-based destination vector can be used to transfect host cells and direct expression of the variants of a protein of interest in the suitable cellular environment for functional analysis.

Figure 2a illustrates one example of how SpCas9 variants are screened for their functionalities: a cell line stably expressing a red fluorescent protein (RFP) and a gRNA that targets the RFP gene sequence was transfected with lentiviral vectors containing coding sequence for SpCas9 variants to indicate on-target activity of each variant, and another cell line stably expressing a RFP harboring synonymous mutations and the gRNA was transfected to indicate off-target activity of the variants. As the CombiSEAL platform is designed for potentially generating useful variants of any protein, different functional screening assays can be devised to depending on the specific functionality of the protein of interest. Once a clone of desirable functional characteristics (as in the case of a Cas9 protein, the on-target and off-target activity profile) is discovered, sequencing of the composite barcodes is performed to allow immediate identification of the specific mutations in the particular variant.

### III. OPTIMIZED CAS9 ENZYMES

Utilizing the newly improved CombiSEAL combinatorial genetic modification system, the present inventors identified a series of SpCas9 mutants and characterized their functional features. Among the mutants studied, a particular variant termed Opti-SpCas9 has been found to have a highly desirable functional profile: it possesses enhanced gene editing specificity without scarifying potency and broad testing range. In light of its functional attributes, this improved Cas9 enzyme is a highly valuable tool in the CRISPR genome editing schemes.

The wild-type SpCas9 protein has the amino acid sequence set forth in SEQ ID NO:1, and its corresponding DNA coding sequence is set forth in SEQ ID NO:2. Previous research on this endonuclease has provided insight about this protein's structure, including the regions and amino acid residues that interact with DNA. During their studies in developing the CombiSEAL platform, the present inventors confirmed that mutations, in particular substitutions, introduced at certain residues of the SpCas9's amino acid sequence that were previously predicted to interact with the target and non-target DNA strands have direct effects on the performance of the endonuclease. Specifically, substitutions at residues such as R661, Q695, K848, Q926, K1003, and K1060 are found to alter the enzyme's on-target/off-target editing activities. Variant Opti-SpCas9 is a double mutant of the wild-type SpCas9: residue 661 in SEQ ID NO:1 is substituted with Alanine and residue 1003 is substituted with Histidine. Its amino acid sequence is set forth in SEQ ID NO:3. These substitutions are responsible for the modified endonuclease's increased on-target editing efficiency and reduced off-target activity, a highly desirable phenotype.

The inventors have also identified a triple mutant of R661A, K1003H, and Q926A, which further decreases off-target editing from Opti-SpCas9 by about 80%, while its on-target activity is also reduced substantially. This triple mutant may be of value in a situation where avoidance of off-target cleavage is of particular importance. In addition, a second mutant termed OptiHF-SpCas9 has been generated, which has 5 point mutations Q695A, K848A, E923M, T924V, and Q926A (see variant 46 in Table 2). The amino acid sequences of Opti-SpCas9 and OptiHF-SpCas9 are set forth in SEQ ID NO:3 and SEQ ID NO:13, respectively. Table 2 provides a compilation of SpCas9 variants analyzed in this study detailing the point mutation(s) they contain and their on-target and off-target cleavage profile.

The SpCas9 variants disclosed herein are valuable tools in genetic manipulation of live cell genome. To use these variants for targeted DNA cleavage by the CRISPR system, one typically introduces into live cells an expression vector directing the expression of a variant (e.g., Opti-SpCas9) and an expression vector encoding for an sgRNA of the appropriate sequence for directing the SpCas9 variant to a pre-selected target site in the cell's genome in order to cleave the genomic DNA at the target site. In some embodiments, the expression vectors are viral vectors, such as retroviral vector especially lentiviral vectors. While the expression vector encoding the SpCas9 variant and the expression vector encoding the sgRNA are often two separate vectors, in some cases one single expression vector contains both coding sequences for the SpCas9 variant and for the sgRNA, with the two coding sequences operably linked to either the same promoter or two individual promoters. As the promoters are typically heterologous to the coding sequences, further consideration may be given to use promoters suitable for the specific type of recipient cells.

EXAMPLES

The following examples are provided by way of illustration only and not by way of limitation. Those of skill in the art will readily recognize a variety of non-critical parameters that could be changed or modified to yield essentially the same or similar results.

**Example 1: CombiSEAL as a high-throughput platform for seamlessly assembling barcoded combinatorial genetic units, thus offering a novel approach for protein optimization such as screening SpCas9 variants**

The combined effect of multiple mutations on protein function is hard to predict, thus the ability to functionally assess a vast number of protein sequence variants would be practically useful for protein engineering. Herein presented is a high-throughput platform that enables scalable assembly and parallel characterization of barcoded protein variants with combinatorial modifications. This platform CombiSEAL is illustrated by systematically characterizing a library of 948 combination mutants of the widely used *Streptococcus pyogenes* Cas9 (SpCas9) nuclease to optimize its genome-editing activity in human cells. The ease of pool-assessing editing activities of SpCas9 variants at multiple on- and off-target sites accelerates the identification of optimized variants and facilitates the study of mutational epistasis. Opti-SpCas9 was successfully identified, which possesses enhanced editing specificity without sacrificing potency and broad targeting range. This platform is broadly applicable for engineering proteins through combinatorial modifications *en mass.*

INTRODUCTION

Protein engineering has proven to be an important strategy for generating enzymes, antibodies, and genome-editing proteins with new or enhanced properties[1-7]. Combinatorial optimization of a protein sequence relies on strategies for creating and screening a large number of variants, but current approaches are limited in their ability to systematically and efficiently build and test multiple modifications in a high-throughput fashion[8-11]. Conventional site-directed mutagenesis based on structural and biochemical knowledge facilitates generation of functionally relevant mutants, but using such one-by-one approach to screen combination mutants lacks throughput and scalability. Gene synthesis technology can be deployed to make combination mutants in pooled format, but it typically gives 1 to 10 errors per kilo bases synthesized[12,13] and is prohibitively expensive if mutations to be introduced are scattered over different regions of a protein. Methods such as combinatorial DNA assembly[14,15] and recombination and shuffling[16] create combination mutants by fusing multiple mutated sequences together to assemble the entire protein sequence, but subsequent genotyping and characterization of the mutations requires selection of clonal isolates or long-read sequencing and neither of them is feasible for tracking a large number of mutants. Mutagenesis via error-prone polymerase chain reaction and mutator strains for directed evolution allows positive selection of desired mutated variants, but it suffers from selection bias towards a subset of amino acids due to the rare occurrence of two or more specific nucleotide mutations in a codon. Even if a great diversity of protein variants could be achieved with sequence randomization, the very limited throughput to genotype and analyze selected hits one-by-one is a major obstacle in protein engineering. Furthermore, pinpointing the exact mutations that confers a desired phenotype from the rest of the passenger mutations could be useful for accelerating the combinatorial optimization process.

Here the inventors devised a new cloning method to couple seamless combinatorial DNA assembly with the barcode concatenation strategy used in *Combinatorial Genetics En Masse* (CombiGEM)[17-19], a platform we termed CombiSEAL, for pooled assembly of barcoded combination mutants that can be easily tracked by high-throughput short-read sequencing (**Fig. 1**). CombiSEAL works by modularizing the protein sequence into composable parts, each comprising a repertoire of variants tagged with barcodes specifying predetermined mutations at defined positions. Type IIS restriction enzyme sites are used to flank the barcoded parts to create digested overhangs originating from the protein-coding sequence, thereby achieving seamless ligation upon fusing with the preceding parts. Unique barcodes are concatenated and appended to each protein-coding sequence variants in the resultant library after iterative pooled cloning of the parts. This method is advantageous over other strategies as it circumvents the need to perform long-read sequencing over the whole protein-coding region covering multiple mutations, which offers a cost-effective way to quantitatively track each variant in a pool by high-throughput sequencing of short (e.g., ~50-base pair) barcodes without the need to select clonal isolates. In addition, pooled characterization of variants allows their head-to-head comparisons under the same experimental condition, and facilitates the study of mutational epistasis. Unlike CombiGEM that only allows combinatorial assembly of discrete genetic components, CombiSEAL does not leave behind a fusion scar sequence to seamlessly link consecutive sequences (e.g., different segments of proteins). Therefore, this new platform has tremendous potentials for protein engineering.

RESULTS

**High-throughput screening of SpCas9 combination mutants.** CombiSEAL was applied to assemble a combination mutant library for SpCas9, the widely used Clustered Regularly Interspaced Short Palindromic Repeats (CRISPR) nuclease for genome engineering[20-23], with an aim to identify optimized variants with high editing specificity and activity. Previously, SpCas9 nucleases carrying specific combination of mutations, including eSpCas9(1.1)[3], SpCas9-HF1[4], HypaCas9[5] and evoCas9[6], were engineered to minimize their off-target editing. However, these variants have less targetable sites due to their incompatibility with gRNAs starting with a mismatched 5'-guanine (5'G)[3-6,24-2]. Limited number of combination mutants have been generated and tested to date **(Table 1)**, and thus necessitates a more systematic exploration of other SpCas9 variants with better compatibility with gRNAs bearing an extra 5'G.

Using CombiSEAL, the SpCas9 sequence was modularized into four parts, and barcoded inserts comprising different random and specific mutations at individual parts were cloned into storage vectors (Fig. 1a; **Fig. 7a, b**; see **METHODS** for details). A combinatorial barcoded library (with $4 \times 2 \times 17 \times 7 = 952$ SpCas9 variants, with wild-type (WT) SpCas9 and eSpCas9(1.1) sequences included) was then pooled assembled into a lentiviral vector. The individual parts and assembled constructs in the libraries were sequenced to confirm the highly accurate assembly of barcoded variants (See **METHODS** for details). The inventors detected high coverage for the library within both the plasmid pools stored in *Escherichia coli (E. coli)* (i.e., 951 out of 952 variants) and infected human cell pools (i.e., 948 out of 952 variants) **(Fig. 1b)**, and a highly reproducible representation between the plasmid and infected cell pools, as well as between biological replicates of infected cell pools **(Fig. 7c)**.

To search for robust and specific SpCas9 variants, a reporter system was established using monoclonal human cell lines to stably express red fluorescent protein (RFP) and a gRNA targeting the *RFP* gene sequence (referred to as RFPsg5-ON and RFPsg8-ON hereafter; **Fig. 2a**). Unlike previous screens that primarily used 20-nucleotide gRNAs starting with a 5'G[3-6], gRNAs carrying an additional 5'G in the reporter system were used to look for compatible SpCas9 variants that do not sacrifice targeting range. Cells were then infected with the SpCas9 variant library, and sorted into bins based on the RFP fluorescence levels at 14 days post-infection. The loss of RFP fluorescence reflects DNA cleavage and indel-mediated disruption of the target site, and thus cells harboring active SpCas9 variants would be enriched in the sorted bin with low RFP level. Using Illumina HiSeq to track the barcoded SpCas9 variants, a subpopulation of variants was found to be enriched by >1.5-fold in the sorted bin that encompasses ~5% of the cell population with the lowest level of RFP (i.e., Bin A) when compared to the unsorted population **(Fig. 2b; Fig. 8)**. WT SpCas9 is among one of those that were enriched for both reporter systems RFPsg5-ON and RFPsg8-ON, while eSpCas9(1.1) was enriched for RFPsg8-ON. To facilitate parallel characterization of the on- and off- target activities of SpCas9 variants, cell lines were further generated harboring synonymous mutations at *RFP,* such that targeting of the mismatched site indicates off-target activity of the SpCas9 variant (i.e., RFPsg5-OFF5-2 and RFPsg8-OFF5; **Fig. 2a**). WT SpCas9, but not eSpCas9(1.1), was enriched for both RFPsg5-OFF5-2 and RFPsg8-OFF5 **(Fig. 2b; Fig. 8)**.

The on- and off- target activities for the library of SpCas9 variants were ranked and plotted based on their enrichment in the sorted bin relative to the unsorted population, and found that a majority of the mutants impairs both the on- and off- target activities of SpCas9 **(Fig. 3a).** Activity-optimized variants were defined as those with enrichment ratios that were at least 90% of WT for both RFPsg5-ON and RFPsg8-ON, and less than 60% of WT for both RFPsg5-OFF5-2 and RFPsg8-OFF5. nOne variant (hereafter referred to as Opti-SpCas9) met these criteria and was selected for further characterization **(Table 2)**. Also identified is a variant with high fidelity, named OptiHF-SpCas9, based on the enrichment ratios of at least >50% of WT for both RFPsg5-ON and RFPsg8-ON, and <90% of WT for both RFPsg5-OFF5-2 and RFPsg8-OFF5 **(Table 2)**. The efficiency and specificity of Opti-SpCas9 and OptiHF-SpCas9 were verified by individual validation assays to measure their on- and off- target activity. Using multiple cell lines each expressing a gRNA that targets the matched or mismatched *RFP* site, it was confirmed that when compared to WT, Opti-SpCas9 exhibited comparable on-target activity (i.e., 94.6%; averaged from three matched sites) and substantially reduced off-target activity (i.e., 1.7%; averaged from three mismatched sites), while OptiHF-SpCas9 showed reduced activities at both on-target (i.e., 63.6%; averaged from two matched sites) and off-target (i.e., 2.0%; averaged from two mismatched sites) sites **(Fig. 3b)**.

**Studying the mutational epistasis for SpCas9's editing efficiency.** Systematic construction of protein variants by CombiSEAL allows us to classify sets of amino acid substitutions as neutral, beneficial or deleterious and explore their hard-to-predict epistatic interactions. Using the enrichment ratio as an index for editing activity of SpCas9 **(Fig. 9)**, heatmaps were constructed presenting on- and off-target activities conferred by the combinations of mutations and the epistatic interactions involved **(Fig. 4; Fig. 10)**. It was revealed that the number and type of substitutions introduced at SpCas9's amino acid residues predicted to interact with the target and non-target DNA strands (such as R661, Q695, K848, Q926, K1003, and K1060) govern the optimal balance between maximizing on-target efficiency and minimizing off-target activity. The activity-optimized variant Opti-SpCas9 differs from WT by two substitution mutations at these DNA-contacting residues (i.e., R661A and K1003H). A comparison among the three conservative basic residues (i.e., lysine, arginine, and histidine) introduced at the 1003rd amino acid position of SpCas9 revealed that K1003H is the preferred substitution that exhibited a positive epistatic interaction with the R661A mutation and conferred Opti-SpCas9 with high editing efficiency at the on-target sites **(Fig. 4)**. Addition of the Q926A substitution, which was shown to confer higher specificity for SpCas9-HF1[4], onto Opti-SpCas9 slightly decreased its off-target effect (i.e., from 1.0% for Opti-SpCas9 to 0.2% for Opti-SpCas9 + Q926A; averaged from three mismatched target sites), and considerably reduced its on-target activity by 21.6%, 62.4%, and 99.9% across three matched sites tested **(Fig. 3b)**. Moreover, it was discovered that most SpCas9 variants bearing three or more mutations at these DNA-contacting residues generated less edits at both on- and off- target sites **(Fig. 4)**. These results are consistent with previous findings that excessive alanine substitutions at these DNA-contacting residues severely reduced SpCas9's editing activity[25]. Interestingly though, with additional substitutions introduced at residues responsible for conformational control of SpCas9's HNH and RuvC nuclease domains[28] such as the E923M + T924V and E923H + T924L mutations located at the linker region connecting the two domains, some of the SpCas9 variants carrying three or more mutations at the DNA-contacting residues restored their on-target editing at the RFPsg5-ON site **(Fig. 4)**. The high-fidelity variant OptiHF-SpCas9 also contains E923M + T924V mutations in addition to Q695A, K848A, and Q926A substitutions, and it showed a slightly higher on-target activity at the RFPsg8-ON site than the variant with only Q695A, K848A, and Q926A triple mutations **(Fig. 4)**. These data support the model that SpCas9's DNA binding and cleavage activities are functionally coupled to determine its editing specificity and efficiency[5,29], and highlight the potential to program SpCas9's editing performance by modifying the linker residues.

**Characterizing the optimized SpCas9 variants.** In the gRNA design and construction, a 5'G is commonly included or added to the start of a gRNA sequence to facilitate efficient transcription under the U6 promoter. WT SpCas9 is compatible with gRNAs having an additional 5'G that is mismatched to the protospacer sequence. On the other hand, eSpCas9(1.1), SpCas9-HF1, HypaCas9, and evoCas9 lose their editing efficiency when using a 20-nucleotide gRNA bearing an additional 5'G (i.e., $G-N_{20}$) or lacking a starting guanine (i.e., $H-N_{19}$)[4,6,24-26,30]. The use of gRNAs with a 5'G matched to the protospacer sequence could dramatically reduce the number of editable sites in the human genome by ~4.3-fold based on the availability of $G-N_{19}$-NGG sites compared to $N_{20}$-NGG **(Fig. 11)**. The editing activities of Opti-SpCas9 were further characterized with gRNAs carrying an additional 5'G, and it was found that Opti-SpCas9 exhibited on-target DNA cleavage activity comparable (i.e., 95.1%) to WT based on assaying endogenous loci that we and others have previously studied[3-5,18,31], while eSpCas9(1.1) and HypaCas9 exhibited largely reduced activity (i.e., 32.4% and 25.6%, respectively) **(Fig. 5a; Fig. 12)**. The reduced editing was not due to decreased protein expression levels of the two SpCas9 variants **(Fig. 13)**. These results corroborate with the on-target activities observed for these variants in our screening systems in which gRNAs bearing an additional 5'G were used **(Fig. 2; 3a)**, as well as based on independent validation experiments using green fluorescent protein (GFP) disruption assays **(Fig. 3b; Fig. 14)**. In addition, Opti-SpCas9, eSpCas9(1.1), and HypaCas9 exhibited editing activity comparable (i.e., 109.1%, 103.3%, and 106.8%, respectively) to WT when 20-nucleotide gRNAs starting with a matched 5'G were used **(Fig. 5a)**. Opti-SpCas9 was further compared with OptiHF-SpCas9 and the more recently characterized high-fidelity variants - evoCas9[6] and Sniper-Cas9[32], and it was discovered that OptiHF-SpCas9, evoCas9, and Sniper-Cas9 generated less on-target edits than Opti-SpCas9 (i.e., reduced by 60.7%, 99.8%, and 51.7%, respectively, when expressed with gRNAs carrying an additional 5'G, and reduced by 40.1%, 87.7% and 63.9%, respectively, when using gRNAs starting with a matched 5'G at the 20-nucleotide gRNA sequence) **(Fig. 5b; Figs. 12; 13)**. Altogether, the restriction of harboring a matched 5'G as the first base of the 20-nucleotide gRNA sequence for transcription under U6, which limits the practical usefulness of other previously engineered SpCas9s with improved specificity, does not apply to Opti-SpCas9 that work compatibly with gRNAs carrying an additional 5'G. These findings highlight that engineered SpCas9s do not necessarily have to sacrifice targeting range for specificity.

The off-target activity of the different SpCas9 variants was further examined. Eight potential off-target loci that are edited by WT SpCas9 using the *VEGFA* site 3 and *DNMT1* site 4 gRNAs were amplified[3-5,31], and genomic indels induced by WT SpCas9 were detected at four of those sites (i.e., *VEGFA* OFF1, *VEGFA* OFF2, *VEGFA* OFF3, and *DAMT1* OFF1) in OVCAR8-ADR cells. When Opti-SpCas9, eSpCas9(1.1), and HypaCas9 were used instead of WT, off-target edits were detected only at the *VEGFA* OFF1 site **(Fig. 15)**. Among the four variants, Opti-SpCas9 showed the greatest on- to off-target activities at that site **(Fig. 15)**. To compare mismatch tolerance of different SpCas9 variants, gRNAs containing one- to four- base mismatches against the reporter gene target (i.e., a genomically integrated *GFP* gene sequence) were generated. These mismatched bases span across different positions of the gRNA's spacer sequence. The loss of GFP fluorescence was measured to reflect DNA cleavage and indel-mediated disruption of the target site. It was discovered that Opti-SpCas9 is largely intolerant to gRNAs with two or more mismatched bases, albeit a relatively low level of activity (i.e., 3.5% for Opti-SpCas9 versus 73.2% for WT) was detected in 1 of the 8 sites carrying two-base mismatches **(Fig. 16)**. It was observed that eSpCas9(1.1) and HypaCas9 exerted less edits at both the on-target site (i.e., reduced by >60%) and the off-target sites in our reporter systems **(Fig. 16)**. With similar level of on-target activity between WT and Opti-SpCas9 (i.e., 97.6% of WT), Opti-SpCas9 showed a higher specificity than WT, indicated by the generation of significantly less off-target edits at 13 of the 20 sites containing a single-base mismatch and yet there were still a considerable amount of off-target edits being detected **(Fig. 16)**. Others have also reported editing activity at single-base mismatched sites using eSpCas9(1.1), SpCas9-HF1, HypaCas9, evoCas9, and Sniper-Cas9[3,5,6,32]. Nevertheless, a majority of the *in silico* predicted off-target sites in the genome contains two or more mismatches against the gRNA sequence[33], and thus tolerance towards single-base mismatch should not limit SpCas9's utility to achieve accurate genome editing. GUIDE-Seq was further performed to look at genome-wide cleavage activities brought by Opti-SpCas9 and other engineered SpCas9 variants. These results indicate that Opti-SpCas9 generated substantially less off-target cleavage than WT, and OptiHF-SpCas9 showed increased on-to-off target ratios comparable to other reported high-fidelity variants such as eSpCas9(1.1), HypaCas9, evoCas9, and Sniper-Cas9 **(Fig 5c; Table 3)**. As compared to eSpCas9(1.1) and HypaCas9, Opti-SpCas9 exhibited better compatibility with the use of truncated gRNAs **(Fig. 17),** which could offer a complementary strategy to improve Opti-SpCas9's editing specificity[34].

DISCUSSIONS

The present inventors have established a simple yet extremely powerful platform, named CombiSEAL, to address the unmet need for rapid and simultaneous profiling of high-order combinatorial mutations for protein engineering. This strategy uses a pooled assembly approach to bypass the laborious steps for building individual combination mutants one-by-one, and exploits barcoding tactics to allow parallel experimentations on and identification of the top performers from a large number of protein variants to facilitate protein engineering. Furthermore, the method can be applied to map epistasis relationships between mutations. Using the CombiSEAL method, the inventors successfully identified Opti-SpCas9 and OptiHF-SpCas9 - novel variants with superior genome editing efficiency and specificity across a broad range of endogenous targets in human cells **(Table 3)**. The CombiSEAL pipeline can be readily applied to build even more Cas9 variants to broaden the search for variants with multifaceted or other properties, such as those having broader protospacer adjacent motif flexibilty[7] and enhanced compatibility with ribonucleoprotein delivery[35]. It is envisioned that CombiSEAL will accelerate the engineering of CRISPR enzymes (including SaCas9[36] and Cpf1[37]) and their derivatives (e.g., base editors[38-41]) for precise editing of the genome. The generalizability of this approach will also expand our scope to systematically engineer diverse proteins, as well as other biological molecules and systems including synthetic DNAs and genetic regulatory circuits, relevant to many biomedical and biotechnology applications.

METHODS

*Construction of DNA vectors*

The vectors used in this study **(Table 4)** were constructed using standard molecular cloning techniques, including PCR, restriction enzyme digestion, ligation, and Gibson assembly. Custom oligonucleotides were purchased from Integrated DNA Technologies and Genewiz. The vector constructs were transformed into *E. coli* strain DH5$\alpha$, and 50 $\mu$g/ml of carbenicillin/ampicillin was used to isolate colonies harboring the constructs. DNA was extracted and purified using Plasmid Mini (Takara) or Midi (Qiagen) kits. Sequences of the vector constructs were verified with Sanger sequencing.
To create the lentiviral expression vector encoding eSpCas9(1.1), HypaCas9, or SpCas9-HF1, together with Zeocin as the selection marker, the *SpCas9* sequences were amplified/mutated from pAWp30 (Addgene #73857), eSpCas9(1.1) (Addgene #71814), and VP12 (Addgene #72247) by PCR using Phusion DNA polymerase (New England Biolabs) and cloned into the pFUGW lentiviral vector backbone using Gibson Assembly Master Mix (New England Biolabs). Lentiviral expression vectors encoding evoCas9, Sniper-Cas9, and xCas9(3.7) were created by amplifying their SpCas9 sequences from Addgene constructs #107550, #113912, and #1803380, respectively, and cloning into the pFUGW vector backbone. To construct a storage vector containing U6 promoter-driven expression of a gRNA that targeted a specific gene, oligo pairs with the gRNA target sequences were synthesized, annealed, and cloned in the BbsI-digested pAWp28 vector (Addgene #73850) using T4 DNA ligase (New England Biolabs) as previously described[18]. In search of SpCas9 variants that work compatibly with gRNAs carrying an additional 5'G at the start of the 20-nucleotide spacer sequence to favor transcription under the U6 promoter, gRNAs containing an extra 5'G were used in this study, except for some of those used in **Fig. 5** and **Fig. 14**. The gRNA spacer sequences are listed in **Table 5.** To construct a lentiviral vector for U6-driven expression of gRNA, U6-gRNA expression cassettes were prepared from digestion of the storage vector with BglII and MfeI enzymes (ThermoFisher Scientific), and inserted into the pAWp12 (Addgene #72732) vector backbone using ligation via the compatible sticky ends generated by digestion of the vector with BamHI and EcoRI enzymes (ThermoFisher Scientific). To express the gRNAs together with the dual RFP and GFP fluorescent protein reporters, the U6-driven gRNA expression cassettes were inserted into the pAWp9 (Addgene #73851), instead of pAWp12, lentiviral vector backbone using the same strategy described above.

### Creation of barcoded DNA parts for SpCas9

Guided by the prior knowledge available when we started this study, the inventors focused on building a library of combination mutants at amino acid residues that were predicted to make contacts with the target and non-target DNA strands at the gRNA-directed genomic sites (including those identified in SpCas9-HF1[4] and eSpCas9(1.1)[3], respectively) or to control the conformational dynamics of SpCas9's HNH and RuvC nuclease domains for DNA cleavage[28]. Eight amino acid residues were selected and modified to harbor specified or randomly generated substitution mutations (Fig. 1a). The basic residues were mutated to alanine to evaluate the role of those charged residues. In additional to alanine substitution at K1003 that was previously introduced to eSpCas9(1.1), this residue was also mutated to other positively charged residues (i.e., arginine and histidine) to minimize its impact on protein stability. It was hypothesized that specific combinations of these mutations on SpCas9 could maximize its on-target editing efficiency and enhance compatibility with gRNAs, while minimizing the undesirable off-target activity.

The SpCas9 sequence was modularized into four parts (i.e., P1, P2, P3, and P4) for building combination mutants, and created four inserts for P1, two inserts for P2, seventeen inserts for P3, and seven inserts for P4. Each of the inserts was amplified and mutated from pAWp30 (Addgene #73857) or eSpCas9(1.1) (Addgene #71814) by PCR using Phusion (New England Biolabs) or Kapa HiFi (Kapa Biosystems) DNA polymerases. To generate site-directed mutations at amino acid positions 923, 924 and 926 of SpCas9, the three original codon sequences were replaced with the degenerate codon NNS in the PCR primer. An 8-base-pair barcode unique to each DNA insert was added after cloning into the storage vector (pAWp61 or pAWp62). Restriction enzyme sites BsaI were added to flank the ends (and BbsI sites and a primer-binding site for barcode sequencing were introduced in between the insert and the barcode for pAWp61 and pAWp62, respectively). Each pAWp61 and pAWp62 storage vector herein was thus configured as "BsaI-Insert-BbsI-BbsI-Barcode-BsaI" and "BsaI-Insert-Primer-binding site-Barcode-BsaI", respectively. Sanger sequencing was performed to confirm the sequence identity of individual inserts and their barcodes. In cases where the engineered sequence of interest contains BsaI or BbsI sites, other type IIS restriction enzyme sites could be used instead of BsaI and BbsI, or synonymous mutations could be introduced to the protein-coding sequence to remove the restriction sites while encoding the same amino acid residues.

### Creation of barcoded combination mutant library for SpCas9

Storage vectors harboring the inserts for each part of SpCas9 were mixed at equal molar ratio. Pooled inserts were generated by single-pot digestion reactions of the mixed storage vectors with BsaI. The destination vector (pAWp60) was digested with BbsI. The digested P1 inserts and vectors were ligated to create a pooled P1 library in destination vector. The P1 library was digested again with BbsI, and ligated with the digested P2 inserts to assemble the library with two-way combinations (P1 × P2). Sequential rounds of ligation reactions were performed to generate the three-way (P1 × P2 × P3) and four-way (P1 × P2 × P3 × P4) combination libraries. After the pooled assembly steps, the protein-coding parts of the inserts were seamlessly linked and localized to one end of the vector construct and their respective barcodes were concatenated at the other end. A four-way (4 × 2 × 17 × 7) combination library of 952 SpCas9 variants was built, each carrying one to eight mutations (except for WT) at amino acid residues that were predicted to interact with the target and non-target DNA strand of the gRNA-directed genomic site[3,4] or alter the conformational dynamics of SpCas9's nuclease domains[28] (Fig. 1a). The combinatorial complexity could be expanded by introducing additional barcoded parts and scaled up to simultaneously study tens of thousands or even more combinatorial modifications. Sanger sequencing analysis was performed, and a majority of the assembled barcoded combination mutant constructs was verified to carry the expected mutations in the two-way (i.e., 20/20 colonies), three-way (i.e., 14/15 colonies), and four-way (i.e., 8/8 colonies) libraries. Except for the one three-way combination mutant construct that carry an unintended base substitution, no other random mutation was detected in the other constructs. The final library was subcloned into pFUGW lentiviral vector to express the SpCas9 variants together with selection marker Zeocin under EFS promoter. Sanger sequencing of the full-length sequence of the barcoded SpCas9 variants assembled in the lentiviral vector (7 out of 7 colonies sampled from the library) confirmed that only expected mutations, and no random mutations, were present.

*Generation of SpCas9 variants for individual validation*

Lentiviral vectors encoding individual SpCas9 variants, including Opti-SpCas9, were constructed with the same strategy that was being used for the generation of combinatorial mutant library described above, except that the assembly was performed one-by-one with individual inserts and vectors.

*Human cell culture*

HEK293T cells were obtained from American Type Culture Collection (ATCC). OVCAR8-ADR cells were gifts from T. Ochiya (Japanese National Cancer Center Research Institute, Japan)[42]. The identity of the OVCAR8-ADR cells was confirmed by a cell line authentication test (Genetica DNA Laboratories). Monoclonal stable OVCAR8-ADR cell lines were generated by transducing cells with lentiviruses encoding *RFP* and *GFP* genes expressed from UBC and CMV promoters, respectively, and a tandem U6 promoter-driven expression cassette of gRNA targeting *RFP* site. RFPsg5-ON, RFPsg8-ON, and RFP-sg6-ON lines harbor target sites on *RFP* that match completely with the gRNA's spacer, while RFPsg5-OFF5-2, RFPsg8-OFF5, and RFPsg5-OFF5 lines harbor target sites on *RFP* carrying synonymous mutations and are mismatched to the gRNA's spacer **(Table 6)**. HEK293T cells were cultured in DMEM supplemented with 10% heat-inactivated FBS and $1 \times$ antibiotic-antimycotic (Life Technologies) at 37°C with 5% $CO_2$. OVCAR8-ADR cells were cultured in RPMI supplemented with 10% heat-inactivated FBS and $1 \times$ antibiotic-antimycotic (Life Technologies) at 37°C with 5% $CO_2$.

*Lentivirus production and transduction*

Lentiviruses were produced in 6-well plates with $2.5 \times 10^5$ HEK293T cells per well. Cells were transfected using FuGENE HD transfection reagents (Promega) with 0.5 $\mu$g of lentiviral vector, 1 $\mu$g of pCMV-dR8.2-dvpr vector, and 0.5 $\mu$g of pCMV-VSV-G vector mixed in 100 $\mu$l of OptiMEM medium (Life Technologies) for 15 minutes. The medium was replaced with fresh culture medium 1 day after transfection. Viral supernatants were then collected every 24 hours between 48 to 96 hours after transfection, pooled together and filtered through a 0.45 $\mu$m polyethersulfone membrane. For transduction with individual vector constructs, 500 $\mu$l filtered viral supernatant was used to infect $2.5 \times 10^5$ cells in the presence of 8 $\mu$g/ml polybrene (Sigma) overnight. For transduction with the pooled library into human cells (i.e., OVCAR8-ADR), lentivirus production was scaled up using the same experimental conditions. To ensure high-coverage library containing a sufficient representation for most combinations, infection was carried out with a starting cell population containing ~300-fold more cells than the library size to be tested. Lentiviruses were titrated to a multiplicity of infection of ~0.3 to give an infection efficiency of ~30% in the presence of 8 $\mu$g/ml polybrene, such that the SpCas9 variant library was delivered at low-copy numbers.

*Cell sorting*

Cell sorting was performed on a BD Influx cell sorter (BD Biosciences). Drop delay was determined using BD Accudrop beads. Cells were filtered through 70$\mu$m nylon mesh filters before sorting through a 100-$\mu$m nozzle using 1.0 Drop Pure sorting mode. Cells were gated for GFP-positive signals and sorted based on the fluorescence level of RFP into three bins (i.e., A, B, and C) such that approximately 5% cells of the population were collected into each bin encompassing cells with lower RFP level. The percentage of cells in the population to be sorted into each bin could be adjusted to balance the trade-off between the representation of individual combinations in the sorted population and the sensitivity of detecting enrichment of variants between bins. About 0.2 - 0.3 million cells were collected for each sorted bin in each sample.

*Sample preparation for barcode sequencing*

For the combination mutant vector library, plasmid DNA was extracted from *E. coli* transformed with the vector library using Plasmid Mini kit (Qiagen). For the human cell pools infected with the combination mutant library, genomic DNA of cells collected from various experimental conditions was extracted using DNeasy Blood & Tissue Kit (Qiagen). DNA concentrations were measured by Quant-iT PicoGreen dsDNA Assay Kit (Life Technologies). PCR amplification of 3 93-base-pair fragments, each containing a unique barcode representing an individual combination mutant, Illumina anchor sequences, and an 8-base-pair indexing barcode for multiplexed sequencing, was performed using Kapa HiFi Hotstart Ready-mix (Kapa Biosystems). The forward and reverse primers used were 5'-AATGATACGGCGACCACCGA-GATCTACACGGAACCGCAACGGTATTC-3' and 5'-CAAGCAGAAGACGGCATACGAGATNNNNNNNNGGTTGCGT-CAGCAAACACAG-3', where NNNNNNNN denotes a specific indexing barcode assigned for each experimental sample. To avoid bias in PCR that could skew the population distribution, PCR conditions were optimized to ensure the amplification occurred during the exponential phase. The PCR amplicons were purified with two rounds of size selection using a 1:0.5 and 1:0.95 ratio of Agencourt AMPure XP beads (Beckman Coulter Genomics) prior to real-time PCR quantification using Kapa SYBR Fast qPCR Master Mix (Kapa Biosystems) with a StepOnePlus Real Time PCR system (Applied Biosystems). Forward and reverse primers used for quantitative PCR were 5'-AATGATACGGCGACCACCGA-3' and 5'-CAAGCAGAAGACGGCATACGA-3' respectively. The quantified samples were then pooled at desired ratio for multiplexing, assessed using the high-sensitivity DNA chip (Agilent) on an Agilent 2100 Bioanalyzer, and run for Illumina HiSeq using primer (5'-CCACCGAGATCTACACGGAACCGCAACGGTATTC-3') and indexing barcode primer (5'-GTGGCGTGGTGTGCACTGTGTTTGCTGACGCAACC-3').

### Barcode sequencing data analysis

Barcode reads for each combination mutant were processed from sequencing data. Barcode reads representing each combination were normalized per million reads for each sample categorized by the indexing barcodes. Profiling was performed in two biological replicates. The frequency of each combination mutant between the sorted Bin A and the unsorted population was measured, and the enrichment ratio (E) between them relative to the rest of the population was calculated. Bin A was selected because enrichment of variants was most obvious in this bin **(Fig. 2b).** Equation used is as follow:

$$E = \frac{(N\,bin\,/\,N\,unsorted)}{(1 - N\,bin)\,/\,(1 - N\,unsorted)}$$

where $N_{bin}$ represents the frequency of the combination mutant in the sorted bin, and $N_{unsorted}$ represents the frequency of the combination mutant in the unsorted bin.

Log-transformed mean score determined from the replicates (i.e., $\log_2(E)$) comparing the sorted bin A against the unsorted population was used as a measure of target editing activity. Only barcodes that gave more than 300 absolute reads in the unsorted population were analyzed to improve data reliability. The correlation between $\log_2(E)$ score determined from the pooled screen and individual validation data **(Fig. 9)** could be improved by increasing the fold representation of cells per combination in the pooled screen to reduce the experimental noises[43]. Activity-optimized variants (i.e., Opti-SpCas9 identified in this study) were defined as those with $\log_2(E)$ (for Bin A versus unsorted population) that were at least >90% of WT for both RFPsg5-ON and RFPsg8-ON, and <60% of WT for both RFPsg5-OFF5-2 and RFPsg8-OFF5. OptiHF-SpCas9 was identified as a variant with high fidelity based on the enrichment ratios of at least >50% of WT for both RFPsg5-ON and RFPsg8-ON, and <90% of WT for both RFPsg5-OFF5-2 and RFPsg8-OFF5. The full list is presented in **Table 2.**

To determine epistasis, we applied a scoring system similar to ones previously described for protein fitness[44,45], and calculated epistasis ($\varepsilon$) scores for each combination in **Fig. 4.** The $\varepsilon$ scores were determined as: observed fitness - expected fitness, where the expected fitness for the combination [X,Y] is ($\log_2(E_{[X]}) + \log_2(E_{[Y]})$) according to the additive model. In general terms, combinations that exhibited better fitness than predicted were defined as positive epistasis, whereas combinations that were less fit than expected were defined as negative epistasis. The $\log_2(E)$ values for a lethal or nearly lethal combination mutant was set equal to a SpCas9 variant with 8 mutations (i.e., R661A + Q695A + K848A + E923M + T924V + Q926A + K1003A + R1060A) in this work for comparison, and our individual validation data confirmed its minimal activity in disrupting the target RFP sequences **(Fig. 3b).** The expected fitness was capped at the $\log_2(E)$ values for a lethal or nearly lethal combination mutant to minimize spurious epistasis values resulting from non-meaningful predicted fitness. In future work, it could be beneficial to include a nuclease-dead mutant of SpCas9 in the pooled screens as a lethal mutant for comparison.

### Fluorescent protein disruption assay

Fluorescent protein disruption assays were performed to evaluate DNA cleavage and indel-mediated disruption at the target site of the fluorescent protein (i.e., GFP or RFP) brought by SpCas9 and gRNA expressions, which results in loss of cell fluorescence. Cells harboring an integrated *GFP* or *RFP* reporter gene and together with SpCas9 and gRNA were washed and resuspended with $1 \times$ PBS supplemented with 2% heat-inactivated FBS, and assayed with a LSR Fortessa analyzer (Becton Dickinson). Cells were gated on forward and side scatter. At least $1 \times 10^4$ cells were recorded per sample in each data set.

### Immunoblot analysis

Cells were lysed in $2\times$ RIPA buffer supplemented with protease inhibitors (Gold Biotechnology #GB-108-2).

Lysates were collected by scrapping the culture plate on ice, and then centrifuged at 15,000 rpm for 15 minutes at 4°C. Supernatants were quantified using the Bradford assay (BioRad). Protein was denatured at 99°C for 5 minutes before gel electrophoresis on a 10% polyacrylamide gel (Bio-Rad). Proteins were transferred to polyvinylidene difluoride membranes at 110V for 2 hours at 4°C. Primary antibodies used were: anti-Cas9 (7A9-3A3) (1:2,000, Cell Signaling #14697), and anti-beta actin (1:10,000, Sigma #A2228). Secondary antibody used was HRP-linked anti-mouse IgG (1:20,000, Cell Signaling #7076). Membranes were developed by WesternBright ECL HRP substrate (Advansta #K-12045-D20).

*T7 Endonuclease I assay*

T7 endonuclease I assay was carried out to evaluate DNA mismatch cleavage at genomic loci targeted by the gRNAs. Genomic DNA was extracted from cell cultures using QuickExtract DNA extraction solution (Epicentre) or DNeasy Blood & Tissue Kit (Qiagen). Amplicons harboring the targeted loci were generated by PCR using primers and PCR conditions listed in **Table 7,** followed by purification using Agencourt AMPure XP beads (Beckman Coulter Genomics). About 400 ng of the PCR amplicons were denatured, self-annealed, and incubated with 4 units of T7 endonuclease I (New England Biolabs) at 37°C for ~40 minutes. The reaction products were resolved using on a 2% agarose gel electrophoresis. Quantification was based on relative band intensities measured using ImageJ. Indel percentage was estimated by the formula, $100 \times (1 - (1 - (b + c)/(a + b + c))^{1/2})$ as previously described[46], where $a$ is the integrated intensity of the uncleaved PCR product, and b and c are the integrated intensities of each cleavage product.

*GUIDE-Seq detection of genome-wide off-targets*

Genome-wide off-targets were accessed using the GUIDE-Seq method[47]. For each GUIDE-Seq sample, 1.5 million OVCAR8-ADR cells infected with SpCas9 variants and gRNAs were electroporated with 1,000 pmol freshly annealed GUIDE-seq end-protected dsODN using 100 μl Neon tips (ThermoFisher Scientific) according to the manufacturer's protocol. The dsODN oligo sequences used were:

5'-P-G*T*TTAATTGAGTTGTCATATGTTAATAACGGT*A*T-3' and
5'-P-A*T*ACCGTTATTAACATATGACAACTCAATTAA*A*C-3', where P represents a 5' phosphorylation and * indicates a phosphorothioate linkage. Genomic DNA was extracted using the DNeasy Blood and Tissue kit (Qiagen) 72 hours after electroporation. Genomic DNA concentration was quantified by Qubit fluorometer dsDNA HS assay (ThermoFisher Scientific), and 400 ng was used for library construction following the GUIDE-Seq protocol with minor modifications. Briefly, DNA was enzymatically fragmented by KAPA Frag Kit (KAPA Biosystems), followed by adaptor ligation and two rounds of hemi-nested PCR enrichment for dsODN integration sequences. To unify Illumina sequencing workflows for obtaining dual indexed data using Single-Indexed sequencing workflow across various Illumina platforms, the half-functional adaptors were redesigned with sample index (Index 2) placed at the head of Read 1, following unique molecular index **(Table 8).** Final sequencing libraries were quantified by KAPA Library Quantification Kits for Illumina and sequenced on Illumina NextSeq 500 System. Data de-multiplexing of Index 1 was performed by bcl2fq v2.19, followed by custom scripts for Index 2 demultiplexing and formatting for analysis using the GUIDE-Seq software[48].

All patents, patent applications, and other publications, including GenBank Accession Numbers or equivalent sequence identification numbers, cited in this application are incorporated by reference in the entirety of their contents for all purposes.

**Table 1**

| Engineered SpCas9 variant(s) | Publication | Methods for screening combination mutants | Screening host | Selection criteria of sites for mutagenesis | Construction of combination mutants with defined genotypes | Functional characterization of SpCas9 variants with defined genotypes |
|---|---|---|---|---|---|---|
| eSpCas9 (1.1) | Slaymaker et al., Science, 2016 | Site-directed mutagenesis | Human cells (U2OS) | Based on protein structure predictions, 31 positively charged residues within the non-target DNA strand groove were selected. | 65 (including variants with single mutation) | 65 (including variants with single mutation) |
| SpCas9-HF1 | Kleinstiver et al., Nature, 2016 | Site-directed mutagenesis | Human cells (HEK293T) | Based on protein structure predictions, 4 residues that form direct hydrogen bonds made to the phosphate backbone of the target DNA strand were selected. | 15 (including variants with single mutation) | 15 (including variants with single mutation) |
| HypaCas9 | Chen et al., Nature, 2017 | Site-directed mutagenesis | Human cells (U2OS) | Based on protein structure predictions, five clusters of residues containing conserved residues within 5 A of the RNA-DNA interface were selected for mutagenesis, and tested with or without Q926A mutation. | 36 (including variants with single mutation) | 36 (including variants with single mutation) |
| evoCas9 | Casini et al., Nature Biotechnology, 2018 | Random mutagenesis using error-prone PCR | Yeast | Random mutations introduced at the REC3 domain of SpCas9. | 62, positively selected by clonal isolation and genotyped by full-length sequencing | 37 (including variants with single mutation) |
| xCas9(3.7), xCas9(3.6) | Hu et al., Nature, 2018 | Random mutagenesis using phage-assisted continuous evolution | E.coli. | Random mutations introduced at full-length SpCas9. | 95, positively selected by clonal isolation and genotyped by full-length sequencing | 2 |
| Sniper-Cas9 | Lee et al., Nature Communications, 2018 | Random mutagenesis using error-prone PCR | E.coli. | Random mutations introduced at full-length SpCas9. | 100, positively selected by clonal isolation and genotyped by full-length sequencing | 19 (including variants with single mutation) |

| Engineered SpCas9 variant(s) | Publication | Methods for screening combination mutants | Screening host | Selection criteria of sites for mutagenesis | Construction of combination mutants with defined genotypes | Functional characterization of SpCas9 variants with defined genotypes |
|---|---|---|---|---|---|---|
| Opti-SpCas9, OptiHF-SpCas9 | The study | Site-directed mutagenesis | Human cells (OVCAR8-ADR) | Based on selected residues from Slaymaker et al., Science, 2016 and Kleinstiver et al., Nature, 2016, in addition to sites responsible for the conformational control of SpCas9's nuclease domains (Sternberg et al., Nature, 2015). | 948 (including variants with single mutation) | 948 (including variants with single mutation) |

## Table 2

This file contains enrichment scores determined for SpCas9 variants based on the pooled characterization.

| Cas9 variant # | Amino acid residue | | | | | | | | log2(E) sgRNA target | | | | Key |
| | 661 | 695 | 848 | 923 | 924 | 926 | 100 3 | 106 0 | RFPsg 5 ON | RFPsg 5 OFF5-2 | RFPsg 8 ON | RFPsg 8 OFF5 | |
|---|---|---|---|---|---|---|---|---|---|---|---|---|---|
| 1 | R | Q | K | E | T | Q | K | R | 0.60 | 1.09 | 2.18 | 1.71 | WT |
| 2 | R | A | K | E | T | Q | K | R | 0.95 | -0.73 | 0.93 | 0.47 | |
| 3 | A | Q | K | E | T | Q | K | R | 1.12 | 1.01 | 1.07 | NA | |
| 4 | A | A | K | E | T | Q | K | R | 0.76 | 0.00 | 1.18 | 0.07 | |
| 5 | R | Q | A | E | T | Q | K | R | 1.06 | -0.21 | 1.03 | 0.34 | |
| 6 | R | A | A | E | T | Q | K | R | -0.17 | 0.14 | 0.19 | 0.39 | |
| 7 | A | Q | A | E | T | Q | K | R | 0.88 | 0.42 | 0.30 | 0.44 | |
| 8 | A | A | A | E | T | Q | K | R | -0.81 | -0.07 | -0.44 | 0.66 | |
| 9 | R | Q | K | E | T | A | K | R | 1.10 | -0.03 | 0.36 | 1.20 | |
| 10 | R | A | K | E | T | A | K | R | 0.87 | -0.20 | -0.27 | 0.62 | |
| 11 | A | Q | K | E | T | A | K | R | 1.06 | -0.23 | 1.15 | 0.13 | |
| 12 | A | A | K | E | T | A | K | R | 0.24 | -0.51 | 0.24 | 0.29 | |
| 13 | R | Q | A | E | T | A | K | R | -0.32 | -0.34 | -0.26 | 0.69 | |
| 14 | R | A | A | E | T | A | K | R | 0.49 | -0.72 | 0.53 | 0.40 | |
| 15 | A | Q | A | E | T | A | K | R | -0.82 | 0.53 | 0.03 | 0.40 | |
| 16 | A | A | A | E | T | A | K | R | -0.18 | -0.83 | -0.31 | -0.10 | |
| 17 | R | Q | K | Q | G | P | K | R | -0.90 | 0.02 | -0.63 | -0.52 | |
| 18 | R | A | K | Q | G | P | K | R | -0.11 | -0.14 | -0.54 | 0.21 | |
| 19 | A | Q | K | Q | G | P | K | R | 0.11 | -0.69 | -0.79 | -0.20 | |
| 20 | A | A | K | Q | G | P | K | R | 0.44 | 0.11 | -0.88 | -0.67 | |
| 21 | R | Q | A | Q | G | P | K | R | 0.44 | -0.38 | -0.37 | -0.82 | |
| 22 | R | A | A | Q | G | P | K | R | -0.64 | -0.39 | -1.25 | 0.04 | |
| 23 | A | Q | A | Q | G | P | K | R | NA | -0.90 | 0.47 | 0.82 | |
| 24 | A | A | A | Q | G | P | K | R | 0.12 | 0.09 | 0.31 | 0.41 | |
| 25 | R | Q | K | V | R | E | K | R | NA | -2.41 | NA | NA | |
| 26 | R | A | K | V | R | E | K | R | NA | 1.07 | -7.21 | NA | |
| 27 | A | Q | K | V | R | E | K | R | NA | NA | NA | NA | |
| 28 | A | A | K | V | R | E | K | R | NA | NA | NA | NA | |
| 29 | R | Q | A | V | R | E | K | R | -0.32 | NA | NA | 0.15 | |
| 30 | R | A | A | V | R | E | K | R | NA | -0.40 | NA | NA | |

EP 4 253 549 A2

This file contains enrichment scores determined for SpCas9 variants based on the pooled characterization.

| Cas9 variant # | Amino acid residue | | | | | | | | log2(E) | | | | Key |
| --- | --- | --- | --- | --- | --- | --- | --- | --- | --- | --- | --- | --- | --- |
| | 661 | 695 | 848 | 923 | 924 | 926 | 100 3 | 106 0 | sgRNA target | | | | |
| | | | | | | | | | RFPsg 5 ON | RFPsg 5 OFF5-2 | RFPsg 8 ON | RFPsg 8 OFF5 | |
| 31 | A | Q | A | V | R | E | K | R | NA | NA | NA | NA | |
| 32 | A | A | A | V | R | E | K | R | NA | NA | -1.30 | NA | |
| 33 | R | Q | K | A | W | E | K | R | NA | -2.52 | NA | -0.98 | |
| 34 | R | A | K | A | W | E | K | R | NA | NA | NA | NA | |
| 35 | A | Q | K | A | W | E | K | R | NA | NA | NA | NA | |
| 36 | A | A | K | A | W | E | K | R | NA | NA | NA | NA | |
| 37 | R | Q | A | A | W | E | K | R | -0.93 | -0.10 | NA | NA | |
| 38 | R | A | A | A | W | E | K | R | NA | 0.60 | NA | NA | |
| 39 | A | Q | A | A | W | E | K | R | NA | NA | NA | NA | |
| 40 | A | A | A | A | W | E | K | R | NA | -0.79 | NA | NA | |
| 41 | R | Q | K | M | V | A | K | R | 1.23 | 1.29 | 0.81 | 1.69 | |
| 42 | R | A | K | M | V | A | K | R | 0.95 | 0.49 | 0.64 | 0.15 | |
| 43 | A | Q | K | M | V | A | K | R | 1.41 | 1.30 | 0.98 | 0.74 | |
| 44 | A | A | K | M | V | A | K | R | 0.94 | -0.17 | 0.50 | 0.82 | |
| 45 | R | Q | A | M | V | A | K | R | 2.16 | -0.06 | 0.98 | 0.90 | OptiHF-SpCas9 |
| 46 | R | A | A | M | V | A | K | R | 0.50 | 0.06 | 1.16 | 0.05 | |
| 47 | A | Q | A | M | V | A | K | R | 1.05 | -0.51 | 0.82 | 0.79 | |
| 48 | A | A | A | M | V | A | K | R | NA | -0.99 | 0.41 | 1.22 | |
| 49 | R | Q | K | K | S | A | K | R | -0.21 | 0.33 | -0.84 | -0.51 | |
| 50 | R | A | K | K | S | A | K | R | -0.96 | -0.19 | -0.34 | 1.82 | |
| 51 | A | Q | K | K | S | A | K | R | 0.23 | 0.08 | -0.62 | -1.49 | |
| 52 | A | A | K | K | S | A | K | R | -0.39 | -0.71 | -0.48 | -0.45 | |
| 53 | R | Q | A | K | S | A | K | R | 0.21 | -0.58 | -0.25 | -0.30 | |
| 54 | R | A | A | K | S | A | K | R | -0.79 | 0.51 | -0.38 | -0.54 | |
| 55 | A | Q | A | K | S | A | K | R | 0.34 | -0.84 | 0.67 | 0.90 | |
| 56 | A | A | A | K | S | A | K | R | -0.79 | -0.55 | -0.25 | -0.38 | |
| 57 | R | Q | K | R | K | Q | K | R | -0.08 | -0.21 | -0.69 | -0.76 | |
| 58 | R | A | K | R | K | Q | K | R | -0.31 | -0.52 | -0.54 | -0.82 | |
| 59 | A | Q | K | R | K | Q | K | R | -1.33 | -0.34 | -0.39 | -0.08 | |
| 60 | A | A | K | R | K | Q | K | R | -0.96 | -0.67 | -0.35 | -0.10 | |

EP 4 253 549 A2

EP 4 253 549 A2

(continued)

This file contains enrichment scores determined for SpCas9 variants based on the pooled characterization.

| | | | | | | | | | log2(E) | | | | |
|---|---|---|---|---|---|---|---|---|---|---|---|---|---|
| | Amino acid residue | | | | | | | | sgRNA target | | | | Key |
| Cas9 variant # | 661 | 695 | 848 | 923 | 924 | 926 | 100 3 | 106 0 | RFPsg 5 ON | RFPsg 5 OFF5-2 | RFPsg 8 ON | RFPsg 8 OFF5 | |
| 61 | R | Q | A | R | K | Q | K | R | -0.60 | 0.16 | -0.93 | 0.65 | |
| 62 | R | A | A | R | K | Q | K | R | -1.16 | 0.71 | -0.32 | -0.15 | |
| 63 | A | Q | A | R | K | Q | K | R | NA | -0.04 | NA | -0.05 | |
| 64 | A | A | A | R | K | Q | K | R | -0.88 | -0.20 | -0.61 | 0.24 | |
| 65 | R | Q | K | C | R | E | K | R | -0.23 | -0.28 | 0.35 | 0.22 | |
| 66 | R | A | K | C | R | E | K | R | -0.70 | -0.13 | -0.79 | 0.85 | |
| 67 | A | Q | K | C | R | E | K | R | -0.13 | -0.41 | 0.15 | 0.52 | |
| 68 | A | A | K | C | R | E | K | R | -0.88 | 0.79 | -0.20 | 0.25 | |
| 69 | R | Q | A | C | R | E | K | R | -1.07 | 0.03 | -0.22 | 0.42 | |
| 70 | R | A | A | C | R | E | K | R | -0.01 | 0.16 | -0.30 | 0.06 | |
| 71 | A | Q | A | C | R | E | K | R | 0.14 | 0.53 | -0.81 | -0.12 | |
| 72 | A | A | A | C | R | E | K | R | 1.33 | 0.56 | -0.35 | 0.65 | |
| 73 | R | Q | K | Q | W | Q | K | R | 0.08 | -0.16 | -0.35 | 1.27 | |
| 74 | R | A | K | Q | W | Q | K | R | -0.33 | 0.68 | -0.16 | 0.57 | |
| 75 | A | Q | K | Q | W | Q | K | R | 0.12 | -0.12 | NA | 0.43 | |
| 76 | A | A | K | Q | W | Q | K | R | -0.27 | -0.09 | -0.32 | 0.31 | |
| 77 | R | Q | A | Q | W | Q | K | R | 0.11 | -0.26 | -0.02 | -0.04 | |
| 78 | R | A | A | Q | W | Q | K | R | -0.50 | -0.44 | -0.52 | 0.49 | |
| 79 | A | Q | A | Q | W | Q | K | R | 0.35 | 0.49 | -1.75 | -1.40 | |
| 80 | A | A | A | Q | W | Q | K | R | -0.02 | 0.33 | 0.94 | -0.91 | |
| 81 | R | Q | K | L | G | A | K | R | NA | 0.42 | -0.51 | -0.46 | |
| 82 | R | A | K | L | G | A | K | R | NA | NA | NA | NA | |
| 83 | A | Q | K | L | G | A | K | R | NA | -0.41 | 0.00 | 0.33 | |
| 84 | A | A | K | L | G | A | K | R | NA | -0.37 | NA | 0.03 | |
| 85 | R | Q | A | L | G | A | K | R | -0.32 | 0.25 | -1.52 | -0.68 | |
| 86 | R | A | A | L | G | A | K | R | -1.71 | -0.25 | NA | NA | |
| 87 | A | Q | A | L | G | A | K | R | -1.21 | -0.22 | -0.78 | NA | |
| 88 | A | A | A | L | G | A | K | R | 0.37 | 0.05 | NA | 0.53 | |
| 89 | R | Q | K | W | D | E | K | R | 0.03 | -0.40 | 0.23 | 0.36 | |
| 90 | R | A | K | W | D | E | K | R | -0.35 | -0.99 | -0.07 | -0.14 | |

27

This file contains enrichment scores determined for SpCas9 variants based on the pooled characterization.

| Cas9 variant # | Amino acid residue | | | | | | | | log2(E) | | | | Key |
|---|---|---|---|---|---|---|---|---|---|---|---|---|---|
| | | | | | | | | | sgRNA target | | | | |
| | 661 | 695 | 848 | 923 | 924 | 926 | 100 3 | 106 0 | RFPsg 5 ON | RFPsg 5 OFF5-2 | RFPsg 8 ON | RFPsg 8 OFF5 | |
| 91 | A | Q | K | W | D | E | K | R | -0.11 | 0.18 | -0.97 | 0.29 | |
| 92 | A | A | K | W | D | E | K | R | -0.17 | -0.07 | -0.54 | 0.12 | |
| 93 | R | Q | A | W | D | E | K | R | -0.63 | -0.92 | -0.40 | 0.49 | |
| 94 | R | A | A | W | D | E | K | R | -0.72 | -0.50 | -1.21 | -0.55 | |
| 95 | A | Q | A | W | D | E | K | R | -0.21 | -0.40 | -0.21 | -0.24 | |
| 96 | A | A | A | W | D | E | K | R | -0.79 | -0.40 | 0.47 | 0.02 | |
| 97 | R | Q | K | H | L | Q | K | R | 0.85 | 1.80 | 0.48 | 0.89 | |
| 98 | R | A | K | H | L | Q | K | R | 0.85 | 1.05 | 0.30 | 0.52 | |
| 99 | A | Q | K | H | L | Q | K | R | 1.27 | 1.13 | 1.25 | 0.82 | |
| 100 | A | A | K | H | L | Q | K | R | 1.61 | 0.48 | 0.76 | -0.11 | |
| 101 | R | Q | A | H | L | Q | K | R | 1.50 | 0.50 | 1.48 | 1.43 | |
| 102 | R | A | A | H | L | Q | K | R | 0.56 | 0.00 | 1.10 | 1.05 | |
| 103 | A | Q | A | H | L | Q | K | R | 1.79 | 0.27 | 1.00 | 0.49 | |
| 104 | A | A | A | H | L | Q | K | R | 0.89 | 0.05 | 0.81 | 0.30 | |
| 105 | R | Q | K | V | W | A | K | R | NA | -0.55 | NA | NA | |
| 106 | R | A | K | V | W | A | K | R | -1.89 | 0.18 | NA | 0.50 | |
| 107 | A | Q | K | V | W | A | K | R | 0.50 | 0.09 | -0.56 | 0.25 | |
| 108 | A | A | K | V | W | A | K | R | NA | -0.28 | -0.38 | NA | |
| 109 | R | Q | A | V | W | A | K | R | -1.35 | -0.19 | NA | NA | |
| 110 | R | A | A | V | W | A | K | R | NA | 1.22 | NA | NA | |
| 111 | A | Q | A | V | W | A | K | R | -1.60 | 0.92 | NA | NA | |
| 112 | A | A | A | V | W | A | K | R | NA | NA | 0.62 | NA | |
| 113 | R | Q | K | R | R | A | K | R | -0.92 | -0.30 | 0.13 | 0.85 | |
| 114 | R | A | K | R | R | A | K | R | -0.69 | -0.50 | 0.07 | 0.19 | |
| 115 | A | Q | K | R | R | A | K | R | -0.11 | -0.41 | -0.19 | NA | |
| 116 | A | A | K | R | R | A | K | R | 0.15 | -0.90 | 1.07 | 0.56 | |
| 117 | R | Q | A | R | R | A | K | R | -0.03 | -0.81 | 0.27 | 0.84 | |
| 118 | R | A | A | R | R | A | K | R | 0.03 | -0.24 | -1.01 | -0.27 | |
| 119 | A | Q | A | R | R | A | K | R | -0.91 | 0.37 | -0.24 | 0.41 | |
| 120 | A | A | A | R | R | A | K | R | -1.21 | -0.12 | -1.05 | -0.17 | |

EP 4 253 549 A2

This file contains enrichment scores determined for SpCas9 variants based on the pooled characterization.

| | | | | | | | | | log2(E) | | | | |
|---|---|---|---|---|---|---|---|---|---|---|---|---|---|
| | Amino acid residue | | | | | | | | sgRNA target | | | | |
| Cas9 variant # | 661 | 695 | 848 | 923 | 924 | 926 | 100 3 | 106 0 | RFPsg 5 ON | RFPsg 5 OFF5-2 | RFPsg 8 ON | RFPsg 8 OFF5 | Key |
| 121 | R | Q | K | G | D | E | K | R | -1.26 | 0.55 | NA | 0.07 | |
| 122 | R | A | K | G | D | E | K | R | NA | 0.29 | NA | NA | |
| 123 | A | Q | K | G | D | E | K | R | -1.62 | -0.40 | -2.17 | -0.37 | |
| 124 | A | A | K | G | D | E | K | R | 0.26 | 0.29 | NA | NA | |
| 125 | R | Q | A | G | D | E | K | R | -1.04 | -0.68 | 0.18 | 1.31 | |
| 126 | R | A | A | G | D | E | K | R | NA | 0.23 | NA | NA | |
| 127 | A | Q | A | G | D | E | K | R | -0.25 | 0.71 | -0.87 | NA | |
| 128 | A | A | A | G | D | E | K | R | 0.56 | -0.32 | -0.17 | 0.62 | |
| 129 | R | Q | K | M | R | A | K | R | 0.47 | -0.09 | -0.28 | 0.84 | |
| 130 | R | A | K | M | R | A | K | R | 0.26 | -0.33 | -0.10 | 0.25 | |
| 131 | A | Q | K | M | R | A | K | R | NA | -0.25 | -1.37 | 0.41 | |
| 132 | A | A | K | M | R | A | K | R | -0.13 | -0.11 | -0.13 | 0.12 | |
| 133 | R | Q | A | M | R | A | K | R | 0.29 | 0.31 | -0.43 | 0.56 | |
| 134 | R | A | A | M | R | A | K | R | 0.16 | -0.10 | -1.15 | -0.43 | |
| 135 | A | Q | A | M | R | A | K | R | NA | -0.18 | -0.77 | 0.72 | |
| 136 | A | A | A | M | R | A | K | R | NA | -0.72 | -0.68 | 0.46 | |
| 137 | R | Q | K | E | T | Q | K | A | 1.29 | 0.46 | 1.09 | 0.88 | |
| 138 | R | A | K | E | T | Q | K | A | 1.37 | 0.06 | 0.87 | 0.62 | |
| 139 | A | Q | K | E | T | Q | K | A | 0.51 | 0.03 | 1.41 | 0.14 | |
| 140 | A | A | K | E | T | Q | K | A | 0.11 | -0.17 | -0.11 | -0.20 | |
| 141 | R | Q | A | E | T | Q | K | A | 0.52 | 0.16 | 1.20 | 0.91 | |
| 142 | R | A | A | E | T | Q | K | A | 0.10 | -0.52 | 0.33 | 0.50 | |
| 143 | A | Q | A | E | T | Q | K | A | 0.17 | -0.27 | 0.46 | 0.70 | |
| 144 | A | A | A | E | T | Q | K | A | -0.16 | -0.42 | -0.98 | 0.03 | |
| 145 | R | Q | K | E | T | A | K | A | 0.54 | -0.24 | 0.67 | 0.90 | |
| 146 | R | A | K | E | T | A | K | A | -0.48 | -0.48 | 0.34 | -0.06 | |
| 147 | A | Q | K | E | T | A | K | A | 0.48 | -0.56 | 0.68 | NA | |
| 148 | A | A | K | E | T | A | K | A | -0.08 | -0.01 | 0.98 | 0.30 | |
| 149 | R | Q | A | E | T | A | K | A | -0.05 | -0.09 | 0.41 | 0.24 | |
| 150 | R | A | A | E | T | A | K | A | -1.50 | 0.09 | -0.20 | 0.36 | |

This file contains enrichment scores determined for SpCas9 variants based on the pooled characterization.

| Cas9 variant # | Amino acid residue | | | | | | | | log2(E) | | | | Key |
| | 661 | 695 | 848 | 923 | 924 | 926 | 100 3 | 106 0 | sgRNA target | | | | |
| | | | | | | | | | RFPsg 5 ON | RFPsg 5 OFF5-2 | RFPsg 8 ON | RFPsg 8 OFF5 | |
| 151 | A | Q | A | E | T | A | K | A | -1.01 | -0.49 | -0.48 | -0.25 | |
| 152 | A | A | A | E | T | A | K | A | -0.06 | -0.04 | -0.86 | -0.15 | |
| 153 | R | Q | K | Q | G | P | K | A | -0.59 | -0.23 | -0.46 | -0.12 | |
| 154 | R | A | K | Q | G | P | K | A | 0.60 | -0.58 | -0.14 | -0.53 | |
| 155 | A | Q | K | Q | G | P | K | A | -0.11 | -0.59 | -1.21 | -0.82 | |
| 156 | A | A | K | Q | G | P | K | A | NA | -0.48 | -0.89 | 0.50 | |
| 157 | R | Q | A | Q | G | P | K | A | -0.72 | -0.10 | -0.42 | 0.10 | |
| 158 | R | A | A | Q | G | P | K | A | -0.23 | -0.34 | -0.74 | -0.64 | |
| 159 | A | Q | A | Q | G | P | K | A | 0.49 | -0.84 | -0.23 | 0.97 | |
| 160 | A | A | A | Q | G | P | K | A | -0.66 | -0.35 | -0.04 | 0.64 | |
| 161 | R | Q | K | V | R | E | K | A | NA | NA | NA | -0.13 | |
| 162 | R | A | K | V | R | E | K | A | NA | NA | NA | NA | |
| 163 | A | Q | K | V | R | E | K | A | NA | NA | NA | -0.21 | |
| 164 | A | A | K | V | R | E | K | A | NA | NA | NA | NA | |
| 165 | R | Q | A | V | R | E | K | A | NA | 1.10 | NA | NA | |
| 166 | R | A | A | V | R | E | K | A | NA | NA | NA | NA | |
| 167 | A | Q | A | V | R | E | K | A | NA | -0.63 | NA | NA | |
| 168 | A | A | A | V | R | E | K | A | NA | NA | NA | NA | |
| 169 | R | Q | K | A | W | E | K | A | NA | NA | NA | NA | |
| 170 | R | A | K | A | W | E | K | A | NA | NA | NA | NA | |
| 171 | A | Q | K | A | W | E | K | A | NA | NA | NA | NA | |
| 172 | A | A | K | A | W | E | K | A | NA | NA | NA | NA | |
| 173 | R | Q | A | A | W | E | K | A | NA | -1.80 | -0.96 | -1.21 | |
| 174 | R | A | A | A | W | E | K | A | -1.35 | -0.13 | -0.16 | NA | |
| 175 | A | Q | A | A | W | E | K | A | NA | -1.36 | NA | -0.58 | |
| 176 | A | A | A | A | W | E | K | A | NA | NA | NA | NA | |
| 177 | R | Q | K | M | V | A | K | A | NA | 1.38 | 1.68 | 0.75 | |
| 178 | R | A | K | M | V | A | K | A | 1.02 | -0.31 | 0.81 | 0.96 | |
| 179 | A | Q | K | M | V | A | K | A | 1.21 | -0.06 | 0.49 | 0.12 | |
| 180 | A | A | K | M | V | A | K | A | 0.46 | -0.42 | 0.96 | -0.64 | |

EP 4 253 549 A2

(continued)

This file contains enrichment scores determined for SpCas9 variants based on the pooled characterization.

| Cas9 variant # | 661 | 695 | 848 | 923 | 924 | 926 | 100 3 | 106 0 | RFPsg 5 ON | RFPsg 5 OFF5-2 | RFPsg 8 ON | RFPsg 8 OFF5 | Key |
|---|---|---|---|---|---|---|---|---|---|---|---|---|---|
| | | | Amino acid residue | | | | | | log2(E) sgRNA target | | | | |
| 181 | R | Q | A | M | V | A | K | A | 1.47 | -0.30 | 0.58 | -0.63 | |
| 182 | R | A | A | M | V | A | K | A | 0.37 | -0.27 | 0.87 | 0.99 | |
| 183 | A | Q | A | M | V | A | K | A | 1.19 | 0.11 | 0.61 | -0.35 | |
| 184 | A | A | A | M | V | A | K | A | 0.55 | -0.11 | NA | 0.48 | |
| 185 | R | Q | K | K | S | A | K | A | -0.76 | -0.32 | -0.41 | -0.16 | |
| 186 | R | A | K | K | S | A | K | A | -1.08 | 0.50 | -0.92 | -0.92 | |
| 187 | A | Q | K | K | S | A | K | A | -0.28 | 0.19 | -0.71 | -0.13 | |
| 188 | A | A | K | K | S | A | K | A | -0.33 | 0.36 | -0.54 | 0.43 | |
| 189 | R | Q | A | K | S | A | K | A | -0.47 | -0.66 | -1.54 | -0.28 | |
| 190 | R | A | A | K | S | A | K | A | -0.39 | -0.13 | -1.51 | -0.59 | |
| 191 | A | Q | A | K | S | A | K | A | -0.76 | -0.74 | -0.29 | 0.45 | |
| 192 | A | A | A | K | S | A | K | A | NA | -0.03 | NA | -0.71 | |
| 193 | R | Q | K | R | K | Q | K | A | -0.15 | -0.44 | 0.12 | 0.07 | |
| 194 | R | A | K | R | K | Q | K | A | -1.14 | -0.81 | -0.08 | -2.01 | |
| 195 | A | Q | K | R | K | Q | K | A | -0.75 | -0.18 | -0.19 | 0.22 | |
| 196 | A | A | K | R | K | Q | K | A | -1.45 | -0.78 | -0.91 | 0.13 | |
| 197 | R | Q | A | R | K | Q | K | A | -0.03 | -0.15 | -0.13 | -0.18 | |
| 198 | R | A | A | R | K | Q | K | A | -0.39 | -0.79 | -0.93 | -0.81 | |
| 199 | A | Q | A | R | K | Q | K | A | 0.18 | -0.17 | NA | -0.17 | |
| 200 | A | A | A | R | K | Q | K | A | NA | -0.62 | -1.52 | -0.70 | |
| 201 | R | Q | K | C | R | E | K | A | -0.61 | 0.37 | -0.02 | -0.75 | |
| 202 | R | A | K | C | R | E | K | A | -0.27 | -0.17 | 0.63 | 1.17 | |
| 203 | A | Q | K | C | R | E | K | A | -0.03 | 0.38 | 0.63 | -0.71 | |
| 204 | A | A | K | C | R | E | K | A | -0.43 | -0.17 | 0.08 | 0.21 | |
| 205 | R | Q | A | C | R | E | K | A | -0.25 | -0.21 | -0.14 | 0.34 | |
| 206 | R | A | A | C | R | E | K | A | -0.62 | -0.07 | NA | -0.35 | |
| 207 | A | Q | A | C | R | E | K | A | 0.13 | 0.58 | -2.47 | NA | |
| 208 | A | A | A | C | R | E | K | A | -0.76 | -0.39 | -0.82 | 0.45 | |
| 209 | R | Q | K | Q | W | Q | K | A | -0.13 | 0.36 | 0.42 | 0.85 | |
| 210 | R | A | K | Q | W | Q | K | A | -0.81 | -0.10 | -0.19 | 0.07 | |

EP 4 253 549 A2

This file contains enrichment scores determined for SpCas9 variants based on the pooled characterization.

| | Amino acid residue | | | | | | | | log2(E) | | | | Key |
| --- | --- | --- | --- | --- | --- | --- | --- | --- | --- | --- | --- | --- | --- |
| | | | | | | | | | sgRNA target | | | | |
| Cas9 variant # | 661 | 695 | 848 | 923 | 924 | 926 | 100 3 | 106 0 | RFPsg 5 ON | RFPsg 5 OFF5-2 | RFPsg 8 ON | RFPsg 8 OFF5 | |
| 211 | A | Q | K | Q | W | Q | K | A | -0.64 | -0.10 | 0.76 | 0.51 | |
| 212 | A | A | K | Q | W | Q | K | A | 0.04 | -0.64 | -0.18 | 0.39 | |
| 213 | R | Q | A | Q | W | Q | K | A | 0.08 | -0.25 | -0.29 | 0.43 | |
| 214 | R | A | A | Q | W | Q | K | A | -0.04 | 0.04 | 0.08 | -0.08 | |
| 215 | A | Q | A | Q | W | Q | K | A | -1.58 | -0.29 | 0.12 | 0.25 | |
| 216 | A | A | A | Q | W | Q | K | A | -0.44 | 0.09 | -0.33 | 0.93 | |
| 217 | R | Q | K | L | G | A | K | A | NA | 0.20 | 0.06 | 0.83 | |
| 218 | R | A | K | L | G | A | K | A | 0.25 | -0.88 | -0.88 | 0.47 | |
| 219 | A | Q | K | L | G | A | K | A | -0.35 | 0.15 | -0.93 | -0.70 | |
| 220 | A | A | K | L | G | A | K | A | -0.02 | -0.85 | -1.15 | NA | |
| 221 | R | Q | A | L | G | A | K | A | NA | NA | NA | 0.38 | |
| 222 | R | A | A | L | G | A | K | A | NA | -0.18 | -0.20 | 1.25 | |
| 223 | A | Q | A | L | G | A | K | A | -0.33 | -1.06 | 0.07 | NA | |
| 224 | A | A | A | L | G | A | K | A | NA | -0.28 | -0.85 | NA | |
| 225 | R | Q | K | W | D | E | K | A | -0.04 | -1.09 | -0.35 | -0.85 | |
| 226 | R | A | K | W | D | E | K | A | -0.64 | -0.99 | 0.04 | -1.16 | |
| 227 | A | Q | K | W | D | E | K | A | 0.34 | -0.17 | -1.63 | -0.34 | |
| 228 | A | A | K | W | D | E | K | A | -0.29 | -0.23 | 0.07 | -0.51 | |
| 229 | R | Q | A | W | D | E | K | A | -0.63 | -0.24 | -0.09 | 0.06 | |
| 230 | R | A | A | W | D | E | K | A | -0.81 | -0.53 | -0.84 | -0.56 | |
| 231 | A | Q | A | W | D | E | K | A | -1.16 | -0.54 | -0.06 | 0.36 | |
| 232 | A | A | A | W | D | E | K | A | -0.66 | -0.75 | NA | 0.19 | |
| 233 | R | Q | K | H | L | Q | K | A | 1.13 | 1.00 | 1.56 | 1.14 | |
| 234 | R | A | K | H | L | Q | K | A | 1.60 | 0.10 | 1.25 | 0.76 | |
| 235 | A | Q | K | H | L | Q | K | A | 1.02 | 0.51 | -0.04 | 0.48 | |
| 236 | A | A | K | H | L | Q | K | A | 0.79 | 0.36 | 0.66 | -0.73 | |
| 237 | R | Q | A | H | L | Q | K | A | 0.80 | -0.51 | 0.19 | -0.45 | |
| 238 | R | A | A | H | L | Q | K | A | 0.69 | -0.55 | 0.06 | 0.20 | |
| 239 | A | Q | A | H | L | Q | K | A | 1.57 | 0.39 | 0.39 | 0.32 | |
| 240 | A | A | A | H | L | Q | K | A | 1.50 | 0.21 | 0.43 | -0.13 | |

EP 4 253 549 A2

(continued)

This file contains enrichment scores determined for SpCas9 variants based on the pooled characterization.

| | Amino acid residue | | | | | | | | log2(E) | | | | Key |
| | | | | | | | | | sgRNA target | | | | |
| Cas9 variant # | 661 | 695 | 848 | 923 | 924 | 926 | 100 3 | 106 0 | RFPsg 5 ON | RFPsg 5 OFF5-2 | RFPsg 8 ON | RFPsg 8 OFF5 | |
|---|---|---|---|---|---|---|---|---|---|---|---|---|---|
| 241 | R | Q | K | V | W | A | K | A | NA | -1.08 | -1.04 | -0.14 | |
| 242 | R | A | K | V | W | A | K | A | NA | NA | NA | NA | |
| 243 | A | Q | K | V | W | A | K | A | NA | -0.12 | NA | NA | |
| 244 | A | A | K | V | W | A | K | A | -0.96 | -0.01 | 0.96 | 0.70 | |
| 245 | R | Q | A | V | W | A | K | A | NA | -0.18 | NA | NA | |
| 246 | R | A | A | V | W | A | K | A | NA | NA | NA | NA | |
| 247 | A | Q | A | V | W | A | K | A | NA | NA | NA | NA | |
| 248 | A | A | A | V | W | A | K | A | NA | -0.48 | -0.13 | NA | |
| 249 | R | Q | K | R | R | A | K | A | -0.26 | -0.18 | -0.05 | 0.70 | |
| 250 | R | A | K | R | R | A | K | A | 0.41 | -0.37 | -0.92 | -0.96 | |
| 251 | A | Q | K | R | R | A | K | A | NA | -0.85 | -0.10 | 0.08 | |
| 252 | A | A | K | R | R | A | K | A | -0.57 | -0.24 | -0.50 | -0.40 | |
| 253 | R | Q | A | R | R | A | K | A | -0.47 | -0.13 | NA | -0.23 | |
| 254 | R | A | A | R | R | A | K | A | -0.55 | -0.22 | -0.63 | -0.71 | |
| 255 | A | Q | A | R | R | A | K | A | -1.31 | -0.69 | -1.28 | -0.16 | |
| 256 | A | A | A | R | R | A | K | A | -1.18 | 0.24 | 0.04 | -0.05 | |
| 257 | R | Q | K | G | D | E | K | A | -2.09 | -0.35 | -2.11 | NA | |
| 258 | R | A | K | G | D | E | K | A | -0.93 | 0.11 | 1.84 | -0.23 | |
| 259 | A | Q | K | G | D | E | K | A | 1.29 | 0.68 | NA | 0.70 | |
| 260 | A | A | K | G | D | E | K | A | -0.41 | 0.01 | 1.84 | 0.12 | |
| 261 | R | Q | A | G | D | E | K | A | NA | -0.07 | -0.17 | NA | |
| 262 | R | A | A | G | D | E | K | A | -0.65 | -1.40 | NA | 1.59 | |
| 263 | A | Q | A | G | D | E | K | A | NA | 0.11 | -0.18 | -0.83 | |
| 264 | A | A | A | G | D | E | K | A | -0.75 | -0.89 | 0.27 | -0.20 | |
| 265 | R | Q | K | M | R | A | K | A | -0.79 | 0.01 | NA | -0.29 | |
| 266 | R | A | K | M | R | A | K | A | -0.89 | -0.80 | 0.46 | -0.76 | |
| 267 | A | Q | K | M | R | A | K | A | -0.87 | 0.06 | 0.59 | -0.94 | |
| 268 | A | A | K | M | R | A | K | A | 0.42 | -0.04 | -0.23 | -0.57 | |
| 269 | R | Q | A | M | R | A | K | A | 0.02 | 0.12 | -0.60 | 0.53 | |
| 270 | R | A | A | M | R | A | K | A | 0.92 | 0.27 | -0.62 | 0.33 | |

This file contains enrichment scores determined for SpCas9 variants based on the pooled characterization.

| Cas9 variant # | Amino acid residue | | | | | | | | log2(E) | | | | Key |
| --- | --- | --- | --- | --- | --- | --- | --- | --- | --- | --- | --- | --- | --- |
| | | | | | | | | | sgRNA target | | | | |
| | 661 | 695 | 848 | 923 | 924 | 926 | 100 3 | 106 0 | RFPsg 5 ON | RFPsg 5 OFF5-2 | RFPsg 8 ON | RFPsg 8 OFF5 | |
| 271 | A | Q | A | M | R | A | K | A | -1.40 | -0.13 | -0.05 | 0.15 | |
| 272 | A | A | A | M | R | A | K | A | -1.20 | 0.24 | -0.49 | 0.62 | |
| 273 | R | Q | K | E | T | Q | A | A | 1.24 | 0.16 | 0.82 | 0.30 | |
| 274 | R | A | K | E | T | Q | A | A | 0.29 | -0.70 | 1.31 | -0.12 | |
| 275 | A | Q | K | E | T | Q | A | A | 0.79 | -0.40 | 0.87 | 0.45 | |
| 276 | A | A | K | E | T | Q | A | A | -0.28 | -0.32 | -0.20 | -0.23 | |
| 277 | R | Q | A | E | T | Q | A | A | -0.36 | -0.41 | 0.16 | 0.06 | eSpCas9(1.1) |
| 278 | R | A | A | E | T | Q | A | A | 0.44 | -0.64 | -1.31 | 0.41 | |
| 279 | A | Q | A | E | T | Q | A | A | -0.31 | -0.70 | -0.19 | 0.00 | |
| 280 | A | A | A | E | T | Q | A | A | 0.58 | 0.67 | -0.47 | -0.10 | |
| 281 | R | Q | K | E | T | A | A | A | 0.59 | -0.40 | 0.32 | 0.87 | |
| 282 | R | A | K | E | T | A | A | A | -0.08 | -0.03 | -1.09 | 0.88 | |
| 283 | A | Q | K | E | T | A | A | A | 0.47 | -0.50 | 0.44 | -0.62 | |
| 284 | A | A | K | E | T | A | A | A | -0.26 | -1.40 | 0.38 | -0.55 | |
| 285 | R | Q | A | E | T | A | A | A | -0.45 | -0.11 | 0.65 | -0.98 | |
| 286 | R | A | A | E | T | A | A | A | -0.55 | -0.65 | -0.01 | 0.37 | |
| 287 | A | Q | A | E | T | A | A | A | -0.76 | -0.91 | -0.67 | 0.31 | |
| 288 | A | A | A | E | T | A | A | A | 0.28 | -0.60 | -0.33 | -0.23 | |
| 289 | R | Q | K | Q | G | P | A | A | -0.80 | -0.24 | -0.31 | -0.77 | |
| 290 | R | A | K | Q | G | P | A | A | 0.11 | -0.50 | -0.83 | -0.18 | |
| 291 | A | Q | K | Q | G | P | A | A | -0.12 | -0.40 | NA | -0.80 | |
| 292 | A | A | K | Q | G | P | A | A | -0.77 | -0.96 | -0.43 | -1.07 | |
| 293 | R | Q | A | Q | G | P | A | A | -0.56 | -0.83 | -0.74 | 0.45 | |
| 294 | R | A | A | Q | G | P | A | A | 0.02 | -0.38 | -0.33 | -0.58 | |
| 295 | A | Q | A | Q | G | P | A | A | -0.36 | -1.49 | -0.92 | 0.45 | |
| 296 | A | A | A | Q | G | P | A | A | -0.53 | 0.25 | 0.35 | 0.34 | |
| 297 | R | Q | K | V | R | E | A | A | NA | NA | NA | -6.55 | |
| 298 | R | A | K | V | R | E | A | A | NA | NA | NA | NA | |
| 299 | A | Q | K | V | R | E | A | A | NA | NA | NA | NA | |
| 300 | A | A | K | V | R | E | A | A | NA | NA | NA | NA | |

EP 4 253 549 A2

(continued)

This file contains enrichment scores determined for SpCas9 variants based on the pooled characterization.

| | Amino acid residue | | | | | | | | log2(E) | | | | |
| | | | | | | | | | sgRNA target | | | | Key |
| Cas9 variant # | 661 | 695 | 848 | 923 | 924 | 926 | 100 3 | 106 0 | RFPsg 5 ON | RFPsg 5 OFF5-2 | RFPsg 8 ON | RFPsg 8 OFF5 | |
|---|---|---|---|---|---|---|---|---|---|---|---|---|---|
| 301 | R | Q | A | V | R | E | A | A | NA | NA | NA | NA | |
| 302 | R | A | A | V | R | E | A | A | NA | NA | NA | NA | |
| 303 | A | Q | A | V | R | E | A | A | NA | NA | NA | NA | |
| 304 | A | A | A | V | R | E | A | A | NA | -0.26 | -5.47 | 0.58 | |
| 305 | R | Q | K | A | W | E | A | A | NA | NA | NA | NA | |
| 306 | R | A | K | A | W | E | A | A | NA | NA | NA | NA | |
| 307 | A | Q | K | A | W | E | A | A | NA | NA | NA | NA | |
| 308 | A | A | K | A | W | E | A | A | -1.60 | -0.89 | -0.83 | NA | |
| 309 | R | Q | A | A | W | E | A | A | NA | 0.32 | NA | 1.32 | |
| 310 | R | A | A | A | W | E | A | A | -0.72 | -1.63 | NA | -1.51 | |
| 311 | A | Q | A | A | W | E | A | A | NA | NA | NA | NA | |
| 312 | A | A | A | A | W | E | A | A | NA | NA | NA | NA | |
| 313 | R | Q | K | M | V | A | A | A | 0.84 | 0.29 | 0.66 | 0.27 | |
| 314 | R | A | K | M | V | A | A | A | 0.00 | -0.46 | 0.43 | 0.43 | |
| 315 | A | Q | K | M | V | A | A | A | 1.64 | 0.31 | 0.38 | 0.50 | |
| 316 | A | A | K | M | V | A | A | A | 0.48 | -0.45 | 0.37 | NA | |
| 317 | R | Q | A | M | V | A | A | A | 0.88 | -0.46 | -1.04 | 0.83 | |
| 318 | R | A | A | M | V | A | A | A | -0.16 | -0.34 | -0.71 | -0.19 | |
| 319 | A | Q | A | M | V | A | A | A | 0.40 | -0.02 | -0.33 | 0.03 | |
| 320 | A | A | A | M | V | A | A | A | -0.38 | -0.22 | 0.31 | 0.16 | |
| 321 | R | Q | K | K | S | A | A | A | 0.25 | -0.35 | -0.74 | 0.45 | |
| 322 | R | A | K | K | S | A | A | A | -0.71 | -1.27 | -1.30 | NA | |
| 323 | A | Q | K | K | S | A | A | A | -0.99 | -0.57 | -0.75 | -0.40 | |
| 324 | A | A | K | K | S | A | A | A | -1.22 | 0.18 | -0.05 | -0.46 | |
| 325 | R | Q | A | K | S | A | A | A | -0.32 | -0.41 | -0.26 | -0.18 | |
| 326 | R | A | A | K | S | A | A | A | -0.91 | 0.00 | -0.55 | 0.64 | |
| 327 | A | Q | A | K | S | A | A | A | -0.53 | -0.33 | -0.63 | 0.49 | |
| 328 | A | A | A | K | S | A | A | A | NA | -1.08 | NA | NA | |
| 329 | R | Q | K | R | K | Q | A | A | 0.05 | -0.94 | 0.32 | -0.14 | |
| 330 | R | A | K | R | K | Q | A | A | -0.07 | -0.03 | -0.12 | 0.39 | |

(continued)

This file contains enrichment scores determined for SpCas9 variants based on the pooled characterization.

| | Amino acid residue | | | | | | | | log2(E) sgRNA target | | | | Key |
|---|---|---|---|---|---|---|---|---|---|---|---|---|---|
| Cas9 variant # | 661 | 695 | 848 | 923 | 924 | 926 | 100 3 | 106 0 | RFPsg 5 ON | RFPsg 5 OFF5-2 | RFPsg 8 ON | RFPsg 8 OFF5 | |
| 331 | A | Q | K | R | K | Q | A | A | -1.29 | -0.72 | 0.66 | 0.61 | |
| 332 | A | A | K | R | K | Q | A | A | -0.63 | -0.54 | -1.02 | -0.08 | |
| 333 | R | Q | A | R | K | Q | A | A | -0.21 | -0.15 | 0.08 | -0.59 | |
| 334 | R | A | A | R | K | Q | A | A | -1.52 | -0.56 | 0.23 | -0.71 | |
| 335 | A | Q | A | R | K | Q | A | A | -0.26 | -0.23 | 0.44 | -0.72 | |
| 336 | A | A | A | R | K | Q | A | A | -1.12 | -0.74 | 0.16 | 0.30 | |
| 337 | R | Q | K | C | R | E | A | A | -0.56 | 0.07 | 0.43 | 0.25 | |
| 338 | R | A | K | C | R | E | A | A | -0.79 | -1.41 | 0.28 | -0.77 | |
| 339 | A | Q | K | C | R | E | A | A | 0.06 | -1.09 | 0.51 | 0.28 | |
| 340 | A | A | K | C | R | E | A | A | -0.34 | -0.73 | 0.68 | -0.44 | |
| 341 | R | Q | A | C | R | E | A | A | NA | -0.55 | 0.05 | -0.26 | |
| 342 | R | A | A | C | R | E | A | A | 0.15 | -0.60 | -0.46 | 0.44 | |
| 343 | A | Q | A | C | R | E | A | A | NA | -0.37 | -1.00 | 1.32 | |
| 344 | A | A | A | C | R | E | A | A | -0.92 | -2.09 | -0.25 | 0.55 | |
| 345 | R | Q | K | Q | W | Q | A | A | 0.22 | -0.61 | -1.34 | 0.43 | |
| 346 | R | A | K | Q | W | Q | A | A | -0.99 | -0.73 | NA | -0.57 | |
| 347 | A | Q | K | Q | W | Q | A | A | -0.33 | -0.76 | -1.44 | -0.42 | |
| 348 | A | A | K | Q | W | Q | A | A | NA | 0.21 | -0.58 | 0.82 | |
| 349 | R | Q | A | Q | W | Q | A | A | -0.56 | 0.36 | -0.32 | 0.79 | |
| 350 | R | A | A | Q | W | Q | A | A | 0.18 | 0.06 | -0.31 | 0.01 | |
| 351 | A | Q | A | Q | W | Q | A | A | -1.29 | -0.77 | -1.54 | -0.50 | |
| 352 | A | A | A | Q | W | Q | A | A | NA | -0.42 | -0.19 | 0.08 | |
| 353 | R | Q | K | L | G | A | A | A | NA | -0.21 | -0.15 | NA | |
| 354 | R | A | K | L | G | A | A | A | -0.26 | -0.39 | 1.17 | -1.27 | |
| 355 | A | Q | K | L | G | A | A | A | -0.11 | -0.05 | NA | -1.17 | |
| 356 | A | A | K | L | G | A | A | A | -1.26 | 0.05 | 0.13 | 0.44 | |
| 357 | R | Q | A | L | G | A | A | A | -1.10 | -0.21 | -0.74 | 0.26 | |
| 358 | R | A | A | L | G | A | A | A | 0.43 | -0.71 | 0.33 | 0.76 | |
| 359 | A | Q | A | L | G | A | A | A | NA | -1.34 | -0.74 | -2.13 | |
| 360 | A | A | A | L | G | A | A | A | -0.32 | -0.81 | 0.16 | 0.54 | |

This file contains enrichment scores determined for SpCas9 variants based on the pooled characterization.

| Cas9 variant # | Amino acid residue | | | | | | | | log2(E) sgRNA target | | | | Key |
| | 661 | 695 | 848 | 923 | 924 | 926 | 100 3 | 106 0 | RFPsg 5 ON | RFPsg 5 OFF5-2 | RFPsg 8 ON | RFPsg 8 OFF5 | |
|---|---|---|---|---|---|---|---|---|---|---|---|---|---|
| 361 | R | Q | K | W | D | E | A | A | NA | 0.21 | -1.19 | -0.33 | |
| 362 | R | A | K | W | D | E | A | A | -1.74 | -0.55 | 0.21 | 0.13 | |
| 363 | A | Q | K | W | D | E | A | A | NA | -1.30 | -1.36 | -1.38 | |
| 364 | A | A | K | W | D | E | A | A | -0.58 | -0.38 | 0.13 | -0.38 | |
| 365 | R | Q | A | W | D | E | A | A | -0.82 | -0.42 | -0.80 | -0.21 | |
| 366 | R | A | A | W | D | E | A | A | -0.79 | -0.91 | -0.62 | 0.40 | |
| 367 | A | Q | A | W | D | E | A | A | 0.21 | 0.43 | -0.54 | 0.08 | |
| 368 | A | A | A | W | D | E | A | A | -0.24 | -0.16 | -0.09 | 0.05 | |
| 369 | R | Q | K | H | L | Q | A | A | 1.83 | 0.61 | NA | -0.01 | |
| 370 | R | A | K | H | L | Q | A | A | 0.01 | NA | 0.22 | NA | |
| 371 | A | Q | K | H | L | Q | A | A | 1.00 | 0.39 | 0.24 | 0.83 | |
| 372 | A | A | K | H | L | Q | A | A | 0.00 | -0.51 | -0.06 | 1.57 | |
| 373 | R | Q | A | H | L | Q | A | A | 1.13 | -0.19 | -0.30 | -0.57 | |
| 374 | R | A | A | H | L | Q | A | A | -0.46 | -0.55 | -0.45 | 0.18 | |
| 375 | A | Q | A | H | L | Q | A | A | 0.74 | -1.02 | 0.51 | -0.52 | |
| 376 | A | A | A | H | L | Q | A | A | 0.62 | -0.24 | -0.23 | -0.69 | |
| 377 | R | Q | K | V | W | A | A | A | 0.32 | -0.25 | -1.58 | -0.57 | |
| 378 | R | A | K | V | W | A | A | A | NA | -0.63 | -0.13 | -0.10 | |
| 379 | A | Q | K | V | W | A | A | A | NA | NA | NA | NA | |
| 380 | A | A | K | V | W | A | A | A | NA | NA | NA | NA | |
| 381 | R | Q | A | V | W | A | A | A | -1.00 | -0.77 | 0.75 | -0.20 | |
| 382 | R | A | A | V | W | A | A | A | -0.58 | -0.69 | -0.25 | 0.42 | |
| 383 | A | Q | A | V | W | A | A | A | NA | NA | NA | NA | |
| 384 | A | A | A | V | W | A | A | A | NA | -0.17 | -1.25 | 0.27 | |
| 385 | R | Q | K | R | R | A | A | A | -0.62 | -0.41 | -0.77 | -0.29 | |
| 386 | R | A | K | R | R | A | A | A | -1.04 | -0.03 | 0.23 | -0.50 | |
| 387 | A | Q | K | R | R | A | A | A | -0.17 | -0.60 | -0.92 | 0.56 | |
| 388 | A | A | K | R | R | A | A | A | 0.31 | -0.39 | -0.28 | -0.11 | |
| 389 | R | Q | A | R | R | A | A | A | 0.59 | -1.33 | -0.16 | 0.11 | |
| 390 | R | A | A | R | R | A | A | A | -1.45 | -1.07 | -0.70 | -0.07 | |

This file contains enrichment scores determined for SpCas9 variants based on the pooled characterization.

| | Amino acid residue | | | | | | | | log2(E) sgRNA target | | | | Key |
|---|---|---|---|---|---|---|---|---|---|---|---|---|---|
| Cas9 variant # | 661 | 695 | 848 | 923 | 924 | 926 | 100 3 | 106 0 | RFPsg 5 ON | RFPsg 5 OFF5-2 | RFPsg 8 ON | RFPsg 8 OFF5 | |
| 391 | A | Q | A | R | R | A | A | A | 0.27 | -1.46 | -0.63 | -0.47 | |
| 392 | A | A | A | R | R | A | A | A | -0.77 | -0.36 | -0.62 | -0.09 | |
| 393 | R | Q | K | G | D | E | A | A | NA | -0.69 | NA | 0.40 | |
| 394 | R | A | K | G | D | E | A | A | -0.82 | -0.22 | -0.97 | -2.10 | |
| 395 | A | Q | K | G | D | E | A | A | NA | -0.49 | NA | 0.51 | |
| 396 | A | A | K | G | D | E | A | A | NA | -1.46 | 0.53 | 1.23 | |
| 397 | R | Q | A | G | D | E | A | A | -0.68 | 0.38 | 0.67 | 0.94 | |
| 398 | R | A | A | G | D | E | A | A | NA | NA | NA | 1.77 | |
| 399 | A | Q | A | G | D | E | A | A | NA | 0.67 | NA | NA | |
| 400 | A | A | A | G | D | E | A | A | -0.69 | -1.29 | 0.74 | 1.28 | |
| 401 | R | Q | K | M | R | A | A | A | -0.69 | -0.34 | 0.34 | 0.62 | |
| 402 | R | A | K | M | R | A | A | A | -0.58 | -0.67 | -0.49 | -0.58 | |
| 403 | A | Q | K | M | R | A | A | A | -0.41 | -0.54 | -1.13 | NA | |
| 404 | A | A | K | M | R | A | A | A | -0.93 | -0.23 | -0.11 | -0.66 | |
| 405 | R | Q | A | M | R | A | A | A | NA | -0.21 | -1.26 | 0.11 | |
| 406 | R | A | A | M | R | A | A | A | -0.39 | -0.24 | -0.99 | -0.75 | |
| 407 | A | Q | A | M | R | A | A | A | 0.49 | 0.15 | -1.42 | 0.27 | |
| 408 | A | A | A | M | R | A | A | A | -0.04 | 0.10 | -1.51 | 1.40 | |
| 409 | R | Q | K | E | T | Q | R | R | 0.50 | 1.89 | 0.84 | 1.71 | |
| 410 | R | A | K | E | T | Q | R | R | 1.82 | -0.56 | 1.01 | 0.59 | |
| 411 | A | Q | K | E | T | Q | R | R | 0.97 | 1.17 | 1.40 | 0.65 | |
| 412 | A | A | K | E | T | Q | R | R | 1.32 | 0.18 | 0.52 | 0.01 | |
| 413 | R | Q | A | E | T | Q | R | R | 1.09 | -0.49 | 0.65 | 0.21 | |
| 414 | R | A | A | E | T | Q | R | R | 0.31 | -0.26 | 0.19 | 0.52 | |
| 415 | A | Q | A | E | T | Q | R | R | 0.74 | 0.20 | 0.19 | 0.96 | |
| 416 | A | A | A | E | T | Q | R | R | 0.14 | 0.39 | 0.89 | -0.15 | |
| 417 | R | Q | K | E | T | A | R | R | 1.30 | 0.48 | 1.23 | -0.07 | |
| 418 | R | A | K | E | T | A | R | R | 1.27 | -0.09 | 0.36 | 0.40 | |
| 419 | A | Q | K | E | T | A | R | R | 2.18 | 0.02 | 0.63 | -0.05 | |
| 420 | A | A | K | E | T | A | R | R | -0.17 | -0.59 | 0.45 | 0.33 | |

(continued)

This file contains enrichment scores determined for SpCas9 variants based on the pooled characterization.

| | Amino acid residue | | | | | | | | log2(E) | | | | Key |
| | | | | | | | | | sgRNA target | | | | |
| Cas9 variant # | 661 | 695 | 848 | 923 | 924 | 926 | 100 3 | 106 0 | RFPsg 5 ON | RFPsg 5 OFF5-2 | RFPsg 8 ON | RFPsg 8 OFF5 | |
|---|---|---|---|---|---|---|---|---|---|---|---|---|---|
| 421 | R | Q | A | E | T | A | R | R | -0.05 | -0.10 | 0.22 | -0.26 | |
| 422 | R | A | A | E | T | A | R | R | 0.09 | 0.34 | 0.25 | -0.11 | |
| 423 | A | Q | A | E | T | A | R | R | -0.40 | -0.68 | 0.61 | 0.41 | |
| 424 | A | A | A | E | T | A | R | R | -0.25 | -0.76 | 0.35 | 0.48 | |
| 425 | R | Q | K | Q | G | P | R | R | -0.03 | -0.05 | 0.09 | 0.01 | |
| 426 | R | A | K | Q | G | P | R | R | 0.30 | -0.36 | -0.79 | 0.64 | |
| 427 | A | Q | K | Q | G | P | R | R | -0.95 | -0.40 | -0.36 | -0.71 | |
| 428 | A | A | K | Q | G | P | R | R | -0.20 | -0.02 | -0.61 | 0.33 | |
| 429 | R | Q | A | Q | G | P | R | R | 0.33 | -0.59 | -0.43 | -0.19 | |
| 430 | R | A | A | Q | G | P | R | R | -0.20 | -1.00 | 0.48 | -0.01 | |
| 431 | A | Q | A | Q | G | P | R | R | -0.34 | -0.47 | 0.91 | -0.29 | |
| 432 | A | A | A | Q | G | P | R | R | -0.04 | 0.34 | 0.62 | 0.04 | |
| 433 | R | Q | K | V | R | E | R | R | #DIV/0! | -1.43 | NA | NA | |
| 434 | R | A | K | V | R | E | R | R | NA | NA | NA | NA | |
| 435 | A | Q | K | V | R | E | R | R | NA | NA | -0.01 | NA | |
| 436 | A | A | K | V | R | E | R | R | NA | -1.01 | NA | 0.43 | |
| 437 | R | Q | A | V | R | E | R | R | NA | NA | NA | NA | |
| 438 | R | A | A | V | R | E | R | R | -0.48 | 0.07 | NA | NA | |
| 439 | A | Q | A | V | R | E | R | R | NA | NA | NA | NA | |
| 440 | A | A | A | V | R | E | R | R | NA | NA | NA | NA | |
| 441 | R | Q | K | A | W | E | R | R | NA | NA | NA | NA | |
| 442 | R | A | K | A | W | E | R | R | NA | NA | NA | NA | |
| 443 | A | Q | K | A | W | E | R | R | NA | NA | NA | NA | |
| 444 | A | A | K | A | W | E | R | R | NA | -0.76 | -0.76 | -0.66 | |
| 445 | R | Q | A | A | W | E | R | R | NA | 0.23 | 1.03 | 0.69 | |
| 446 | R | A | A | A | W | E | R | R | NA | -0.81 | 0.21 | NA | |
| 447 | A | Q | A | A | W | E | R | R | NA | NA | NA | NA | |
| 448 | A | A | A | A | W | E | R | R | NA | NA | NA | NA | |
| 449 | R | Q | K | M | V | A | R | R | 1.46 | 1.61 | 1.01 | 1.71 | |
| 450 | R | A | K | M | V | A | R | R | 1.53 | 0.39 | 0.60 | 0.17 | |

(continued)

This file contains enrichment scores determined for SpCas9 variants based on the pooled characterization.

| Cas9 variant # | Amino acid residue | | | | | | | | log2(E) sgRNA target | | | | Key |
| --- | --- | --- | --- | --- | --- | --- | --- | --- | --- | --- | --- | --- | --- |
| | 661 | 695 | 848 | 923 | 924 | 926 | 100 3 | 106 0 | RFPsg 5 ON | RFPsg 5 OFF5-2 | RFPsg 8 ON | RFPsg 8 OFF5 | |
| 451 | A | Q | K | M | V | A | R | R | 1.01 | 1.09 | 1.87 | 0.89 | |
| 452 | A | A | K | M | V | A | R | R | 0.68 | 0.53 | 1.08 | -0.16 | |
| 453 | R | Q | A | M | V | A | R | R | 0.82 | -0.28 | 0.84 | 0.70 | |
| 454 | R | A | A | M | V | A | R | R | 0.84 | -0.05 | 0.43 | 0.78 | |
| 455 | A | Q | A | M | V | A | R | R | 0.91 | 0.34 | -0.04 | 0.82 | |
| 456 | A | A | A | M | V | A | R | R | 0.39 | 0.16 | 0.36 | 0.28 | |
| 457 | R | Q | K | K | S | A | R | R | 0.00 | -0.51 | -0.18 | -0.15 | |
| 458 | R | A | K | K | S | A | R | R | -0.41 | -0.28 | -1.75 | -0.69 | |
| 459 | A | Q | K | K | S | A | R | R | 0.33 | -0.03 | NA | 0.52 | |
| 460 | A | A | K | K | S | A | R | R | -0.84 | 0.63 | -1.16 | -0.81 | |
| 461 | R | Q | A | K | S | A | R | R | -0.84 | -0.25 | -0.24 | 1.45 | |
| 462 | R | A | A | K | S | A | R | R | -1.02 | -0.66 | -0.41 | NA | |
| 463 | A | Q | A | K | S | A | R | R | NA | -0.62 | 0.61 | 0.25 | |
| 464 | A | A | A | K | S | A | R | R | 0.27 | -0.37 | -0.22 | 0.01 | |
| 465 | R | Q | K | R | K | Q | R | R | -0.67 | -0.85 | -0.71 | -0.12 | |
| 466 | R | A | K | R | K | Q | R | R | 0.04 | -0.62 | -0.32 | 0.70 | |
| 467 | A | Q | K | R | K | Q | R | R | -0.82 | -0.43 | -0.34 | -0.33 | |
| 468 | A | A | K | R | K | Q | R | R | -0.21 | -0.61 | -0.93 | 0.01 | |
| 469 | R | Q | A | R | K | Q | R | R | -0.92 | 0.11 | 0.05 | -0.01 | |
| 470 | R | A | A | R | K | Q | R | R | -1.14 | -0.39 | -0.06 | -0.23 | |
| 471 | A | Q | A | R | K | Q | R | R | 0.07 | 0.14 | -0.31 | -0.90 | |
| 472 | A | A | A | R | K | Q | R | R | 0.59 | -0.65 | -0.20 | 0.30 | |
| 473 | R | Q | K | C | R | E | R | R | -0.42 | 0.36 | -0.94 | 0.62 | |
| 474 | R | A | K | C | R | E | R | R | NA | -0.33 | 0.24 | 0.58 | |
| 475 | A | Q | K | C | R | E | R | R | -0.85 | -0.26 | 0.20 | 0.75 | |
| 476 | A | A | K | C | R | E | R | R | -0.95 | -0.53 | 0.07 | -0.09 | |
| 477 | R | Q | A | C | R | E | R | R | 0.21 | 0.19 | 0.81 | 1.02 | |
| 478 | R | A | A | C | R | E | R | R | 0.01 | 0.22 | -0.30 | 0.95 | |
| 479 | A | Q | A | C | R | E | R | R | 0.55 | 0.53 | -0.81 | 1.10 | |
| 480 | A | A | A | C | R | E | R | R | -0.47 | -0.19 | -0.40 | 0.25 | |

This file contains enrichment scores determined for SpCas9 variants based on the pooled characterization.

| Cas9 variant # | Amino acid residue | | | | | | | | log2(E) | | | | Key |
| | 661 | 695 | 848 | 923 | 924 | 926 | 100 3 | 106 0 | sgRNA target | | | | |
| | | | | | | | | | RFPsg 5 ON | RFPsg 5 OFF5-2 | RFPsg 8 ON | RFPsg 8 OFF5 | |
| 481 | R | Q | K | Q | W | Q | R | R | -0.50 | 0.42 | 0.18 | 0.46 | |
| 482 | R | A | K | Q | W | Q | R | R | -0.38 | -0.19 | -0.10 | -0.52 | |
| 483 | A | Q | K | Q | W | Q | R | R | -0.22 | 0.23 | -0.13 | 0.61 | |
| 484 | A | A | K | Q | W | Q | R | R | -0.98 | 0.20 | -0.89 | -0.14 | |
| 485 | R | Q | A | Q | W | Q | R | R | 0.72 | 0.37 | 0.39 | 0.13 | |
| 486 | R | A | A | Q | W | Q | R | R | -0.07 | -0.40 | -0.02 | 0.60 | |
| 487 | A | Q | A | Q | W | Q | R | R | -0.32 | 0.34 | -0.22 | 0.53 | |
| 488 | A | A | A | Q | W | Q | R | R | 0.64 | -1.00 | -0.06 | 0.42 | |
| 489 | R | Q | K | L | G | A | R | R | 0.41 | -0.13 | 1.47 | 0.38 | |
| 490 | R | A | K | L | G | A | R | R | NA | -1.96 | -0.21 | 0.34 | |
| 491 | A | Q | K | L | G | A | R | R | NA | -0.16 | 0.00 | -0.47 | |
| 492 | A | A | K | L | G | A | R | R | 0.32 | -1.49 | NA | -0.21 | |
| 493 | R | Q | A | L | G | A | R | R | 0.00 | 0.34 | 0.28 | -1.24 | |
| 494 | R | A | A | L | G | A | R | R | -1.32 | -0.92 | NA | 0.32 | |
| 495 | A | Q | A | L | G | A | R | R | NA | -1.01 | 1.01 | NA | |
| 496 | A | A | A | L | G | A | R | R | 0.44 | -0.98 | 0.83 | -0.84 | |
| 497 | R | Q | K | W | D | E | R | R | 0.10 | -0.50 | -0.42 | -0.43 | |
| 498 | R | A | K | W | D | E | R | R | 0.03 | -1.16 | -0.44 | -0.42 | |
| 499 | A | Q | K | W | D | E | R | R | -0.40 | -0.59 | 0.51 | 0.42 | |
| 500 | A | A | K | W | D | E | R | R | -1.30 | -0.80 | -0.17 | -0.04 | |
| 501 | R | Q | A | W | D | E | R | R | -0.68 | -0.26 | -0.65 | 0.18 | |
| 502 | R | A | A | W | D | E | R | R | -0.42 | -0.60 | -0.70 | -0.09 | |
| 503 | A | Q | A | W | D | E | R | R | -0.53 | -0.87 | -0.44 | 0.26 | |
| 504 | A | A | A | W | D | E | R | R | -0.87 | -0.56 | -0.48 | 0.56 | |
| 505 | R | Q | K | H | L | Q | R | R | 1.02 | 1.48 | 0.62 | 0.32 | |
| 506 | R | A | K | H | L | Q | R | R | 1.01 | 0.78 | 0.62 | 1.35 | |
| 507 | A | Q | K | H | L | Q | R | R | 1.52 | 1.12 | 1.24 | 0.26 | |
| 508 | A | A | K | H | L | Q | R | R | 1.17 | 0.58 | 0.61 | 0.45 | |
| 509 | R | Q | A | H | L | Q | R | R | 1.35 | 0.27 | 1.46 | 0.75 | |
| 510 | R | A | A | H | L | Q | R | R | 1.09 | -0.17 | 0.96 | 0.83 | |

This file contains enrichment scores determined for SpCas9 variants based on the pooled characterization.

| | Amino acid residue | | | | | | | | log2(E) sgRNA target | | | | Key |
|---|---|---|---|---|---|---|---|---|---|---|---|---|---|
| Cas9 variant # | 661 | 695 | 848 | 923 | 924 | 926 | 100 3 | 106 0 | RFPsg 5 ON | RFPsg 5 OFF5-2 | RFPsg 8 ON | RFPsg 8 OFF5 | |
| 511 | A | Q | A | H | L | Q | R | R | 1.72 | -0.30 | -0.47 | 0.35 | |
| 512 | A | A | A | H | L | Q | R | R | 0.08 | 0.01 | 0.27 | -0.72 | |
| 513 | R | Q | K | V | W | A | R | R | NA | 1.24 | 0.22 | -0.67 | |
| 514 | R | A | K | V | W | A | R | R | -0.39 | -0.63 | -0.86 | 1.18 | |
| 515 | A | Q | K | V | W | A | R | R | NA | -0.17 | -2.66 | NA | |
| 516 | A | A | K | V | W | A | R | R | NA | NA | 0.44 | NA | |
| 517 | R | Q | A | V | W | A | R | R | NA | 0.25 | -0.86 | 0.46 | |
| 518 | R | A | A | V | W | A | R | R | NA | 0.26 | NA | -1.57 | |
| 519 | A | Q | A | V | W | A | R | R | -0.49 | -0.22 | -0.63 | NA | |
| 520 | A | A | A | V | W | A | R | R | 1.53 | 0.24 | -0.04 | 0.42 | |
| 521 | R | Q | K | R | R | A | R | R | -0.93 | -0.51 | 0.50 | 0.12 | |
| 522 | R | A | K | R | R | A | R | R | -0.40 | -0.64 | -0.91 | -1.28 | |
| 523 | A | Q | K | R | R | A | R | R | -0.72 | -0.86 | -0.52 | 0.20 | |
| 524 | A | A | K | R | R | A | R | R | -0.47 | -0.62 | -0.12 | 0.32 | |
| 525 | R | Q | A | R | R | A | R | R | -0.81 | -0.09 | -0.68 | -0.26 | |
| 526 | R | A | A | R | R | A | R | R | -0.91 | -0.34 | -0.19 | -0.42 | |
| 527 | A | Q | A | R | R | A | R | R | 0.36 | -0.86 | 0.49 | 0.13 | |
| 528 | A | A | A | R | R | A | R | R | -0.06 | -0.03 | -0.03 | -0.46 | |
| 529 | R | Q | K | G | D | E | R | R | 0.76 | NA | 0.85 | -0.22 | |
| 530 | R | A | K | G | D | E | R | R | -1.03 | 0.08 | NA | 0.17 | |
| 531 | A | Q | K | G | D | E | R | R | -0.06 | -0.12 | -0.19 | 0.78 | |
| 532 | A | A | K | G | D | E | R | R | -0.83 | -1.22 | -1.40 | 0.01 | |
| 533 | R | Q | A | G | D | E | R | R | NA | -0.69 | 0.91 | 0.66 | |
| 534 | R | A | A | G | D | E | R | R | 0.01 | -0.14 | -0.64 | 0.98 | |
| 535 | A | Q | A | G | D | E | R | R | NA | 0.23 | 0.78 | -0.48 | |
| 536 | A | A | A | G | D | E | R | R | 0.04 | 0.07 | -1.21 | 1.21 | |
| 537 | R | Q | K | M | R | A | R | R | 0.02 | NA | -0.25 | -0.26 | |
| 538 | R | A | K | M | R | A | R | R | -0.49 | 0.29 | -0.92 | 0.88 | |
| 539 | A | Q | K | M | R | A | R | R | 0.28 | -0.47 | -0.69 | 0.46 | |
| 540 | A | A | K | M | R | A | R | R | -0.04 | -0.06 | -0.09 | 1.14 | |

EP 4 253 549 A2

(continued)

This file contains enrichment scores determined for SpCas9 variants based on the pooled characterization.

| Cas9 variant # | Amino acid residue | | | | | | | | log2(E) | | | | Key |
| | 661 | 695 | 848 | 923 | 924 | 926 | 100 3 | 106 0 | sgRNA target | | | | |
| | | | | | | | | | RFPsg 5 ON | RFPsg 5 OFF5-2 | RFPsg 8 ON | RFPsg 8 OFF5 | |
| 541 | R | Q | A | M | R | A | R | R | 0.38 | -0.16 | -0.65 | 1.07 | |
| 542 | R | A | A | M | R | A | R | R | -0.98 | -0.65 | -0.60 | 0.44 | |
| 543 | A | Q | A | M | R | A | R | R | 1.43 | 0.57 | 0.23 | 0.18 | |
| 544 | A | A | A | M | R | A | R | R | NA | -0.20 | -0.28 | 0.40 | |
| 545 | R | Q | K | E | T | Q | R | A | NA | 0.60 | NA | NA | |
| 546 | R | A | K | E | T | Q | R | A | NA | -0.27 | 1.29 | -0.17 | |
| 547 | A | Q | K | E | T | Q | R | A | 0.12 | -0.87 | NA | 0.69 | |
| 548 | A | A | K | E | T | Q | R | A | -0.36 | -0.42 | 1.30 | -0.65 | |
| 549 | R | Q | A | E | T | Q | R | A | 0.79 | -0.58 | 0.16 | 0.17 | |
| 550 | R | A | A | E | T | Q | R | A | -0.35 | -1.15 | -0.05 | -0.43 | |
| 551 | A | Q | A | E | T | Q | R | A | NA | -0.41 | 0.72 | 0.30 | |
| 552 | A | A | A | E | T | Q | R | A | 0.19 | 0.26 | -0.08 | NA | |
| 553 | R | Q | K | E | T | A | R | A | NA | 0.01 | 0.66 | 0.29 | |
| 554 | R | A | K | E | T | A | R | A | -0.40 | -0.81 | -0.26 | NA | |
| 555 | A | Q | K | E | T | A | R | A | 0.75 | -0.93 | -0.09 | 0.14 | |
| 556 | A | A | K | E | T | A | R | A | -0.82 | -1.31 | NA | 0.39 | |
| 557 | R | Q | A | E | T | A | R | A | NA | -0.58 | -0.26 | 1.05 | |
| 558 | R | A | A | E | T | A | R | A | 0.05 | -0.55 | -3.16 | -0.42 | |
| 559 | A | Q | A | E | T | A | R | A | NA | 0.51 | 0.24 | 1.22 | |
| 560 | A | A | A | E | T | A | R | A | -0.13 | 0.08 | -0.95 | -0.27 | |
| 561 | R | Q | K | Q | G | P | R | A | -0.74 | -0.38 | 0.23 | -0.41 | |
| 562 | R | A | K | Q | G | P | R | A | -0.39 | 0.31 | NA | 0.23 | |
| 563 | A | Q | K | Q | G | P | R | A | NA | -0.04 | -1.79 | NA | |
| 564 | A | A | K | Q | G | P | R | A | -0.07 | -0.61 | NA | -0.84 | |
| 565 | R | Q | A | Q | G | P | R | A | NA | -1.13 | NA | 0.20 | |
| 566 | R | A | A | Q | G | P | R | A | -1.53 | -0.30 | -0.03 | 0.80 | |
| 567 | A | Q | A | Q | G | P | R | A | NA | 0.74 | NA | NA | |
| 568 | A | A | A | Q | G | P | R | A | NA | 0.00 | NA | -0.65 | |
| 569 | R | Q | K | V | R | E | R | A | NA | -1.39 | NA | NA | |
| 570 | R | A | K | V | R | E | R | A | #DIV/0! | #DIV/0! | NA | #DIV/0! | |

EP 4 253 549 A2

(continued)

This file contains enrichment scores determined for SpCas9 variants based on the pooled characterization.

| | Amino acid residue | | | | | | | | log2(E) | | | | Key |
| | | | | | | | | | sgRNA target | | | | |
| Cas9 variant # | 661 | 695 | 848 | 923 | 924 | 926 | 100 3 | 106 0 | RFPsg 5 ON | RFPsg 5 OFF5-2 | RFPsg 8 ON | RFPsg 8 OFF5 | |
| 571 | A | Q | K | V | R | E | R | A | NA | -0.35 | NA | NA | |
| 572 | A | A | K | V | R | E | R | A | #DIV/0! | NA | NA | #DIV/0! | |
| 573 | R | Q | A | V | R | E | R | A | NA | NA | NA | #DIV/0! | |
| 574 | R | A | A | V | R | E | R | A | NA | NA | NA | NA | |
| 575 | A | Q | A | V | R | E | R | A | #DIV/0! | NA | NA | NA | |
| 576 | A | A | A | V | R | E | R | A | NA | NA | NA | NA | |
| 577 | R | Q | K | A | W | E | R | A | NA | NA | NA | NA | |
| 578 | R | A | K | A | W | E | R | A | NA | NA | NA | NA | |
| 579 | A | Q | K | A | W | E | R | A | NA | NA | NA | NA | |
| 580 | A | A | K | A | W | E | R | A | NA | NA | NA | NA | |
| 581 | R | Q | A | A | W | E | R | A | NA | -7.30 | NA | NA | |
| 582 | R | A | A | A | W | E | R | A | NA | NA | NA | NA | |
| 583 | A | Q | A | A | W | E | R | A | NA | NA | NA | NA | |
| 584 | A | A | A | A | W | E | R | A | NA | NA | NA | NA | |
| 585 | R | Q | K | M | V | A | R | A | NA | 1.03 | 1.07 | NA | |
| 586 | R | A | K | M | V | A | R | A | NA | -0.47 | 0.93 | NA | |
| 587 | A | Q | K | M | V | A | R | A | 0.34 | -0.81 | 2.04 | 0.62 | |
| 588 | A | A | K | M | V | A | R | A | NA | NA | NA | 0.30 | |
| 589 | R | Q | A | M | V | A | R | A | 0.41 | -0.67 | NA | 1.17 | |
| 590 | R | A | A | M | V | A | R | A | 0.47 | -1.32 | -0.93 | 0.56 | |
| 591 | A | Q | A | M | V | A | R | A | NA | -1.35 | -0.62 | NA | |
| 592 | A | A | A | M | V | A | R | A | NA | -0.59 | -1.01 | NA | |
| 593 | R | Q | K | K | S | A | R | A | -1.65 | -1.16 | -0.49 | -1.12 | |
| 594 | R | A | K | K | S | A | R | A | NA | 1.06 | 1.20 | 0.50 | |
| 595 | A | Q | K | K | S | A | R | A | NA | -0.26 | NA | NA | |
| 596 | A | A | K | K | S | A | R | A | 0.13 | NA | -2.44 | NA | |
| 597 | R | Q | A | K | S | A | R | A | NA | 0.17 | NA | 0.91 | |
| 598 | R | A | A | K | S | A | R | A | NA | -0.79 | NA | -2.04 | |
| 599 | A | Q | A | K | S | A | R | A | -0.22 | 0.00 | NA | NA | |
| 600 | A | A | A | K | S | A | R | A | NA | -0.46 | 0.00 | NA | |

This file contains enrichment scores determined for SpCas9 variants based on the pooled characterization.

| | | | Amino acid residue | | | | | | log2(E) | | | | |
| | | | | | | | | | | sgRNA target | | | |
| Cas9 variant # | 661 | 695 | 848 | 923 | 924 | 926 | 100 3 | 106 0 | RFPsg 5 ON | RFPsg 5 OFF5-2 | RFPsg 8 ON | RFPsg 8 OFF5 | Key |
|---|---|---|---|---|---|---|---|---|---|---|---|---|---|
| 601 | R | Q | K | R | K | Q | R | A | -0.20 | -0.50 | -1.09 | -0.60 | |
| 602 | R | A | K | R | K | Q | R | A | -0.90 | -1.18 | 0.04 | NA | |
| 603 | A | Q | K | R | K | Q | R | A | NA | 0.08 | 1.59 | -0.24 | |
| 604 | A | A | K | R | K | Q | R | A | 0.08 | -0.81 | -0.12 | NA | |
| 605 | R | Q | A | R | K | Q | R | A | NA | NA | -2.00 | 0.75 | |
| 606 | R | A | A | R | K | Q | R | A | -0.39 | -0.57 | -1.78 | -0.53 | |
| 607 | A | Q | A | R | K | Q | R | A | NA | NA | NA | NA | |
| 608 | A | A | A | R | K | Q | R | A | NA | -0.50 | NA | -1.17 | |
| 609 | R | Q | K | C | R | E | R | A | -0.97 | 0.77 | 0.58 | 1.44 | |
| 610 | R | A | K | C | R | E | R | A | NA | -0.46 | 1.07 | 0.72 | |
| 611 | A | Q | K | C | R | E | R | A | NA | -0.63 | NA | NA | |
| 612 | A | A | K | C | R | E | R | A | NA | -0.29 | NA | 0.44 | |
| 613 | R | Q | A | C | R | E | R | A | NA | 0.70 | -2.64 | 0.51 | |
| 614 | R | A | A | C | R | E | R | A | NA | 1.39 | 0.57 | 1.11 | |
| 615 | A | Q | A | C | R | E | R | A | -0.39 | -0.28 | NA | 0.69 | |
| 616 | A | A | A | C | R | E | R | A | NA | -0.02 | NA | NA | |
| 617 | R | Q | K | Q | W | Q | R | A | -1.27 | 1.18 | -0.78 | 0.18 | |
| 618 | R | A | K | Q | W | Q | R | A | -0.65 | -1.16 | -0.30 | NA | |
| 619 | A | Q | K | Q | W | Q | R | A | NA | -0.47 | NA | -1.06 | |
| 620 | A | A | K | Q | W | Q | R | A | NA | -0.98 | -0.37 | 0.58 | |
| 621 | R | Q | A | Q | W | Q | R | A | NA | -0.01 | NA | NA | |
| 622 | R | A | A | Q | W | Q | R | A | NA | -0.36 | -0.20 | 0.17 | |
| 623 | A | Q | A | Q | W | Q | R | A | NA | 1.17 | 0.07 | 0.86 | |
| 624 | A | A | A | Q | W | Q | R | A | 0.00 | NA | -0.41 | 0.51 | |
| 625 | R | Q | K | L | G | A | R | A | NA | -1.01 | NA | NA | |
| 626 | R | A | K | L | G | A | R | A | NA | -1.00 | -0.65 | -1.38 | |
| 627 | A | Q | K | L | G | A | R | A | NA | -0.48 | NA | 1.07 | |
| 628 | A | A | K | L | G | A | R | A | NA | -0.17 | 0.19 | NA | |
| 629 | R | Q | A | L | G | A | R | A | NA | -0.47 | NA | NA | |
| 630 | R | A | A | L | G | A | R | A | NA | -1.65 | NA | NA | |

EP 4 253 549 A2

This file contains enrichment scores determined for SpCas9 variants based on the pooled characterization.

| Cas9 variant # | Amino acid residue | | | | | | | | log2(E) sgRNA target | | | | Key |
|---|---|---|---|---|---|---|---|---|---|---|---|---|---|
| | 661 | 695 | 848 | 923 | 924 | 926 | 100 3 | 106 0 | RFPsg 5 ON | RFPsg 5 OFF5-2 | RFPsg 8 ON | RFPsg 8 OFF5 | |
| 631 | A | Q | A | L | G | A | R | A | NA | NA | NA | NA | |
| 632 | A | A | A | L | G | A | R | A | -0.85 | -0.33 | NA | NA | |
| 633 | R | Q | K | W | D | E | R | A | NA | -2.39 | 0.87 | 1.02 | |
| 634 | R | A | K | W | D | E | R | A | NA | NA | 0.23 | -0.46 | |
| 635 | A | Q | K | W | D | E | R | A | NA | 0.67 | 0.42 | -0.42 | |
| 636 | A | A | K | W | D | E | R | A | -1.24 | 0.34 | -0.51 | NA | |
| 637 | R | Q | A | W | D | E | R | A | NA | -0.33 | NA | NA | |
| 638 | R | A | A | W | D | E | R | A | NA | -3.58 | NA | NA | |
| 639 | A | Q | A | W | D | E | R | A | NA | 0.01 | 0.43 | NA | |
| 640 | A | A | A | W | D | E | R | A | -0.46 | -1.63 | -0.80 | 0.42 | |
| 641 | R | Q | K | H | L | Q | R | A | NA | NA | 0.55 | NA | |
| 642 | R | A | K | H | L | Q | R | A | 0.40 | -0.08 | 0.67 | 0.43 | |
| 643 | A | Q | K | H | L | Q | R | A | 0.16 | 0.97 | -0.38 | NA | |
| 644 | A | A | K | H | L | Q | R | A | NA | -0.69 | -0.99 | -0.18 | |
| 645 | R | Q | A | H | L | Q | R | A | NA | -0.83 | -0.10 | NA | |
| 646 | R | A | A | H | L | Q | R | A | NA | -1.35 | 0.47 | -0.17 | |
| 647 | A | Q | A | H | L | Q | R | A | NA | NA | 0.54 | NA | |
| 648 | A | A | A | H | L | Q | R | A | NA | -0.37 | -1.39 | 0.21 | |
| 649 | R | Q | K | V | W | A | R | A | NA | NA | -1.27 | NA | |
| 650 | R | A | K | V | W | A | R | A | NA | NA | -0.80 | NA | |
| 651 | A | Q | K | V | W | A | R | A | NA | NA | NA | NA | |
| 652 | A | A | K | V | W | A | R | A | NA | NA | NA | NA | |
| 653 | R | Q | A | V | W | A | R | A | NA | -0.06 | NA | NA | |
| 654 | R | A | A | V | W | A | R | A | NA | NA | NA | NA | |
| 655 | A | Q | A | V | W | A | R | A | NA | NA | NA | NA | |
| 656 | A | A | A | V | W | A | R | A | NA | NA | NA | NA | |
| 657 | R | Q | K | R | R | A | R | A | NA | -0.22 | -2.26 | 0.03 | |
| 658 | R | A | K | R | R | A | R | A | NA | -0.77 | -1.52 | NA | |
| 659 | A | Q | K | R | R | A | R | A | NA | 0.88 | NA | NA | |
| 660 | A | A | K | R | R | A | R | A | 0.50 | -0.42 | 0.52 | NA | |

EP 4 253 549 A2

(continued)

This file contains enrichment scores determined for SpCas9 variants based on the pooled characterization.

| Cas9 variant # | Amino acid residue | | | | | | | | log2(E) | | | | Key |
|---|---|---|---|---|---|---|---|---|---|---|---|---|---|
| | | | | | | | | | sgRNA target | | | | |
| | 661 | 695 | 848 | 923 | 924 | 926 | 100 3 | 106 0 | RFPsg 5 ON | RFPsg 5 OFF5-2 | RFPsg 8 ON | RFPsg 8 OFF5 | |
| 661 | R | Q | A | R | R | A | R | A | NA | NA | -0.88 | NA | |
| 662 | R | A | A | R | R | A | R | A | NA | -0.20 | -0.58 | 1.20 | |
| 663 | A | Q | A | R | R | A | R | A | NA | -1.60 | NA | 0.75 | |
| 664 | A | A | A | R | R | A | R | A | NA | -0.65 | NA | NA | |
| 665 | R | Q | K | G | D | E | R | A | NA | NA | NA | NA | |
| 666 | R | A | K | G | D | E | R | A | NA | NA | NA | NA | |
| 667 | A | Q | K | G | D | E | R | A | NA | NA | NA | NA | |
| 668 | A | A | K | G | D | E | R | A | NA | NA | NA | NA | |
| 669 | R | Q | A | G | D | E | R | A | NA | NA | NA | NA | |
| 670 | R | A | A | G | D | E | R | A | NA | NA | -0.68 | NA | |
| 671 | A | Q | A | G | D | E | R | A | NA | NA | NA | NA | |
| 672 | A | A | A | G | D | E | R | A | NA | NA | -1.26 | NA | |
| 673 | R | Q | K | M | R | A | R | A | NA | -0.19 | -2.17 | -0.22 | |
| 674 | R | A | K | M | R | A | R | A | -0.86 | 0.40 | NA | -0.09 | |
| 675 | A | Q | K | M | R | A | R | A | -0.46 | -0.48 | NA | -0.15 | |
| 676 | A | A | K | M | R | A | R | A | 0.28 | -0.67 | NA | 1.60 | |
| 677 | R | Q | A | M | R | A | R | A | NA | -0.80 | -1.08 | 1.37 | |
| 678 | R | A | A | M | R | A | R | A | NA | -0.13 | NA | 0.42 | |
| 679 | A | Q | A | M | R | A | R | A | NA | 0.27 | -0.44 | NA | |
| 680 | A | A | A | M | R | A | R | A | -0.50 | 0.77 | NA | 0.72 | |
| 681 | R | Q | K | E | T | Q | H | R | 0.37 | 1.43 | 1.11 | 1.11 | |
| 682 | R | A | K | E | T | Q | H | R | 1.28 | -1.27 | 1.05 | 0.63 | |
| 683 | A | Q | K | E | T | Q | H | R | 1.41 | 0.44 | 2.76 | 0.88 | Opti-SpCas9 |
| 684 | A | A | K | E | T | Q | H | R | NA | -0.03 | 0.92 | 0.54 | |
| 685 | R | Q | A | E | T | Q | H | R | 0.75 | 0.44 | 0.69 | 0.43 | |
| 686 | R | A | A | E | T | Q | H | R | -1.03 | -0.57 | 0.29 | -0.41 | |
| 687 | A | Q | A | E | T | Q | H | R | 0.72 | -0.07 | 0.27 | -0.20 | |
| 688 | A | A | A | E | T | Q | H | R | 0.20 | -0.33 | -0.45 | -0.37 | |
| 689 | R | Q | K | E | T | A | H | R | 1.42 | -0.53 | 1.45 | 0.97 | |
| 690 | R | A | K | E | T | A | H | R | 0.20 | -1.05 | 0.52 | 0.18 | |

EP 4 253 549 A2

(continued)

This file contains enrichment scores determined for SpCas9 variants based on the pooled characterization.

| Cas9 variant # | Amino acid residue | | | | | | | | log2(E) sgRNA target | | | | Key |
|---|---|---|---|---|---|---|---|---|---|---|---|---|---|
| | 661 | 695 | 848 | 923 | 924 | 926 | 100 3 | 106 0 | RFPsg 5 ON | RFPsg 5 OFF5-2 | RFPsg 8 ON | RFPsg 8 OFF5 | |
| 691 | A | Q | K | E | T | A | H | R | 0.95 | 0.13 | 1.43 | 0.06 | |
| 692 | A | A | K | E | T | A | H | R | -0.12 | -1.28 | 0.31 | 0.20 | |
| 693 | R | Q | A | E | T | A | H | R | -0.37 | -0.42 | 0.32 | 0.38 | |
| 694 | R | A | A | E | T | A | H | R | -0.14 | -0.57 | 0.39 | NA | |
| 695 | A | Q | A | E | T | A | H | R | -0.21 | -0.15 | -0.95 | -0.23 | |
| 696 | A | A | A | E | T | A | H | R | -0.06 | -0.52 | 0.07 | 0.06 | |
| 697 | R | Q | K | Q | G | P | H | R | 0.25 | -1.01 | -0.09 | 0.18 | |
| 698 | R | A | K | Q | G | P | H | R | 0.57 | -0.90 | -1.30 | -0.93 | |
| 699 | A | Q | K | Q | G | P | H | R | -0.77 | -0.60 | -0.28 | 0.74 | |
| 700 | A | A | K | Q | G | P | H | R | -0.27 | -0.42 | -0.37 | -0.62 | |
| 701 | R | Q | A | Q | G | P | H | R | -0.32 | -0.10 | -0.32 | -0.18 | |
| 702 | R | A | A | Q | G | P | H | R | -0.60 | 0.05 | -0.18 | -0.64 | |
| 703 | A | Q | A | Q | G | P | H | R | -1.72 | 0.03 | 0.55 | 0.37 | |
| 704 | A | A | A | Q | G | P | H | R | -0.64 | -1.29 | -0.85 | -0.21 | |
| 705 | R | Q | K | V | R | E | H | R | NA | NA | 1.01 | NA | |
| 706 | R | A | K | V | R | E | H | R | NA | 0.04 | NA | NA | |
| 707 | A | Q | K | V | R | E | H | R | NA | NA | NA | NA | |
| 708 | A | A | K | V | R | E | H | R | #DIV/0! | NA | NA | #DIV/0! | |
| 709 | R | Q | A | V | R | E | H | R | NA | NA | NA | NA | |
| 710 | R | A | A | V | R | E | H | R | NA | NA | NA | NA | |
| 711 | A | Q | A | V | R | E | H | R | NA | NA | NA | NA | |
| 712 | A | A | A | V | R | E | H | R | NA | NA | NA | NA | |
| 713 | R | Q | K | A | W | E | H | R | NA | NA | NA | NA | |
| 714 | R | A | K | A | W | E | H | R | NA | NA | NA | NA | |
| 715 | A | Q | K | A | W | E | H | R | NA | -2.10 | NA | -1.00 | |
| 716 | A | A | K | A | W | E | H | R | NA | -0.22 | NA | NA | |
| 717 | R | Q | A | A | W | E | H | R | NA | 0.17 | NA | -0.07 | |
| 718 | R | A | A | A | W | E | H | R | NA | 0.19 | -1.13 | NA | |
| 719 | A | Q | A | A | W | E | H | R | NA | NA | NA | NA | |
| 720 | A | A | A | A | W | E | H | R | NA | -1.47 | NA | NA | |

This file contains enrichment scores determined for SpCas9 variants based on the pooled characterization.

| | | | | | | | | | log2(E) | | | | |
|---|---|---|---|---|---|---|---|---|---|---|---|---|---|
| | Amino acid residue | | | | | | | | sgRNA target | | | | |
| Cas9 variant # | 661 | 695 | 848 | 923 | 924 | 926 | 100 3 | 106 0 | RFPsg 5 ON | RFPsg 5 OFF5-2 | RFPsg 8 ON | RFPsg 8 OFF5 | Key |
| 721 | R | Q | K | M | V | A | H | R | 0.45 | 0.60 | 0.28 | 1.10 | |
| 722 | R | A | K | M | V | A | H | R | 1.19 | 0.63 | 1.68 | 0.75 | |
| 723 | A | Q | K | M | V | A | H | R | NA | 0.38 | 0.24 | 0.03 | |
| 724 | A | A | K | M | V | A | H | R | 1.27 | 0.16 | 0.23 | 0.66 | |
| 725 | R | Q | A | M | V | A | H | R | 1.71 | -0.40 | 0.01 | -0.46 | |
| 726 | R | A | A | M | V | A | H | R | 0.20 | -0.01 | 1.06 | 0.37 | |
| 727 | A | Q | A | M | V | A | H | R | 1.27 | -0.22 | 0.70 | -0.46 | |
| 728 | A | A | A | M | V | A | H | R | -0.12 | 0.07 | -0.47 | -0.38 | |
| 729 | R | Q | K | K | S | A | H | R | -1.15 | -1.10 | 0.17 | 0.90 | |
| 730 | R | A | K | K | S | A | H | R | -0.83 | 0.33 | -1.18 | -0.53 | |
| 731 | A | Q | K | K | S | A | H | R | -0.23 | -0.37 | -1.24 | 0.55 | |
| 732 | A | A | K | K | S | A | H | R | -0.03 | -0.20 | -0.10 | -0.17 | |
| 733 | R | Q | A | K | S | A | H | R | -0.21 | -0.30 | NA | 0.70 | |
| 734 | R | A | A | K | S | A | H | R | -0.66 | 1.31 | NA | -0.49 | |
| 735 | A | Q | A | K | S | A | H | R | -0.94 | -0.46 | -0.49 | -0.58 | |
| 736 | A | A | A | K | S | A | H | R | -1.33 | -1.39 | -2.02 | 0.88 | |
| 737 | R | Q | K | R | K | Q | H | R | -0.75 | -0.50 | -0.30 | -0.99 | |
| 738 | R | A | K | R | K | Q | H | R | -0.36 | -0.97 | -0.29 | -0.90 | |
| 739 | A | Q | K | R | K | Q | H | R | -1.76 | 0.08 | -0.20 | -0.06 | |
| 740 | A | A | K | R | K | Q | H | R | -0.17 | -0.77 | 0.04 | -0.90 | |
| 741 | R | Q | A | R | K | Q | H | R | -0.43 | -0.20 | -0.19 | 0.70 | |
| 742 | R | A | A | R | K | Q | H | R | -0.10 | NA | -0.10 | -0.59 | |
| 743 | A | Q | A | R | K | Q | H | R | NA | -0.16 | -0.81 | NA | |
| 744 | A | A | A | R | K | Q | H | R | -0.40 | -0.85 | -0.42 | -0.37 | |
| 745 | R | Q | K | C | R | E | H | R | -1.09 | 0.40 | -0.20 | 0.05 | |
| 746 | R | A | K | C | R | E | H | R | -2.00 | -0.25 | -1.32 | 0.36 | |
| 747 | A | Q | K | C | R | E | H | R | NA | -0.08 | 0.24 | NA | |
| 748 | A | A | K | C | R | E | H | R | 0.25 | -0.53 | 0.64 | 0.45 | |
| 749 | R | Q | A | C | R | E | H | R | -0.31 | -0.29 | 0.23 | 0.14 | |
| 750 | R | A | A | C | R | E | H | R | NA | NA | 0.49 | 0.93 | |

EP 4 253 549 A2

This file contains enrichment scores determined for SpCas9 variants based on the pooled characterization.

| Cas9 variant # | 661 | 695 | 848 | 923 | 924 | 926 | 100 3 | 106 0 | RFPsg 5 ON | RFPsg 5 OFF5-2 | RFPsg 8 ON | RFPsg 8 OFF5 | Key |
|---|---|---|---|---|---|---|---|---|---|---|---|---|---|
| | | | Amino acid residue | | | | | | | log2(E) sgRNA target | | | |
| 751 | A | Q | A | C | R | E | H | R | NA | 0.29 | -0.49 | NA | |
| 752 | A | A | A | C | R | E | H | R | -0.10 | 0.12 | -0.58 | 0.44 | |
| 753 | R | Q | K | Q | W | Q | H | R | -0.41 | -0.38 | -0.23 | -0.62 | |
| 754 | R | A | K | Q | W | Q | H | R | -0.01 | 0.33 | -0.29 | -0.29 | |
| 755 | A | Q | K | Q | W | Q | H | R | NA | 0.08 | -0.84 | -0.04 | |
| 756 | A | A | K | Q | W | Q | H | R | 0.11 | -0.33 | -0.05 | 0.18 | |
| 757 | R | Q | A | Q | W | Q | H | R | -0.19 | -0.62 | -0.54 | NA | |
| 758 | R | A | A | Q | W | Q | H | R | -0.15 | -0.19 | -0.26 | 0.74 | |
| 759 | A | Q | A | Q | W | Q | H | R | 0.47 | 0.06 | 0.01 | NA | |
| 760 | A | A | A | Q | W | Q | H | R | -0.09 | -0.44 | NA | -0.16 | |
| 761 | R | Q | K | L | G | A | H | R | 0.09 | 0.22 | 0.19 | NA | |
| 762 | R | A | K | L | G | A | H | R | -0.70 | -1.24 | -0.28 | 0.63 | |
| 763 | A | Q | K | L | G | A | H | R | NA | -0.21 | NA | -0.28 | |
| 764 | A | A | K | L | G | A | H | R | NA | 0.10 | -0.12 | NA | |
| 765 | R | Q | A | L | G | A | H | R | -0.41 | -1.61 | -0.65 | -0.98 | |
| 766 | R | A | A | L | G | A | H | R | -0.42 | 0.36 | NA | 0.16 | |
| 767 | A | Q | A | L | G | A | H | R | NA | 0.98 | -3.30 | -0.76 | |
| 768 | A | A | A | L | G | A | H | R | 0.10 | -2.15 | -0.86 | NA | |
| 769 | R | Q | K | W | D | E | H | R | NA | -0.52 | 0.51 | -0.18 | |
| 770 | R | A | K | W | D | E | H | R | 0.37 | -0.08 | 0.46 | -0.18 | |
| 771 | A | Q | K | W | D | E | H | R | -0.50 | -0.24 | -0.24 | -0.57 | |
| 772 | A | A | K | W | D | E | H | R | 0.37 | -0.12 | -0.78 | 0.16 | |
| 773 | R | Q | A | W | D | E | H | R | -0.21 | 0.41 | 0.14 | 0.03 | |
| 774 | R | A | A | W | D | E | H | R | -0.64 | -0.59 | 0.02 | 0.01 | |
| 775 | A | Q | A | W | D | E | H | R | 0.00 | -0.64 | -0.45 | -0.11 | |
| 776 | A | A | A | W | D | E | H | R | 0.13 | -1.53 | 0.34 | -0.24 | |
| 777 | R | Q | K | H | L | Q | H | R | 0.65 | 1.48 | 0.90 | 1.66 | |
| 778 | R | A | K | H | L | Q | H | R | 1.08 | -0.16 | 0.51 | -0.41 | |
| 779 | A | Q | K | H | L | Q | H | R | 1.93 | 1.38 | 0.57 | 0.34 | |
| 780 | A | A | K | H | L | Q | H | R | 0.79 | -0.44 | 0.79 | -1.23 | |

EP 4 253 549 A2

(continued)

This file contains enrichment scores determined for SpCas9 variants based on the pooled characterization.

| | Amino acid residue | | | | | | | | log2(E) sgRNA target | | | | Key |
|---|---|---|---|---|---|---|---|---|---|---|---|---|---|
| Cas9 variant # | 661 | 695 | 848 | 923 | 924 | 926 | 100 3 | 106 0 | RFPsg 5 ON | RFPsg 5 OFF5-2 | RFPsg 8 ON | RFPsg 8 OFF5 | |
| 781 | R | Q | A | H | L | Q | H | R | 0.93 | 0.30 | -0.16 | 0.62 | |
| 782 | R | A | A | H | L | Q | H | R | 1.69 | -0.63 | 0.00 | -0.95 | |
| 783 | A | Q | A | H | L | Q | H | R | 1.00 | -1.32 | 1.24 | 0.24 | |
| 784 | A | A | A | H | L | Q | H | R | 0.26 | 0.89 | 0.26 | 0.23 | |
| 785 | R | Q | K | V | W | A | H | R | NA | NA | NA | 0.53 | |
| 786 | R | A | K | V | W | A | H | R | NA | NA | NA | NA | |
| 787 | A | Q | K | V | W | A | H | R | NA | -1.06 | NA | NA | |
| 788 | A | A | K | V | W | A | H | R | NA | NA | NA | NA | |
| 789 | R | Q | A | V | W | A | H | R | NA | 0.20 | -1.16 | NA | |
| 790 | R | A | A | V | W | A | H | R | NA | NA | NA | 0.31 | |
| 791 | A | Q | A | V | W | A | H | R | NA | -1.72 | NA | -1.12 | |
| 792 | A | A | A | V | W | A | H | R | NA | NA | NA | NA | |
| 793 | R | Q | K | R | R | A | H | R | -0.41 | -0.36 | 1.08 | NA | |
| 794 | R | A | K | R | R | A | H | R | -0.31 | 0.10 | -0.38 | 1.02 | |
| 795 | A | Q | K | R | R | A | H | R | NA | 0.38 | 0.27 | -0.40 | |
| 796 | A | A | K | R | R | A | H | R | -0.21 | -0.35 | -0.18 | -0.77 | |
| 797 | R | Q | A | R | R | A | H | R | NA | -0.40 | -1.16 | NA | |
| 798 | R | A | A | R | R | A | H | R | -1.02 | -0.32 | 0.78 | 0.07 | |
| 799 | A | Q | A | R | R | A | H | R | -0.14 | -0.12 | -0.09 | 0.31 | |
| 800 | A | A | A | R | R | A | H | R | 0.15 | -0.66 | -1.07 | 0.10 | |
| 801 | R | Q | K | G | D | E | H | R | NA | 0.29 | 0.36 | NA | |
| 802 | R | A | K | G | D | E | H | R | -0.60 | -0.92 | -2.11 | 0.50 | |
| 803 | A | Q | K | G | D | E | H | R | NA | NA | NA | NA | |
| 804 | A | A | K | G | D | E | H | R | NA | 0.75 | 0.65 | 0.93 | |
| 805 | R | Q | A | G | D | E | H | R | -0.75 | -1.06 | -0.24 | 0.53 | |
| 806 | R | A | A | G | D | E | H | R | NA | 0.04 | -0.40 | -1.30 | |
| 807 | A | Q | A | G | D | E | H | R | NA | -2.23 | NA | NA | |
| 808 | A | A | A | G | D | E | H | R | NA | -0.07 | NA | NA | |
| 809 | R | Q | K | M | R | A | H | R | -1.51 | -0.30 | -0.92 | 0.67 | |
| 810 | R | A | K | M | R | A | H | R | -0.47 | 0.26 | NA | -0.20 | |

This file contains enrichment scores determined for SpCas9 variants based on the pooled characterization.

| | Amino acid residue | | | | | | | | log2(E) sgRNA target | | | | Key |
|---|---|---|---|---|---|---|---|---|---|---|---|---|---|
| Cas9 variant # | 661 | 695 | 848 | 923 | 924 | 926 | 100 3 | 106 0 | RFPsg 5 ON | RFPsg 5 OFF5-2 | RFPsg 8 ON | RFPsg 8 OFF5 | |
| 811 | A | Q | K | M | R | A | H | R | -0.70 | -0.91 | NA | -0.53 | |
| 812 | A | A | K | M | R | A | H | R | -0.99 | -0.10 | NA | 0.06 | |
| 813 | R | Q | A | M | R | A | H | R | -0.33 | 0.29 | 0.30 | 0.20 | |
| 814 | R | A | A | M | R | A | H | R | -0.52 | -0.52 | NA | -0.62 | |
| 815 | A | Q | A | M | R | A | H | R | 0.51 | -0.18 | -0.25 | -0.08 | |
| 816 | A | A | A | M | R | A | H | R | -0.27 | -0.71 | -0.50 | -0.19 | |
| 817 | R | Q | K | E | T | Q | H | A | 1.25 | 0.56 | 0.90 | -0.04 | |
| 818 | R | A | K | E | T | Q | H | A | 1.12 | -0.30 | 0.85 | NA | |
| 819 | A | Q | K | E | T | Q | H | A | 0.89 | -0.15 | 1.42 | 0.75 | |
| 820 | A | A | K | E | T | Q | H | A | -0.13 | -0.07 | 0.07 | -0.15 | |
| 821 | R | Q | A | E | T | Q | H | A | -0.03 | -0.85 | 0.12 | -0.16 | |
| 822 | R | A | A | E | T | Q | H | A | NA | -0.50 | -0.13 | 0.40 | |
| 823 | A | Q | A | E | T | Q | H | A | 0.36 | -0.09 | -0.52 | 0.21 | |
| 824 | A | A | A | E | T | Q | H | A | -0.16 | -0.86 | -0.14 | 0.30 | |
| 825 | R | Q | K | E | T | A | H | A | 0.67 | -1.07 | 0.33 | 0.13 | |
| 826 | R | A | K | E | T | A | H | A | 0.14 | 0.06 | 0.86 | -0.09 | |
| 827 | A | Q | K | E | T | A | H | A | 0.21 | -0.36 | 0.10 | 0.60 | |
| 828 | A | A | K | E | T | A | H | A | -0.37 | -0.60 | -0.67 | -0.33 | |
| 829 | R | Q | A | E | T | A | H | A | -0.07 | -0.34 | -0.26 | 0.33 | |
| 830 | R | A | A | E | T | A | H | A | -1.07 | -0.61 | -1.40 | 0.61 | |
| 831 | A | Q | A | E | T | A | H | A | -0.45 | -0.54 | 0.05 | -0.48 | |
| 832 | A | A | A | E | T | A | H | A | -1.10 | -0.23 | -0.20 | 0.30 | |
| 833 | R | Q | K | Q | G | P | H | A | -0.65 | 0.19 | -1.00 | -0.81 | |
| 834 | R | A | K | Q | G | P | H | A | -0.83 | -0.79 | -1.08 | -0.19 | |
| 835 | A | Q | K | Q | G | P | H | A | -0.13 | -0.09 | -1.48 | -0.41 | |
| 836 | A | A | K | Q | G | P | H | A | -0.18 | 0.07 | -0.39 | 0.05 | |
| 837 | R | Q | A | Q | G | P | H | A | -0.32 | -0.85 | -0.02 | 0.27 | |
| 838 | R | A | A | Q | G | P | H | A | -0.58 | -0.39 | -1.24 | -0.90 | |
| 839 | A | Q | A | Q | G | P | H | A | 0.22 | -0.22 | -0.67 | 0.01 | |
| 840 | A | A | A | Q | G | P | H | A | -1.18 | 0.06 | -0.32 | 0.03 | |

This file contains enrichment scores determined for SpCas9 variants based on the pooled characterization.

| | Amino acid residue | | | | | | | | log2(E) sgRNA target | | | | Key |
|---|---|---|---|---|---|---|---|---|---|---|---|---|---|
| Cas9 variant # | 661 | 695 | 848 | 923 | 924 | 926 | 100 3 | 106 0 | RFPsg 5 ON | RFPsg 5 OFF5-2 | RFPsg 8 ON | RFPsg 8 OFF5 | |
| 841 | R | Q | K | V | R | E | H | A | NA | #DIV/0! | NA | NA | |
| 842 | R | A | K | V | R | E | H | A | NA | NA | NA | NA | |
| 843 | A | Q | K | V | R | E | H | A | NA | NA | NA | NA | |
| 844 | A | A | K | V | R | E | H | A | NA | NA | NA | NA | |
| 845 | R | Q | A | V | R | E | H | A | NA | NA | NA | NA | |
| 846 | R | A | A | V | R | E | H | A | NA | -1.55 | NA | NA | |
| 847 | A | Q | A | V | R | E | H | A | NA | NA | NA | NA | |
| 848 | A | A | A | V | R | E | H | A | NA | -0.12 | -0.83 | NA | |
| 849 | R | Q | K | A | W | E | H | A | NA | NA | NA | NA | |
| 850 | R | A | K | A | W | E | H | A | NA | NA | NA | NA | |
| 851 | A | Q | K | A | W | E | H | A | NA | -0.18 | NA | NA | |
| 852 | A | A | K | A | W | E | H | A | NA | NA | NA | NA | |
| 853 | R | Q | A | A | W | E | H | A | NA | 0.50 | -0.57 | -0.12 | |
| 854 | R | A | A | A | W | E | H | A | -0.24 | -0.27 | -1.17 | NA | |
| 855 | A | Q | A | A | W | E | H | A | NA | 0.44 | NA | 0.57 | |
| 856 | A | A | A | A | W | E | H | A | NA | 0.31 | 0.06 | NA | |
| 857 | R | Q | K | M | V | A | H | A | 1.41 | 0.91 | NA | -0.22 | |
| 858 | R | A | K | M | V | A | H | A | 0.85 | 0.20 | -0.76 | -0.18 | |
| 859 | A | Q | K | M | V | A | H | A | 0.54 | 0.12 | -0.04 | -0.35 | |
| 860 | A | A | K | M | V | A | H | A | 0.54 | -0.67 | 0.74 | -0.60 | |
| 861 | R | Q | A | M | V | A | H | A | NA | 0.49 | -0.57 | -0.01 | |
| 862 | R | A | A | M | V | A | H | A | -0.18 | -0.94 | -0.35 | -0.22 | |
| 863 | A | Q | A | M | V | A | H | A | 2.25 | 1.15 | NA | -0.03 | |
| 864 | A | A | A | M | V | A | H | A | 0.18 | -0.70 | -0.54 | -0.33 | |
| 865 | R | Q | K | K | S | A | H | A | -0.33 | -0.02 | -0.26 | -0.53 | |
| 866 | R | A | K | K | S | A | H | A | NA | -1.25 | 1.13 | -1.32 | |
| 867 | A | Q | K | K | S | A | H | A | -0.07 | -0.95 | -0.49 | 0.23 | |
| 868 | A | A | K | K | S | A | H | A | -0.07 | -0.05 | -1.08 | -0.86 | |
| 869 | R | Q | A | K | S | A | H | A | -0.40 | -0.47 | -0.69 | -0.01 | |
| 870 | R | A | A | K | S | A | H | A | -0.61 | -0.23 | -0.14 | NA | |

This file contains enrichment scores determined for SpCas9 variants based on the pooled characterization.

| | Amino acid residue | | | | | | | | log2(E) sgRNA target | | | | Key |
|---|---|---|---|---|---|---|---|---|---|---|---|---|---|
| Cas9 variant # | 661 | 695 | 848 | 923 | 924 | 926 | 100 3 | 106 0 | RFPsg 5 ON | RFPsg 5 OFF5-2 | RFPsg 8 ON | RFPsg 8 OFF5 | |
| 871 | A | Q | A | K | S | A | H | A | -0.16 | 0.14 | 0.90 | 0.25 | |
| 872 | A | A | A | K | S | A | H | A | -1.32 | -0.29 | -0.96 | -0.46 | |
| 873 | R | Q | K | R | K | Q | H | A | -1.37 | -0.22 | -0.51 | 0.10 | |
| 874 | R | A | K | R | K | Q | H | A | 0.20 | -0.30 | -0.21 | -0.49 | |
| 875 | A | Q | K | R | K | Q | H | A | -0.31 | -0.70 | 0.18 | 0.25 | |
| 876 | A | A | K | R | K | Q | H | A | -0.76 | -1.15 | -0.30 | -0.13 | |
| 877 | R | Q | A | R | K | Q | H | A | -1.35 | -1.53 | 0.31 | -0.27 | |
| 878 | R | A | A | R | K | Q | H | A | -0.98 | -0.31 | -1.18 | 0.10 | |
| 879 | A | Q | A | R | K | Q | H | A | -0.64 | -0.86 | 0.09 | -0.37 | |
| 880 | A | A | A | R | K | Q | H | A | -1.78 | -0.86 | -0.58 | -1.54 | |
| 881 | R | Q | K | C | R | E | H | A | -0.28 | -0.80 | -1.20 | -0.26 | |
| 882 | R | A | K | C | R | E | H | A | NA | -0.22 | -0.15 | -0.04 | |
| 883 | A | Q | K | C | R | E | H | A | -0.43 | -1.41 | -1.00 | 1.29 | |
| 884 | A | A | K | C | R | E | H | A | -0.14 | -0.04 | 0.08 | -0.35 | |
| 885 | R | Q | A | C | R | E | H | A | -0.56 | -0.50 | 0.53 | -0.16 | |
| 886 | R | A | A | C | R | E | H | A | NA | -0.36 | -0.09 | -0.31 | |
| 887 | A | Q | A | C | R | E | H | A | -0.29 | -0.77 | NA | 0.10 | |
| 888 | A | A | A | C | R | E | H | A | -0.42 | -0.03 | -0.51 | 0.51 | |
| 889 | R | Q | K | Q | W | Q | H | A | -1.60 | -0.89 | -0.60 | -0.41 | |
| 890 | R | A | K | Q | W | Q | H | A | -1.25 | 0.25 | 1.16 | 0.63 | |
| 891 | A | Q | K | Q | W | Q | H | A | -0.32 | -1.00 | 0.32 | -0.55 | |
| 892 | A | A | K | Q | W | Q | H | A | -0.89 | -0.57 | -0.26 | -0.02 | |
| 893 | R | Q | A | Q | W | Q | H | A | -0.70 | -0.56 | -0.54 | 0.58 | |
| 894 | R | A | A | Q | W | Q | H | A | -0.55 | 0.12 | 0.04 | -0.54 | |
| 895 | A | Q | A | Q | W | Q | H | A | 0.06 | 0.02 | 0.62 | 0.12 | |
| 896 | A | A | A | Q | W | Q | H | A | 0.34 | -0.14 | -0.33 | 0.34 | |
| 897 | R | Q | K | L | G | A | H | A | -0.77 | -0.78 | 0.35 | NA | |
| 898 | R | A | K | L | G | A | H | A | NA | -0.33 | -0.54 | 0.50 | |
| 899 | A | Q | K | L | G | A | H | A | -0.62 | -1.32 | 0.28 | -0.28 | |
| 900 | A | A | K | L | G | A | H | A | 0.29 | 0.04 | -0.01 | -0.25 | |

EP 4 253 549 A2

(continued)

This file contains enrichment scores determined for SpCas9 variants based on the pooled characterization.

| Cas9 variant # | Amino acid residue | | | | | | | | log2(E) sgRNA target | | | | Key |
|---|---|---|---|---|---|---|---|---|---|---|---|---|---|
| | 661 | 695 | 848 | 923 | 924 | 926 | 100 3 | 106 0 | RFPsg 5 ON | RFPsg 5 OFF5-2 | RFPsg 8 ON | RFPsg 8 OFF5 | |
| 901 | R | Q | A | L | G | A | H | A | NA | -0.28 | 0.62 | 1.32 | |
| 902 | R | A | A | L | G | A | H | A | -0.83 | -0.60 | 0.99 | -1.23 | |
| 903 | A | Q | A | L | G | A | H | A | NA | -0.34 | -2.93 | 0.95 | |
| 904 | A | A | A | L | G | A | H | A | NA | 0.11 | NA | NA | |
| 905 | R | Q | K | W | D | E | H | A | -0.90 | -0.12 | -0.84 | 0.11 | |
| 906 | R | A | K | W | D | E | H | A | NA | -0.04 | 0.25 | -0.81 | |
| 907 | A | Q | K | W | D | E | H | A | -0.39 | -1.38 | 0.18 | -1.56 | |
| 908 | A | A | K | W | D | E | H | A | -0.94 | -0.74 | -0.89 | 0.22 | |
| 909 | R | Q | A | W | D | E | H | A | -0.80 | -0.81 | 0.63 | -0.64 | |
| 910 | R | A | A | W | D | E | H | A | 0.54 | -0.01 | -0.21 | -0.01 | |
| 911 | A | Q | A | W | D | E | H | A | 0.31 | -0.79 | 0.09 | -0.07 | |
| 912 | A | A | A | W | D | E | H | A | -0.96 | -0.74 | 0.61 | -0.28 | |
| 913 | R | Q | K | H | L | Q | H | A | 1.40 | 0.52 | 1.37 | 0.21 | |
| 914 | R | A | K | H | L | Q | H | A | 0.38 | -1.09 | 0.25 | 0.10 | |
| 915 | A | Q | K | H | L | Q | H | A | 1.31 | -0.12 | -0.84 | -0.02 | |
| 916 | A | A | K | H | L | Q | H | A | 0.37 | 0.18 | -0.46 | -0.63 | |
| 917 | R | Q | A | H | L | Q | H | A | 0.61 | -0.38 | -0.81 | 0.28 | |
| 918 | R | A | A | H | L | Q | H | A | 0.10 | -0.33 | -0.04 | -1.47 | |
| 919 | A | Q | A | H | L | Q | H | A | 1.18 | 0.35 | -0.57 | -0.07 | |
| 920 | A | A | A | H | L | Q | H | A | -0.44 | -0.31 | -0.36 | -0.73 | |
| 921 | R | Q | K | V | W | A | H | A | NA | -0.65 | -0.53 | 0.51 | |
| 922 | R | A | K | V | W | A | H | A | NA | -1.47 | -0.51 | NA | |
| 923 | A | Q | K | V | W | A | H | A | NA | 0.06 | NA | NA | |
| 924 | A | A | K | V | W | A | H | A | -1.05 | -0.89 | 0.17 | 1.10 | |
| 925 | R | Q | A | V | W | A | H | A | NA | NA | -0.72 | 1.91 | |
| 926 | R | A | A | V | W | A | H | A | NA | -0.44 | 0.33 | NA | |
| 927 | A | Q | A | V | W | A | H | A | -0.31 | 0.35 | 1.75 | NA | |
| 928 | A | A | A | V | W | A | H | A | NA | NA | NA | 0.84 | |
| 929 | R | Q | K | R | R | A | H | A | -0.06 | -0.20 | -0.11 | -0.06 | |
| 930 | R | A | K | R | R | A | H | A | -0.55 | 0.26 | -0.81 | -0.56 | |

This file contains enrichment scores determined for SpCas9 variants based on the pooled characterization.

| Cas9 variant # | Amino acid residue | | | | | | | | log2(E) | | | | Key |
|---|---|---|---|---|---|---|---|---|---|---|---|---|---|
| | 661 | 695 | 848 | 923 | 924 | 926 | 100 3 | 106 0 | sgRNA target | | | | |
| | | | | | | | | | RFPsg 5 ON | RFPsg 5 OFF5-2 | RFPsg 8 ON | RFPsg 8 OFF5 | |
| 931 | A | Q | K | R | R | A | H | A | -0.33 | 0.69 | -0.73 | -1.97 | |
| 932 | A | A | K | R | R | A | H | A | -0.58 | -0.19 | -0.37 | 0.02 | |
| 933 | R | Q | A | R | R | A | H | A | -0.57 | -0.69 | NA | -0.64 | |
| 934 | R | A | A | R | R | A | H | A | -1.12 | -0.48 | 0.32 | 0.68 | |
| 935 | A | Q | A | R | R | A | H | A | 0.37 | -0.44 | -0.91 | 0.33 | |
| 936 | A | A | A | R | R | A | H | A | 0.41 | 0.08 | -0.69 | 0.63 | |
| 937 | R | Q | K | G | D | E | H | A | NA | -0.06 | NA | NA | |
| 938 | R | A | K | G | D | E | H | A | -1.58 | -0.57 | NA | -0.86 | |
| 939 | A | Q | K | G | D | E | H | A | NA | NA | -0.70 | -1.01 | |
| 940 | A | A | K | G | D | E | H | A | NA | -0.66 | NA | -1.28 | |
| 941 | R | Q | A | G | D | E | H | A | NA | -0.66 | -1.34 | 0.29 | |
| 942 | R | A | A | G | D | E | H | A | NA | -0.91 | 0.31 | -2.39 | |
| 943 | A | Q | A | G | D | E | H | A | NA | NA | NA | NA | |
| 944 | A | A | A | G | D | E | H | A | -0.94 | -0.20 | -0.10 | NA | |
| 945 | R | Q | K | M | R | A | H | A | 0.02 | 0.08 | -1.29 | -1.01 | |
| 946 | R | A | K | M | R | A | H | A | -0.27 | -0.77 | -1.30 | -0.14 | |
| 947 | A | Q | K | M | R | A | H | A | -0.51 | -0.32 | 0.50 | NA | |
| 948 | A | A | K | M | R | A | H | A | 0.20 | -0.85 | -0.34 | -0.26 | |
| 949 | R | Q | A | M | R | A | H | A | -0.50 | 0.12 | -0.34 | -0.05 | |
| 950 | R | A | A | M | R | A | H | A | -0.67 | -0.03 | -0.03 | -0.79 | |
| 951 | A | Q | A | M | R | A | H | A | -0.36 | -0.24 | -0.53 | NA | |
| 952 | A | A | A | M | R | A | H | A | -0.46 | -1.15 | -1.19 | 0.12 | |

**Table 3**

A table comparing the on- and off- target activities of SpCas9 variants

1. At endogenous loci

| Cas9 variants | gRNAs with additional 5'G (gN20/GN20) | | | gRNAs without additional 5'G (GN19) | |
|---|---|---|---|---|---|
| | On-target activity by T7E1 assay (GN20)*^ | On-target activity by T7E1 assay (gN20)*^ | On-to-off target ratio by GUIDE-Seq (gN20)* | On-target activity by T7E1 assay*^ | On-to-off target ratio by GUIDE-Seq* |
| WT SpCas9 | | | 52.6 | | 35.6 |
| Opti-SpCas9 | | | 93.7 | | 73.8 |
| OptiHF-SpCas9 | | | 99.4 | | 90.6 |
| Sniper-Cas9 | | | 96.3 | | 64.2 |
| evoCas9 | | 0.0 | n.d. | | 100.0 |
| HypaCas9 | | | n.d. | | 95.5 |
| eSpCas9(1.1) | | | n.d. | | 99.9 |

2. At GFP reporter gene

| Cas9 variants | gRNAs with additional 5'G (gN20) | gRNAs without additional 5'G (GN19) |
|---|---|---|
| | On-target activity*^ | On-target activity*^ |
| WT SpCas9 | | |
| Opti-SpCas9 | | |
| eSpCas9(1.1) | | |
| HypaCas9 | | |

| Cas9 variants | GFPsg1 gRNA (with additional 5'G; gN20) | | | | |
|---|---|---|---|---|---|
| | On-target activity Perfect match | Off-target activity* 1 mismatch | 2 mismatches | 3 mismatches | 4 mismatches |
| WT SpCas9 | | 67.4 | 29.6 | 0.1 | 0.0 |
| Opti-SpCas9 | | 47.9 | 3.3 | 0.0 | 0.1 |
| eSpCas9(1.1) | | 6.4 | 0.1 | 0.0 | 0.1 |
| HypaCas9 | | 3.6 | 0.0 | 0.0 | 0.1 |

\* Averaged score across multiple sites indicated in Fig.5 (for endogenous loci) and Extended Data Figs. 9 and 11 (for GFP reporter gene).
^ On-target sites that showed at least 5% editing activities are included in the calculation and normalized against WT SpCas9.
gN20: gRNA with an additional 5'G that does not match to the target DNA; GN20: gRNA with an additional 5'G that matches to the target DNA.
n.d.: not determined due to low on-target activities

**Table 4**

| This file contains a list of constructs used in this work | | |
|---|---|---|
| **Construct ID** | **Design** | **Reference** |
| pAWp30 | pFUGW-EFS-SpCas9-Zco | Wong et al., PNAS, 2016; 113(9):2544-9 |
| pAWp58 | pFUGW-EFS-eSpCas9(1.1)-Zeo | This study; Slavmaker et al., Science 2016; 351 (6268):84-8 |
| pAWp59 | pFUGW-EFS-SpCas9-HFI-Zeo | This study; Kleinstiver et al., Nature, 2016; 529 (7587):490-5 |
| pAWp130 | pFUGW-EFS-HypaCas9-Zeo | This study; Chen et al., Nature, 2017; 550(7676): 407-10 |
| pAWp145 | pFUGW-EFS-xCas9(3.7) | This study; Hu ct al., Nature, 2018; 556(7699): 57-63 |
| pAWp149D | pFUGW-EFS-evoCas9 | This study; Casini et al., Nat. Biotechnol., 2018; 36(3): 265-271 |
| pAWp151 | pFUGW-CMV-SniperCas9 | This study; Lee eL al., Nat. Commun., 2018; 9 (1):3048 |

(continued)

| This file contains a list of constructs used in this work | | |
|---|---|---|
| **Construct ID** | **Design** | **Reference** |
| pAWp28 | pBT264-U6p-{2xBbsI}-sgRNA scaffold-{MfeI} | Wong et al., PNAS, 2016; 113(9):2544-9 |
| pAWp9 | pFUGW-UBCp-RFP-CMVp-GFP-{BamHI+EcoRI} | Wong et al., PNAS, 2016; 113(9):2544-9 |
| pAWp9-R5 | pFUGW-UBCp-RFP-CMVp-GFP-U6p-RFPsg5 | This study |
| pAWp97-clone5 | pFUGW-UBCp-RFP(OFF5)-CMVp-GFP-U6p-RFPsg5 | This study |
| pAWp97-clone5-2nd | pFUGW-UBCp-RFP(OFF5-2)-CMVp-GFP-U6p-RFPsg5 | This study |
| pAWp9-R6 | pFUGW-UBCp-RFP-CMVp-GFP-U6p-RFPsg6 | This study |
| pAWp9-R8 | pFUGW-UBCp-RFP-CMVp-GFP-U6p-RFPsg8 | This study |
| pAWp98-clone5 | pFUGW-UBCp-RFP(OFF5)-CMVp-GFP-U6p-RFPsg8 | This study |
| pAWp9-1 | pFUGW-UBCp-RFP-CMVp-GFP-U6p-GFPsg1 | Wong et al., PNAS, 2016; 113(9):2544-9 |
| pPZp112-2MM1 | pFUGW-UBCp-RFP-CMVp-GFP-U6p-GFPsg1-2MM1 | This study |
| pPZp112-2MM3 | pFUGW-UBCp-RFP-CMVp-GFP-U6p-GFPsg1-2MM3 | This study |
| pPZp112-2MM6 | pFUGW-UBCp-RFP-CMVp-GFP-U6p-GFPsg1-2MM6 | This study |
| pPZp112-2MM8 | pFUGW-UBCp-RFP-CMVp-GFP-U6p-GFPsg1-2MM8 | This study |
| pPZp112-2MM9 | pFUGW-UBCp-RFP-CMVp-GFP-U6p-GFPsg1-2MM9 | This study |
| pPZp112-2MM10 | pFUGW-UBCp-RFP-CMVp-GFP-U6p-GFPsg1-2MM10 | This study |
| pPZp112-2MM13 | pFUGW-UBCp-RFP-CMVp-GFP-U6p-GFPsg1-2MM13 | This study |
| pPZp112-2MM14 | pFUGW-UBCp-RFP-CMVp-GFP-U6p-GFPsg1-2MM14 | This study |
| pPZp112-3MM5 | pFUGW-UBCp-RFP-CMVp-GFP-U6p-GFPsg1-3MM5 | This study |
| pPZp112-3MM6 | pFUGW-UBCp-RFP-CMVp-GFP-U6p-GFPsg1-3MM6 | This study |
| pPZp112-3MM8 | pFUGW-UBCp-RFP-CMVp-GFP-U6p-GFPsg1-3MM8 | This study |
| pPZp112-4MM1 | pFUGW-UBCp-RFP-CMVp-GFP-U6p-GFPsg1-4MM1 | This study |
| pPZp112-4MM5 | pFUGW-UBCp-RFP-CMVp-GFP-U6p-GFPsg1-4MM5 | This study |
| pPZp112-4MM6 | pFUGW-UBCp-RFP-CMVp-GFP-U6p-GFPsg1-4MM6 | This study |
| pPZp112-4MM8 | pFUGW-UBCp-RFP-CMVp-GFP-U6p-GFPsg1-4MM8 | This study |
| pPZp112-M1 | pFUGW-UBCp-RFP-CMVp-GFP-U6p-GFPsg1-M1 | This study |
| pPZp112-M2 | pFUGW-UBCp-RFP-CMVp-GFP-U6p-GFPsg1-M2 | This study |
| pPZp112-M3 | pFUGW-UBCp-RFP-CMVp-GFP-U6p-GFPsg1-M3 | This study |
| pPZp112-M4 | pFUGW-UBCp-RFP-CMVp-GFP-U6p-GFPsg1-M4 | This study |
| pPZp112-M5 | pFUGW-UBCp-RFP-CMVp-GFP-U6p-GFPsg1-M5 | This study |
| pPZp112-M6 | pFUGW-UBCp-RFP-CMVp-GFP-U6p-GFPsg1-M6 | This study |
| pPZp112-M7 | pFUGW-UBCp-RFP-CMVp-GFP-U6p-GFPsg1-M7 | This study |
| pPZp112-M8 | pFUGW-UBCp-RFP-CMVp-GFP-U6p-GFPsg1-M8 | This study |
| pPZp112-M9 | pFUGW-UBCp-RFP-CMVp-GFP-U6p-GFPsg1-M9 | This study |
| pPZp112-M10 | pFUGW-UBCp-RFP-CMVp-GFP-U6p-GFPsg1-M10 | This study |

(continued)

| This file contains a list of constructs used in this work | | |
|---|---|---|
| **Construct ID** | **Design** | **Reference** |
| pPZp112-M11 | pFUGW-UBCp-RFP-CMVp-GFP-U6p-GFPsg1-M11 | This study |
| pPZp112-M12 | pFUGW-UBCp-RFP-CMVp-GFP-U6p-GFPsg1-M12 | This study |
| pPZp112-M13 | pFUGW-UBCp-RFP-CMVp-GFP-U6p-GFPsg1-M13 | This study |
| pPZp112-M14 | pFUGW-UBCp-RFP-CMVp-GFP-U6p-GFPsg1-M14 | This study |
| pPZp112-M15 | pFUGW-UBCp-RFP-CMVp-GFP-U6p-GFPsg1-M15 | This study |
| pPZp112-M16 | pFUGW-UBCp-RFP-CMVp-GFP-U6p-GFPsg1-M16 | This study |
| pPZp112-M17 | pFUGW-UBCp-RFP-CMVp-GFP-U6p-GFPsg1-M17 | This study |
| pPZp112-M18 | pFUGW-UBCp-RFP-CMVp-GFP-U6p-GFPsg1-M18 | This study |
| pPZp112-M19 | pFUGW-UBCp-RFP-CMVp-GFP-U6p-GFPsg1-M19 | This study |
| pPZp112-M20 | pFUGW-UBCp-RFP-CMVp-GFP-U6p-GFPsg1-M20 | This study |
| pPZp114-1 | pFUGW-UBCp-RFP-CMVp-GFP-U6p-GFP-site20 | This study; Casini et al., Nat. Biotechnol., 2018; 36:265-71 |
| pPZp114-2 | pFUGW-UBCp-RFP-CMVp-GFP-UGp-GFPon-19nt | This study; Casini et al., Nat. Biotechnol.. 2018; 36:265-71 |
| pPZp114-3 | pFUGW-UBCp-RFP-CMVp-GFP-U6p-GFPB-18nt | This study; Casini et al., Nat. Biotechnol., 2018; 36:265-71 |
| pPZp114-4 | pFUGW-UBCp-RFP-CMVp-GFP-U6p-GFPW-17nt | This study; Casini et al., Nat. Biotechnol., 2018; 36:265-71 |
| pPZp114-5 | pFUGW-UBCp-RFP-CMVp-GFP-U6p-GFP 5'-G site20 | This study; Casini et al., Nat. Biotechnol., 2018; 36:265-71 |
| pPZp114-6 | pFUGW-UBCp-RFP-CMVp-GFP-U6p-GFP-site25 | This study; Kleinstiver et al., Nature, 2016; 529 (7587):490-5 |
| pPZp114-7 | pFUGW-UBCp-RFP-CMVp-GFP-U6p-GFP 5'-G site25 | This study; Casini et al., Nat. Biotechnol., 2018; 36:265-71 |
| pPZp114-9 | pFUGW-UBCp-RFP-CMVp-GFP-U6p-GFP 5'-C | This study; Casini et al., Nat. Biotechnol., 2018; 36:265-71 |
| pPZp114-10 | pFUGW-UBCp-RFP-CMVp-GFP-U6p-GFP 5'-GC | This study; Casini et al., Nat. Biotechnol.. 2018; 36:265-71 |
| pPZp114-13 | pFUGW-UBCp-RFP-CMVp-GFP-U6p-GFP 5'-A | This study; Casini et al., Nat. Biotechnol., 2018; 36:265-71 |
| pPZp114-14 | pFUGW-UBCp-RFP-CMVp-GFP-U6p-GFP 5'-GA | This study; Casini et al., Nat. Biotechnol., 2018; 36:265-71 |

(continued)

| This file contains a list of constructs used in this work | | |
| --- | --- | --- |
| **Construct ID** | **Design** | **Reference** |
| pAWp12 | pFUGW-CMVp-GFP | Wong et al., Nat. Biotechnol., 2015; 33(9): 952-61 |
| pAWp12-5 | pFUGW-CMVp-GFP-[U6p-BRD4sg3 gN20] | Wong et al., PNAS, 2016; 113(9):2544-9 |
| pAWp12-6 | pFUGW-CMVp-GFP-[U6p-KDM4Csg1 gN20] | Wong et al., PNAS, 2016; 113(9):2544-9 |
| pAWp12-10 | pFUGW-CMVp-GFP-[U6p-PRMT2sg3 gN20] | Wong et al., PNAS, 2016; 113(9):2544-9 |
| pAWp12-11 | pFUGW-CMVp-GFP-[U6p-HDAC2sg1 gN20] | Wong et al., PNAS, 2016; 113(9):2544-9 |
| pAWp12-15 | pFUGW-CMVp-GFP-[U6p-PRMT6sg1 gN20] | Wong et al., PNAS, 2016; 113(9):2544-9 |
| pAWp12-27 | pFUGW-CMVp-GFP-[U6p-NF1sg1 gN20] | Wong et al., PNAS, 2016; 113(9):2544-9 |
| pAWp12-29 | pFUGW-CMVp-GFP-[U6p-NF2sg1 gN20] | Wong et al., PNAS, 2016; 113(9):2544-9 |
| pPZp115 | pFUGW-CMVp-GFP-[U6p-VEGFAsg-site3 gN20] | This study |
| pPZp116 | pFUGW-CMVp-GFP-[U6p-DNMT1sg-site4 gN20] | This study |
| pPZp132 | pFUGW-CMVp-GFP-[U6p-FANCFsg-site6 gN20] | This study |
| pPZp133 | pFUGW-CMVp-GFP-[U6p-EMX1sg-site3 gN20] | This study |
| pPZp139-2 | pFUGW-CMVp-GFP-[U6p-FANCFsg-site2 GN19] | This study |
| pPZp140-2 | pFUGW-CMVp-GFP-[U6p-FANCFsg-site6 GN19] | This study |
| pPZp141-2 | pFUGW-CMVp-GFP-[U6p-EMX1sg-site3 GN19] | This study |
| pPZp143-2 | pFUGW-CMVp-GFP-[U6p-DNMT1sg-site4 GN19] | This study |
| pPZp144-2 | pFUGW-CMVp-GFP-[U6p-NF1sg1 GN19] | This study |
| pPZp145-2 | pFUGW-CMVp-GFP-[U6p-CXCR4sg GN19] | This study |
| pPZp146-2 | pFUGW-CMVp-GFP-[U6p-PD1sg GN19] | This study |
| pPZp147-2 | pFUGW-CMVp-GFP-[U6p-EMX1sg-site2 GN19] | This study |
| pPZp149-2 | pFUGW-CMVp-GFP-[U6p-ZSCAN2sg GN19] | This study |
| pPZp150-2 | pFUGW-CMVp-GFP-[U6p-FANCFsg-site1 GN19] | This study |
| pPZp151-2 | pFUGW-CMVp-GFP-[U6p-RUNXsg-site1 GN19] | This study |
| pPZp154-2 | pFUGW-CMVp-GFP-[U6p-EMX1sg1 GN19] | This study |
| pPZp155-2 | pFUGW-CMVp-GFP-[U6p-CXCR4sg gN20] | This study |
| pPZp156-2 | pFUGW-CMVp-GFP-[U6p-PD1sg gN20] | This study |
| pPZp157-2 | pFUGW-CMVp-GFP-[U6p-EMX1sg-site2 gN20] | This study |
| pPZp158-2 | pFUGW-CMVp-GFP-[U6p-FANCFsg-site3 gN20] | This study |
| pPZp159-2 | pFUGW-CMVp-GFP-[U6p-ZSCAN2sg gN20] | This study |
| pPZp160-2 | pFUGW-CMVp-GFP-[U6p-FANCFsg-site1 gN20] | This study |

(continued)

| This file contains a list of constructs used in this work | | |
|---|---|---|
| Construct ID | Design | Reference |
| pPZp161-2 | pFUGW-CMVp-GFP-[U6p-RUNXsg-site1 gN20] | This study |
| pPZp164-2 | pFUGW-CMVp-GFP-[U6p-EMX1sg1 gN20] | This study |
| pPZp165-2 | pFUGW-CMVp-GFP-[U6p-BRD4sg3 GN19] | This study |
| pPZp166-2 | pFUGW-CMVp-GFP-[U6p-PRMT2sg3 gN19] | This study |
| pPZp167-2 | pFUGW-CMVp-GFP-[U6p-KDM4Csg1 gN19] | This study |
| pPZp168-2 | pFUGW-CMVp-GFP-[U6p-HDAC2sg1 gN19] | This study |
| pPZp175-2 | pFUGW-CMVp-GFP-[U6p-HPRTsg gN19] | This study |
| pPZp176-2 | pFUGW-CMVp-GFP-[U6p-HPRT38087sg gN19] | This study |
| pPZp177-2 | pFUGW-CMVp-GFP-[U6p-DMDsg gN20] | This study |
| pPZp178-2 | pFUGW-CMVp-GFP-[U6p-AAVSsg gN20] | This study |
| pPZp182-2 | pFUGW-CMVp-GFP-[U6p-HPRTsg gN20] | This study |
| pPZp183-2 | pFUGW-CMVp-GFP-[U6p-HPRT38087sg gN20] | This study |
| pPZp186-2 | pFUGW-CMVp-GFP-[U6p-DNMT1sg-site4-19nt] | This study |
| pPZp197-2 | pFUGW-CMVp-GFP-[U6p-DNMT1sg-site4-17nt] | This study |
| pPZp188-2 | pFUGW-CMVp-GFP-[U6p-PRMT2sg3-18nt] | This study |
| pPZp189-2 | pFUGW-CMVp-GFP-[U6p-EMX1-site3-18nt] | This study |
| pPZp190-2 | pFUGW-CMVp-GFP-[U6p-BRD4sg3-19nt] | This study |
| pPZp191-2 | pFUGW-CMVp-GFP-[U6p-BRD4sg3-18nt] | This study |
| pAWp60 | p-EFS-Cas9 Nter | This study |
| pAWp61 | p-{BsaI-2xBbsI}-barcode-(BsaI) | This study |
| pAWp62 | p-{BsaI}-barcode-{BsaI} | This study |
| pAWp63-clone4 | pFUGW-EFS-SpCas9(R661A+KS48A+Q926A)-Zeo | This study |
| pAWp63-clone6 | pFUGW-EFS-SpCas9(R661A+Q695A+K848A+ E923M+T924V+ Q926A+K1003A+R1060A)-Zeo | This study |
| pAWp63-clonc23 | pFUGW-EFS-SpCas9(Q926A+K1003H+R1060A)-Zeo | This study |
| pAWp63-clonc26 | pFUGW-EFS-SpCas9(Q695A+K1003A+R1060A)-Zeo | This study |
| pAWp63-clone27 | pFUGW-EFS-SpCas9(Q695A+E923H+T924L+R1060A)-Zeo | This study |
| pAWp63-clonc28 | pFUGW-EFS-SpCas9 (Q695A+E923M+T924V+Q926A+K1003H)-Zeo | This study |
| pAWp63-clone29 | pFUGW-EFS-SpCas9(K1003R+R1060A)-Zeo | This study |
| pAWp63-clone30 | pFUGW-EFS-SpCas9(E923M+T924V+Q926A+K1060A)-Zeo | This study |
| pAWp63-clone31 | pFUGW-EFS-SpCas9(K848A+E923H+T924L+K1003R)-Zeo | This study |
| pAWp63-clone32 (Opti-Cas9) | pFUGW-EFS-SpCas9(R661A+K1003H)-Zeo | This study |
| pAWp63-clone33 | pFUGW-EFS-SpCas9(R661A+Q926A)-Zeo | This study |
| pAWp63-clone34 | pFUGW-EFS-SpCas9(R661A+Q926A+K1003H)-Zeo | This study |

(continued)

| Construct ID | Design | Reference |
|---|---|---|
| This file contains a list of constructs used in this work | | |
| pAWp63-clone3-12 (OptiHF-SpCas9) | pFUGW-EFS-SpCas9 (Q695A+K848A+E923M+T924V+Q926A)-Zeo | This study |
| pAWp63-clone3-14 | pFUGW-EFS-SpCas9(R661A+Q926A)-Zeo | This study |
| pAWp63-clone3-16 | pFUGW-EFS-SpCas9(R661A+Q695A)-Zeo | This study |
| pAWp63-clone3-18 | pFUGW-EFS-SpCas9(Q695A+K848A)-Zeo | This study |
| pAWp63-clone3-19 | pFUGW-EFS-SpCas9(Q695A+K848A+R1060A)-Zeo | This study |
| pAWp63-clone3-21 | pFUGW-EFS-SpCas9 (R661A+Q695A+E923M+T924V+Q926A+R1060A)-Zeo | This study |

**Table 5**

This file contains a list of gRNA protospacer sequences used in this study

| sgRNA ID | 3' end of U6 | 5' G (*) | sgRNA protospacer sequence (*) |
|---|---|---|---|
| BRD4sg3 gN20 | ...CACC | g | GGGAACAATAAAGAAGCGCT |
| KDM4Csg1 gN20 | ...CACC | g | CCTTTGCAAGACCCGCACGA |
| PRMT2sg3 gN20 | ...CACC | g | CTGTCCCAGAAGTGAATCGC |
| HDAC2sg1 gN20 | ...CACC | g | TCCGTAATGTTGCTCGATGT |
| PRMT6sg1 GN20 | ...CACC | G | ATTGTCCGGCGAGGACGTGC |
| NF1sg1 gN20 | ...CACC | g | GTTGTGCTCAGTACTGACTT |
| NF2sg1 GN20 | ...CACC | G | AAACATCTCGTACAGTGACA |
| VEGFAsg-site3 GN20 | ...CACC | G | GGTGAGTGAGTGTGTGCGTG |
| DNMT1sg-site 4 GN20 | ...CACC | G | GGAGTGAGGGAAACGGCCCC |
| FANCFsg-site 6 gN20 | ...CACC | g | GCTTGAGACCGCCAGAAGCT |
| EMX1sg-site 3 gN20 | ...CACC | g | GAGTCCGAGCAGAAGAAGAA |
| CXCR4sg GN20 | ...CACC | G | GAAGCGTGATGACAAAGAGG |
| PD1sg gN20 | ...CACC | g | GGCCAGGATGGTTCTTAGGT |
| EMX1sg site 2 gN20 | ...CACC | g | GTCACCTCCAATGACTAGGG |
| FANCFsg-site 3 gN20 | ...CACC | g | GGCGGCTGCACAACCAGTGG |
| ZSCAN2sg gN20 | ...CACC | g | GTGCGGCAAGAGCTTCAGCC |
| FANCFsg-Site 1 gN20 | ...CACC | g | GGAATCCCTTCTGCAGCACC |
| RUNXsg-Site 1 gN20 | ...CACC | g | GCATTTTCAGGAGGAAGCGA |
| EMX1sg1 GN20 | ...CACC | G | GCCTCCCCAAAGCCTGGCCA |
| DMDsggN20 | ...CACC | g | CTTTCTACCTACTGAGTCTG |
| AAVSsggN20 | ...CACC | g | CTCCCTCCCAGGATCCTCTC |
| HPRTsg gN20 | ...CACC | g | TCGAGATGTGATGAAGGAGA |
| HPRT38087sg gN20 | ...CACC | g | AATTATGGGGATTACTAGGA |
| FANCFsg-site 2 GN19 | ...CACC | - | GCTGCAGAAGGGATTCCATG |
| FANCFsg-site 6 GN19 | ...CACC | - | GCTTGAGACCGCCAGAAGCT |
| EMX1sg-site 3 GN19 | ...CACC | - | GAGTCCGAGCAGAAGAAGAA |
| DNMT1sg-site 4 GN19 | ...CACC | - | GGAGTGAGGGAAACGGCCCC |
| NF1sg1 GN19 | ...CACC | - | GTTGTGCTCAGTACTGACTT |
| CXCR4sg GN19 | ...CACC | - | GAAGCGTGATGACAAAGAGG |

(continued)

This file contains a list of gRNA protospacer sequences used in this study

| sgRNA ID | 3' end of U6 | 5' G (*) | sgRNA protospacer sequence (*) |
|---|---|---|---|
| PD1sg GN19 | ...CACC | - | GGCCAGGATGGTTCTTAGGT |
| EMX1sg-site 2 GN19 | ...CACC | - | GTCACCTCCAATGACTAGGG |
| ZSCAN2sg GN19 | ...CACC | - | GTGCGGCAAGAGCTTCAGCC |
| FANCFsg Site 1 GN19 | ...CACC | - | GGAATCCCTTCTGCAGCACC |
| RUNXsg Site 1 GN19 | ...CACC | - | GCATTTTCAGGAGGAAGCGA |
| EMX1sg1 GN19 | ...CACC | - | GCCTCCCCAAAGCCTGGCCA |
| BRD4sg3 GN19 | ...CACC | - | GGGAACAATAAAGAAGCGCT |
| PRMT2sg3 gN19 | ...CACC | g | TGTCCCAGAAGTGAATCGC |
| KDM4Csg1 gN19 | ...CACC | g | CTTTGCAAGACCCGCACGA |
| HDAC2sg1 gN19 | ...CACC | g | CCGTAATGTTGCTCGATGT |
| HPRTsg gN19 | ...CACC | g | CGAGATGTGATGAAGGAGA |
| HPRT38087sg gN19 | ...CACC | g | ATTATGGGGATTACTAGGA |
| DNMT1sg-site 4-19nt | ...CACC | - | GAGTGAGGGAAACGGCCCC |
| DNMT1sg-site 4-17nt | ...CACC | - | GTGAGGGAAACGGCCCC |
| PRMT2sg3-18nt | ...CACC | - | GTCCCAGAAGTGAATCGC |
| EMX1-site 3-18nt | ...CACC | - | GTCCGAGCAGAAGAAGAA |
| BRD4sg3-19nt | ...CACC | - | GGAACAATAAAGAAGCGCT |
| BRD4sg3-18nt | ...CACC | - | GAACAATAAAGAAGCGCT |
| RFPsg5 | ...CACC | G | CACCCAGACCATGAAGATCA |
| RFPsg6 | ...CACC | g | CCACTTCAAGTGCACATCCG |
| RFPsg8 | ...CACC | g | CTGGCTACCAGCTTCATGTA |
| GFPsg1 | ...CACC | g | GGGCGAGGAGCTGTTCACCG |
| GFPsg1-2MM1 | ...CACC | g | GGGtGAGGAGCTGTTtACCG |
| GFPsg1-2MM3 | ...CACC | g | GGGtGAcGAGCTGTTCACCG |
| GFPsg1-2MM6 | ...CACC | g | GGGCGAGGAGCaGaTCACCG |
| GFPsg1-2MM8 | ...CACC | g | GtGCGAGGAGCTGTTCgCCG |
| GFPsg1-2MM9 | ...CACC | g | GaGCGAGtAGCTGTTCACCG |
| GFPsg1-2MM10 | ...CACC | g | GGGaGAGGAGCTGTgCACCG |
| GFPsg1-2MM13 | ...CACC | g | GGGCcAGGAGgTGTTCACCG |
| GFPsg1-2MM14 | ...CACC | g | GGGCGAGGtGCTaTTCACCG |
| GFPsg1-3MM5 | ...CACC | g | tGGgGAGGAGCTGTTCACCc |
| GFPsg1-3MM6 | ...CACC | g | GGaCGcGGAtCTGTTCACCG |
| GFPsg1-3MM8 | ...CACC | g | GGGCGgGcAGCgGTTCACCG |
| GFPsg1-4MM1 | ...CACC | g | GGGCcAtGAGCTGgTtACCG |
| GFPsg1-4MM5 | ...CACC | g | GGGCGcGaAGCatTTCACCG |
| GFPsg1-4MM6 | ...CACC | g | GGGacAGcAGCaGTTCACCG |
| GFPsg1-4MM8 | ...CACC | g | GGagGAcGAGCTGcTCACCG |
| GFPsg1-M1 | ...CACC | g | GGGCGAGGAGCTGTTCACCa |
| GFPsg1-M2 | ...CACC | g | GGGCGAGGAGCTGTTCACtG |
| GFPsg1-M3 | ...CACC | g | GGGCGAGGAGCTGTTCAtCG |
| GFPsg1-M4 | ...CACC | g | GGGCGAGGAGCTGTTCgCCG |
| GFPsg1-M5 | ...CACC | g | GGGCGAGGAGCTGTTtACCG |
| GFPsg1-M6 | ...CACC | g | GGGCGAGGAGCTGTcCACCG |
| GFPsg1-M7 | ...CACC | g | GGGCGAGGAGCTGcTCACCG |
| GFPsg1-M8 | ...CACC | g | GGGCGAGGAGCTaTTCACCG |
| GFPsg1-M9 | ...CACC | g | GGGCGAGGAGCcGTTCACCG |
| GFPsg1-M10 | ...CACC | g | GGGCGAGGAGtTGTTCACCG |
| GFPsg1-M11 | ...CACC | g | GGGCGAGGAaCTGTTCACCG |
| GFPsg1-M12 | ...CACC | g | GGGCGAGGgGCTGTTCACCG |

(continued)

This file contains a list of gRNA protospacer sequences used in this study

| sgRNA ID | 3' end of U6 | 5' G (*) | sgRNA protospacer sequence (*) |
|---|---|---|---|
| GFPsg1-M13 | ...CACC | g | GGGCGAGaAGCTGTTCACCG |
| GFPsg1-M14 | ...CACC | g | GGGCGAaGAGCTGTTCACCG |
| GFPsg1-M15 | ...CACC | g | GGGCGgGGAGCTGTTCACCG |
| GFPsg1-M16 | ...CACC | g | GGGCaAGGAGCTGTTCACCG |
| GFPsg1-M17 | ...CACC | g | GGGtGAGGAGCTGTTCACCG |
| GFPsg1-M18 | ...CACC | g | GGaCGAGGAGCTGTTCACCG |
| GFPsg1-M19 | ...CACC | g | GaGCGAGGAGCTGTTCACCG |
| GFPsg1-M20 | ...CACC | g | aGGCGAGGAGCTGTTCACCG |
| GFP-site20 | ...CACC | - | GAAGTTCGAGGGCGACACCC |
| GFP 5'-G site20 | ...CACC | g | GAAGTTCGAGGGCGACACCC |
| GFP-site25 | ...CACC | - | CCTCGAACTTCACCTCGGCG |
| GFP 5'-G site25 | ...CACC | g | CCTCGAACTTCACCTCGGCG |
| GFP 5'-C | ...CACC | - | CTCGTGACCACCCTGACCTA |
| GFP 5'-GC | ...CACC | g | CTCGTGACCACCCTGACCTA |
| GFP 5'-A | ...CACC | - | ACCATCTTCTTCAAGGACGA |
| GFP 5'-GA | ...CACC | g | ACCATCTTCTTCAAGGACGA |
| GFPon-19nt | ...CACC | - | GGCACGGGCAGCTTGCCGG |
| GFPB-18nt | ...CACC | - | GGCAAGCTGCCCGTGCCC |
| GFPW-17nt | ...CACC | - | GTGACCACCCTGACCTA |

(*) Lowercase indicates non-matching additional 5' guanines. Uppercase indicates matching additional 5' guanines. '-' indicates no additional 5' guanine.

## Table 6

This file contains a list of reporter cell lines used in this work

| Cell line ID | Reporter design | Target sequence on RFP |
|---|---|---|
| RFPsg5-ON | UBCp-RFP-CMVp-GFP-U6p-RFPsg5 | CACCCAGACCATGAAGATCA |
| RFPsg5-OFF5 | UBCp-RFP(OFFS)-CMVp-GFP-U6p-RFPsgS | gACCCAaACCATGAAGATCA |
| RFPsg5-OFF5-2 | UBCp-RFP(OFF5-2)-CMVp-GFP-U6p-RFPsg5 | CACCCAaACCATGAAGATCA |
| RFPsg6-ON | UBCp-RFP-CMVp-GFP-U6p-RFPsg6 | CCACTTCAAGTGCACATCCG |
| RFPsg8-ON | UBCp-RFP-CMVp-GFP-U6p-RFPsg8 | CTGGCTACCAGCTTCATGTA |
| RFPsg8-OFF5 | UBCp-RFP(OFF5)-CMVp-GFP-U6p-RFPsg8 | gTGcCTcCCtGCTTCATGTA |

## Table 7

This file contains a list of primers and PCR conditions used for T7E1 assay

| Target gene | Forward primer (5' to 3') | Reverse primer (5' to 3') |
|---|---|---|
| BRD4 | CACTTGCTGATGCCAGTAGGAG | AAGCACATGCTTCAGGCTAACA |
| DNMT1 site 4 | CCACTTGACAGGCGAGTAACAG | CCAAGGATCTTGTGCTGG |
| DNMT1 site 4 OFF1 | CCAACAAGCCCTAACCAGGA | AGAACGAGAATGCTCGGCAG |
| HDAC2 | GACTTTTCCATCAGGGACACCT | AACCATGCACAGAATCCAGATTTA |
| KDM4C | AGCCACCCTTGGTTGGTTTT | TTCTCTCCAGACACTGCCCT |
| NF1 | GCCATTATTGACAAGAAGTCTAGGGC | GCAAATTCCCCAAAACACAGTAACCC |
| NF2 | GGGACCTGCTGAAACTTGTCACATG | CCAGTCTGGGCATGCATAATGAAATCC |
| PRMT2 | ATTGCCTTAAGTCGACACCTGAT | CACCTTACAGGCACTGCGTT |
| PRMT6 | GACTGTAGAGTTGCCGGAACAG | CTCCCTCCCTAGAGGCTATGAG |
| VEGFA site 3 | TGCCAGACTCCACAGTGCATACG | AGTGAGGTTACGTGCGGACAG |
| VEGFA site 3 OFF1 | ATGCGGTTTCTTCCGGGATT | GAGAGGATCGCAGTCCGAAG |
| VEGFA site 3 OFF2 | GCTTGCAGCAGAACACATGTTGG | GTTGCCTGGGGATGGGGTAT |

(continued)

This file contains a list of primers and PCR conditions used for T7E1 assay

| Target gene | Forward primer (5' to 3') | Reverse primer (5' to 3') |
|---|---|---|
| VEGFA site 3 OFF3 | ACAGTGAGGTGCGGTCTTTGGG | GCACCTAATTGATGCAGTTTGGCTC |
| VEGFA site 3 OFF4 | TTAGCTCCCTTGTGCTGATGAGAC | GAGATGCCTGATGCCGATGTAACC |
| VEGFA site 3 OFF5 | TCTCACCACCTGGCTCCCATTTC | CCAATCCAGGATGATTCCGC |
| VEGFA site 3 OFF6 | ACAGAGTAGCTGACCCACCT | GCTGCCGTCCGAACCCAAGA |
| VEGFA site 3 OFF7 | GGAGGCTGACAGTACTTCATGGT | CGGGACTTTCACCAGGTCCAGAG |
| FANCF site 6 | CAGCATGTGCACCGCAGACC | TCATCTCGCACGTGGTTCCGG |
| EMX1 site 3 | TGCTTGTCCCTCTGTCAATGG | TTAGGCCCTGTGGGAGATCA |
| CXCR4 | GGAGTGGCCTCTTTGTGTGT | ATCTGCCTCACTGACGTTGG |
| PD1 | CGGGATATGGAAAGAGGCCA | AAGCCAAGGTTAGTCCCACA |
| EMX1 site 2 | CCTCCTAGTTATGAAACCATGCCC | AGGGAGATTGGAGACACGGA |
| FANCF site 3 | CGGTAGGATGCCCTACATCTG | AGTCCTCCTGGAGATTTGGGT |
| ZSCAN2 | AGTGTGGGGTGTGTGGGAAG | GCAAGGGGAAGACTCTGGCA |
| FANCF site 1 | CAGAATTCAGCATAGCGCCT | CTGCACCAGGTGGTAACGAG |
| RUNX site 1 | CAAACCACAGGGTTTCGCAG | ACTCAGACACAGGCATTCCG |
| EMX1sg1 | AGGAGCTAGGATGCACAGCA | GAACGCGTTTGCTCTACCAG |
| DMD | GCTTATTCTTCCCCAGGGTGAT | AGTTCCTGCTCTTCGCTACA |
| AAVS | GGCTGGCTACTGGCCTTATC | CTCCTGTGGATTCGGGTCAC |
| HPRT | GGCAAAGGATGTGTTACGTGG | CGCCAATACTCTAGCTCTCCA |
| HPRT38087 | GTGATGCTCACCTCTCCCACA | ACAAGAAGTGTCACCCTAGCC |
| FANCF site 2 | CAGCATGTGCACCGCAGACC | TCATCTCGCACGTGGTTCCGG |
| FANCF site 1 | CAGAATTCAGCATAGCGCCT | CTGCACCAGGTGGTAACGAG |

## Table 8

This file contains adaptor and primer sequences for GUIDE-Seq

| | | |
|---|---|---|
| OFF_GSP1_- | GGATCTCGACGCTCTCCCTGTTTAATTGAGTTGTCATATGTTAATAAC | for PCR1 |
| OFF_GSP1_+ | GGATCTCGACGCTCTCCCTATACCGTTATTAACATATGACA | for PCR1 |
| OFF GSP2 - | CCTCTCTATGGGCAGTCGGTGATACATATGACAACTCAATTAAAC Nuclease off 3 GSP2 | for PCR2 |
| OFF_GSP2_+ | CCTCTCTATGGGCAGTCGGTGATTTGAGTTGTCATATGTTAATAACGGTA | for PCR2 |
| P558 | AATGATACGGCGACCACCGAGATCTACACTCTTTCCCTACACGACGCTCTT CCGATCT | for PCR2 |
| P701 | CAAGCAGAAGACGGCATACGAGATTCGCCTTAGTGACTGGAGTCCTCTCTA TGGGCAGTCGGTGA | for PCR2: use one of the P7##s for each sample (Index 1) |
| P702 | CAAGCAGAAGACGGCATACGAGATCTAGTACGGTGACTGGAGTCCTCTCTATGGGCAGTCGGTGA | |
| P703 | CAAGCAGAAGACGGCATACGAGATTTCTGCCTGTGACTGGAGTCCTCTCTATGGGCAGTCGGTGA | |
| P704 | CAAGCAGAAGACGGCATACGAGATGCTCAGGAGTGACTGGAGTCCTCTCTATGGGCAGTCGGTGA | |
| P705 | CAAGCAGAAGACGGCATACGAGATAGGAGTCCGTGACTGGAGTCCTCTCTATGGGCAGTCGGTGA | |
| P706 | CAAGCAGAAGACGGCATACGAGATCATGCCTAGTGACTGGAGTCCTCTCTATGGGCAGTCGGTGA | |
| P707 | CAAGCAGAAGACGGCATACGAGATGTAGAGAGGTGACTGGAGTCCTCTCTATGGGCAGTCGGTGA | |
| P708 | CAAGCAGAAGACGGCATACGAGATCCTCTCTGGTGACTGGAGTCCTCTCTATGGGCAGTCGGTGA | |
| P709 | CAAGCAGAAGACGGCATACGAGATAGCGTAGCGTGACTGGAGTCCTCTCTATGGGCAGTCGGTGA | |
| P710 | CAAGCAGAAGACGGCATACGAGATCAGCCTCGGTGACTGGAGTCCTCTCTATGGGCAGTCGGTGA | |
| P711 | CAAGCAGAAGACGGCATACGAGATTGCCTCTTGTGACTGGAGTCCTCTCTATGGGCAGTCGGTGA | |
| P712 | CAAGCAGAAGACGGCATACGAGATTCCTCTACGTGACTGGAGTCCTCTCTATGGGCAGTCGGTGA | |
| P713 | CAAGCAGAAGACGGCATACGAGATAACTTCACGTGACTGGAGTCCTCTCTATGGGCAGTCGGTGA | |
| P714 | CAAGCAGAAGACGGCATACGAGATTGGAGAGGGTGACTGGAGTCCTCTCTATGGGCAGTCGGTGA | |
| P715 | CAAGCAGAAGACGGCATACGAGATACGCATCGGTGACTGGAGTCCTCTCTATGGGCAGTCGGTGA | |
| P716 | CAAGCAGAAGACGGCATACGAGATGTACCGTTGTGACTGGAGTCCTCTCTATGGGCAGTCGGTGA | |
| P717 | CAAGCAGAAGACGGCATACGAGATTACAGTTAGTGACTGGAGTCCTCTCTATGGGCAGTCGGTGA | |
| P718 | CAAGCAGAAGACGGCATACGAGATAATCAACTGTGACTGGAGTCCTCTCTATGGGCAGTCGGTGA | |
| P719 | CAAGCAGAAGACGGCATACGAGATGTACCTAGGTGACTGGAGTCCTCTCTATGGGCAGTCGGTGA | |
| P720 | CAAGCAGAAGACGGCATACGAGATCTGGAACAGTGACTGGAGTCCTCTCTATGGGCAGTCGGTGA | |
| P721 | CAAGCAGAAGACGGCATACGAGATGGTGACTAGTGACTGGAGTCCTCTCTATGGGCAGTCGGTGA | |
| P722 | CAAGCAGAAGACGGCATACGAGATGTGCAACCGTGACTGGAGTCCTCTCTATGGGCAGTCGGTGA | |
| P723 | CAAGCAGAAGACGGCATACGAGATGCCTGTCTGTGACTGGAGTCCTCTCTATGGGCAGTCGGTGA | |
| P724 | CAAGCAGAAGACGGCATACGAGATACTGATGGGTGACTGGAGTCCTCTCTATGGGCAGTCGGTGA | |
| P725 | CAAGCAGAAGACGGCATACGAGATATGCTAACGTGACTGGAGTCCTCTCTATGGGCAGTCGGTGA | |
| P726 | CAAGCAGAAGACGGCATACGAGATCACTGAGTGTGACTGGAGTCCTCTCTATGGGCAGTCGGTGA | |
| P727 | CAAGCAGAAGACGGCATACGAGATTAGGCCATGTGACTGGAGTCCTCTCTATGGGCAGTCGGTGA | |
| P728 | CAAGCAGAAGACGGCATACGAGATCAGCAGTCGTGACTGGAGTCCTCTCTATGGGCAGTCGGTGA | |
| P729 | CAAGCAGAAGACGGCATACGAGATTTCTGAGAGTGACTGGAGTCCTCTCTATGGGCAGTCGGTGA | |
| P730 | CAAGCAGAAGACGGCATACGAGATGGACGTTAGTGACTGGAGTCCTCTCTATGGGCAGTCGGTGA | |
| P731 | CAAGCAGAAGACGGCATACGAGATGTGTAGGTGTGACTGGAGTCCTCTCTATGGGCAGTCGGTGA | |
| P732 | CAAGCAGAAGACGGCATACGAGATCATCTCAGGTGACTGGAGTCCTCTCTATGGGCAGTCGGTGA | |
| P733 | CAAGCAGAAGACGGCATACGAGATGCATAGCAGTGACTGGAGTCCTCTCTATGGGCAGTCGGTGA | |

| | |
|---|---|
| P734 | CAAGCAGAAGACGGCATACGAGATCAGTGCACGTGACTGGAGTCCTCTCTATGGGCAGTCGGTGA |
| P735 | CAAGCAGAAGACGGCATACGAGATTTCGGCATGTGACTGGAGTCCTCTCTATGGGCAGTCGGTGA |
| P736 | CAAGCAGAAGACGGCATACGAGATCAACAGGTGTGACTGGAGTCCTCTCTATGGGCAGTCGGTGA |
| P737 | CAAGCAGAAGACGGCATACGAGATAACACTCGGTGACTGGAGTCCTCTCTATGGGCAGTCGGTGA |
| P738 | CAAGCAGAAGACGGCATACGAGATGTCCTGACGTGACTGGAGTCCTCTCTATGGGCAGTCGGTGA |
| P739 | CAAGCAGAAGACGGCATACGAGATGACGTAGAGTGACTGGAGTCCTCTCTATGGGCAGTCGGTGA |
| P740 | CAAGCAGAAGACGGCATACGAGATGATTGGCAGTGACTGGAGTCCTCTCTATGGGCAGTCGGTGA |
| P741 | CAAGCAGAAGACGGCATACGAGATGCCACGACGTGACTGGAGTCCTCTCTATGGGCAGTCGGTGA |
| P742 | CAAGCAGAAGACGGCATACGAGATTTGTTACGGTGACTGGAGTCCTCTCTATGGGCAGTCGGTGA |
| P743 | CAAGCAGAAGACGGCATACGAGATACGACCTAGTGACTGGAGTCCTCTCTATGGGCAGTCGGTGA |
| P744 | CAAGCAGAAGACGGCATACGAGATTGATAATGGTGACTGGAGTCCTCTCTATGGGCAGTCGGTGA |
| P745 | CAAGCAGAAGACGGCATACGAGATGGTTCCATGTGACTGGAGTCCTCTCTATGGGCAGTCGGTGA |
| P746 | CAAGCAGAAGACGGCATACGAGATCCAGTATCGTGACTGGAGTCCTCTCTATGGGCAGTCGGTGA |
| P747 | CAAGCAGAAGACGGCATACGAGATGTCCAGCTGTGACTGGAGTCCTCTCTATGGGCAGTCGGTGA |
| P748 | CAAGCAGAAGACGGCATACGAGATTAACCTTCGTGACTGGAGTCCTCTCTATGGGCAGTCGGTGA |
| Index1 | ATCACCGACTGCCCATAGAGAGGACTCCAGTCAC | Custom sequencing primer Index1 |
| Read2 | GTGACTGGAGTCCTCTCTATGGGCAGTCGGTGAT | Custom sequencing primer Read2 |

**SEQUENCE LISTING**

    **SEQ ID NO:1** amino acid sequence of wild-type SpCas9 protein (WP_115355356.1 type II CRISPR RNA-guided endonuclease Cas9 [*Streptococcus pyogenes*] R661 and K1003 underlined)

MDKKYSIGLDIGTNSVGWAVITDEYKVPSKKFKVLGNTDRHSIKKNLIGALLFDSGETA
EATRLKRTARRRYTRRKNRICYLQEIFSNEMAKVDDSFFHRLEESFLVEEDKKHERHPIF
GNIVDEVAYHEKYPTIYHLRKKLVDSTDKADLRLIYLALAHMIKFRGHFLIEGDLNPDNS
DVDKLFIQLVQTYNQLFEENPINASGVDAKAILSARLSKSRRLENLIAQLPGEKKNGLFG
NLIALSLGLTPNFKSNFDLAEDAKLQLSKDTYDDDLDNLLAQIGDQYADLFLAAKNLSD
AILLSDILRVNTEITKAPLSASMIKRYDEHHQDLTLLKALVRQQLPEKYKEIFFDQSKNGY
AGYIDGGASQEEFYKFIKPILEKMDGTEELLVKLNREDLLRKQRTFDNGSIPHQIHLGEL
HAILRRQEDFYPFLKDNREKIEKILTFRIPYYVGPLARGNSRFAWMTRKSEETITPWNFEE
VVDKGASAQSFIERMTNFDKNLPNEKVLPKHSLLYEYFTVYNELTKVKYVTEGMRKPA
FLSGEQKKAIVDLLFKTNRKVTVKQLKEDYFKKIECFDSVEISGVEDRFNASLGTYHDLL
KIIKDKDFLDNEENEDILEDIVLTLTLFEDREMIEERLKTYAHLFDDKVMKQLKRRRYTG
WGRLSRKLINGIRDKQSGKTILDFLKSDGFANRNFMQLIHDDSLTFKEDIQKAQVSGQG
DSLHEHIANLAGSPAIKKGILQTVKVVDELVKVMGRHKPENIVIEMARENQTTQKGQKN
SRERMKRIEEGIKELGSQILKEHPVENTQLQNEKLYLYYLQNGRDMYVDQELDINRLSD
YDVDHIVPQSFLKDDSIDNKVLTRSDKNRGKSDNVPSEEVVKKMKNYWRQLLNAKLIT
QRKFDNLTKAERGGLSELDKAGFIKRQLVETRQITKHVAQILDSRMNTKYDENDKLIRE
VKVITLKSKLVSDFRKDFQFYKVREINNYHHAHDAYLNAVVGTALIKKYPKLESEFVYG
DYKVYDVRKMIAKSEQEIGKATAKYFFYSNIMNFFKTEITLANGEIRKRPLIETNGETGEI
VVWDKGRDFATVRKVLSMPQVNIVKKTEVQTGGFSKESILPKRNSDKLIARKKDWDPKK
YGGFDSPTVAYSVLVVAKVEKGKSKKLKSVKELLGITIMERSSFEKNPIDFLEAKGYKE
VKKDLIIKLPKYSLFELENGRKRMLASAGELQKGNELALPSKYVNFLYLASHYEKLKGS
PEDNEQKQLFVEQHKHYLDEIIEQISEFSKRVILADANLDKVLSAYNKHRDKPIREQAENI
IHLFTLTNLGAPAAFKYFDTTIDRKRYTSTKEVLDATLIHQSITGLYETRIDLSQLGGD

**SEQ ID NO:2** polynucleotide sequence encoding the wild-type SpCas9 protein (GenBank Accession No. KM099237.1)

ATGGCCCCAAAGAAGAAGCGGAAGGTCGGTATCCACGGAGTCCCAGCAGCCGACAAGAAGTACAG
CATCGGCCTGGACATCGGCACCAACTCTGTGGGCTGGGCCGTGATCACCGACGAGTACAAGGTGC
CCAGCAAGAAATTCAAGGTGCTGGGCAACACCGACCGGCACAGCATCAAGAAGAACCTGATCGGAG
CCCTGCTGTTCGACAGCGGCGAAACAGCCGAGGCCACCCGGCTGAAGAGAACCGCCAGAAGAAGA
TACACCAGACGGAAGAACCGGATCTGCTATCTGCAAGAGATCTTCAGCAACGAGATGGCCAAGGTG
GACGACAGCTTCTTCCACAGACTGGAAGAGTCCTTCCTGGTGGAAGAGGATAAGAAGCACGAGCGG
CACCCCATCTTCGGCAACATCGTGGACGAGGTGGCCTACCACGAGAAGTACCCCACCATCTACCAC
CTGAGAAAGAAACTGGTGGACAGCACCGACAAGGCCGACCTGCGGCTGATCTATCTGGCCCTGGCC
CACATGATCAAGTTCCGGGGGCCACTTCCTGATCGAGGGCGACCTGAACCCCGACAACAGCGACGTG
GACAAGCTGTTCATCCAGCTGGTGCAGACCTACAACCAGCTGTTCGAGGAAAACCCCATCAACGCC
AGCGGCGTGGACGCCAAGGCCATCCTGTCTGCCAGACTGAGCAAGAGCAGACGGCTGGAAAATCT
GATCGCCCAGCTGCCCGGCGAGAAGAAGAATGGCCTGTTCGGAAACCTGATTGCCCTGAGCCTGG
GCCTGACCCCCAACTTCAAGAGCAACTTCGACCTGGCCGAGGATGCCAAACTGCAGCTGAGCAAGG
ACACCTACGACGACGACCTGGACAACCTGCTGGCCCAGATCGGCGACCAGTACGCCGACCTGTTTC
TGGCCGCCAAGAACCTGTCCGACGCCATCCTGCTGAGCGACATCCTGAGAGTGAACACCGAGATCA
CCAAGGCCCCCCTGAGCGCCTCTATGATCAAGAGATACGACGAGCACCACCAGGACCTGACCCTGC
TGAAAGCTCTCGTGCGGCAGCAGCTGCCTGAGAAGTACAAAGAGATTTTCTTCGACCAGAGCAAGAA

```
CGGCTACGCCGGCTACATTGACGGCGGAGCCAGCCAGGAAGAGTTCTACAAGTTCATCAAGCCCAT
CCTGGAAAAGATGGACGGCACCGAGGAACTGCTCGTGAAGCTGAACAGAGAGGACCTGCTGCGGA
AGCAGCGGACCTTCGACAACGGCAGCATCCCCCACCAGATCCACCTGGGAGAGCTGCACGCCATTC
TGCGGCGGCAGGAAGATTTTTACCCATTCCTGAAGGACAACGGGAAAGATCGAGAAGATCCTGA
CCTTCCGCATCCCCTACTACGTGGGCCCTCTGGCCAGGGGAAACAGCAGATTCGCCTGGATGACCA
GAAAGAGCGAGGAAACCATCACCCCCTGGAACTTCGAGGAAGTGGTGGACAAGGGCGCTTCCGCC
CAGAGCTTCATCGAGCGGATGACCAACTTCGATAAGAACCTGCCCAACGAGAAGGTGCTGCCCAAG
CACAGCCTGCTGTACGAGTACTTCACCGTGTATAACGAGCTGACCAAAGTGAAATACGTGACCGAGG
GAATGAGAAAGCCCGCCTTCCTGAGCGGCGAGCAGAAAAAGGCCATCGTGGACCTGCTGTTCAAGA
CCAACCGGAAAGTGACCGTGAAGCAGCTGAAAGAGGACTACTTCAAGAAAATCGAGTGCTTCGACT
CCGTGGAAATCTCCGGCGTGGAAGATCGGTTCAACGCCTCCCTGGGCACATACCACGATCTGCTGA
AAATTATCAAGGACAAGGACTTCCTGGACAATGAGGAAACGAGGACATTCTGGAAGATATCGTGCT
GACCCTGACACTGTTTGAGGACAGAGAGATGATCGAGGAACGGCTGAAAACCTATGCCCACCTGTT
CGACGACAAAGTGATGAAGCAGCTGAAGCGGCGGAGATACACCGGCTGGGGCAGGCTGAGCCGGA
AGCTGATCAACGGCATCCGGGACAAGCAGTCCGGCAAGACAATCCTGGATTTCCTGAAGTCCGACG
GCTTCGCCAACAGAAACTTCATGCAGCTGATCCACGACGACAGCCTGACCTTTAAAGAGGACATCCA
GAAAGCCCAGGTGTCCGGCCAGGGCGATAGCCTGCACGAGCACATTGCCAATCTGGCCGGCAGCC
CCGCCATTAAGAAGGGCATCCTGCAGACAGTGAAGGTGGTGGACGAGCTCGTGAAAGTGATGGGC
CGGCACAAGCCCGAGAACATCGTGATCGAAATGGCCAGAGAGAACCAGACCACCCAGAAGGGACA
GAAGAACAGCCGCGAGAGAATGAAGCGGATCGAAGAGGGCATCAAAGAGCTGGGCAGCCAGATCC
TGAAAGAACACCCCGTGGAAAACACCCAGCTGCAGAACGAGAAGCTGTACCTGTACTACCTGCAGA
ATGGGCGGGATATGTACGTGGACCAGGAACTGGACATCAACCGGCTGTCCGACTACGATGTGGACC
ATATCGTGCCTCAGAGCTTTCTGAAGGACGACTCCATCGACAACAAGGTGCTGACCAGAAGCGACAA
GAACCGGGGCAAGAGCGACAACGTGCCCTCCGAAGAGGTCGTGAAGAAGATGAAGAACTACTGGC
GGCAGCTGCTGAACGCCAAGCTGATTACCCAGAGAAAGTTCGACAATCTGACCAAGGCCGAGAGAG
GCGGCCTGAGCGAACTGGATAAGGCCGGCTTCATCAAGAGACAGCTGGTGGAAACCCGGCAGATC
ACAAAGCACGTGGCACAGATCCTGGACTCCCGGATGAACACTAAGTACGACGAGAATGACAAGCTG
ATCCGGGAAGTGAAAGTGATCACCCTGAAGTCCAAGCTGGTGTCCGATTTCCGGAAGGATTTCCAGT
TTTACAAAGTGCGCGAGATCAACAACTACCACCACGCCCACGACGCCTACCTGAACGCCGTCGTGG
GAACCGCCCTGATCAAAAAGTACCCTAAGCTGGAAAGCGAGTTCGTGTACGGCGACTACAAGGTGT
ACGACGTGCGGAAGATGATCGCCAAGAGCGAGCAGGAAATCGGCAAGGCTACCGCCAAGTACTTCT
TCTACAGCAACATCATGAACTTTTTCAAGACCGAGATTACCCTGGCCAACGGCGAGATCCGGAAGCG
GCCTCTGATCGAGACAAACGGCGAAACCGGGGAGATCGTGTGGGATAAGGGCCGGGATTTTGCCA
CCGTGCGGAAAGTGCTGAGCATGCCCCAAGTGAATATCGTGAAAAAGACCGAGGTGCAGACAGGCG
GCTTCAGCAAAGAGTCTATCCTGCCCAAGAGGAACAGCGATAAGCTGATCGCCAGAAAGAAGGACT
GGGACCCTAAGAAGTACGGCGGCTTCGACAGCCCCACCGTGGCCTATTCTGTGCTGGTGGTGGCC
AAAGTGGAAAAGGGCAAGTCCAAGAAACTGAAGAGTGTGAAAGAGCTGCTGGGGATCACCATCATG
GAAAGAAGCAGCTTCGAGAAGAATCCCATCGACTTTCTGGAAGCCAAGGGCTACAAAGAAGTGAAAA
AGGACCTGATCATCAAGCTGCCTAAGTACTCCCTGTTCGAGCTGGAAAACGGCCGGAAGAGAATGC
TGGCCTCTGCCGGCGAACTGCAGAAGGGAAACGAACTGGCCCTGCCCTCCAAATATGTGAACTTCC
TGTACCTGGCCAGCCACTATGAGAAGCTGAAGGGCTCCCCCGAGGATAATGAGCAGAAACAGCTGT
TTGTGGAACAGCACAAGCACTACCTGGACGAGATCATCGAGCAGATCAGCGAGTTCTCCAAGAGAG
TGATCCTGGCCGACGCTAATCTGGACAAAGTGCTGTCCGCCTACAACAAGCACCGGGATAAGCCCA
TCAGAGAGCAGGCCGAGAATATCATCCACCTGTTTACCCTGACCAATCTGGGAGCCCCTGCCGCCT
TCAAGTACTTTGACACCACCATCGACCGGAAGAGGTACACCAGCACCAAAGAGGTGCTGGACGCCA
CCCTGATCCACCAGAGCATCACCGGCCTGTACGAGACACGGATCGACCTGTCTCAGCTGGGAGGC
GACAAGCGTCCTGCTGCTACTAAGAAAGCTGGTCAAGCTAAGAAAAAGAAAGCTAGCGGCAGCGGC
GCCACCAACTTCAGCCTGCTGAAGCAGGCCGGCGACGTGGAGGAGAACCCCGGCCCCATGGCCAA
GTTGACCAGTGCCGTTCCGGTGCTCACCGCGCGCGACGTCGCCGGAGCGGTCGAGTTCTGGACCG
ACCGGCTCGGGTTCTCCCGGGACTTCGTGGAGGACGACTTCGCCGGTGTGGTCCGGGACGACGTG
ACCCTGTTCATCAGCGCGGTCCAGGACCAGGTGGTGCCGGACAACACCCTGGCCTGGGTGTGGGT
GCGCGGCCTGGACGAGCTGTACGCCGAGTGGTCGGAGGTCGTGTCCACGAACTTCCGGGACGCCT
CCGGGCCGGCCATGACCGAGATCGGCGAGCAGCCGTGGGGCGGGAGTTCGCCCTGCGCGACCC
GGCCGGCAACTGCGTGCACTTCGTGGCCGAGGAGCAGGACTGA
```

**SEQ ID NO:3** amino acid sequence of Opti-SpCas9 protein (base seqeuence SEQ ID NO:1, residue 1003 substituted with Histidine and residue 661 substituted with Alanine)

MDKKYSIGLDIGTNSVGWAVITDEYKVPSKKFKVLGNTDRHSIKKNLIGALLFDSGETA
EATRLKRTARRRYTRRKNRICYLQEIFSNEMAKVDDSFFHRLEESFLVEEDKKHERHPIF
GNIVDEVAYHEKYPTIYHLRKKLVDSTDKADLRLIYLALAHMIKFRGHFLIEGDLNPDNS
DVDKLFIQLVQTYNQLFEENPINASGVDAKAILSARLSKSRRLENLIAQLPGEKKNGLFG
NLIALSLGLTPNFKSNFDLAEDAKLQLSKDTYDDDLDNLLAQIGDQYADLFLAAKNLSD
AILLSDILRVNTEITKAPLSASMIKRYDEHHQDLTLLKALVRQQLPEKYKEIFFDQSKNGY
AGYIDGGASQEEFYKFIKPILEKMDGTEELLVKLNREDLLRKQRTFDNGSIPHQIHLGEL
HAILRRQEDFYPFLKDNREKIEKILTFRIPYYVGPLARGNSRFAWMTRKSEETITPWNFEE
VVDKGASAQSFIERMTNFDKNLPNEKVLPKHSLLYEYFTVYNELTKVKYVTEGMRKPA
FLSGEQKKAIVDLLFKTNRKVTVKQLKEDYFKKIECFDSVEISGVEDRFNASLGTYHDLL
KIIKDKDFLDNEENEDILEDIVLTLTLFEDREMIEERLKTYAHLFDDKVMKQLKRRRYTG
WG**A**LSRKLINGIRDKQSGKTILDFLKSDGFANRNFMQLIHDDSLTFKEDIQKAQVSGQG
DSLHEHIANLAGSPAIKKGILQTVKVVDELVKVMGRHKPENIVIEMARENQTTQKGQKN
SRERMKRIEEGIKELGSQILKEHPVENTQLQNEKLYLYYLQNGRDMYVDQELDINRLSD
YDVDHIVPQSFLKDDSIDNKVLTRSDKNRGKSDNVPSEEVVKKMKNYWRQLLNAKLIT
QRKFDNLTKAERGGLSELDKAGFIKRQLVETRQITKHVAQILDSRMNTKYDENDKLIRE
VKVITLKSKLVSDFRKDFQFYKVREINNYHHAHDAYLNAVVGTALIKKYP**H**LESEFVYG
DYKVYDVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDWDPKK
YGGFDSPTVAYSVLVVAKVEKGKSKKLKSVKELLGITIMERSSFEKNPIDFLEAKGYKE
VKKDLIIKLPKYSLFELENGRKRMLASAGELQKGNELALPSKYVNFLYLASHYEKLKGS
PEDNEQKQLFVEQHKHYLDEIIEQISEFSKRVILADANLDKVLSAYNKHRDKPIREQAENI
IHLFTLTNLGAPAAFKYFDTTIDRKRYTSTKEVLDATLIHQSITGLYETRIDLSQLGGD

**SEQ ID NO:4** amino acid sequence >WP_002279859.1 type II CRISPR RNA-guided endonuclease Cas9 [*Streptococcus mutans*]

```
MKKPYSIGLDIGTNSVGWAVVTDDYKVPAKKMKVLGNTDKSHIKKNLLGALLFDSGNTAEDRRLKRTARR
RYTRRRNRILYLQEIFSEEMGKVDDSFFHRLDESFLTDDDKNFDSHPIFGNKAEEDAYHQKFPTIYHLRK
HLADSTEKADLRLVYLALAHMIKFRGHFLIEGELNAENTDVQKLFADFVGVYDRTFDDSHLSEITVDASS
ILTEKISKSRRLEKLINNYPKEKKNTLFGNLIALSLGLQPNFKTNFKLSEDAKLQFSKDTYEEELEVLLA
QIGDNYAELFLSAKKLYDSILLSGILTVTDVSTKAPLSASMIQRYNEHQMDLAQLKQFIRQKLSDKYNEV
FSDVSKDGYAGYIDGKTNQEAFYKYLKGLLNKIEGSGYFLDKIEREDFLRKQRTFDNGSIPHQIHLQEMR
AIIRRQAEFYPFLADNQDRIEKILTFRIPYYVGPLARGKSDFAWLSRKSADKITPWNFDEIVDKESSVEA
FINRMTNYDLYLPNQKVLPKHSLLYEKFTVYNELTKVKYKTEQGKTAFFDANMKQEIFDGVFKVYRKVTK
DKLMDFLEKEFDEFRIVDLTGLDKENKAFNASYGTYHDLRKILDKDFLDNSKNEKILEDIVLTLTLFEDR
EMIRKRLKNYSDLLTKEQLKKLERRHYTGWGRLSAELIHGIRNKESRKTILDYLIDDGNSNRNFMQLIND
DALSFKEEIAKAQVIGETDNLNQVVSDIAGSPAIKKGILQSLKIVDELVKIMGHQPENIVVEMARENQFT
NQGRRNSQQRLKGLTDSIKEFGSQILKEHPVENSQLQNDRLFYYLQNGRDMYTGEELDIDYLSQYDIDH
IIPQAFIKDNSIDNRVLTSSKENRGKSDDVPSKDVVRKMKSYWSKLLSAKLITQRKFDNLTKGERGGLTD
DDKAGFIKRQLVETRQITKHVARILDERFNTETDENNKKIRQVKIVTLKSNLVSNFRKEFELYKVREIND
YHHAHDAYLNAVIGKALLGVYPQLEPEFVYGDYPHFHGHKENKATAKKFFYSNIMNFFKKDDVRTDKNGE
IIWKKDEYISNIKKVLSYPQVNIVKKVEEQTGGFSKESILPKGDSDKLIPRKTKKFYWDTKKYGGFDSPI
VAYSILVIADIEKGKSKKLKTVKALVGVTIMEKMTFERDPVAFLERGYRNVQEENIIKLPKYSLFKLEN
GRKRLLASARELQKGNEIVLPNHLGTLLYHAKNIHKVDEPKHLDYVDKHKDEFKELLDVVSNFSKKYTLA
EGNLEKIKELYAQNNGEDLKELASSFINLLTFTAIGAPATFKFFDKNIDRKRYTSTTEILNATLIHQSIT
GLYETRIDLSKLGGD
```

**SEQ ID NO:5** amino acid sequence >WP_111681791.1 type II CRISPR RNA-guided endonuclease Cas9 [*Streptococcus dysgalactiae*]

```
MDKKYSIGLDIGTNSVGWAVITDDYKVPSKKFKVLGNTDRHSIKKNLIGALLFDSGETAEATRLKRTARR
RYTRRKNRIRYLQEIFSSEMSKVDDSFFHRLEESFLVEEDKKHERHPIFGNIVDEVAYHEKYPTIYHLRK
KLADSTDKADLRLIYLALAHMIKFRGHFLIEGDLNPDNSDMDKLFIQLVQTYNQLFEENPINASRVDAKA
ILSARLSKSRRLENLIAQLPGEKRNGLFGNLIALSLGLTPNFKSNFDLAEDAKLQLSKDTYDDDLDNLLA
QIGDQYADLFLAAKNLSDAILLSDILRVNSEITKAPLSASMIKRYDEHHQDLTLLKALVRQQLPEKYKEI
FFDQSKNGYAGYIDGGASQEEFYKFIKPILEKMDGTEELLAKLNREDLLRKQRTFDNGSIPHQIHLGELH
AILRRQEDFYPFLKDNREKIEKILTFRIPYYVGPLARGNSRFAWMTRKSEETITPWNFEEVVDKGASAQS
FIERMTNFDKNLPNEKVLPKHSLLYEYFTVYNELTKVKYVTEGMRKPAFLSGEQKKAIVDLLFKTNRKVT
VKQLKEDYFKKIECFDSVEISGVEDRFNASLGTYHDLLKIIKDKDFLDNEENEDILEDIVLTLTLFEDKE
MIEERLKKYANLFDDKVMKQLKRRHYTGWGRLSRKLINGIRDKQSGKTILDFLKSDGFANRNFMQLINDD
SLTFKEAIQKAQVSGQGHSLHEQIANLAGSPAIKKGILQSVKVVDELVKVMGHKPENIVIEMARENQTTQ
KGQKNSRERMKRIEEGIKELGSQILKEHPVENTQLQNEKLYLYYLQNGRDMYVDQELDINRLSDYDVDHI
VPQSFIKDDSIDNKVLTRSDKNRGKSDNVPSEEVVKKMKNYWRQLLNAKLITQRKFDNLTKAERGGLSEL
DKAGFIKRQLVETRQITKHVAQILDSRMNTKYDENDKLIREVKVITLKSKLVSDFRKDFQFYKVREINNY
HHAHDAYLNAVVGTALIKKYPKLESEFVYGDYKVYDVRKMIAKSEQEIGKATAKRFFYSNIMNFFKTEIT
LANGEIRKRPLIETNEETGEIVWDKGRDFATVRKVLSMPQVNIVKKTEVQTGALTNESIYARGSFDKLIS
RKHRFESSKYGGFGSPTVTYSVLVVAKSKVQDGKVKKIKTGKELIGITLLDKLVFEKNPLKFIEDKGYGN
VQIDKCIKLPKYSLFEFENGTRRMLASVMANNNSRGDLQKANEMFLPAKLVTLLYHAHKIESSKELEHEA
YILDHYNDLYQLLSYIERFASLYVDVEKNISKVKELFSNIESYSISEICSSVINLLTLTASGAPADFKFL
GTTIPRKRYGSPQSILSSTLIHSITGLYETRIDLSQLGGD
```

**SEQ ID NO:6** amino acid sequence >WP_037581760.1 type II CRISPR RNA-guided endonuclease Cas9 [*Streptococcus equi*]

```
MKKPYTIALDIGTNSVGWVVVTDDYRVPTKKMKVLGNTERKTIKKNLIGALLFDSGDTAEGTRLKRTARP
RYTRRKNRLRFLKEIFTEEMAKVDDGFFQRLEDSFYVLEDKEGNKHPIFANLADEVAYHKKYPTIYHLRK
ELVDNPQKADLRLIYLAVAHIIKFRGHFLIEGTLSSKNNNLQKSFDHLVDTYNLLFEEQRLLTEGINAKE
LLSAALSKSKRLENLISLIPGQKKTGIFGNIIALSLGLTPNFKANFGLSKDVKLQLAKDTYADDLDSLLA
QIGDQYADLFLAAKNLSDAILLSDILTESDEITRAPLSASMVKRYREHHKDLVTLKTLIKDQLPEKYQEI
FLDKTKNGYAGYIEGQVSQEEFYKYLKPILARLDGSEPLLLKIDREDFLRKQRTFDNGSIPHQIHLEELH
AILRRQEVFYPFLKDNRKKIESLLTFRIPYYVGPLARGHSRFAWVKRKFDGAIRPWNFEEIVDEEASAQI
FIEKMTKNDLYLPNEKVLPKHSLLYETFTVYNELTKVKYATEGMTRPQFLSADQKQAIVDLLFKTNRKVT
VKQLKENYFKKIECWDSVEITGVEDSFNASLGTYHDLLKIIQDKDFLDNPDNQKIIEDIILTLTLFEDKK
MISKRLDQYAHLFDKVVLNKLERHHYTGWGRLSGKLINGIRDKQSGKTILDFLKADGFANRNFMQLIHDS
ELSFIDEIAKAQVIGKTEYSKDLVGNLAGSPAIKKGISQTIKIVDELVKIMGYLPQQIVIEMARENQTTA
QGIKNARQRMRKLEETAKKLGSNILKEHPVDNSQLQNDKRYLYYLQNGKDMYTGDDLDIDYLSSYDIDHI
IPQSFIKNNSIDNKVLTSQGANRGKLDNVPSEAIVRKMKGYWQSLLRAGAISKQKFDNLTKAERGGLTQV
DKAGFIQLQLVETRQITKHVAQILDSRFNTEFDDHNKRIRKVHIITLKSKLVSDFRKEFGLYKIRDINHY
HHAHDAYLNAVVAKAILGKYPQLAPEFVYGDYPKYNSFKERQKATQKTLFYSNILKFFKDQESLHVNSDG
EEIWNANKHLPIIKNVLSIPQVNIVKKTEVQTGGFYKESILSKGNSDKLIPRKNNWDTRKYGGFDSPTVA
YSVLVIAKMEKGKAKVLKPVKEMVGITIMERIAFEENPVVFLEAKGYREIQEHLIIKLPKYSLFELENGR
RRLLASASELQKGNELFLPVDYMTFLYLAAHYHELTGSSEDVLRKKYFVERHLHYFDDIIQMINDFAERH
ILASSNLEKINHTYHNNSDLPVNERAENIINVFTFVALGAPAAFKFFDATIDRKRYTSTKEVLNATLIHQ
SVTGLYETRIDLSQLGEN
```

**SEQ ID NO:7** amino acid sequence >WP_061588516.1 type II CRISPR RNA-guided endonuclease Cas9 [*Streptococcus oralis*]

```
MNNKPYSIGLDIGTNSVGWAVITDDYKVPSKKMKVLGNTDKHFIKKNLLGALLFDEGTTAEDRRLKRTAR
RRYTRRKNRLRYLQEIFTEEMSKVDSNFFHRLDDSFLVPEDKRGSKYPIFATLEEEKEYHKNFPTIYHLR
KHLADSKEKADFRLIYLALAHMIKYRGHFLYEESFDIKNNDIQKIFNEFISIYDNTFEGSSLNGQNAQVE
AIFTDKISKSAKRERVLKLFPDEKSTGLFSEFLKLIVGNQADFKKHFDLEEKAPLQFSKDTYDEDLENLL
GQIGDDFADLFLVAKKLYDAILLSGILTVTDPSTKAPLSASMIERYENHQKDLATLKQFIKNNLPEKYDE
VFSDQSKDGYAGYIDGKTTQEAFYKYIKNLLSKLEGADYFLDKIEREDFLRKQRTFDNGSIPHQIHLQEM
NAIIRRQGEHYPFLQENKEKIEKILTFRIPYYVGPLARGNRDFAWLTRNSDQAIRPWNFEEVVDKARSAE
DFINKMTNYDLYLPEEKVLPKHSLLYETFAVYNELTKVKFIAEGLRDYQFLDSGQKKQIVTQLFKEKRKV
TEKDIIQYLHTVDGYDGIELKGIEKQFNASLSTYHDLLKIIKDKEFMDDSKNEAILENIVHTLTIFEDRE
MIRQHLTQYASIFDEKVIKALTRRHYTGWGKLSAKLINGICDKQTGDTILDYLIDDGEINRNFMQLIHDD
GLSFKEIIQKAQVVGKTDDVKQVVQELPGSPAIKKGILQSIKIVDELVKVMGHEPESIVIEMARENQTTA
RGKKNSQQRYKRIEDALKNLAPELDSNILKEHPTDNIQLQNDRLFLYYLQNGKDMYTGEALDINQLSSCD
IDHIIPQAFIKDDSLDNRVLTSSKDNRGKSDNVPSLEIVQKRKAFWQQLLDSKLISERKFNNLTKAERGG
LDERDKVGFIRRQLVETRQITKHVAQILDARFNTEVTEKDKKDRSVKIITLKSNLVSNFRKEFRLYKVRE
INDYHHAHDAYLNAVVAKAILKKYPKLEPEFVYGDYQKYDLKRYISRTKDPKEVEKATEKYFFYSNLLNF
FKEEVHYADGTIVKRENIEYSKDTGEIAWNKEKDFATIKKVLSLPQVNIVKKTEEQTVGQNGGLFDNNIV
SKKKVVDASKLTPIKSGLSPEKYGGYARPTIAYSVLVIADIEKGKAKKLKRIKEMVGITVQDKKKFEANP
IAYLEECGYKNINPNLIIKLPKYSLFEFNNGQRRLLASSIELQKGNELIVPYHFTALLYHAQRINKISEP
IHKQYVETHQSEFKELLTAIISLSKKYIQKPNVESLLQQAFDQSDKDIYQLSESFISLLKLISFGAPGTF
KFLGVEISQSNVRYQSVSSCFNATLIHQSITGLYETRIDLSKLGED
```

**SEQ ID NO:8** amino acid sequence >WP_042900171.1 type II CRISPR RNA-guided endonuclease Cas9 [*Streptococcus mitis*]

```
MNNNNYSIGLDIGTNSVGWAVITDDYKVPSKKMKVLGNTDKHFIKKNLIGALLFDEGTTAEDRRLKRTAR
RRYTRRKNRLRYLQEIFSPEISKVDSSFFHRLDDSFLVPEDKRGSKYPIFATLAEEKEYHKNFPTIYHLR
KQLADSKEKADLRLIYLALAHMIKYRGHFLYEESFDIKNNDIQKIFNEFISIYDNTFEGSSLSGQNAQVE
AIFTDKISKSAKRERVLKLFPDEKSTGLFSEFLKLIVGNQAEFKKHFDLEEKAPLQFSKDTYDDDLENLL
GQIGDGFAELFVAAKKLYDAILLSGILTVTDPSTKAPLSASMIERYENHQKDLAALKQFIQNNLQEKYDE
VFSDQSKDGYAGYINGKTTQEAFYKYIKNLLSKFEGSDYFLDKIEREDFLKKQRTFDNGSIPHQIHLQEM
NAIIRRQGEHYPFLQENKEKIKKILTFRIPYYVGPLARGNGDFAWLTRNSDQAIRPWNFEEIVDQASSAE
DFINKMTNYDLYLPEEKVLPKHSLLYETFAVYNELTKVKFIAEGLRDYQFLDSGQKKQIVNQLFKEKRKV
TEKDITQYLHNVDGYDGIELKGIEKQFNASLSTYHDLLKIIKDKAFMDDAENEATLENIIHTLTIFEDRE
MIKQRLAQYDSLFDEKVIKALIRRHYTGWGKLSAKLINGICDKTGKTILDYLIDDGYSNRNFMQLINDD
GLSFKDIIQKAQVVGRTNDVKQIVHELPGSPAIKKGILQSIKIVDELVKIMGHTPESIVIEMARENQTTA
RGKKNSQQRYKRIEDALKNLAPGLDSNILKEYPTDNIQLQNDRLFLYYLQNGKDMYTGEPLDINQLSSYD
IDHIVPQAFIKDDSLDNRVLTSSKDNRGKSDNVPSLEVVQKRKAFWQQLLDSKLISERKFNNLTKAERGG
LDERDKVGFIRRQLVETRQITKHVAQILDARFNTEVTEKDKKNRNVKIITLKSNLVSNFRKEFKLYKVRE
INDYHHAHDAYLNAVVAKAILKKYPKLEPEFVYGDYQKYDLKRYISRSKDPKDVEKATEKYFFYSNLLNF
FKEEVHYADGTIVKRENIEYSKDTGEIAWNKEKDFATIKKVLSLPQVNIVKKTEIQTHGLDRGKPRGLFN
SNPSPKPSEDSKENLVPIKQGLDPRKYGGYAGISNSYAVLVKAIIEKGAKKQQKTVLEFQGISILDKINF
EKNKENYLLEKGYIKILSTITLPKYSLFEFPDGTRRRLASILSTNNKRGEIHKGNELVISEKYTTLLYHA
KNINKTLEPEHLEYVEKHRNDFAKLLESVLDFNDKYVGALKNGERIRQAFIDWETVDIEKLCFSFIGPRN
SKNAGLFELTSQGSASDFEFLGVKIPRYRDYTPSSLLNATLIHQSITGLYETRIDLSKLGED
```

**SEQ ID NO:9** amino acid sequence >WP_003739838.1 type II CRISPR RNA-guided endonuclease Cas9 [*Listeria monocytogenes*]

```
MKNPYTIGLDIGTNSVGWAVLTDQYDLVKRKMKVAGNSDKKQIKKNFWGVRLFDEGETAADRRMNRTARR
RIERRRNRISYLQEIFALEMANIDANFFCRLNDSFYVDSEKRNSRHPFFATIEEEVAYHKNYRTIYHLRE
```

```
ELVNSSEKADLRLVYLALAHIIKYRGNFLIEGALDTKNTSVDGVYKQFIQTYNQVFISNIEEGTLAKMEE
NTTVADILAGKFTRKEKLERILQLYPGEKSTGMFAQFISLIVGSKGNFQKVFDLVEKTDIECAKDSYEED
LEALLAIIGDEYAELFVAAKNTYNAVVLSSIITVTDTETNAKLSASMIERFDAHEKDLSELKAFIKLHLP
KQYEEIFSNVAIDGYAGYIDGKTKQVDFYKYLKTLLENIEGADYFIAKIEEENFLRKQRTFDNGAIPHQL
HLEELEAILHQQAKYYPFLKEAYDKIKSLVTFRIPYFVGPLANGQSDFAWLTRKADGEIRPWNIEEKVDF
GKSAVDFIEKMTNKDTYLPKENVLPKHSLYYQKYMVYNELTKVRYIDDQGKTNYFSGQEKQQIFNDYFKQ
KRKVSKKDLEQFLRNMSHIESPTIEGLEDSFNSSYATYHDLLKVGIKQEVLENPLNTEMLEDIVKILTVF
EDKRMIKEQLQQFSDVLDGAVLKKLERRHYTGWGRLSAKLLVGIRDKQSHLTILDYLMNDDGLNRNLMQL
INDSNLSFKSIIEKEQVSTTDKDLQSIVADLAGSPAIKKGILQSLKIVDELVSIMGYPPQTIVVEMAREN
QTTVKGKNNSRPRYKSLEKAIKEFGSQILKEHPTDNQELRNNRLYLYYLQNGKDMYTGQELDIHNLSNYD
IDHIVPQSFITDNSIDNLVLTSSAGNREKGDDVPPLEIVRKRKVFWEKLFQGNLMSKRKFDYLTKAERGG
LTEADKATFIHRQLVETRQITKNVANILHQRFNNETDNHGNNMEQVRIVMLKSALVSQFRKQFQLYKVRE
VNDYHHAHDAYLNGVVANTLLKVYPQLEPEFVYGEYHQFDWFKANKATAKKQFYTNIMLFFAQKERIIDE
NGEILWDKKYLETIKKVLDYRQMNIVKKTEIQKGEFSKATIKPKGNSSKLIPRKENWDPMKYGGLDSPNM
AYAVIIEHAKGKKKVVFEKKIIRITIMERKAFEKDEKSFLEKQGYRQPKVLTKLPKYTLYECENGRRRML
ASANEAQKGNQQVLKGQLITLLHHAKNCEASDGKSLDYIESNREMFGELLAHVSEFAKRYTLADANLSKI
NQLFEQNKDNDIKVIAQSFVNLMAFNAMGAPASFKFFEATIERKRYTNLKELLSATIIYQSITGLYEARK
RLDG
```

**SEQ ID NO:10** amino acid sequence >WP_071131842.1 type II CRISPR RNA-guided endonuclease Cas9 [*Enterococcus timonensis*]

```
MGKDYTIGLDIGTNSVGWAVLRDDLDLVKKKMKVFGNTDKKALKKNFWGVSLFDEGQTAADARMKRTMRR
RLARRHQRIVFLQEEFFQKAMNEKDANFFHRLNESFLVEEDKEFNRHPIFGKLEEEKAYYKKYPTIYHLR
KELADSTQQADLRLVYLAMAHIIKYRGHFLIEGKLSTENTSVSETFKVFLDKFNEASKIADNELKLDTTI
DVEKVLTEKSSRSRKAENVLNFFPTEKKNDTFDQFLKMIVGNQGNFKKTFDLDEDAKLQFSKEDYDTELE
NLLGMAGDGYGDVFEAAKNAYNAVELSGILTVQDSLTKAKLSAGMIKRYDDHKEDLALLKKFFLNNLGYE
EYVSYFKGDGKKDNNGYASYIDGHTKQDDFYSYTKKMLDKVEGADYFLAKIDQEDFLRKQRTFDNGVIPH
QIHLEELKAIMEHQGEFYPFLKENFQKIVDLFNFRIPYYVGPLASKENHGRFAWLERNSDEPITPWNITE
VVDMNKSAEKFIERMTNFDTYLPNEKVLPKHSMLYEKFTVYNELTKVSYTDEQEKTHNFSSIEKEKIFKE
LFCKNRKVTKDRLQKFLYNEYNLENVTINGIENEFNAKLATYHDFLKLNVSPEMLNDPENEDMFEEIVKM
LTIFEDRKMLAKQLASFKSYFDEKTMKELVRRYYTGWGRLSAKLINGLYDQQTGKTVIDFLVMDDAPGKN
TNRNFMQLINDNMLSFKEEIQKAQKEVGTKNDLNQIVQELAGSPALKKGILQSLKIVDEIVDIMGYAPTN
IVIEMARENQTTGRGKINSQPRYKNLEKSLNEMQSKILKDYPTDNKAIQKDRLYLYYLQNGRDMYTGHDL
DINNLSNYDIDHIIPQSFIVDNSIDNRVLVSSKENRGKSDDVLNIDIVKSRKGFWEQLLHSKLMSKKKFD
NLTKAERGGITEDDKAGFIKRQLVETRQITKHVARILDERFNTEKDQTGKKIRTVRIVTLKSALTSQFRK
NYQIYKVREINDYHHAHDAYLNGVVANTLLKIYPQLEPEFVYGEYHRYDSFKENRATAKKNMYSNIMQFT
KKDVTLDKEGNGEILWDNKSVAMVKKVIDYRQMNIVKKTEIQRGGFSNETVLPKGPSDKLIPRKNNWDPA
KYGGVGSPTEAYSIIISYEKGKSKKVVKEIVGITIMQRKAFEENELGFLKTRGYENPKVLAKLPKYTLFE
FADGRRRLLASSKESQKGNQLVLSKDLNELVYHAKNSDKKSESLEFVTNNSTMFFDFLEYVDIFAQKYII
ATKNSERIQIVAENNKDSEGKDLATSFFNLLQFTAMGAPADFKFFNETIPRKRYSSTSELLNATIIYQSV
TGLYETRRNLGD
```

**SEQ ID NO:11** amino acid sequence >WP_082309079.1 type II CRISPR RNA-guided endonuclease Cas9 [*Streptococcus thermophilus*]

```
MTKPYSIGLDIGTNSVGWAVTTDNYKVPSKKMKVLGNTSKKYIKKNLLGVLLFDSGITAEGRRLKRTARR
RYTRRRNRILYLQEIFSTEMATLDDAFFQRLDDSFLVPDDKRDSKYPIFGNLVEEKAYHDEFPTIYHLRK
YLADSTKKADLRLVYLALAHMIKYRGHFLIEGEFNSKNNDIQKNFQDFLDTYNAIFESDLSLENSKQLEE
IVKDKISKLEKKDRILKLFPGEKNSGIFSEFLKLIVGNQADFRKCFNLDEKASLHFSKESYDEDLETLLG
YIGDDYSDVFLKAKKLYDAILLSGFLTVTDNETEAPLSSAMIKRYNEHKEDLALLKEYIRNISLKTYNEV
FKDDTKNGYAGYIDGKTNQEDFYVYLKKLLAKFEGADYFLEKIDREDFLRKQRTFDNGSIPYQIHLQEMR
AILDKQAKFYPFLAKNKERIEKILTFRIPYYVGPLARGNSDFAWSIRKRNEKITPWNFEDVIDKESSAEA
```

```
FINRMTSFDLYLPEEKVLPKHSLLYETFNVYNELTKVRFIAESMRDYQFLDSKQKKDIVRLYFKDKRKVT
DKDIIEYLHAIYGYDGIELKGIEKQFNSSLSTYHDLLNIINDKEFLDDSSNEAIIEEIIHTLTIFEDREM
IKQRLSKFENIFDKSVLKKLSRRHYTGWGKLSAKLINGIRDEKSGNTILDYLIDDGISNRNFMQLIHDDA
LSFKKKIQKAQIIGDEDKGNIKEVVKSLPGSPAIKKGILQSIKIVDELVKVMGGRKPESIVVEMARENQY
TNQGKSNSQQRLKRLEKSLKELGSKILKENIPAKLSKIDNNALQNDRLYLYYLQNGKDMYTGDDLDIDRL
SNYDIDHIIPQAFLKDNSIDNKVLVSSASNRGKSDDVPSLEVVKKRKTFWYQLLKSKLISQRKFDNLTKA
ERGGLSPEDKAGFIQRQLVETRQITKHVARLLDEKFNNKKDENNRAVRTVKIITLKSTLVSQFRKDFELY
KVREINDFHHAHDAYLNAVVASALLKKYPKLEPEFVYGDYPKYNSFRERKSATEKVYFYSNIMNIFKKSI
SLADGRVIERPLIEVNEETGESVWNKESDLATVRRVLSYPQVNVVKKVEEQNHGLDRGKPKGLFNANLSS
KPKPNSNENLVGAKEYLDPKKYGGYAGISNSFAVLVKGTIEKGAKKKITNVLEFQGISILDRINYRKDKL
NFLLEKGYKDIELIIELPKYSLFELSDGSRRMLASILSTNNKRGEIHKGNQIFLSQKFVKLLYHAKRISN
TINENHRKYVENHKKEFEELFYYILEFNENYVGAKKNGKLLNSAFQSWQNHSIDELCSSFIGPTGSERKG
LFELTSRGSAADFEFLGVKIPRYRDYTPSSLLKDATLIHQSVTGLYETRIDLAKLGEG
```

**SEQ ID NO:12** amino acid sequence >WP_049523028.1 type II CRISPR RNA-guided endonuclease Cas9 [*Streptococcus parasanguinis*]

```
MKKPYSIGLDIGTNSVGWAVITDDYKVPAKKMKVLGNTNKESIKKNLIGALLFDAGNTAADRRLKRTARR
RYTRRRNRILYLQEIFAAEMNKVDESFFHRLDDSFLVPEDKRGSKYPIFGTLEEEKEYHKQFPTIYYLRK
ILADSKEKVDLRLIYLALAHIIKYRGHFLYEDSFDIKNNDIQKIFNEFTILYDNTFEESSLSKGNAQVEE
IFTDKISKSAKRDRVLKLFPDEKSTGLFSEFLKLIVGNQADFKKHFDLEEKAPLQFSKDTYEEDLESLLG
QIGDVYADLFVVAKKLYDAILLAGILSVKDPGTKAPLSASMIERYDNHQNDLSALKQFVRRNLPEKYAEV
FSDDSKDGYAGYIDGKTTQEGFYKYIKNLISKIEGAEYFLEKIEREDFLRKQRTFDNGSIPHQIHLQEMN
AILRHQGEYYPFLKENKDKIEQILTFRIPYYVGPLARGNSDFAWLSRNSDEAIRPWNFEEMVDKSSSAED
FIHRMTNYDLYLPEEKVLPKHSLLYETFTVYNELTKVKYIAEGMKDYQFLDSGQKKQIVNQLFKEKRKVT
EKDIIHYLHNVDGYDGIELKGIEKHFNSSLSTYHDLLKIIKDKEFMDDPKNEEIFENIVHTLTIFEDRVM
IKQRLNQYDSIFDEKVIKALTRRHYTGWGKLSAKLINGIRDKKTSKTILDYLIDDGYSNRNFMQLINDDG
LSFKETIQKAQVVGETNDVKQVVQELPGSPAIKKGILQSIKIVDELVKVMGHAPESVVIEMARENQTTNK
GKSKSQQRLKTLSDAISELGSNILKEHPTDNIQLQNDRLFLYYLQNGKDMYTGEALDINQLSNYDIDHII
PQAFIKDDSLDNRVLTSSKDNRGKSDNVPSLEIVEKMKGFWQQLLDSKLISERKFNNLTKAERGGLDERD
KVGFIKRQLVETRQITKHVAQILDDRFNAEVNEKNQKLRSVKIITLKSNLVSNFRKEFGLYKVREINDYH
HAHDAYLNAVVAKAILKKYPKLEPEFVYGDYQKYDLKRYISRTKDPKEIEKATEKYFFYSNLLNFFKDKV
YYADGTIIQRGNVEYSKDTGEIAWNKKRDFAIVRKVLSYPQVNIVKKTEEQTGGFSKESILPKGNSDKLI
PRKTKNVQLDTTKYGGFDSPVIAYSILLVADVEKGKSKKLKTVKSLIGITIMEKVKFEANPVAFLEGKGY
QNVVEENIIRLPKYSLFELENGRRRMLASAKELQKGNEMVLPSYLIALLYHAKRIQKKDEPEHLEYIKQH
HSEFNDLLNFVSEFSQKYVLAESNLEKIKNLYIDNEQTNMEEIANSFINLLTFTAFGAPAVFKFFGKDIE
RKRYSTVTEILKATLIHQSLTGLYETRIDLSKLGEE
```

**SEQ ID NO:13** amino acid sequence of OptiHF-SpCas9 protein (Base sequence SEQ ID NO:1, residues 695, 848, and 926 substituted with Alanine, residue 923 substituted with Methionine, and residue 924 substituted with Valine)

MDKKYSIGLDIGTNSVGWAVITDEYKVPSKKFKVLGNTDRHSIKKNLIGALLFDSGETA

EATRLKRTARRRYTRRKNRICYLQEIFSNEMAKVDDSFFHRLEESFLVEEDKKHERHPIF

GNIVDEVAYHEKYPTIYHLRKKLVDSTDKADLRLIYLALAHMIKFRGHFLIEGDLNPDNS

DVDKLFIQLVQTYNQLFEENPINASGVDAKAILSARLSKSRRLENLIAQLPGEKKNGLFG

NLIALSLGLTPNFKSNFDLAEDAKLQLSKDTYDDDLDNLLAQIGDQYADLFLAAKNLSD

AILLSDILRVNTEITKAPLSASMIKRYDEHHQDLTLLKALVRQQLPEKYKEIFFDQSKNGY

AGYIDGGASQEEFYKFIKPILEKMDGTEELLVKLNREDLLRKQRTFDNGSIPHQIHLGEL
HAILRRQEDFYPFLKDNREKIEKILTFRIPYYVGPLARGNSRFAWMTRKSEETITPWNFEE
VVDKGASAQSFIERMTNFDKNLPNEKVLPKHSLLYEYFTVYNELTKVKYVTEGMRKPA
FLSGEQKKAIVDLLFKTNRKVTVKQLKEDYFKKIECFDSVEISGVEDRFNASLGTYHDLL
KIIKDKDFLDNEENEDILEDIVLTLTLFEDREMIEERLKTYAHLFDDKVMKQLKRRRYTG
WGRLSRKLINGIRDKQSGKTILDFLKSDGFANRNFMÁLIHDDSLTFKEDIQKAQVSGQG
DSLHEHIANLAGSPAIKKGILQTVKVVDELVKVMGRHKPENIVIEMARENQTTQKGQKN
SRERMKRIEEGIKELGSQILKEHPVENTQLQNEKLYLYYLQNGRDMYVDQELDINRLSD
YDVDHIVPQSFLÄDDSIDNKVLTRSDKNRGKSDNVPSEEVVKKMKNYWRQLLNAKLIT
QRKFDNLTKAERGGLSELDKAGFIKRQLVMVRÄITKHVAQILDSRMNTKYDENDKLIRE
VKVITLKSKLVSDFRKDFQFYKVREINNYHHAHDAYLNAVVGTALIKKYPKLESEFVYG
DYKVYDVRKMIAKSEQEIGKATAKYFFYSNIMNFFKTEITLANGEIRKRPLIETNGETGEI
VWDKGRDFATVRKVLSMPQVNIVKKTEVQTGGFSKESILPKRNSDKLIARKKDWDPKK
YGGFDSPTVAYSVLVVAKVEKGKSKKLKSVKELLGITIMERSSFEKNPIDFLEAKGYKE
VKKDLIIKLPKYSLFELENGRKRMLASAGELQKGNELALPSKYVNFLYLASHYEKLKGS
PEDNEQKQLFVEQHKHYLDEIIEQISEFSKRVILADANLDKVLSAYNKHRDKPIREQAENI
IHLFTLTNLGAPAAFKYFDTTIDRKRYTSTKEVLDATLIHQSITGLYETRIDLSQLGGD

References

1 Bornscheuer, U. T. et al. Engineering the third wave of biocatalysis. Nature 485, 185-194, doi:10.1038/nature11117 (2012).

2 Weinreich, D. M., Delaney, N. F., Depristo, M. A. & Hartl, D. L. Darwinian evolution can follow only very few mutational paths to fitter proteins. Science 312, 111-114, doi:10.1126/science.1123539 (2006).

3 Slaymaker, I. M. et al. Rationally engineered Cas9 nucleases with improved specificity. Science 351, 84-88, doi:10.1126/science.aad5227 (2016).

4 Kleinstiver, B. P. et al. High-fidelity CRISPR-Cas9 nucleases with no detectable genome-wide off-target effects. Nature 529, 490-495, doi:10.1038/nature16526 (2016).

5 Chen, J. S. et al. Enhanced proofreading governs CRISPR-Cas9 targeting accuracy. Nature 550, 407-410, doi:10.1038/nature24268 (2017).

6 Casini, A. et al. A highly specific SpCas9 variant is identified by in vivo screening in yeast. Nat Biotechnol, doi:10.1038/nbt.4066 (2018).

7 Hu, J. H. et al. Evolved Cas9 variants with broad PAM compatibility and high DNA specificity. Nature, doi:10.1038/nature26155 (2018).

8 Packer, M. S. & Liu, D. R. Methods for the directed evolution of proteins. Nat Rev Genet 16, 379-394, doi:10.1038/nrg3927 (2015).

9 Romero, P. A. & Arnold, F. H. Exploring protein fitness landscapes by directed evolution. Nat Rev Mol Cell Biol 10, 866-876, doi:10.1038/nrm2805 (2009).

10 Gasperini, M., Starita, L. & Shendure, J. The power of multiplexed functional analysis of genetic variants. Nat Protoc 11, 1782-1787, doi:10.1038/nprot.2016.135 (2016).

11 Fowler, D. M. & Fields, S. Deep mutational scanning: a new style of protein science. Nat Methods 11, 801-807, doi:10.1038/nmeth.3027 (2014).

12 Ma, S., Saaem, I. & Tian, J. Error correction in gene synthesis technology. Trends Biotechnol 30, 147-154, doi:10.1016/j.tibtech.2011.10.002 (2012).

13 Kosuri, S. & Church, G. M. Large-scale de novo DNA synthesis: technologies and applications. Nat Methods 11, 499-507, doi:10.1038/nmeth.2918 (2014).

14 Engler, C., Kandzia, R. & Marillonnet, S. A one pot, one step, precision cloning method with high throughput capability. PLoS One 3, e3647, doi:10.1371/journal.pone.0003647 (2008).

15 Gibson, D. G. et al. Enzymatic assembly of DNA molecules up to several hundred kilobases. Nat Methods 6, 343-345, doi:10.1038/nmeth.1318 (2009).

16 Trudeau, D. L., Smith, M. A. & Arnold, F. H. Innovation by homologous recombination. Curr Opin Chem Biol 17, 902-909, doi:10.1016/j.cbpa.2013.10.007 (2013).

17 Wong, A. S., Choi, G. C., Cheng, A. A., Purcell, O. & Lu, T. K. Massively parallel high-order combinatorial genetics in human cells. Nat Biotechnol 33, 952-961, doi:10.1038/nbt.3326 (2015).

18 Wong, A. S. et al. Multiplexed barcoded CRISPR-Cas9 screening enabled by CombiGEM. Proc Natl Acad Sci USA 113, 2544-2549, doi:10.1073/pnas.1517883113 (2016).

19 Cheng, A. A., Ding, H. & Lu, T. K. Enhanced killing of antibiotic-resistant bacteria enabled by massively parallel combinatorial genetics. Proc Natl Acad Sci USA 111, 12462-12467, doi:10.1073/pnas.1400093111 (2014).

20 Doudna, J. A. & Charpentier, E. Genome editing. The new frontier of genome engineering with CRISPR-Cas9. Science 346, 1258096, doi:10.1126/science.1258096 (2014).

21 Hsu, P. D., Lander, E. S. & Zhang, F. Development and applications of CRISPR-Cas9 for genome engineering. Cell 157, 1262-1278, doi:10.1016/j.cell.2014.05.010 (2014).

22 Mali, P., Esvelt, K. M. & Church, G. M. Cas9 as a versatile tool for engineering biology. Nat Methods 10, 957-963, doi:10.1038/nmeth.2649 (2013).

23 Barrangou, R. & Horvath, P. A decade of discovery: CRISPR functions and applications. Nat Microbiol 2, 17092, doi:10.1038/nmicrobiol.2017.92 (2017).

24 Kim, S., Bae, T., Hwang, J. & Kim, J. S. Rescue of high-specificity Cas9 variants using sgRNAs with matched 5' nucleotides. Genome Biol 18, 218, doi:10.1186/s13059-017-1355-3 (2017).

25 Kulcsar, P. I. et al. Crossing enhanced and high fidelity SpCas9 nucleases to optimize specificity and cleavage. Genome Biol 18, 190, doi:10.1186/s13059-017-1318-8 (2017).

26 Zhang, D. et al. Perfectly matched 20-nucleotide guide RNA sequences enable robust genome editing using high-fidelity SpCas9 nucleases. Genome Biol 18, 191, doi:10.1186/s13059-017-1325-9 (2017).

27 Kato-Inui, T., Takahashi, G., Hsu, S. & Miyaoka, Y. Clustered regularly interspaced short palindromic repeats (CRISPR)/CRISPR-associated protein 9 with improved proofreading enhances homology-directed repair. Nucleic Acids Res 46, 4677-4688, doi:10.1093/nar/gky264 (2018).

28 Sternberg, S. H., LaFrance, B., Kaplan, M. & Doudna, J. A. Conformational control of DNA target cleavage by CRISPR-Cas9. Nature 527, 110-113, doi:10.1038/nature15544 (2015).

29 Singh, D. et al. Mechanisms of improved specificity of engineered Cas9s revealed by single-molecule FRET analysis. Nat Struct Mol Biol 25, 347-354, doi:10.1038/s41594-018-0051-7 (2018).

30 Kato-Inui, T., Takahashi, G., Hsu, S. & Miyaoka, Y. Clustered regularly interspaced short palindromic repeats (CRISPR)/CRISPR-associated protein 9 with improved proofreading enhances homology-directed repair. Nucleic Acids Res, doi:10.1093/nar/gky264 (2018).

31 Fu, Y. et al. High-frequency off-target mutagenesis induced by CRISPR-Cas nucleases in human cells. Nat Biotechnol 31, 822-826, doi:10.1038/nbt.2623 (2013).

32 Lee, J. K. et al. Directed evolution of CRISPR-Cas9 to increase its specificity. Nat Commun 9, 3048, doi:10.1038/s41467-018-05477-x (2018).

33 Haeussler, M. et al. Evaluation of off-target and on-target scoring algorithms and integration into the guide RNA selection tool CRISPOR. Genome Biol 17, 148, doi:10.1186/s13059-016-1012-2 (2016).

34 Fu, Y., Sander, J. D., Reyon, D., Cascio, V. M. & Joung, J. K. Improving CRISPR-Cas nuclease specificity using truncated guide RNAs. Nat Biotechnol 32, 279-284, doi:10.1038/nbt.2808 (2014).

35 Vakulskas, C. A. et al. A high-fidelity Cas9 mutant delivered as a ribonucleoprotein complex enables efficient gene editing in human hematopoietic stem and progenitor cells. Nat Med 24, 1216-1224, doi:10.1038/s41591-018-0137-0 (2018).

36 Ran, F. A. et al. In vivo genome editing using Staphylococcus aureus Cas9. Nature 520, 186-191, doi:10.1038/nature14299 (2015).

37 Zetsche, B. et al. Cpf1 is a single RNA-guided endonuclease of a class 2 CRISPR-Cas system. Cell 163, 759-771, doi:10.1016/j.cell.2015.09.038 (2015).

38 Komor, A. C., Kim, Y. B., Packer, M. S., Zuris, J. A. & Liu, D. R. Programmable editing of a target base in genomic DNA without double-stranded DNA cleavage. Nature 533, 420-424, doi:10.1038/nature17946 (2016).

39 Nishida, K. et al. Targeted nucleotide editing using hybrid prokaryotic and vertebrate adaptive immune systems. Science 353, doi:10.1126/science.aaf8729 (2016).

40 Gaudelli, N. M. et al. Programmable base editing of A∗T to G∗C in genomic DNA without DNA cleavage. Nature 551, 464-471, doi:10.1038/nature24644 (2017).

41 Li, X. et al. Base editing with a Cpfl-cytidine deaminase fusion. Nat Biotechnol 36, 324-327, doi:10.1038/nbt.4102 (2018).

42 Honma, K. et al. RPN2 gene confers docetaxel resistance in breast cancer. Nat Med 14, 939-948, doi:10.1038/nm.1858 (2008).

43 Kampmann, M., Bassik, M. C. & Weissman, J. S. Functional genomics platform for pooled screening and generation of mammalian genetic interaction maps. Nat Protoc 9, 1825-1847, doi:10.1038/nprot.2014.103 (2014).

44 Olson, C. A., Wu, N. C. & Sun, R. A comprehensive biophysical description of pairwise epistasis throughout an entire protein domain. Curr Biol 24, 2643-2651, doi:10.1016/j.cub.2014.09.072 (2014).

45 Aakre, C. D. et al. Evolving new protein-protein interaction specificity through promiscuous intermediates. Cell 163, 594-606, doi:10.1016/j.cell.2015.09.055 (2015).

46 Guschin, D. Y. et al. A rapid and general assay for monitoring endogenous gene modification. Methods Mol Biol 649, 247-256, doi:10.1007/978-1-60761-753-2_15 (2010).

47 Tsai, S. Q. et al. GUIDE-seq enables genome-wide profiling of off-target cleavage by CRISPR-Cas nucleases. Nat Biotechnol 33, 187-197, doi:10.1038/nbt.3117 (2015).

48 Tsai, S. Q., Topkar, V. V., Joung, J. K. & Aryee, M. J. Open-source guideseq software for analysis of GUIDE-seq data. Nat Biotechnol 34, 483, doi:10.1038/nbt.3534 (2016).

The following paragraphs are not claims, but represent preferred aspects and embodiments of the invention.

1. A DNA construct comprising from 5' to 3':

a first recognition site for a first type IIS restriction enzyme,

a DNA element,

a first and a second recognition sites for a second type IIS restriction enzyme,

a barcode uniquely assigned to the DNA element, and

a second recognition site for the first type IIS restriction enzyme.

2. The DNA construct of claim 1, which is a DNA vector.

3. A library comprising two or more of the DNA constructs of paragraph 1.

4. A DNA construct comprising from 5' to 3':

a recognition site for a first type IIS restriction enzyme,

a plurality of DNA elements,

a primer binding site, and

a plurality of barcodes each uniquely assigned to one of the plurality of DNA elements, and a recognition site for a second type IIS restriction enzyme,

wherein the plurality of DNA elements are connected to each other to form a coding sequence for a protein without any extraneous sequence at any connection point between any two of the plurality of DNA elements, and wherein the plurality of barcodes are placed in the reverse order of their assigned DNA elements.

5. The DNA construct of paragraph 4, which is a DNA vector.

6. The DNA construct of any one of paragraphs 1, 2, 4 and 5, wherein the first type IIS restriction enzyme and the second type IIS restriction enzyme generate compatible ends upon cleaving a DNA molecule.

7. The DNA construct of any one of paragraphs 1, 2, 4 and 5, wherein the first type IIS restriction enzyme is BsaI and the second type IIS restriction enzyme is BbsI.

8. A method for generating a combinatorial genetic construct, comprising:

(a) cleaving a first DNA vector of paragraph 2 with the first type IIS restriction enzyme to release a first DNA fragment comprising the first DNA segment, the first and second recognition sites for a second type IIS restriction enzyme, and the first barcode flanked by a first and a second ends generated by the first type IIS restriction enzyme;

(b) cleaving an initial expression vector comprising a promoter with the second type IIS restriction enzyme to linearize the initial expression vector near 3' end of the promoter and generate two ends that are compatible with the first and second ends of DNA fragment of (a);

(c) annealing and ligating the first DNA fragment of (a) into the linearized expression vector of (b) to form a 1-way composite expression vector in which the first DNA fragment and the first barcode are operably linked to the promoter at its 3' end;

(d) cleaving a second DNA vector of paragraph 2 with the first type IIS restriction enzyme to release a second DNA fragment comprising the second DNA segment, the first and second recognition sites for the second type IIS restriction enzyme, and the second barcode flanked by a first and a second ends generated by the first type IIS restriction enzyme;

(e) cleaving the composite expression vector of (c) with the second type IIS restriction enzyme to linearize the composite expression vector between the first DNA element and the first barcode and generate two ends that are compatible with the first and second ends of DNA fragment of (d);

(f) annealing and ligating the second DNA fragment of (d) into linearized composite expression vector of (e) between the first DNA element and the first barcode to form a 2-way composite expression vector in which the first DNA fragment, the second DNA fragment, the second barcode, and the first barcode are operably linked in this order to the promoter at its 3' end,

wherein the first and second DNA elements encode the first and second segments of a pre-selected protein from its N-terminus that are immediately adjacent to each other, and wherein the first and second DNA fragments are joined to each other in the 2-way composite expression vector without any extraneous nucleotide sequence resulting in any amino acid residue not found in the pre-selected protein, and wherein each of the first and second DNA elements comprises one or more mutations.

9. The method of paragraph 6, wherein steps (d) to (f) are repeated until the nth time to incorporate the nth DNA fragment comprising the nth DNA element, the first and second recognition sites for the second type IIS restriction enzyme, and the nth barcode into an n-way composite expression vector, the nth DNA element encoding for the nth or the second to the last segment of the pre-selected protein from its C-terminus, further comprising the steps of:

(x) providing a final DNA vector, which comprises between a first and a second recognition sites for a first type IIS restriction enzyme, a (n+1)th DNA element, a primer-binding site, and a (n+1)th barcode;

(y) cleaving the final DNA vector with the first type IIS restriction enzyme to release a final DNA fragment comprising from 5' to 3': the (n+1)th DNA element, the primer-binding site, and the (n+1)th barcode, flanked by a first and a second ends generated by the first type IIS restriction enzyme;

(z) annealing and ligating the final DNA fragment into the n-way composite expression vector, which is produced after steps (d) to (f) are repeated for the nth time and has been linearized by the second type IIS restriction enzyme, to form a final composite expression vector,

wherein the first, second, and so on up to the nth and the (n+1)th DNA elements encode the first, second, and so on up to the nth and the last segments of the pre-selected protein from its N-terminus that are immediately adjacent to each other, and wherein the first, second, and so on up to the nth and the last DNA fragments are joined to each other in the final composite expression vector without any extraneous nucleotide sequence resulting in any amino acid residue not found in the pre-selected protein, and wherein each of the DNA elements comprises one or more mutations.

10. The method of paragraph 8 or 9, wherein the first type IIS restriction enzym and the second type IIS restriction enzyme generate compatible ends upon cleaving a DNA molecule.

11. The method of paragraph 8 or 9, wherein the first type IIS restriction enzym is BsaI and the second type IIS restriction enzyme is BbsI.

12. A library comprising two or more of the final composite expression vectors generated by the method of paragraph 9.

13. A polypeptide comprising the amino acid sequence set forth in any one of SEQ ID NOs:1 and 4-13, wherein residue corresponding to residue 1003 of SEQ ID NO:1 is substituted and residue corresponding to residue 661 of SEQ ID NO:1 is substituted.

14. The polypeptide of paragraph 13, wherein the residue corresponding to residue 1003 of SEQ ID NO:1 is substituted with Histidine and the residue corresponding to residue 661 of SEQ ID NO:1 is substituted with Alanine.

15. The polypeptide of paragraph 14, comprising the amino acid sequence set forth in SEQ ID NO:1, wherein residue 1003 is substituted with Histidine and residue 661 is substituted with Alanine, optionally further comprising a substitution with Alanine at residue 926.

16. The polypeptide of paragraph 13, wherein the residues corresponding to residues 695, 848, and 926 of SEQ ID NO:1 are substituted with Alanine, the residue corresponding to residue 923 of SEQ ID NO:1 is substitued with Methionine, and the residue corresponding to residue 924 of SEQ ID NO:1 is substituted with Valine.

17. The polypeptide of paragraph 16, comprising the amino acid sequence set forth in SEQ ID NO:1, wherein the residues corresponding to residues 695, 848, and 926 of SEQ ID NO:1 are substituted with Alanine, the residue corresponding to residue 923 of SEQ ID NO:1 is substitued with Methionine, and the residue corresponding to residue 924 of SEQ ID NO:1 is substituted with Valine.

18. A composition comprising the polypeptide of paragraph 13 and a physiologically acceptable excipient.

19. A nucleic acid comprising a polynucleotide sequence encoding the polypeptide of any one of paragraphs 13-17.

20. A composition comprising the nucleic acid of paragraph 17 and a physiologically acceptable excipient..

21. An expression cassette comprising a promoter operably linked to a polynucleotide sequence encoding the polypeptide of any one of paragraphs 13-17.

22. A vector comprising the expression cassette of paragraph 21.

23. The vector of paragraph 22, which is a viral vector.

24. A host cell comprising the expression cassette of claim 19 or the polypeptide of any one of paragraphs 13-17.

25. A method for cleaving a DNA molecule at a target site, comprising contacting the DNA molecule comprising the target DNA site with the polypeptide of any one of paragraphs 13-17. and a short guide RNA (sgRNA) that specifically binds the target DNA site, thereby causing the DNA molecule to be cleaved at the target DNA site.

26. The method of paragraph 25, wherein the DNA molecule is a genomic DNA within a live cell, and wherein the cell has been transfected with polynucleotide sequences encoding the sgRNA and the polypeptide.

27. The method of paragraph 26, wherein the cell has been transfected with a first vector encoding the sgRNA and a second vector encoding the polypeptide.

28. The method of paragraph 26, wherein the cell has been transfected with a vector encoding both the sgRNA and the polypeptide.

29. The method of paragraph 27, wherein each of the first and second vectors is a viral vector.

30. The method of paragraph 28, wherein the vector is a viral vector.

31. The method of paragraph 29 or 30, wherein the viral vector is a retroviral vector.

32. The method of paragraph 31, wherein the retroviral vector is a lentviral vector.

**Claims**

1. A polypeptide comprising the amino acid sequence set forth in any one of SEQ ID NOs: 1 and 4-13, wherein a:

   (a) residue corresponding to residue 1003 of SEQ ID NO:1 is substituted with histidine and a residue corresponding to residue 661 of SEQ ID NO:1 is substituted with alanine;
   (b) residue corresponding to residue 695 of SEQ ID NO:1 is substituted with alanine, a residue corresponding to residue 848 of SEQ ID NO:1 is substituted with alanine, a residue corresponding to residue 923 of SEQ ID NO:1 is substituted with methionine, a residue corresponding to residue 924 of SEQ ID NO:1 is substituted with valine, and a residue corresponding to residue 926 of SEQ ID NO:1 is substituted with alanine;
   (c) residue corresponding to residue 661 of SEQ ID NO:1 is substituted with alanine, and a residue corresponding to residue 1060 of SEQ ID NO:1 is substituted with alanine;
   (d) a residue corresponding to residue 661 of SEQ ID NO:1 is substituted with alanine, and a residue corresponding to residue 695 of SEQ ID NO:1 is substituted with alanine;
   (e) residue corresponding to residue 661 of SEQ ID NO:1 is substituted with alanine, a residue corresponding to residue 926 of SEQ ID NO:1 is substituted with alanine, and a residue corresponding to residue 1003 of SEQ ID NO:1 is substituted with histidine;
   (f) residue corresponding to residue 661 of SEQ ID NO:1 is substituted with alanine, a residue corresponding to residue 848 of SEQ ID NO:1 is substituted with alanine, a residue corresponding to residue 923 of SEQ ID NO:1 is substituted with histidine, a residue corresponding to residue 924 of SEQ ID NO:1 is substituted with leucine, and a residue corresponding to residue 1003 of SEQ ID NO:1 is substituted with histidine; or
   (g) the amino acid sequence comprises substitutions consisting of a residue corresponding to residue 661 of SEQ ID NO:1 substituted with alanine, and the amino acid sequence consists of a residue corresponding to residue 926 of SEQ ID NO:1 substituted with alanine;

2. A nucleic acid comprising a polynucleotide sequence encoding the polypeptide of claim 1.

3. A composition comprising the polypeptide of claim 1, or the nucleic acid of claim 2 and a physiologically acceptable excipient.

4. An expression cassette comprising a promoter operably linked to a polynucleotide sequence encoding the polypeptide of claim 1.

5. A vector comprising the expression cassette of claim 4; preferably wherein the vector is a viral vector.

6. A host cell comprising the expression cassette of claim 4 or the polypeptide of claim 1.

7. An in vitro method for cleaving a DNA molecule at a target site, comprising contacting the DNA molecule comprising the target DNA site with the polypeptide of claim 1, and a short guide RNA (sgRNA) that specifically binds the target DNA site, thereby causing the DNA molecule to be cleaved at the target DNA site.

8. A polypeptide according to claim 1 and a short guide RNA (sgRNA) that specifically binds a target DNA site for use in a method for cleaving a DNA molecule at a target site, comprising contacting the DNA molecule comprising the target DNA site with the polypeptide of claim 1, and a short guide RNA (sgRNA) that specifically binds the target DNA site, thereby causing the DNA molecule to be cleaved at the target DNA site.

9. The method of claim 7 or polypeptide for use according to claim 8, wherein the DNA molecule is a genomic DNA within a live cell, and wherein the cell has been transfected with polynucleotide sequences encoding the sgRNA and the polypeptide.

10. The method of claim 9 or the polypeptide for use according to claim 9, wherein the cell has been transfected with a first vector encoding the sgRNA and a second vector encoding the polypeptide.

11. The method of claims 10 or polypeptide for use according to claim 10 wherein each of the first and second vectors is a viral vector, for example a retroviral vector, more particularly a lentiviral vector.

12. The method of claims 7 to 11 or polypeptide for use according to any of claims 9 to 11,wherein the cell has been transfected with a vector encoding both the sgRNA and the polypeptide.

# Figure 1a

**Figure 1b**

# Figure 2a

## Figure 2b

**Figure 3a**

**Figure 3b**

# Figure 4

**Figures 5a and 5b**

## Figure 5c

# Figure 6

Combinatorial golden-gate strategy without barcode

The high error rate of high-throughput long-read sequencing poses challenges to discriminate random mutations from sequencing errors, and to verify if they are the intended mutated sequences (e.g., the nucleotides that code for 8 substituted amino acid residues in this example). It also has lower throughput and higher cost per base when compared to short-read sequencing. Alternatively, a huge number of clonal isolates is to be selected for Sanger sequencing over the whole mutated region (e.g., ~1.2 kb in this example). This approach is practically infeasible for large library screening.

Combinatorial golden-gate strategy with random-sequence barcode

A look-up table that links up the identity of mutation combinations spanning over the protein-coding sequence and the barcode (e.g., ~2.2 kb in this study) is needed. The drawback of using this strategy lies in the requirement of high-throughput long-read sequencing that has a high error rate, or the need to profile a massive number of clonal isolates one-by-one using Sanger sequencing, for the creation of a look-up table.

CombiSEAL

CombiSEAL modularizes a single protein into composable parts and create seamless barcoded combinatorial libraries of the protein variants from mutagenized parts. This method enables high-throughput sequencing of short barcode sequences (i.e., ~50 bp) specifying predetermined mutations at defined positions of the protein to analyze massive number of mutated sequences for the rapid establishment of sequence–activity relationship. This method circumvents the need to perform long-read sequencing and select clonal isolates, which offers a systematic, highly scalable, and cost-effective approach for engineering proteins.

(Diagrams not in scale)

## Figure 7a-b

**Figure 7c**

**Figure 8**

**Figure 9**

# Figure 10

**Figure 11**

# Figure 12

**Figure 13**

**Figure 14**

**Figure 15**

Figure 16

Figure 17

**Figure 18**

**Multiple Sequence Alignment – Comparision of Cas9 homologues of *Streptococcus pyogenes***

```
S.pyogenes          1 -DKSGASE
S.dysgalactiae      1 -DKSFSASE
S.equi              1 -KPVTTASD
S.mutans            1 -KPASHASN
S.oralis            1 MNNPSFAET
S.mitis             1 MNNNNSFAET
S.parasanguinis     1 -KPANESAAN
S.thermophilus      1 -TPTNSSYVSI
L.monocytogenes     1 -KNPQDVKAQFWVREE
E.timonensis        1 -GDRLDVKFAFWVSEQ
consensus           1   . . *.*.**********.*..*..... .*.**.**.... .***..* .*** * **


S.pyogenes         60 EATCSNAK-DSHEKHEH
S.dysgalactiae     60 EATRSSSK-DSHEKHEH
S.equi             60 EGTPRKTEAK-DGQYLEGNH
S.mutans           60 EDRLSEGK-DSHTDNFDSH
S.oralis           61 EDRRTESK-SNHPRGSY
S.mitis            61 EDRRSPSK-SSHPRGSY
S.parasanguinis    60 ADRLAANK-ESHPRGSY
S.thermophilus     60 EGRLSTAT-DAQPRDSY
L.monocytogenes    60 ADRNIESALAN-ANCNYDSRNSHF
E.timonensis       60 ADAMLAVEFQKAMNEKANHNEEFNH
consensus          61    *..**.*.*..**..*. .*.*.*  ..   .*  ** **..**.. ..*   . *.


S.pyogenes        119 NVVAEKKTDVIGDLNPD
S.dysgalactiae    119 NVVAEKKTDIGDLNPD
S.equi            119 NAVAKKEVNPQIGTLSSK
S.mutans          119 NKADAQKHTEIGELNAE
S.oralis          120 TEKEKNHKEFYESFDIK
S.mitis           120 TAKEKNQKEYESFDIK
S.parasanguinis   119 TEKEKQYIKEVYDSFDIK
S.thermophilus    119 NVKADEYTKIGEFNSK
L.monocytogenes   119 TEVAKNREEVNSENIGALDTK
E.timonensis      120 KEKAYKKETQQIGKLSTE
consensus         121 *.  . .*  *.  ..***.**. *...   ..*.**.***.**.**.**.** *        *
```

```
S.pyogenes          179 SDDLIQLQTNQLENPINASRVDAKA------LARLNAQLG
S.dysgalactiae      179 SDDLIQLQTNQLENPINASRVDAKA------LARLNAQLG
S.equi              179 NNQSDHLDTNLLEQRLLTEGINAKE------LAAKLNSLIGQ
S.mutans            179 TDQLADFGVDRTDSHLSEITVDASS------LERLKNNYK
S.oralis            180 NDQINEFSIDNTGSSLNGQNAQVEA------FDARRKLFD
S.mitis             180 NDQINEFSIDNTGSSLSGQNAQVEA------FDARRKLFD
S.parasanguinis     179 NDQINEFTILDNTESSLSKGNAQVEE------FDARRKLFD
S.thermophilus      179 NDQNQDFDTNAISDLSLENSKQLEE------VKDLEKRKLFG
L.monocytogenes     179 TSDGVKQFQTNQVISNIEEGTLAKMEENTTVADLAGFKELRQLYG
E.timonensis        180 TSSETKVFDKNEASKIADNELKLDTTID---VEKLESRANNFFT
consensus           181     . .  . .     ..                 . . .... . . ..     * .


S.pyogenes          233 KNGNLASLTPNSNDADKLNQQA
S.dysgalactiae      233 RNGNLASLTPNSNDADKLNQQA
S.equi              233 KTGNIASLTPNANGSKDVKLAASQQA
S.mutans            233 KNTGNLASLQPNTNKSDKFVQNS
S.oralis            234 STSEFKINQADKHDEKPFNQDV
S.mitis             234 STSEFKINQAEKHDEKPFNQGA
S.parasanguinis     233 STSEFKINQADKHDEKPFSQVV
S.thermophilus      233 NSSEFKINQADKCNDKSHFTYDSK
L.monocytogenes     239 STAQFSISKGNQKVDVKTDECAAIEA
E.timonensis        237 KNDTDQFKINQGNKTDDDKFDTNMAGEA
consensus           241 *   ..*    . . .*      *.  * * .  . .. .*..*...*. **. .** ....*.


S.pyogenes          293 NSTDRNSEIKDEHQTLALQQPEKKF
S.dysgalactiae      293 NSDRNSEIKDEHQTLALQQPEKKF
S.equi              293 NSDESEIKREHKVTTLDQPEKQL
S.mutans            293 KYSGTVSQNEQMAQQFQKSDKNS
S.oralis            294 KYGTPSEENQKATQFNNPEKDS
S.mitis             294 KYGTPSEENQKAAQFQNNQEKDS
S.parasanguinis     293 KYAGKPGEDNQNSAQFRNPEKAS
S.thermophilus      293 KYGFTNEESAKNEKEALEYNISLKTNK
L.monocytogenes     299 NTYNSTTENKEDAEKSEAFLHPKQES
E.timonensis        297 NAYNEGQSLKGKDDKEALKFFLNNGYEEYVSF
consensus           301 **  . ....*. .... .  *.*.**..*. *.  *  ** **  ..  .   . ...


S.pyogenes          353 ----QNGASEPETELANL
S.dysgalactiae      353 ----QNGASEPETELANL
S.equi              353 ----KNQVSEPASPLL
S.mutans            353 ----VDKTNAGNSGYFD
S.oralis            354 ----QDKTTANSAYFD
S.mitis             354 ----QDNKTTANSFSYFD
S.parasanguinis     353 ----DDKTTGNSAYFE
S.thermophilus      353 ----DNKTNDVKAFAYFE
L.monocytogenes     359 N----VAIDKTKVDTENAYFAEN
E.timonensis        357 KGDGKKDNNSHTKDSTKDAYFAQ
consensus           361 .      .. ***.**.*   *. **...* .. ....*  .  . *...*..*.*******
```

```
S.pyogenes        409 YGHEDKREKNSR--T
S.dysgalactiae    409 GHEDKREKNSR--T
S.equi            409 EHEVKRKESHSR--K
S.mutans          409 QRAEAQEKKSD--S
S.oralis          410 QNGEHQKEKNRD--T
S.mitis           410 QNGEHQKKKNGD--T
S.parasanguinis   409 QNGEKKEQNSD--S
S.thermophilus    409 YQRDAKAKKEKNSD--SI
L.monocytogenes   415 AEEQAKKAYKSNQSD--T
E.timonensis      417 VEKEGEKFQVDFNSKENHGRE
consensus         421 .**.*.** *. **...*   .**** .. ..*  ...****.*****..    ***.


S.pyogenes        467 KSETTKGAQSEKNNYT
S.dysgalactiae    467 KSETTKGAQSEKNNYT
S.equi            467 KFGAREEAQIEKNLNTT
S.mutans          467 KSADKTKESVEANLNQKT
S.oralis          468 NSQARKAREDNLETA
S.mitis           468 NSQARQASEDNLETA
S.parasanguinis   467 NSEARKSSEDHLETT
S.thermophilus    467 KRNEKTKESEANSLETN
L.monocytogenes   473 KAGERIKFGKVDEKTKNYQKM
E.timonensis      477 NSEPTITMNKEKETNKT
consensus         481 *  .   * ***.....*   *.  ** .**..* .** ..******..*. . *******


S.pyogenes        527 VTGMKPALSGEQKAVDLTNVQKEDYFKKECSSDR
S.dysgalactiae    527 VTGMKPALSGEQKAVDLTNVQKEDYFKKECSSDR
S.equi            527 ATGMTRPQLSADQQAVDLTNVQKENYFKKECSTDS
S.mutans          527 KT-QGKTAFDANMQEFDGVYKDKMDFLEKEFDERITKE
S.oralis          528 IAGLDYQLDSGQKQVTQEKEDIQYLHT-DGGKKQ
S.mitis           528 IAGLDYQLDSGQKQVNQEKEDTQYLHN-DGGKKQ
S.parasanguinis   527 IAGMDYQLDSGQKQVNQEKEDIHYLHN-DGGKKH
S.thermophilus    527 IASMDYQLDSKQKDVRLYDKDDIEYLHA-YGGKKQ
L.monocytogenes   533 IDQGTNYSGQ-EQQFNDYQKKDEQFLRN-SHISPTEDS
E.timonensis      537 STDQETHNSSI-EEKFKECKNKDRQKFLYN-EYNLNTNNE
consensus         541 *..  .  .    *     *  *   .*. ***...    .  .. ... *..


S.pyogenes        587 ---TGKDNEEDDLEEEKKAN
S.dysgalactiae    587 ---AGKDNEEDDLEEEKKAN
S.equi            587 ---AGQDNPDQKDLKSKDQAH
S.mutans          586 NKAAYGRD-NSKKDLERKKNSDL
S.oralis          587 ---ASKDDSKANHERQTQAS
S.mitis           587 ---ASKDADAEATNHEKQAQDS
S.parasanguinis   586 ---SSKDDPKEFNHVKQNQDS
S.thermophilus    586 ---SSNNDDSSAEHEKQSKEN
L.monocytogenes   591 ---SYAGIKQVNPLTEDKIRKEQQQSDL
E.timonensis      595 ---AKAFNVSPMNDPEDFEKMKAKQASKSY
consensus         601    ** .. ****.....  .....    *. ..* *. .**.***. *.   .*   . ..
```

```
S.pyogenes          644 DKQKDRRQGKKSF----AN
S.dysgalactiae      644 DKQKRRQGKKSF----AN
S.equi              644 KVNKEHRQGKKAF----AH
S.mutans            645 TKEQKEEHRNERKIDN----SN
S.oralis            644 EKATCQGDIDE----IH
S.mitis             644 EKAICKGKIDY----SN
S.parasanguinis     643 EKATRKSKIDY----SN
S.thermophilus      643 KSKSREKGNIDI----SH
L.monocytogenes     648 GAKEVRQHLMNDG----LLN
E.timonensis        652 EKTEVYYQQGKVMDAPGKNTN
consensus           661 .   ... * *..**.**.**..*...*.  ...  .  *..*.* *.      ***.**** *


S.pyogenes          700 DSAQS--QGHSHEQANA-HKE
S.dysgalactiae      700 DSAQS--QGHSHEQANA-HKE
S.equi              700 SEIEAI--KTEYSKDGNAS-YLQ
S.mutans            701 DAEAI--ETDNNQSDA-HQE
S.oralis            700 DGIQV--KTDDKQQEP-HEE
S.mitis             700 DGIQV--RTNDKQHEP-HTE
S.parasanguinis     699 DGTQV--ETNDKQQEP-HAE
S.thermophilus      699 DAKKQIDEDKGNKEKSPGRKE
L.monocytogenes     704 SNSIEEST--TDKDQSADAS-YPQ
E.timonensis        712 NMEQKEVG-TKNDNQQEAD-YAT
consensus           721    *.*.. * *.*. .         .  ..  . ****.****.*...*.***.*...**    *


S.pyogenes          757 NQKQKREMREGESQLKEHPVNTQ------
S.dysgalactiae      757 NQKQKREMREGESQLKEHPVNTQ------
S.equi              757 QAQIKARQMKETAKSNLKEHPVNSQ------R
S.mutans            758 NFNQRRQQLGTDSEFSQLKEHPVNSQ------
S.oralis            757 SARKKQQYRDANPEDSNILKHPT--DNIQ
S.mitis             757 SARKKQQYRDANPGDSNILKYPT--DNIQ
S.parasanguinis     756 SNKKSKQQLTSDASE----SNILKHPT--DNIQ
S.thermophilus      759 SYNQKSQQLRKSESKLKENIPAKLSKIDNNA
L.monocytogenes     761 TVKKNRPYSKAEFSQLKEHPTNQE------RN
E.timonensis        770 NGRKIQPYNKSNEQSKLKDYPTNKA------K
consensus           781  .*.*******.*  *  ..  *  . ..  .. ..  .      .         ......


S.pyogenes          811 VDQENRDSDRDKN
S.dysgalactiae      811 VDQENRDSDRDKN
S.equi              811 DDDYSSNNQGALN
S.mutans            812 EEDYQANKED
S.oralis            815 EANQSCADKDN
S.mitis             815 EPNQSADKDN
S.parasanguinis     810 EANQNADKDN
S.thermophilus      819 DDDRNANVASD
L.monocytogenes     815 QEHNNSTNLAGEGD
E.timonensis        824 HDNNSVNVKEDL
consensus           841 .*******.***..  ***  ** .*.***.** *... *.**.**...  **.*.* *.
```

```
S.pyogenes        871  EKMNRQNAQDSLA
S.dysgalactiae    871  EKMNRQNAQDSLA
S.equi            871  EARMGQSRAGAKQDTQVAQL
S.mutans          872  KRMSSKSAQDTDA
S.oralis          875  LQRAQQDSENDRV
S.mitis           875  LQRAQQDSENDRV
S.parasanguinis   870  LEMGQQDSENDRV
S.thermophilus    879  LKRTYQKSQDSPEAQ
L.monocytogenes   875  PLRRVEKFQGNKDYTAATN
E.timonensis      884  NIKSRGEQHSKDTDA
consensus         901  . ..* . * .* *.  .... .** .***.****. . ** .**..*********.*


S.pyogenes        931  QSMTKYDNDLEKDQFN
S.dysgalactiae    931  QSMTKYDNDLEKDQFN
S.equi            931  QSTEFDHNRKKDGH
S.mutans          932  RETETDNNKQNNED
S.oralis          935  QATEVTKDKDSNNRD
S.mitis           935  QATEVTKDKNNNNKD
S.parasanguinis   930  QDAEVNKNQKSNNGD
S.thermophilus    939  RENKKDNNATTQED
L.monocytogenes   935  NHQNETDNHGNNEQMAQQQD
E.timonensis      944  RETEKDQTGKTATQNQD
consensus         961  * .*. ..*    .  . .. *....*** *.* ***.. .**.*...* .********.


S.pyogenes        991  TKKSKVDVRMIAKSE--QEIGKAYFTE
S.dysgalactiae    991  TKKSKVDVRMIAKSE--QEIGKARFTE
S.equi            991  KGKQAPKNSFERQK---------QTLKDQ
S.mutans          992  KGVQPHHGHENKA----------AKFKD
S.oralis          995  KKKQKDLKYISRTKDPKEVEKEYFEE
S.mitis           995  KKKQKDLKYISRSKDPKDVEKEYFEE
S.parasanguinis   990  KKKQKDLKYISRTKDPKEIEKEYFDK
S.thermophilus    999  SKKPKNSFER---------KSEVYIKS
L.monocytogenes   995  NTVQHQDWFAN----------KAKQLAQ-
E.timonensis     1004  NTIQHRDSFEN----------RAKNMTK-
consensus        1021  *.. .... ** *..*****.*  .  .         .* *  .*.*......


S.pyogenes       1049  ITLANGEIRKRPLIETNGTGIVDKGDFATMVTGFSKE-
S.dysgalactiae   1049  ITLANGEIRKRPLIETNETGIVDKGDFATMVTGLTNE-
S.equi           1042  ESLHVNSD------------GEINANHLPINIVTGFYKE-
S.mutans         1042  DVRTDKNG-------------IIKKDEYISNYVETGFSKE-
S.oralis         1055  VHYADGTIVKRENIEYSKTGIANKEDFATLETVQNGG-
S.mitis          1055  VHYADGTIVKRENIEYSKTGIANKEDFATLITHLDRGK
S.parasanguinis  1050  VYYADGTIIQRGNVEYSKTGIANKKDFAIYETGFSKE-
S.thermophilus   1050  ISLADGRVIERPLIEVNETGSVNKESDLATYVENHLDRGK
L.monocytogenes  1044  ------------KERIID-NGEILWDKYLETDYRIKGEFSKAT
E.timonensis     1053  ------------DVTLDKGNGEILWDNSVAMDYRIRGFSNET
consensus        1081           . . .    .     ...*.. .*.*.***.* *   .
```

```
S.pyogenes       1108 -------SLPRNSDKLIA---RKDWPKDSTVASVKVE--KS
S.dysgalactiae   1108 -------SYAGSFDKLIS---RHRFSSGSTVTSVKSKVQDKV
S.equi           1089 -------SLSGNSDKLIP---RNNWTRDSTVASIKME--KAV
S.mutans         1088 -------SLPGDSDKLIPRKTKFYWTKDSIVASIDIE--KS
S.oralis         1114 ---LFDNNVSKKVVDASKLTPISGLSPEARTIASIDIE--KA
S.mitis          1115 PRGLFNSNPSPPSEDSKENLVPIQGLPRAGISNSAKIIE--AKQ
S.parasanguinis  1109 -------SLPGNSDKLIPRKTKNVQLTTDSVIASVDVE--KS
S.thermophilus   1110 PKGLFNANSSPKPNSNENLVGAEYLPKAGISNSAKTIE--AK
L.monocytogenes  1091 --------KPGNSSKLIP---RENWPMLDSNMAAEHAKG-----
E.timonensis     1101 --------LPGPSDKLIP---RNNWPAVGSTEASSYEKG-----S
consensus        1141            .   .          .     .  ****.  .    .  ....  .    .    ..


S.pyogenes       1156 KSVKELSSKNPDEAKERKDLLENSR
S.dysgalactiae   1158 KTGKEILVKNPKEDGNQIDKCFENT
S.equi           1137 KPVKEVIAENPVEAREQEHLLENR
S.mutans         1139 KTVKAVMTRDPAERRNQEENKLENR
S.oralis         1169 KRIKEVQKKANPAEECKNNPNLFNNQ
S.mitis          1173 QKTVLEFQINKNKENLEIKLS--TTFPDTR
S.parasanguinis  1160 KTVKSIVKANPAEGQNVEENLENR
S.thermophilus   1168 TNVLEFQINRKDKNLEKDEL--ELSDS
L.monocytogenes  1135 VFEKKIRKAKDEKSEKQRQPKV--TCENR
E.timonensis     1145 KVVKEIG-QKAENEGKTENPKV--AFADR
consensus        1201 .          .  .......  ..    .  ...  .**   .       ...****.*..    .  .*.***


S.pyogenes       1216 AGEL--KGNELALPSKYNFLYLASHEKLKGSPEDNEQQLEQKHYLDEQS
S.dysgalactiae   1218 VMANNSRGDLQEMFPAKLVTHKIESSKELEEALDYNDLYQSYE
S.equi           1197 ASEL--KGNELFLPVDYTFLYLAAHELTGSSEDVLRKYERLHYDQMN
S.mutans         1199 AREL-------EIVPNHLGTKNIHKVDEPKLDDKKDEKDVS
S.oralis         1229 SIEL-------ELIPYHFTAQRINKISEPIKQETQSEKTAI
S.mitis          1231 ILSTNKRGEIHELVSEKYTTKNINKTLEPELEEKRNDAKESL
S.parasanguinis  1220 AKEL-------EMVPSYLIAKRIQKKDEPELEKQHSENNFS
S.thermophilus   1226 ILSTNKRGEIHQIFSQKFVKKRISNTINENRKENKKEEFYYL
L.monocytogenes  1193 ANEA-------QQVKGQLITHKNCEAS-DGKSLDESNREMGAHS
E.timonensis     1202 SKES-------QLVSKDLNEKNSDK--KSESLETNNSTMFFEYD
consensus        1261       .            ...    .        .....          .  ..   .   . ...  .


S.pyogenes       1274 ESKVLDALDLSANKHRDKPREQAENIH--------FTLTNLA
S.dysgalactiae   1278 RASLDVEKISKELSNIESYSSECSVN--------LTLTASD
S.equi           1255 DAEHLSSLENHTHNNSDLPNERAENIN--------FTFVALA
S.mutans         1252 NSKTLEGLEKELAQNNGEDKEASFN--------LTFTAIT
S.oralis         1282 SLSKQKP-VESLQQAFDQSDKDYQSEFS--------LKLISFT
S.mitis          1291 DNDGLKGERQAIDWETVDEKCFFGPRNSKNAGFELTSQSASDE
S.parasanguinis  1273 ESQLESLEKNLIDNEQTNEEANFN--------LTFTAFV
S.thermophilus   1286 ENENGKKGKLNSAQSWQNHSDECSFGPTGSERKGFELTSRSADE
L.monocytogenes  1245 EAKTLDALSNQLEQNKDNDKVAQFN--------MAFNAMS
E.timonensis     1253 IAQITKSEQIVAENNKDSEGKDATFFN--------LQFTAMD
consensus        1321 .  ...  .  *  ..    .      .   .   . .        .           *... *..
```

```
S.pyogenes          1326 FDTTD--TTKEDSQGD
S.dysgalactiae      1330 LGTTP--GPQSSSSQGD
S.equi              1307 FDATD--TTKENSQEN
S.mutans            1304 FDKND--TTTENSKGD
S.oralis            1333 LGVESQSNVQVSSCFNSKED
S.mitis             1351 LGVKP--YDYPSSNSKED
S.parasanguinis     1325 FGKDE--SVTEKSKEE
S.thermophilus      1346 LGVKP-YDTPSSLKDAKEG
L.monocytogenes     1297 FEATE--TNLKESYAKRDG----
E.timonensis        1305 FNETP--STSENYRNGD----
consensus           1381      * .  ... .   .. .*.*.**.*****.*..*  ..
```

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62733410
- WO 2016070037 A
- WO 2016115033 A
- US 9315806 B
- US 5965408 A

**Non-patent literature cited in the description**

- **WONG et al.** *Nat. Biotechnol.,* September 2015, vol. 33 (9), 952-961
- **WONG et al.** *Proc. Nat. Acad. Sci.,* 01 March 2016, vol. 113 (9), 2544-2549
- **BATZER et al.** *Nucleic Acid Res.,* 1991, vol. 19, 5081
- **OHTSUKA et al.** *J. Biol. Chem.,* 1985, vol. 260, 2605-2608
- **ROSSOLINI et al.** *Mol. Cell. Probes,* 1994, vol. 8, 91-98
- **SAMBROOK ; RUSSELL.** Molecular Cloning, A Laboratory Manual. 2001
- **KRIEGLER.** *Gene Transfer and Expression: A Laboratory Manual,* 1990
- Current Protocols in Molecular Biology. 1994
- **BEAUCAGE ; CARUTHERS.** *Tetrahedron Lett.,* 1981, vol. 22, 1859-1862
- **VAN DEVANTER.** *Nucleic Acids Res.,* 1984, vol. 12, 6159-6168
- **PEARSON ; REANIER.** *J. Chrom,* 1983, vol. 255, 137-149
- **WALLACE et al.** *Gene,* 1981, vol. 16, 21-26
- **ZHANG et al.** *Proc. Natl. Acad. Sci. USA,* 1997, vol. 94, 4504-4509
- **STEMMER.** *Nature,* 1994, vol. 370, 389-391
- **BOTSTEIN ; SHORTLE.** *Science,* 1985, vol. 229, 1193-1201
- **KUNKEL.** *Proc. Natl. Acad. Sci. USA,* 1985, vol. 82, 488-492
- **ZOLLER ; SMITH.** *Nucl. Acids Res.,* 1982, vol. 10, 6487-6500
- **TAYLOR et al.** *Nucl. Acids Res.,* 1985, vol. 13, 8749-8764, 8765-8787
- **KRAMER et al.** *Cell,* 1984, vol. 38, 879-887
- **CARTER et al.** *Nucl. Acids Res,* 1985, vol. 13, 4431-4443
- **HENIKOFF.** *Nucl. Acids Res,* 1986, vol. 14, 5115
- **WELLS et al.** *Phil. Trans. R. Soc. Land. A,* 1986, vol. 317, 415-423
- **NAMBIAR et al.** *Science,* 1984, vol. 223, 1299-1301
- **MANDECKI.** *Proc. Natl. Acad. Sci. USA,* 1986, vol. 83, 7177-7181
- **LEUNG et al.** *Biotechniques,* 1989, vol. 1, 11-15
- **COLLEY et al.** *J. Biol. Chem.,* 1989, vol. 264, 17619-17622
- Guide to Protein Purification, in Methods in Enzymology. 1990, vol. 182
- **MORRISON.** *J. Bact,* 1977, vol. 132, 349-351
- **CLARK-CURTISS ; CURTISS.** *Methods in Enzymology,* vol. 101, 347-362
- **WONG et al.** *PNAS,* 2016, vol. 113 (9), 2544-9
- **SLAVMAKER et al.** *Science,* 2016, vol. 351 (6268), 84-8
- **KLEINSTIVER et al.** *Nature,* 2016, vol. 529 (7587), 490-5
- **CHEN et al.** *Nature,* 2017, vol. 550 (7676), 407-10
- **HU.** *Nature,* 2018, vol. 556 (7699), 57-63
- **CASINI et al.** *Nat. Biotechnol.,* 2018, vol. 36 (3), 265-271
- **LEE EL.** *Nat. Commun.,* 2018, vol. 9 (1), 3048
- **CASINI et al.** *Nat. Biotechnol.,* 2018, vol. 36, 265-71
- **WONG et al.** *Nat. Biotechnol.,* 2015, vol. 33 (9), 952-61
- **BORNSCHEUER, U. T et al.** Engineering the third wave of biocatalysis. *Nature,* 2012, vol. 485, 185-194
- **WEINREICH, D. M. ; DELANEY, N. F. ; DEPRISTO, M. A ; HARTL, D. L.** Darwinian evolution can follow only very few mutational paths to fitter proteins. *Science,* 2006, vol. 312, 111-114
- **SLAYMAKER, I. M et al.** Rationally engineered Cas9 nucleases with improved specificity. *Science,* 2016, vol. 351, 84-88
- **KLEINSTIVER, B. P et al.** High-fidelity CRISPR-Cas9 nucleases with no detectable genome-wide off-target effects. *Nature,* 2016, vol. 529, 490-495

- **CHEN, J. S et al.** Enhanced proofreading governs CRISPR-Cas9 targeting accuracy. *Nature,* 2017, vol. 550, 407-410
- **CASINI, A et al.** A highly specific SpCas9 variant is identified by in vivo screening in yeast. *Nat Biotechnol,* 2018
- **HU, J. H et al.** Evolved Cas9 variants with broad PAM compatibility and high DNA specificity. *Nature,* 2018
- **PACKER, M. S ; LIU, D. R.** Methods for the directed evolution of proteins. *Nat Rev Genet,* 2015, vol. 16, 379-394
- **ROMERO, P. A ; ARNOLD, F. H.** Exploring protein fitness landscapes by directed evolution. *Nat Rev Mol Cell Biol,* 2009, vol. 10, 866-876
- **GASPERINI, M ; STARITA, L ; SHENDURE, J.** The power of multiplexed functional analysis of genetic variants. *Nat Protoc,* 2016, vol. 11, 1782-1787
- **FOWLER, D. M ; FIELDS, S.** Deep mutational scanning: a new style of protein science. *Nat Methods,* 2014, vol. 11, 801-807
- **MA, S. ; SAAEM, I ; TIAN, J.** Error correction in gene synthesis technology. *Trends Biotechnol,* 2012, vol. 30, 147-154
- **KOSURI, S ; CHURCH, G. M.** Large-scale de novo DNA synthesis: technologies and applications. *Nat Methods,* 2014, vol. 11, 499-507
- **ENGLER, C. ; KANDZIA, R ; MARILLONNET, S.** A one pot, one step, precision cloning method with high throughput capability. *PLoS One,* 2008, vol. 3, e3647
- **GIBSON, D. G et al.** Enzymatic assembly of DNA molecules up to several hundred kilobases. *Nat Methods,* 2009, vol. 6, 343-345
- **TRUDEAU, D. L. ; SMITH, M. A ; ARNOLD, F. H.** Innovation by homologous recombination. *Curr Opin Chem Biol,* 2013, vol. 17, 902-909
- **WONG, A. S. ; CHOI, G. C. ; CHENG, A. A ; PURCELL, O ; LU, T. K.** Massively parallel high-order combinatorial genetics in human cells. *Nat Biotechnol,* 2015, vol. 33, 952-961
- **WONG, A. S et al.** Multiplexed barcoded CRISPR-Cas9 screening enabled by CombiGEM. *Proc Natl Acad Sci USA,* 2016, vol. 113, 2544-2549
- **CHENG, A. A. ; DING, H ; LU, T. K.** Enhanced killing of antibiotic-resistant bacteria enabled by massively parallel combinatorial genetics. *Proc Natl Acad Sci USA,* 2014, vol. 111, 12462-12467
- **DOUDNA, J. A ; CHARPENTIER, E.** Genome editing. The new frontier of genome engineering with CRISPR-Cas9. *Science,* 2014, vol. 346, 1258096
- **HSU, P. D. ; LANDER, E. S ; ZHANG, F.** Development and applications of CRISPR-Cas9 for genome engineering. *Cell,* 2014, vol. 157, 1262-1278
- **MALI, P. ; ESVELT, K. M ; CHURCH, G. M.** Cas9 as a versatile tool for engineering biology. *Nat Methods,* 2013, vol. 10, 957-963
- **BARRANGOU, R ; HORVATH, P.** A decade of discovery: CRISPR functions and applications. *Nat Microbiol,* 2017, vol. 2, 17092
- **KIM, S. ; BAE, T. ; HWANG, J. ; KIM, J. S.** Rescue of high-specificity Cas9 variants using sgRNAs with matched 5' nucleotides. *Genome Biol,* 2017, vol. 18, 218
- **KULCSAR, P. I et al.** Crossing enhanced and high fidelity SpCas9 nucleases to optimize specificity and cleavage. *Genome Biol,* 2017, vol. 18, 190
- **ZHANG, D et al.** Perfectly matched 20-nucleotide guide RNA sequences enable robust genome editing using high-fidelity SpCas9 nucleases. *Genome Biol,* 2017, vol. 18, 191
- **KATO-INUI, T. ; TAKAHASHI, G ; HSU, S ; MIYAOKA, Y.** Clustered regularly interspaced short palindromic repeats (CRISPR)/CRISPR-associated protein 9 with improved proofreading enhances homology-directed repair. *Nucleic Acids Res,* 2018, vol. 46, 4677-4688
- **STERNBERG, S. H. ; LAFRANCE, B ; KAPLAN, M ; DOUDNA, J. A.** Conformational control of DNA target cleavage by CRISPR-Cas9. *Nature,* 2015, vol. 527, 110-113
- **SINGH, D et al.** Mechanisms of improved specificity of engineered Cas9s revealed by single-molecule FRET analysis. *Nat Struct Mol Biol,* 2018, vol. 25, 347-354
- **KATO-INUI, T. ; TAKAHASHI, G ; HSU, S ; MIYAOKA, Y.** Clustered regularly interspaced short palindromic repeats (CRISPR)/CRISPR-associated protein 9 with improved proofreading enhances homology-directed repair. *Nucleic Acids Res,* 2018
- **FU, Y et al.** High-frequency off-target mutagenesis induced by CRISPR-Cas nucleases in human cells. *Nat Biotechnol,* 2013, vol. 31, 822-826
- **LEE, J. K et al.** Directed evolution of CRISPR-Cas9 to increase its specificity. *Nat Commun,* 2018, vol. 9
- **HAEUSSLER, M et al.** Evaluation of off-target and on-target scoring algorithms and integration into the guide RNA selection tool CRISPOR. *Genome Biol,* 2016, vol. 17, 148
- **FU, Y. ; SANDER, J. D. ; REYON, D. ; CASCIO, V. M ; JOUNG, J. K.** Improving CRISPR-Cas nuclease specificity using truncated guide RNAs. *Nat Biotechnol,* 2014, vol. 32, 279-284
- **VAKULSKAS, C. A et al.** A high-fidelity Cas9 mutant delivered as a ribonucleoprotein complex enables efficient gene editing in human hematopoietic stem and progenitor cells. *Nat Med,* 2018, vol. 24, 1216-1224
- **RAN, F. A et al.** In vivo genome editing using Staphylococcus aureus Cas9. *Nature,* 2015, vol. 520, 186-191

- **ZETSCHE, B et al.** Cpfl is a single RNA-guided endonuclease of a class 2 CRISPR-Cas system. *Cell,* 2015, vol. 163, 759-771
- **KOMOR, A. C. ; KIM, Y. B. ; PACKER, M. S. ; ZURIS, J. A ; LIU, D. R.** Programmable editing of a target base in genomic DNA without double-stranded DNA cleavage. *Nature,* 2016, vol. 533, 420-424
- **NISHIDA, K. et al.** Targeted nucleotide editing using hybrid prokaryotic and vertebrate adaptive immune systems. *Science,* 2016, vol. 353
- **GAUDELLI, N. M et al.** Programmable base editing of A∗T to G∗C in genomic DNA without DNA cleavage. *Nature,* 2017, vol. 551, 464-471
- **LI, X et al.** Base editing with a Cpfl-cytidine deaminase fusion. *Nat Biotechnol,* 2018, vol. 36, 324-327
- **HONMA, K et al.** RPN2 gene confers docetaxel resistance in breast cancer. *Nat Med,* 2008, vol. 14, 939-948
- **KAMPMANN, M. ; BASSIK, M. C ; WEISSMAN, J. S.** Functional genomics platform for pooled screening and generation of mammalian genetic interaction maps. *Nat Protoc,* 2014, vol. 9, 1825-1847
- **OLSON, C. A. ; WU, N. C ; SUN, R.** A comprehensive biophysical description of pairwise epistasis throughout an entire protein domain. *Curr Biol,* 2014, vol. 24, 2643-2651
- **AAKRE, C. D et al.** Evolving new protein-protein interaction specificity through promiscuous intermediates. *Cell,* 2015, vol. 163, 594-606
- **GUSCHIN, D. Y et al.** A rapid and general assay for monitoring endogenous gene modification. *Methods Mol Biol,* 2010, vol. 649, 247-256
- **TSAI, S. Q et al.** GUIDE-seq enables genome-wide profiling of off-target cleavage by CRISPR-Cas nucleases. *Nat Biotechnol,* 2015, vol. 33, 187-197
- **TSAI, S. Q. ; TOPKAR, V. V. ; JOUNG, J. K ; ARYEE, M. J.** Open-source guideseq software for analysis of GUIDE-seq data. *Nat Biotechnol,* 2016, vol. 34, 483